# EUROPÄISCHE PATENTSCHRIFT

(11) **EP 1 210 333 B1**
(45) Veröffentlichungstag und Bekanntmachung des Hinweises auf die Patenterteilung: **17.11.2004**
(21) Anmeldenummer: 00965923.6
(22) Anmeldetag: 05.09.2000
(51) Int. Cl.: C07D 231/32, C07D 231/34, C07D 231/36, C07D 491/04, C07D 417/12, C07D 403/12, C07D 409/12, C07D 413/12, C07D 487/04, C07D 207/38, C07D 491/10, C07D 209/96, C07D 307/60, C07D 307/94, C07D 493/10

(54) **p-Tolyl-Heterocyclen als Herbizide**
p-Tolyl-heterocycles as herbicides
Héterocyles p-tolyl-substitués utilisés comme herbicides

(30) Priorität: 07.09.1999 CH 164299
(43) Veröffentlichungstag der Anmeldung: 05.06.2002
(60) Teilanmeldung: 04013876.0
(73) Patentinhaber: Syngenta Participations AG, 4058 Basel (CH)
(72) Erfinder: MAETZKE, Thomas, CH-4142 Münchenstein (CH); STOLLER, André, F-68730 Blotzheim (FR); WENDEBORN, Sebastian, CH-4102 Binningen (CH); SZCZEPANSKI, Henry, CH-4323 Wallbach (CH)
(74) Vertreter: Bastian, Werner Maria
(86) Internationale Anmeldenummer: PCT/EP2000/008656
(87) Internationale Veröffentlichungsnummer: WO 2001/017972

(56) Entgegenhaltungen:
- EP-A- 0 508 126
- WO-A-00/78712
- WO-A-96/25395

## Beschreibung

Die vorliegende Erfindung betrifft neue, herbizid wirksame durch eine Phenylgruppe substituierte Heterocyclen, Verfahren zu ihrer Herstellung, Mittel, die diese Verbindungen enthalten, sowie ihre Verwendung zum Bekämpfen von Unkräutern, vor allem in Nutzpflanzenkulturen oder zum Hemmen des Pflanzenwachstums.

3-Hydroxy-4-aryl-5-oxo-pyrazolin-Derivate mit herbizider Wirkung sind beispielsweise in EP-A-0 508 126, WO 96/25395 und WO 96/21652 beschrieben.

Es wurden nun neue durch eine Phenylgruppe substituierte Heterocyclen mit herbiziden und wuchshemmenden Eigenschaften gefunden.

Gegenstand der vorliegenden Erfindung sind somit Verbindungen der Formel I 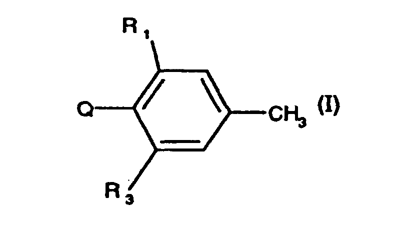 worin
R₁ und R₃ unabhängig voneinander Ethyl, Halogenethyl, Ethinyl, C₁-C₂-Alkoxy, C₁-C₂-Halogenalkoxy oder C₁-C₂-Alkylcarbonyl bedeuten;
Q eine Gruppe 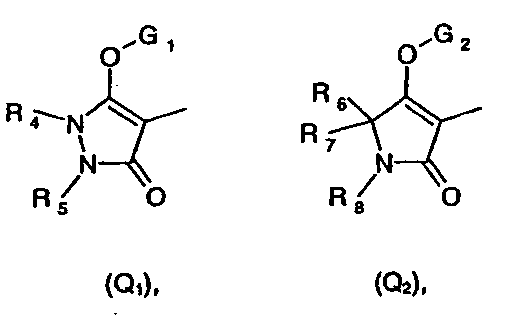 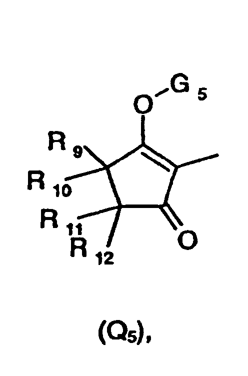 oder 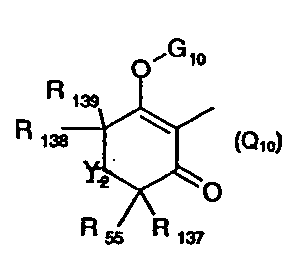 bedeutet;
R₄ und R₅ unabhängig voneinander C₁-C₁₀-Alkyl, C₂-C₁₀-Alkenyl, C₂-C₁₀-Alkinyl, C₁-C₁₀-Halogenalkyl, C₂-C₁₀-Alkoxyalkyl, C₃-C₁₀-Alkenyloxyalkyl, C₃-C₁₀-Alkinyloxyalkyl, C₂-C₁₀-Alkylthioalkyl, C₂-C₁₀-Alkylsulfinylalkyl, C₂-C₁₀-Alkylsulfonylalkyl, C₂-C₁₀-Alkylcarbonyl-alkyl, C₂-C₁₀-N-Alkoxy-iminoalkyl, C₂-C₁₀-Alkoxycarbonylalkyl, C₁-C₁₀-Aminoalkyl, C₃-C₁₀-Dialkylaminoalkyl, C₂-C₁₀-Alkylaminoalkyl, C₁-C₁₀-Cyanoalkyl, C₄-C₁₀-Cycloalkylalkyl, C₁-C₁₀-Phenylalkyl, C₁-C₁₀-Heteroarylalkyl, C₁-C₁₀-Phenoxylalkyl, C₁-C₁₀-Heteroaryloxyalkyl, C₁-C₁₀-Alkylidenaminooxyalkyl, C₁-C₁₀-Nitroalkyl, C₁-C₁₀-Trialkylsilylalkyl, C₂-C₁₀-Alkylaminocarbonylalkyl, C₂-C₁₀-Dialkylaminocarbonylalkyl, C₂-C₁₀-Alkylaminocarbonyloxyalkyl, C₃-C₁₀-Dialkylaminocarbonyloxalkyl, C₂-C₁₀-Alkoxycarbonylaminoalkyl, C₁-C₁₀-N-Alkoxycarbonyl-N-alkylamino-alkyl, C₁-C₁₀-Cycloalkyl, Aryl oder Heteroaryl bedeuten; oder
R₄ und R₅ bilden gemeinsam mit den Atomen, an die sie gebunden sind, einen 5- bis 7-gliedrigen Cyclus, der ein oder zwei Heteroatome ausgewählt aus Stickstoff, Sauerstoff und Schwefel enthalten kann und der zusätzlich eine anellierte oder spirogebundene, aus 2 bis 6 Kohlenstoffatomen bestehende Alkylen- oder Alkenylenkette enthalten kann, die ihrerseits ein oder zwei Heteroatome ausgewählt aus Sauerstoff und Schwefel enthalten kann, wobei dieser Cyclus mit Phenyl oder Benzyl substituiert sein kann, welche ihrerseits durch Halogen, C₁-C₆-Alkyl, C₁-C₆-Halogenalkyl, C₃-C₆-Cycloalkyl, Hydroxy, C₁-C₆-Alkoxy, C₁-C₆-Alkoxy-C₁-C₆-alkoxy, C₁-C₆-Halogenalkoxy oder Nitro substituiert sein können;
R₆ C₁-C₁₀-Alkyl, C₂-C₁₀-Alkenyl, C₂-C₁₀-Alkinyl, C₁-C₁₀-Halogenalkyl, C₂-C₁₀-Alkoxyalkyl, C₃-C₁₀-Alkenyloxyalkyl, C₃-C₁₀-Alkinyloxyalkyl, C₂-C₁₀-Alkylthioalkyl, C₂-C₁₀-Alkylsulfinylalkyl, C₂-C₁₀-Alkylsulfonylalkyl, C₂-C₁₀-Alkylcarbonyl-alkyl, C₃-C₁₀-Cycloalkyl, Aryl oder Heteroaryl bedeutet;
R₇ Wasserstoff, C₁-C₁₀-Alkyl, C₂-C₁₀-Alkenyl, C₂-C₁₀-Alkinyl oder C₂-C₁₀-Alkoxyalkyl;
R₈ Wasserstoff, C₁-C₁₀-Alkyl, C₁-C₁₀-Halogenalkyl, C₂-C₁₀-Alkoxyalkyl, C₃-C₁₀-Alkenyloxyalkyl, C₃-C₁₀-Alkinyloxyalkyl, C₂-C₁₀-Alkylthioalkyl, C₂-C₁₀-Alkylsulfinylalkyl, C₂-C₁₀-Alkylsulfonylalkyl, C₃-C₁₀-Cycloalkyl, Aryl oder Heteroaryl bedeuten; oder
R₆ und R₇ bilden gemeinsam mit dem Atom, an das sie gebunden sind, einen gesättigten 3- bis 7- gliedrigen Cyclus, der ein oder zwei Heteroatome ausgewählt aus Stickstoff, Sauerstoff und Schwefel enthalten kann; oder R₆ und R₈ bilden gemeinsam mit den Atomen, an die sie gebunden sind, einen 5-bis 7-gliedrigen Cyclus, der ein oder zwei Heteroatome ausgewählt aus Stickstoff, Sauerstoff und Schwefel enthalten kann;
R₉, R₁₀, R₁₁ und R₁₂ unabängig voneinander C₁-C₁₀-Alkyl, C₂-C₁₀-Alkenyl, C₂-C₁₀-Alkinyl, C₁-C₁₀-Halogenalkyl, C₂-C₁₀-Alkoxyalkyl, C₃-C₁₀-Alkenyloxyalkyl, C₃-C₁₀-Alkinyloxyalkyl, C₂-C₁₀-Alkylthialkyl, C₂-C₁₀-Alkylsulfinylalkyl, C₂-C₁₀-Alkylsulfonylalkyl, C₂-C₁₀-Alkylcarbonyl-alkyl, C₃-C₁₀-Cyclolalkyl, Aryl oder Heteroaryl bedeuten; oder
R₁₀ und R₁₁ oder R₉ und R₁₀ bilden gemeinsam mit den Atomen, an die sie gebunden sind, einen 5- bis 7- gliedrigen Cyclus, der ein oder zwei Heteroatome ausgewählt aus Stickstoff, Sauerstoff und Schwefel enthalten kann;
G₁, G₂, G₅, und G₁₀ unabhängig voneinander Wasserstoff, -C(X₁)-R₂₀, -C(X₂)-X₃-R₂₁, -C(X₄)-N(R₂₂)-R₂₃, -SO₂-R₂₄, ein Alkali-, Erdalkali-, Sulfonium- oder Ammoniumkation, -P(X₅)(R₂₅)-R₂₆ oder -CH₂-X₆-R₂₇ bedeuten;
X₁, X₂, X₃, X₄ , X₅ und X₆ unabhängig voneinander Sauerstoff oder Schwefel bedeuten;
R₂₀, R₂₁, R₂₂ und R₂₃ unabhängig voneinander Wasserstoff, C₁-C₁₀-Alkyl, C₂-C₁₀-Alkenyl, C₂-C₁₀-Alkinyl, C₁-C₁₀-Halogenalkyl, C₁-C₁₀-Cyanoalkyl, C₁-C₁₀-Nitroalkyl, C₁-C₁₀-Aminoalkyl, C₁-C₅-Alkylamino-C₁-C₅-alkyl, C₂-C₈-Dialkylamino-C₁-C₅-alkyl, C₃-C₇-Cycloalkyl-C₁-C₅-alkyl, C₂-C₁₀-Alkoxy-alkyl, C₄-C₁₀-Alkenyloxy-alkyl, C₄-C₁₀-Alkinyloxy-alkyl, C₂-C₁₀-Alkylthio-alkyl, C₁-C₅-Alkylsulfoxyl-C₁-C₅-alkyl, C₁-C₅-Alkylsulfonyl-C₁-C₅-alkyl, C₂-C₈-Alkylideneamino-oxy-C₁-C₅-alkyl, C₁-C₅-Alkylcarbonyl-C₁-C₅-alkyl, C₁-C₅-Alkoxycarbonyl-C₁-C₅-alkyl, C₁-C₅-Aminocarbonyl-C₁-C₅-alkyl, C₂-C₈-Dialkylamino-carbonyl-C₁-C₅-alkyl, C₁-C₅-Alkylcarbonylamino-C₁-C₅-alkyl, C₁-C₅-Alkylcarbonyl-(C₂-C₅-alkyl)-aminoalkyl, C₃-C₆-Trialkylsilyl-C₁-C₅-alkyl, Phenyl-C₁-C₅-alkyl, Heteroaryl-C₁-C₅-alkyl, Phenoxy- C₁-C₅-alkyl, Heteroaryloxy-C₁-C₅-alkyl, C₂-C₅-Alkenyl, C₂-C₅-Halogenalkenyl, C₃-C₈-Cycloalkyl, Phenyl, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Phenyl, oder Heteroaryl oder Heteroarylamino, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Heteroaryl oder Heteroarylamino, Diheteroarylamino oder durch C₁-C₃-Alkyl, C₁-C₃-Halogenalkyl, C₁-C₃-Alkoxy, C₁-C₃-Halogenalkoxy, Halogen, Cyano oder Nitro substituiertes Diheteroarylamino, Phenylamino oder durch C₁-C₃-Alkyl, C₁-C₃-Halogenalkyl, C₁-C₃-Alkoxy, C₁-C₃-Halogenalkoxy, Halogen, Cyano oder Nitro substituiertes Phenylamino, Diphenylamino oder durch C₁-C₃-Alkyl, C₁-C₃-Halogenalkyl, C₁-C₃-Alkoxy, C₁-C₃-Halogenalkoxy, Halogen, Cyano oder Nitro substituiertes Diphenylamino, oder C₃-C₇-Cycloalkylamino, Di-C₃-C₇-cycloalkylamino oder C₃-C₇-Cycloalkoxy bedeuten;
R₂₄, R₂₅ und R₂₆ Wasserstoff, C₁-C₁₀-Alkyl, C₂-C₁₀-Alkenyl, C₂-C₁₀-Alkinyl, C₁-C₁₀-Halogenalkyl, C₁-C₁₀-Cyanoalkyl, C₁-C₁₀-Nitroalkyl, C₁- C₁₀-Aminoalkyl, C₁-C₅-Alkylamino-C₁-C₅-alkyl, C₂-C₈-Dialkylamino- C₁-C₅-alkyl, C₃-C₇-Cycloalkyl-C₁-C₅-alkyl, C₂-C₁₀-Alkoxy-alkyl, C₄-C₁₀-Alkenyloxy-alkyl, C₄-C₁₀-Alkinyloxy-alkyl, C₂- C₁₀-Alkylthio-alkyl, C₁-C₅-Alkysulfoxyl-C₁-C₅-alkyl, C₁-C₅-Alkylsulfonyl-C₁-C₅-alkyl, C₂-C₈-Alkylideneamino-oxy-C₁-C₅-alkyl, C₁-C₅-Alkylcarbonyl-C₁-C₅-alkyl, C₁-C₅-Alkoxycarbonyl-C₁-C₅-alkyl, C₁-C₅-Amino-carbonyl-C₁-C₅-alkyl, C₂-C₈-Dialkylamino-carbonyl-C₁-C₅-alkyl, C₁-C₅-Alkylcarbonylamino-C₁-C₅-alkyl, C₁-C₅-Alkylcarbonyl-(C₂-C₅-alkyl)-aminoalkyl, C₃-C₆-Trialkylsilyl-C₁-C₅-alkyl, Phenyl-C₁-C₅-alkyl, Heteroaryl-C₁-C₅-alkyl, Phenoxy- C₁-C₅-alkyl, Heteroaryloxy- C₁-C₅-alkyl, C₂-C₅-Alkenyl, C₂-C₅-Halogenalkenyl, C₃-C₈-Cycloalkyl, Phenyl, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Phenyl, oder Heteroaryl oder Heteroarylamino, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Heteroaryl oder Heteroarylamino, Diheteroarylamino, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Diheteroarylamino, Phenylamino, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Phenylamino, Diphenylamino oder durch C₁-C₃-Alkyl, C₁-C₃-Halogenalkyl, C₁-C₃-Alkoxy, C₁-C₃-Halogenalkoxy, Halogen, Cyano oder Nitro substituiertes Diphenylamino, oder C₃-C₇-Cycloalkylamino, Di-C₃-C₇-cycloalkylamino, C₃-C₇-Cycloalkoxy, C₁-C₁₀-Alkoxy, C₁-C₁₀-Halogenalkoxy, C₁-C₅-Alkylamino, C₂-C₈-Dialkylamino, Benzyloxy oder Phenoxy, wobei die Benzyl- und Phenylgruppen ihrerseits durch C₁-C₃-Alkyl, C₁-C₃-Halogenalkyl, C₁-C₃-Alkoxy, C₁-C₃-Halogenalkoxy, Halogen, Cyano oder Nitro substituiert sein können, bedeuten;
R₂₇ C₁-C₁₀-Alkyl, C₂-C₁₀-Alkenyl, C₂-C₁₀-Alkinyl, C₁-C₁₀-Halogenalkyl, C₁- C₁₀-Cyanoalkyl, C₁-C₁₀-Nitroalkyl, C₁-C₁₀-Aminoalkyl, C₁-C₅-Alkylamino-C₁-C₅-alkyl, C₂-C₈-Dialkylamino-C₁-C₅-alkyl, C₃-C₇-Cycloalkyl-C₁-C₅-alkyl, C₂-C₁₀-Alkoxy-alkyl, C₄-C₁₀-Alkenyloxy-alkyl, C₄-C₁₀-Alkinyloxy-alkyl, C₂-C₁₀-Alkylthio-alkyl, C₁-C₅-Alkylsulfoxyl- C₁-C₅-alkyl, C₁-C₅-Alkylsulfonyl-C₁-C₅-alkyl, C₂-C₈-Alkylideneamino-oxy-C₁-C₅-alkyl, C₁-C₅-Alkylcarbonyl-C₁-C₅-alkyl, C₁-C₅-Alkoxycarbonyl-C₁-C₅-alkyl, C₁-C₅-Amino-carbonyl-C₁-C₅-alkyl, C₂-C₈-Dialkylamino-carbonyl-C₁-C₅-alkyl, C₁-C₅-Alkylcarbonylamino-C₁-C₅-alkyl, C₁-C₅-Alkylcarbonyl-(C₂-C₅-alkyl)-aminoalkyl, C₃-C₆-Trialkylsilyl-C₁-C₅-alkyl, Phenyl-C₁-C₅-alkyl, Heteroaryl-C₁-C₅-alkyl, Phenoxy-C₁-C₅-alkyl, Heteroaryloxy-C₁-C₅-alkyl, C₂-C₅-Alkenyl, C₂-C₅-Halogenalkenyl, C₃-C₈-Cycloalkyl, Phenyl, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Phenyl, oder Heteroaryl, oder Heteroarylamino, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Heteroaryl oder Heteroarylamino, Diheteroarylamino, durch C₁-C₃-Alkyl, C₁-C₃-Halogenalkyl, C₁-C₃-Alkoxy, C₁-C₃-Halogenalkoxy, Halogen, Cyano oder Nitro substituiertes Diheteroarylamino, oder Phenylamino, durch C₁-C₃-Alkyl, C₁-C₃-Halogenalkyl, C₁-C₃-Alkoxy, C₁-C₃-Halogenalkoxy, Halogen, Cyano oder Nitro substituiertes Phenylamino, Diphenylamino, durch C₁-C₃-Alkyl, C₁-C₃-Halogenalkyl, C₁-C₃-Alkoxy, C₁-C₃-Halogenalkoxy, Halogen, Cyano oder Nitro substituiertes Diphenylamino, C₃-C₇-Cycloalkylamino, Di-C₃-C₇-cycloalkylamino, C₃-C₇-Cycloalkoxy oder C₁-C₁₀-Alkylcarbonyl bedeutet;
R₅₅ C₁-C₁₀-Alkyl bedeutet;
R₁₃₇ Wasserstoff oder C₁-C₁₀-Alkyl bedeutet; und
R₁₃₈ und R₁₃₉ unabhängig voneinander Wasserstoff oder C₁-C₁₀-Alkyl sind;
sowie agronomisch verträgliche Salze, Isomere und Enantiomere dieser Verbindungen.

Die in den Substituenten definitionen vorkommenden Alkylgruppen können geradkettig oder verzweigt sein und stehen beispielsweise für Methyl, Ethyl, n-Propyl, iso-Propyl, n-Butyl, sek.-Butyl, iso-Butyl, tert.-Butyl sowie die Isomeren Pentyle, Hexyle, Heptyle, Octyle, Nonyle und Decyle. Halogenalkyl ist beispielsweise Fluormethyl, Difluormethyl, Trifluormethyl, Chlormethyl, Dichlormethyl, Trichlormethyl, 2,2,2-Trifluorethyl, 2-Fluorethyl, 2-Chlorethyl,Pentafluorethyl, 1,1-Difluor-2,2,2-trichlorethyl, 2,2,3,3-Tetrafluorethyl und 2,2,2-Trichlorethyl; vorzugsweise Trichlormethyl, Difluorchlormethyl, Difluormethyl, Trifluormethyl und Dichlorfluormethyl. Alkoxyalkyl ist beispielsweise Methoxymethyl, Ethoxymethyl, Propoxyethyl, i-Propoxyethyl, n-Butoxymethyl, iso-Butoxyn-butyl, sek.-Butoxymethyl und tert.-Butoxy-i-propyl, vorzugsweise Methoxymethyl und Ethoxymethyl. Alkoxy-, Alkenyl-, Alkinyl-, Alkoxyalkyl-, Alkylthio-, Alkylsulfonyl-, Alkylaminocarbonyl-, Dialkylaminocarbonyl-Alkylaminoalkyl, Phenylalkyl-, Nitroalkyl-, Aminoalkyl- und N-Alkoxycarbonyl-N-alkylaminoalkylgruppen leiten sich von den genannten Alkylresten ab. Die Alkenyl- und Alkinylgruppen können ein- oder mehrfach ungesättigt sein. Unter Alkenyl ist beispielsweise Vinyl, Allyl, Methallyl, 1-Methylvinyl oder But-2-en-1-yl zu verstehen. Alkinyl bedeutet beispielsweise Ethinyl, Propargyl, But-2-in-1-yl, 2-Methylbutin-2-yl oder But-3-in-2-yl. Alkinyl bedeutet beispielsweise Ethinyl, Propargyl, But-2-in-1-yl, 2-Methylbutin-2-yl oder But-3-in-2-yl. Halogenalkylgruppen haben vorzugsweise eine Kettenlänge von 1 bis 4 Kohlenstoffatomen. Halogenalkyl ist beispielsweise Fluormethyl, Difluormethyl, Trifluormethyl, Chlormethyl, Dichlormethyl, Trichlormethyl, 2,2,2-Trifluorethyl, 2-Fluorethyl, 2-Chlorethyl,Pentafluorethyl, 1,1-Difluor-2,2,2-trichlorethyl, 2,2,3,3-Tetrafluorethyl und 2,2,2-Trichlorethyl; vorzugsweise Trichlormethyl, Difluorchlormethyl, Difluormethyl, Trifluormethyl und Dichlorfluormethyl. Als Halogenalkenyl kommen ein- oder mehrfach durch Halogen substituierte Alkenylgruppen in Betracht, wobei Halogen Fluor, Chlor, Brom und Jod und insbesondere Fluor und Chlor bedeutet, beispielsweise 2,2-Difluor-1-methylvinyl, 3-Fluorpropenyl, 3-Chlorpropenyl, 3-Brompropenyl, 2,3,3-Trifluorpropenyl, 2,3,3-Trichlorpropenyl und 4,4,4-Trifluor-but-2-en-1-yl. Unter den durch Halogen 1-, 2- oder 3-fach substituierten C₂-C₆-Alkenylgruppen sind diejenigen bevorzugt, die eine Kettenlänge von 3 bis 5 Kohlenstoffatomen besitzen. Alkoxygruppen haben vorzugsweise eine Kettenlänge von 1 bis 6 Kohlenstoffatomen. Alkoxy ist beispielsweise Methoxy, Ethoxy, Propoxy, i-Propoxy, n-Butoxy, iso-Butoxy, sek.-Butoxy und tert.-Butoxy sowie die Isomeren Pentyloxy und Hexyloxy; vorzugsweise Methoxy und Ethoxy. Alkylcarbonyl steht vorzugsweise für Acetyl oder Propionyl. Alkoxycarbonyl bedeutet beispielsweise Methoxycarbonyl, Ethoxycarbonyl, Propoxycarbonyl, iso-Propoxycarbonyl, n-Butoxycarbonyl, iso-Butoxycarbonyl, sek.-Butoxycarbonyl oder tert.-Butoxycarbonyl; vorzugsweise Methoxycarbonyl oder Ethoxycarbonyl. Alkylthiogruppen haben vorzugsweise eine Kettenlänge von 1 bis 4 Kohlenstoffatomen. Alkylthio ist beispielsweise Methylthio, Ethylthio, Propylthio, iso-Propylthio, n-Butylthio, iso-Butylthio, sek.-Butylthio oder tert.-Butylthio, vorzugsweise Methylthio und Ethylthio. Alkylsulfinyl ist beispielsweise Methylsulfinyl, Ethylsulfinyl, Propylsulfinyl, iso-Propylsulfinyl, n-Butylsulfinyl, iso-Butylsulfinyl, sek.-Butylsulfinyl, tert.-Butylsulfinyl; vorzugsweise Methylsulfinyl und Ethylsulfinyl. Alkylsulfonyl steht beispielsweise für Methylsulfonyl, Ethylsulfonyl, Propylsulfonyl, iso-Propylsulfonyl, n-Butylsulfonyl, iso-Butylsulfonyl, sek.-Butylsulfonyl oder tert.-Butylsulfonyl; vorzugsweise für Methylsulfonyl oder Ethylsulfonyl. Alkylamino ist beispielsweise Methylamino, Ethylamino, n-Propylamino, iso-Propylamino oder die isomeren Butylamine. Dialkylamino steht beispielsweise für Dimethylamino, Methylethylamino, Diethylamino, n-Propylmethylamino, Di-butylamino und Di-Isopropylamino. Alkoxyalkylgruppen haben vorzugsweise 1 bis 6 Kohlenstoffatome. Alkoxyalkyl bedeutet beispielsweise Methoxymethyl, Methoxyethyl, Ethoxymethyl, Ethoxyethyl, n-Propoxymethyl, n-Propoxyethyl, iso-Propoxymethyl oder iso-Propoxyethyl. Alkylthioalkyl bedeutet beispielsweise Methylthiomethyl, Methylthioethyl, Ethylthiomethyl, Ethylthioethyl, n-Propylthiomethyl, n-Propylthioethyl, iso-Propylthiomethyl, iso-Propylthioethyl, Butylthiomethyl, Butylthioethyl oder Butylthiobutyl. Phenyl, kann substituiert vorliegen. Die Substituenten können dann in ortho-, meta- und/oder para-Stellung stehen. Bevorzugte Substituentenstellungen sind die ortho- und para-Positionen zur Ringverknüpfungsstelle.

Aryl steht beispielsweise für Phenyl oder Naphtyl. Diese Gruppen können auch substituiert sein. Phenyl, auch als Teil eines Substitenten wie Phenylalkyl, kann beispielsweise- wenn in den Definitionen nicht anders angegeben, durch Halogen, Nitro, Cyano, C₁-C₄-Alkyl, C₁-C₄-Alkoxy, C₁-C₄-Alkylthio, C₁-C₄-Alkylsulfoxy, C₁-C₄-Alkylsulfonyl, Carboxyl, C₁-C₄-Alkoxycarbonyl, Amino, C₁-C₄-Alkylamino, C₁-C₄-Dialkylamino oder C₁-C₄-Alkylcarbonylamino substituiert sein.

Heteroarylgruppen sind üblicherweise aromatische Heterocyclen, die vorzugsweise 1 bis 3 Heteroatome wie Stickstoff, Schwefel und Sauerstoff enthalten. Beispiele für geeignete Heterocyclen und Heteroaromaten sind: Pyrrolidin, Piperidin, Pyran, Dioxan, Azetidin, Oxetan, Pyridin, Pyrimidin, Triazin, Thiazol, Thiadiazol, Imidazol, Oxazol, Isoxazol sowie Pyrazin, Furan, Morpholin, Piperazin, Pyrazol, Benzoxazol, Benzthiazol, Chinoxalin und Chinolin. Diese Heterocyclen und Heteroaromaten können weiter substituiert sein, beispielsweise mit Halogen, Alkyl, Alkoxy, Haloalkyl, Haloalkoxy, Nitro, Cyano, Thioalkyl, Alkylamino oder Phenyl.

Unter den 3- bis 7- gliedrigen Cyclen sind im Rahmen der vorliegenden Erfindung Ringsysteme zu verstehen, die zusätzlich zu den gegebenenfalls im Ring der Substituenten Q bereits vorhandenen Heteroatomen neben den Kohlenstoffatomen ein oder mehrere Heteroatome wie Stickstoff, Sauerstoff und/oder Schwefel enthalten können. Sie können gesättigt oder ungesättigt sein. Die ungesättigte Bindung kann beispielsweise bei der Gruppe Q₂ durch die Substituenten R₆ und R₇ gebildet werden. Bevorzugt enthalten solche Ringsysteme 5 bis 7 Ringatome.
3- bis 7- gliedrige Cyclen einschließlich die Cycloalkyle wie z.B. Cyclopropyl, Cyclobutyl, Cyclopentyl, Cyclohexyl, Cycloheptyl oder Cyclooctyl können auch substituiert sein. Geeignete Substituenten sind Halogen, Hydroxy, Nitro, Cyano, C₁-C₄-Alkylcarbonyl, C₁-C₄-Alkoxycarbonyl, C₁-C₄-Alkyl, C₁-C₄-Halogenalkyl, Keto, C₂-C₄-Alkenyloxyimino, C₁-C₄-Alkoxy, C₁-C₄-Alkoxyalkoxy, C₁-C₄-Alkylthio, oder eine der folgenden 3 Gruppen 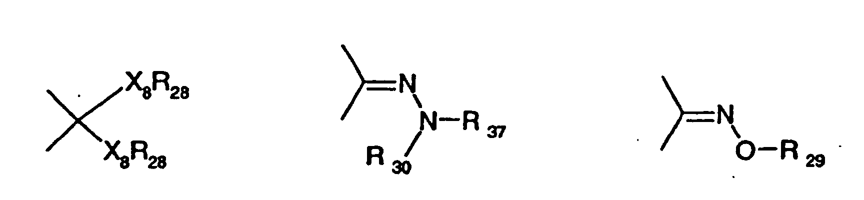 worin X₈ Schwefel oder Sauerstoff bedeutet, R₂₈ C₁-C₄-Alkoxyl oder beide R₂₈ bilden mit der -X₈-C-X₈-Brücke, an die sie gebunden sind, einen 5- oder 6-gliedrigen Ring, der mit Methyl, Ethyl, Methoxy oder einer Ketogruppe substituiert sein kann,
R₂₉ C₁-C₄-Alkyl, C₁-C₄-Halogenalkyl, C₂-C₄-Alkenyl oder C₂-C₄-Halogenalkenyl bedeutet,
R₃₀ und R₃₇ unabhängig voneinander C₁-C₄-Alkyl, Phenyl, C₂-C₄-Alkenyl, oder R₃₀ und R₃₇ bilden zusammen mit dem Stickstoffatom, an das sie gebunden sind, einen 5- bis 6 gliedrigen Ring, der ein Heteroatom ausgewählt aus Stickstoff, Sauerstoff und Schwefel enthalten kann.

In den Substituentendefinitionen bedeutet die Zahl der Kohlenstoffatome die Gesamtzahl der Kohlenstoffatome in den Alkyl-, Alkenyl- und Alkinylgruppen sowie den davon abgeleiteten Gruppen wie z.B. Halogenalkyl oder Alkenyloxy. C₂-C₃-Alkoxyalkyl umfaßt daher Methoxymethyl, Metoxyethyl und Ethoxymethyl. C₃-Alkoxycarbonylalkyl umfaßt Methoxycarbonylethyl und Ethoxycarbonylmethyl.

Alkali-, Erdalkali- oder Ammoniumkationen für die Substituenten G₁, G₂ und G₁₀ sind beispielsweise die Kationen von Natrium, Kalium, Magnesium, Kalzium und Ammonium. Bevorzugte Sulfoniumkationen sind insbesondere Trialkylsulfoniumkationen, worin die Alkylgruppen vorzugsweise je 1 bis 4 Kohlenstoffatome enthalten.

Die Verbindungen der Formel I können, auch in Abhängigkeit von der Art der Substituenten, als geometrische, und/oder optische Isomere und Isomerengemische sowie als Tautomere und Tautomerengemische vorliegen. Diese Verbindungen der Formel I bilden ebenfalls einen Gegenstand der vorliegenden Erfindung. Beispielsweise können die Verbindungen der Formel I, worin Q für Q₁ steht und die Gruppe G₁ Wasserstoff bedeutet, in den folgenden tautomeren Gleichgewichten vorliegen:

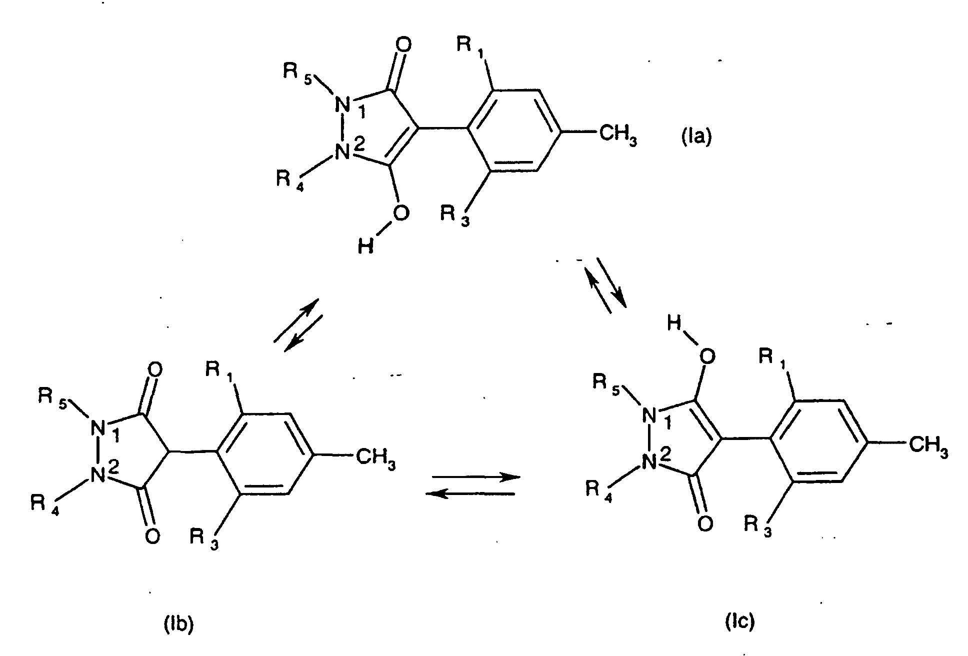

Wenn G₁ bis G₁₀ verschieden von Wasserstoff ist und der von R₄ und R₅ zusammen gebildete Cyclus unsymmetrisch substituiert, anelliert oder spiroverknüpft ist, kann beispielsweise die Verbindung der Formel I als Isomer der Formel Id 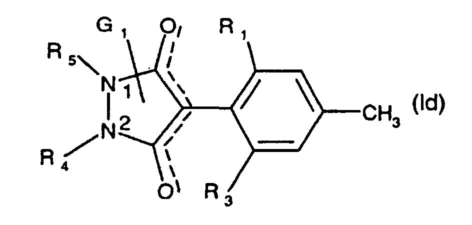 vorliegen.

Die Erfindung umfaßt ebenfalls die Salze, die die Verbindungen der Formel I vorzugsweise mit Aminen, Alkali- und Erdalkalimetallbasen oder quaternären Ammoniumbasen bilden können. Geeignete Salzbildner sind beispielsweise in WO 98/41089 beschrieben.

Die Erfindung umfaßt ebenfalls die Salze, die die Verbindungen der Formel I mit Aminen, Alkali- und Erdalkalimetallbasen oder quaternären Ammoniumbasen bilden können.

Unter den Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Als Beispiele für zur Ammoniumsalzbildung geeignete Amine kommen sowohl Ammoniak wie auch primäre, sekundäre und tertiäre C₁-C₁₈-Alkylamine, C₁-C₄-Hydroxyalkylamine und C₂-C₄-Alkoxyalkylamine in Betracht, beispielsweise Methylamin, Ethylamin, n-Propylamin, iso-Propylamin, die vier isomeren Butylamine, n-Amylamin, iso-Amylamin, Hexylamin, Heptylamin, Octylamin, Nonylamin, Decylamin, Pentadecylamin, Hexadecylamin, Heptadecylamin, Octadecylamin, Methyl-ethylamin, Methyl-iso-propylamin, Methylhexylamin, Methyl-nonylamin, Methyl-pentadecylamin, Methyl-octadecylamin, Ethylbutylamin, Ethyl-heptylamin, Ethyl-octylamin, Hexyl-heptylamin, Hexyl-octylamin, Dimethylamin, Diethylamin, Di-n-propylamin, Di-iso-propylamin, Di-n-butylamin, Di-namylamin, Di-iso-amylamin, Dihexylamin, Diheptylamin, Dioctylamin, Ethanolamin, n-Propanolamin, iso-Propanolamin, N,N-Diethanolamin, N-Ethylpropanolamin, N-Butylethanolamin, Allylamin, n-Butenyl-2-amin, n-Pentenyl-2-amin, 2,3-Dimethylbutenyl-2-amin, Di-butenyl-2-amin, n-Hexenyl-2-amin, Propylendiamin, Trimethylamin, Triethylamin, Tri-n-propylamin, Tri-iso-propylamin, Tri-n-butylamin, Tri-iso-butylamin, Tri-sek.-butylamin, Tri-n-amylamin, Methoxyethylamin und Ethoxyethylamin; heterocyclische Amine wie z.B. Pyridin, Chinolin, iso-Chinolin, Morpholin, Piperidin, Pyrrolidin, Indolin, Chinuclidin und Azepin; primäre Arylamine wie z.B. Aniline, Methoxyaniline, Ethoxyaniline, o,m,p-Toluidine, Phenylendiamine, Benzidine, Naphthylamine und o,m,p-Chloraniline; insbesondere aber Triethylamin, iso-Propylamin und Di-iso-propylamin.

Bevorzugte quartemäre Ammoniumbasen, die zur Salzbildung geeignet sind, entsprechen z.B. der Formel [N(RₐR_{b}R_{c}R_{d})]OH, worin Rₐ, R_{b}, R_{c} und R_{d} unabhängig voneinander C₁-C₄ Alkyl bedeuten. Andere geeignete Tetraalkylammoniumbasen mit anderen Anionen können beispielsweise durch Anionenaustauschreaktionen erhalten werden.

Von den Verbindungen der Formel I sind diejenigen bevorzugt, worin Q für Q₁, Q₂ oder Q₅ steht.
Ferner bevorzugte Verbindungen der Formel I sind dadurch gekennzeichnet, daß R₄ und R₅ unahängig voneinander C₁-C₆-Alkyl, C₁-C₆-Halogenalkyl, C₂-C₆-Alkoxyalkyl, C₄-C₆-Alkenyloxyalkyl, C₄-C₆-Alkinyloxyalkyl, C₂-C₆-Alkylthioalkyl, C₂-C₆-Alkylsulfoxylalkyl, C₂-C₆-Alkylsulfonylalkyl, C₂-C₆-Alkylcarbonyl-alkyl, C₃-C₆-N-Alkoxy-iminoalkyl, C₃-C₆-Alkoxycarbonylalkyl, C₁-C₆-Aminoalkyl, C₂-C₆-Dialkylaminoalkyl, C₃-C₆-Alkylaminoalkyl, C₁-C₆-Cyanoalkyl, C₄-C₈-Cycloalkylalkyl, C₇-C₈-Phenylalkyl, C₇-C₈-Heteroarylalkyl, C₇-C₈-Phenoxylalkyl, C₇-C₈-Heteroaryloxyalkyl, C₄-C₆-Alkylidenaminooxyalkyl, C₁-C₆-Nitroalkyl, C₄-C₈-Trialkylsilylalkyl, C₄-C₆-Alkylaminocarbonyl, C₃-C₆-Dialkylaminocarbonyl, C₄-C₈-Alkylaminocarbonyloxyalkyl, C₄-C₈-Dialkylaminocarbonyloxalkyl, C₄-C₈-Alkoxycarbonylaminoalkyl, C₄-C₈-N-Alkoxycarbonyl-N-alkylamino-alkyl, C₃-C₈-Cycloalkyl, Aryl oder Heteroaryl bedeuten, oder
R₄ und R₅ gemeinsam mit den Atomen, an die sie gebunden sind, einen 5- bis 7- gliedrigen Cyclus bilden.
Femer sind diejenigen Verbindungen der Formel I bevorzugt, worin
R₆ C₁-C₆-Alkyl, C₁-C₆-Halogenalkyl, C₂-C₆-Alkoxyalkyl, C₄-C₆-Alkenyloxyalkyl, C₄-C₆-Alkinyloxyalkyl, C₂-C₆-Alkylthioalkyl, C₂-C₆-Alkylsulfoxylalkyl, C₂-C₆-Alkylsulfonylalkyl, C₃-C₆-Alkylcarbonyl-alkyl,
C₃-C₈-Cyclolalkyl, Aryl oder Heteroaryl;
R₇ C₁-C₆-Alkyl oder C₁-C₆-Alkoxyalkyl;
R₈ Wasserstoff, C₁-C₆-Alkyl, C₁-C₆-Halogenalkyl, C₂-C₆-Alkoxyalkyl, C₄-C₆-Alkenyloxyalkyl, C₄-C₆-Alkinyloxyalkyl, C₁-C₆-Alkylthioalkyl, C₁-C₆-Alkylsulfinylalkyl, C₁-C₆- Alkylsulfonylalkyl, C₃-C₈-Cyclolalkyl, Aryl oder Heteroaryl bedeutet; oder
R₆ und R₇ bilden gemeinsam mit den Atomen, an die sie gebunden sind, einen 5-bis 7-gliedrigen Cyclus, der ein oder zwei Heteroatome ausgewählt aus Stickstoff, Sauerstoff und Schwefel enthalten kann, oder
R₆ und R₈ bilden gemeinsam mit den Atomen, an die sie gebunden sind, einen 5-bis 7-gliedrigen Cyclus, der ein oder zwei Heteroatome ausgewählt aus Stickstoff, Sauerstoff und Schwefel enthalten kann;
R₉, R₁₀, R₁₁ und R₁₂ unabängig voneinander C₁-C₆-Alkyl, C₁-C₆-Halogenalkyl, C₂-C₆-Alkoxyalkyl, C₄-C₆-Alkenyloxyalkyl, C₄-C₆-Alkinyloxyalkyl, C₂-C₆-Alkylthialkyl, C₂-C₆-Alkylsulfinylalkyl, C₂-C₆- Alkylsulfonylalkyl, C₃-C₆-Alkylcarbonyl-alkyl, C₃-C₈-Cyclolalkyl, Aryl oder Heteroaryl bedeuten; oder
R₁₀ und R₁₁ oder R₉ und R₁₀ bilden gemeinsam mit den Atomen, an die sie gebunden sind einen 5- bis 7- gliedrigen Cyclus, der ein oder zwei Heteroatome ausgewählt aus Stickstoff, Sauerstoff und Schwefel enthalten kann, oder
R₂₀, R₂₁, R₂₂, R₂₃, und R₂₇ unabhängig voneinander Wasserstoff,
C₁-C₈-Alkyl, C₁-C₈-Halogenalkyl, C₁-C₈-Cyanoalkyl, C₁-C₈-Nitroalkyl, C₁-C₈-Aminoalkyl, C₁-C₅-Alkylamino-C₁-C₂-alkyl, C₂-C₆-Dialkylamino-C₁-C₂-alkyl, C₃-C₇-Cycloalkyl-C₁-C₂-alkyl, C₂-C₈-Alkoxy-alkyl, C₄- C₈-Alkenyloxy-alkyl, C₄-C₈-Alkinyloxy-alkyl, C₂-C₈-Alkylthio-alkyl, C₁-C₂-Alkysulfoxyl-C₁-C₂-alkyl, C₁-C₂-Alkylsulfonyl-C₁-C₂-alkyl, C₂-C₈-Alkylideneamino-oxy-C₁-C₂alkyl, C₁-C₅-Alkylcarbonyl-C₁-C₂-alkyl, C₁-C₅-Alkoxycarbonyl-C₁-C₂-alkyl, C₁-C₅-Aminocarbonyl-C₁-C₂-alkyl, C₂-C₈-Dialkylamino-carbonyl-C₁-C₂-alkyl, C₁-C₅-Alkylcarbonylamino-C₁-C₂-alkyl, C₁-C₂-Alkylcarbonyl-N-C₁-C₃-alkyl-C₁-C₂-aminoalkyl, C₃-C₆-Trialkylsilyl-C₁-C₃-alkyl, Phenyl-C₁-C₂-alkyl, Heteroaryl-C₁-C₂-alkyl, Phenoxy-C₁-C₂-alkyl, Heteroaryloxy-C₁-C₂-alkyl, C₂-C₅-Alkenyl, C₂-C₅-Halogenalkenyl, C₃-C₈-Cycloalkyl, Phenyl oder Heteroaryl bedeuten;
R₂₄, R₂₅ und R₂₆ unabhängig voneinander Wasserstoff, C₁-C₈-Alkyl, C₁-C₈-Halogenalkyl, C₁-C₈-Cyanoalkyl, C₁- C₈-Nitroalkyl, C₁- C₈-Aminoalkyl, C₁-C₅-Alkylamino-C₁-C₂-alkyl, C₂-C₆-Dialkylamino-C₁-C₂-alkyl, C₃-C₇-Cycloalkyl-C₁-C₂-alkyl, C₂-C₈-Alkoxy-alkyl, C₄- C₈-Alkenyloxyalkyl, C₄-C₈-Alkinyloxy-alkyl, C₂-C₈-Alkylthio-alkyl, C₁-C₂-Alkysulfoxyl-C₁-C₂-alkyl, C₁-C₂-Alkylsulfonyl-C₁-C₂-alkyl, C₂-C₈-Alkylideneamino-oxy-C₁-C₂-alkyl, C₁-C₅-Alkylcarbonyl-C₁-C₂-alkyl, C₁-C₅-Alkoxycarbonyl-C₁-C₂-alkyl, C₁-C₅-Amino-carbonyl-C₁-C₂-alkyl, C₂-C₈-Dialkylamino-carbonyl-C₁-C₂-alkyl, C₁-C₅-Alkylcarbonylamino-C₁-C₂-alkyl, C₁-C₂-Alkylcarbonyl-N-C₁-C₃-alkyl-C₁-C₂-aminoalkyl, C₃-C₆-Trialkylsilyl-C₁-C₃-alkyl, Phenyl-C₁-C₂-alkyl, Heteroaryl-C₁-C₂-alkyl, Phenoxy-C₁-C₂-alkyl, Heteroaryloxy-C₁-C₂-alkyl, C₂-C₅-Alkenyl, C₂-C₅-Halogenalkenyl, C₃-C₈-Cycloalkyl, Phenyl, Heteroaryl, C₁-C₆-Alkoxy, C₁-C₆-Halogenalkoxy, C₁-C₃-Alkylamino, C₂-C₆-Dialkylamino sowie Benzyloxy oder Phenoxy, wobei die Benzyl- und Phenylgruppen ihrerseits durch C₁-C₃-Alkyl, C₁-C₃-Halogenalkyl, C₁-C₃-Alkoxy, C₁-C₃-Halogenalkoxy, Halogen, Cyano oder Nitro substituiert sein können, bedeuten; und
R₂₇ C₁-C₈-Alkyl, C₁-C₈-Halogenalkyl, C₁- C₈-Cyanoalkyl, C₁- C₈-Nitroalkyl, C₁- C₈-Aminoalkyl, C₁-C₅-Alkylamino-C₁-C₂-alkyl, C₂-C₆-Dialkylamino-C₁-C₂-alkyl, C₃-C₇₋Cycloalkyl-C₁-C₂-alkyl, C₂-C₈-Alkoxy-alkyl, C₄- C₈-Alkenyloxy-alkyl, C₄-C₈-Alkinyloxy-alkyl, C₂-C₈-Alkylthio-alkyl, C₁-C₂-Alkysulfoxyl-C₁-C₂-alkyl, C₁-C₂-Alkylsulfonyl-C₁-C₂-alkyl, C₂-C₈-Alkylideneamino-oxy-C₁-C₂-alkyl, C₁-C₅-Alkylcarbonyl-C₁-C₂-alkyl, C₁-C₅-Alkoxycarbonyl-C₁-C₂-alkyl, C₁-C₅-Aminocarbonyl-C₁-C₂-alkyl, C₂-C₈-Dialkylamino-carbonyl-C₁-C₂-alkyl, C₁-C₅-Alkylcarbonylamino-C₁-C₂-alkyl, C₁-C₂-Alkylcarbonyl-N-C₁-C₃-alkyl-C₁-C₂-aminoalkyl, C₃-C₆-Trialkylsilyl-C₁-C₃-alkyl, Phenyl-C₁-C₂-alkyl, Heteroaryl-C₁-C₂-alkyl, Phenoxy-C₁-C₂-alkyl, Heteroaryloxy-C₁-C₂-alkyl, C₂-C₅-Alkenyl, C₂-C₅-Halogenalkenyl, C₃-C₈-Cycloalkyl, Phenyl, Heteroaryl, C₁-C₆-Alkoxy, C₁-C₆-Halogenalkoxy, C₁-C₈-Alkylcarbonyl, C₁-C₃-Alkylamino, C₂-C₆-Dialkylamino sowie Benzyloxy oder Phenoxy, wobei die Benzyl- und Phenylgruppen ihrerseits durch C₁-C₃-Alkyl, C₁-C₃-Halogenalkyl, C₁-C₃-Alkoxy, C₁-C₃-Halogenalkoxy, Halogen, Cyano oder Nitro substituiert sein können, bedeutet.
Besonders bevorzugte Verbindungen der Formel I sind dadurch gekennzeichnet, daß R₁ und R₃ unabhängig voneinander Ethyl, Halogenethyl, Ethinyl, C₁-C₂-Alkoxy, C₁-C₂-Halogenalkoxy oder C₁-C₂-Alkylcarbonyl bedeuten;
R₄ und R₅ unahängig voneinander C₁-C₆-Alkyl, C₁-C₆-Halogenalkyl, C₂-C₆-Alkoxyalkyl, C₂-C₆-Alkylcarbonyl-alkyl, C₃-C₆-Alkoxycarbonylalkyl, C₁-C₆-Aminoalkyl, C₂-C₆-Dialkylaminoalkyl, C₃-C₆-Alkylaminoalkyl, C₁-C₆-Cyanoalkyl, C₃-C₈-Cycloalkyl, Aryl oder Heteroaryl bedeuten; oder
R₄ und R₅ bilden gemeinsam mit den Atomen, an die sie gebunden sind einen 5- bis 7-gliedrigen Cyclus, der ein oder zwei Heteroatome ausgewählt aus Stickstoff, Sauerstoff und Schwefel enthalten kann;
R₆ C₁-C₆-Alkyl, C₁-C₆-Halogenalkyl, C₂-C₆-Alkoxyalkyl, C₃-C₈-Cyclolalkyl, Aryl oder Heteroaryl;
R₇ Wasserstoff, C₁-C₆-Alkyl oder C₁-C₆-Alkoxyalkyl bedeutet;
R₈ Wasserstoff, C₁-C₆-Alkyl, C₁-C₆-Halogenalkyl, C₂-C₆-Alkoxyalkyl, C₁-C₆-Alkylthioalkyl, C₃-C₈-Cyclolalkyl, Aryl oder Heteroaryl bedeutet; oder
R₆ und R₇ bilden gemeinsam mit dem Atom, an das sie gebunden sind, einen gesättigten 3- bis 7- gliedriger Cyclus, der ein oder zwei Heteroatome ausgewählt aus Stickstoff, Sauerstoff und Schwefel enthalten kann; oder
R₆ und R₈ bilden gemeinsam mit den Atomen, an die sie gebunden sind, einen 5- bis 7-gliedrigen Cyclus, der ein oder zwei Heteroatome ausgewählt aus Stickstoff, Sauerstoff und Schwefel enthalten kann;
R₉, R₁₀, R₁₁ und R₁₂ unabängig voneinander C₁-C₆-Alkyl, C₁-C₆-Halogenalkyl, C₂-C₆-Alkoxyalkyl, C₃-C₈-Cyclolalkyl, Aryl oder Heteroaryl bedeuten; oder
R₉ und R₁₁ bilden gemeinsam mit den Atomen, an die sie gebunden sind, einen 5- bis 7-gliedrigen Cyclus bildet, der ein oder zwei Heteroatome ausgewählt aus Stickstoff, Sauerstoff und Schwefel enthalten kann; oder
R₉ und R₁₀ bilden gemeinsam mit dem Atom, an das sie gebunden sind, einen gesättigten 3- bis 7- gliedrigen Cyclus, der ein oder zwei Heteroatome ausgewählt aus Stickstoff, Sauerstoff und Schwefel enthalten kann;
R₂₀, R₂₁, R₂₂ und R₂₃ unabhängig voneinander Wasserstoff, C₁-C₈-Alkyl, C₁-C₈-Halogenalkyl, C₃-C₇-Cycloalkyl-C₁-C₂-alkyl, C₂-C₈-Alkoxy-alkyl, Phenyl-C₁-C₂-alkyl, Heteroaryl-C₁-C₂-alkyl, Phenoxy-C₁-C₂-alkyl, Heteroaryloxy-C₁-C₂-alkyl, C₂-C₅-Alkenyl, C₂-C₅-Halogenalkenyl, C₃-C₈-Cycloalkyl, Phenyl oder Heteroaryl bedeuten;
R₂₄, R₂₅ und R₂₆ unabhängig voneinander Wasserstoff, C₁-C₈-Alkyl, C₁-C₈-Halogenalkyl, C₃-C₇₋Cycloalkyl-C₁-C₂-alkyl, C₂-C₈-Alkoxy-alkyl, Phenyl-C₁-C₂-alkyl, Heteroaryl-C₁-C₂-alkyl, Phenoxy-C₁-C₂-alkyl, Heteroaryloxy-C₁-C₂-alkyl, C₂-C₅-Alkenyl, C₂-C₅-Halogenalkenyl, C₃-C₈-Cycloalkyl, Phenyl, Heteroaryl, C₁-C₆-Alkoxy, C₁-C₃-Alkylamino oder C₂-C₆-Dialkylamino bedeuten; und
R₂₇ C₁-C₈-Alkyl, C₁-C₈-Halogenalkyl, C₃-C₇-Cycloalkyl-C₁-C₂-alkyl, C₂-C₈-Alkoxy-alkyl, Phenyl-C₁-C₂-alkyl, Heteroaryl-C₁-C₂-alkyl, Phenoxy-C₁-C₂-alkyl, Heteroaryloxy-C₁-C₂-alkyl, C₂-C₅-Alkenyl, C₂-C₅-Halogenalkenyl, C₃-C₈-Cycloalkyl, Phenyl, Heteroaryl, C₁-C₆-Alkoxy, C₁-C₃-Alkylamino, C₂-C₆-Dialkylamino oder C₁-C₈-Alkylcarbonyl bedeuten.

Die Verbindungen der Formel I können hergestellt werden, indem man eine Verbindung der Formel XXX

Q-H (XXX)

worin Q für Q₁, Q₂, Q₅ oder Q₁₀ steht, wobei deren Substituenten mit Ausnahme von G₁, G₂, G₅ und G₁₀ die oben angegebene Bedeutung haben und G₁, G₂, G₅, und G₁₀ Wasserstoff bedeutet, mit einer Verbindung der Formel XXXI 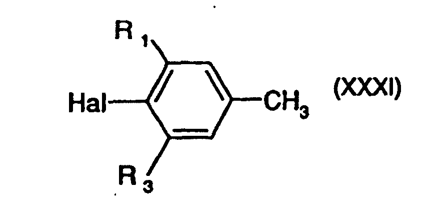 worin R₁ und R₃ die unter Formel I angegebene Bedeutung haben und Hal für Chlor, Brom oder Jod steht, in Gegenwart eines inerten Lösungsmittels, einer Base und eines Palladiumkatalysators bei Temperaturen von 30 bis 250 °C umsetzt. Die Reaktion wird vorzugsweise unter Inertgasatmosphäre durchgeführt.

Es hat sich überraschenderweise gezeigt, dass die Herstellung von Verbindungen der Formel I, worin R₁ und R₃ für Ethyl stehen, mit diesem Verfahren ganz besonders vorteilhaft ist.

Die Verbindungen der Formel XXX sind bekannt oder nach bekannten Verfahren, wie beispielsweise in J. Chem. Soc. Perkin Trans. 1 (1987), (4), 877-884 beschrieben, herstellbar. Die Verbindungen der Formel XXXI können beispielsweise nach bekannten Methoden wie z.B. Sandmeyer-Reaktion aus den entsprechenden Anilinen der Formel XXXII 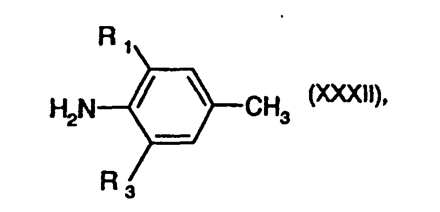 worin R₁ und R₃ die unter Formel I angegebenen Bedeutungen haben, über die Diazoniumsalze hergestellt werden. Derartige Reaktionen sind beispielsweise in Vogel's Textbook of Practical Organic Chemistry, 5*th* Edition, B.S. Furniss, A.J. Hannaford, P.W.G. Smith, A.R. Tatchell; Longman Scientific & Technical 1989, Seite 923 beschrieben. Die Verbindungen der Formel XXXII sind bekannt, teilweise kommerziell erhältlich oder können analog zu bekannten hergestellt werden.

Geeignet für diese Reaktion sind Basen, wie Tri-Alkalimetallphosphate, Alkali- und Erdalkalimetallhydride, Alkali- und Erdalkalimetallamide oder Alkalimetallalkoholate, beispielsweise Tri-Kaliumphosphat, Natriumhydrid, Lithiumdiisopropylamid (LDA), Na-tert.-Butylat oder K-tert.Butylat. Besonders bevorzugt sind Na-tert.-Butylat, K-tert.Butylat sowie Trikaliumphosphat.

Geeignete Lösungsmittel sind beispielsweise aromatische Kohlenwasserstoffe wie z.B. Xylol oder Toluol, Ether wie Tetrahydrofuran, Dioxan oder Ethylenglykoldimethylether, Dimethylsulfoxid oder tertiäre Amide wie Dimethylformamid , N-Methylpyrrolidinon oder Dimethylacetamid oder acyclische Harnstoffe wie N,N'-Dimethylpropylenhamstoff.

Die für die C-C-Verknüpfungsreaktion einer Verbindung der Formel XXX mit einer Verbindung der Formel XXXI in Betracht kommenden Palladiumkatalysatoren sind generell Palladium(II)- oder Palladium(0)-Komplexe wie z.B. Palladium(II)-dihalogenide, Palladium(II)-acetat, Palladium(II)-sulfat, Bis-(triphenylphosphin)-palladium(II)-dichlorid, Bis-(tricyclopentylphosphin)-palladium(II)-dichlorid, Bis-(tricyclohexylphosphin)-palladium(II)-dichlorid, Bis-(dibenzylidenaceton)-palladium(0) oder Tetrakis-(triphenylphosphin)-palladium(0). Der Palladiumkatalysator kann auch aus Palladium(II)- oder Palladium(0)-Verbindungen durch Komplexierung mit den gewünschten Liganden *'in situ'* hergestellt werden, indem z.B. das zu komplexierende Palladium(II)-Salz beispielsweise Palladium(II)-dichlorid (PdCl₂) oder Palladium(II)-acetat (Pd(OAc)₂) zusammen mit dem gewünschten Liganden beispielsweise Triphenylphosphin (PPh₃), tricyclopentylphosphin oder Tricyclohexylphosphin zusammen mit dem gewählten Lösungsmittel, eine Verbindung der Formel XXXI, eine Verbindung der Formel XXX und Base vorgelegt wird. Bidendate Liganden kommen auch in Frage, wie zum Beispiel 1,1'-Bis-(Diphenylphosphino)ferrocen oder 1,2-Bis-(Diphenylphosphino)ethan. Durch Erwärmung des Reaktionsmediums bildet sich dann 'in situ' der für die C-C-Kopplungsreaktion gewünschte Palladium(II)- bzw. Palladium(0)-Komplex, welcher dann die C-C-Kopplungsreaktion startet.
Die Palladiumkatalysatoren werden in einer Menge von 0,001 - 50 Mol%, bevorzugt in einer Menge von 0,1 - 15 Mol% bezogen auf die Verbindung der Formel XXXI eingesetzt.

Die Reaktionstemperaturen werden in Abhängigkeit des verwendeten Lösungsmittels und gegebenenfalls des Drucks gewählt. Vorzugsweise wird die Reaktion bei Atmosphärendruck durchgeführt.

Die Verbindungen der Formel I, worin Q für Q₁ steht, können analog den in WO 96/21652 beschriebenen Verfahren hergestellt werden. Verbindungen der Formel I worin Q für Q₂ steht, können beispielsweise nach den in EP-A-0 415 185, EP-A-0 521 334, EP-A-0 355 599 und EP-A-0 442 077 beschriebenen Verfahren hergestellt werden. Verbindungen der Formel I worin Q für Q₅ steht können z.B. analog den in WO 97/14667 beschriebenen Verfahren hergestellt werden. Verbindungen der Formel I, worin Q für Q₁₀ steht, können nach J. Org. Chem. (1979), 44(26), 4906-4912 oder J. Org. Chem. (1977), 42(7), 1163-1169 oder analog hergestellt werden.

Die Umsetzungen zu Verbindungen der Formel I werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, chlorierte Kohlenwasserstoffe wie Dichlormethan, Trichlormethan, Tetrachlormethan oder Chlorbenzol, Ether wie Diethylether, Ethylenglykoldimethylether, Diethylenglykoldimethylether, Tetrahydrofuran oder Dioxan, Nitrile wie Acetonitril oder Propionitril, Amide wie N,N-Dimethylformamid, Diethylformamid oder N-Methylpyrrolidinon. Die Reaktionstemperaturen liegen vorzugsweise zwischen -20°C und +120°C. Die Umsetzungen verlaufen im allgemeinen leicht exotherm und können in der Regel bei Raumtemperatur durchgeführt werden. Zum Abkürzen der Reaktionszeit oder auch zum Einleiten der Umsetzung kann gegebenenfalls für kurze Zeit bis zum Siedepunkt des Reaktionsgemisches aufgewärmt werden. Die Reaktionszeiten können ebenfalls durch Zugabe einiger Tropfen Base als Reaktionskatalysator verkürzt werden. Als Basen sind insbesondere tertiäre Amine wie Trimethylamin, Triethylamin, Chinuclidin, 1,4-Diazabicyclo-[2.2.2]octan, 1,5-Diazabicyclo[4.3.0]non-5-en oder 1,5-Diazabicyclo[5.4.0]undec-7-en geeignet. Als Basen können aber auch anorganische Basen wie Hydride wie Natrium- oder Calciumhydrid, Hydroxide wie Natrium- oder Kaliumhydroxid, Carbonate wie Natrium- und Kaliumcarbonat oder Hydrogencarbonate wie Kalium- und Natriumhydrogencarbonat verwendet werden. Die Verbindungen der Formel I können auf übliche Weise durch Einengen und/oder Verdampfen des Lösungsmittels isoliert und durch Umkristallisieren oder Zerreiben des festen Rückstandes in Lösungsmitteln, in denen sie sich nicht gut lösen, wie Ether, aromatischen Kohlenwasserstoffe oder chlorierten Kohlenwasserstoffe, gereinigt werden.

Für die erfindungsgemäße Verwendung der Verbindungen der Formel I oder diese enthaltende Mittel kommen alle in der Landwirtschaft üblichen Applikationsmethoden wie z.B. preemergente Applikation, postemergente Applikation und Saatbeizung, sowie verschiedene Methoden und Techniken in Betracht, wie beispielsweise die kontrollierte Wirkstoffabgabe. Dazu wird der Wirkstoff in Lösung auf mineralische Granulatträger oder polymerisierte Granulate (Harnstoff/Formaldehyd) aufgezogen und getrocknet. Gegebenenfalls kann zusätzlich ein Überzug aufgebracht werden (Umhüllungsgranulate), der es erlaubt, den Wirkstoff über einen bestimmten Zeitraum dosiert abzugeben.

Die Verbindungen der Formel I können in unveränderter Form, d.h. wie sie in der Synthese anfallen, als Herbizide eingesetzt werden. Vorzugsweise verarbeitet man sie aber auf übliche Weise mit den in der Formulierungstechnik gebräuchlichen Hilfsmitteln z.B. zu emulgierbaren Konzentraten, direkt versprühbaren oder verdünnbaren Lösungen, verdünnten Emulsionen, Spritzpulvern, löslichen Pulvern, Stäubemitteln, Granulaten oder Mikrokapseln. Solche Formulierungen sind beispielsweise in der WO 97/34485 auf den Seiten 9 bis 13 beschrieben. Die Anwendungsverfahren wie Versprühen, Vernebeln, Verstäuben, Benetzen, Verstreuen oder Gießen werden gleich wie die Art der Mittel, den angestrebten Zielen und den gegebenen Verhältnissen entsprechend gewählt.

Die Formulierungen, d.h. die den Wirkstoff der Formel I bzw. mindestens einen Wirkstoff der Formel I und in der Regel einen oder mehrere feste oder flüssige Formulierungshilfsmittel enthaltenden Mittel, Zubereitungen oder Zusammensetzungen werden in bekannter Weise hergestellt, z.B. durch inniges Vermischen und/oder Vermahlen der Wirkstoffe mit den Formulierungshilfsmitteln wie z.B. Lösungsmittel oder festen Trägerstoffe. Ferner können zusätzlich oberflächenaktive Verbindungen (Tenside) bei der Herstellung der Formulierungen verwendet werden. Beispiele für Lösungsmittel und feste Trägerstoffe sind z.B. in der WO 97/34485 auf der Seite 6 angegeben.

Als oberflächenaktive Verbindungen kommen je nach der Art des zu formulierenden Wirkstoffes der Formel I nichtionogene, kation- und/oder anionaktive Tenside und Tensidgemische mit guten Emulgier-, Dispergier- und Netzeigenschaften in Betracht. Beispiele für geeignete anionische, nichtionische und kationische Tenside sind beispielsweise in der WO 97/34485 auf den Seiten 7 und 8 aufgezählt. Ferner sind auch die in der Formulierungstechnik gebräuchlichen Tenside, die u.a. in "Mc Cutcheon's Detergents and Emulsifiers Annual" MC Publishing Corp., Ridgewood New Jersey, 1981, Stache, H., "Tensid-Taschenbuch", Carl Hanser Verlag, München/Wien, 1981 und M. und J. Ash, "Encyclopedia of Surfactants", Vol I-III, Chemical Publishing Co., New York, 1980-81 beschrieben sind, zur Herstellung der erfindungsgemäßen herbiziden Mittel geeignet.

Erfindungsgemäße herbizide und den Pflanzenwuchs hemmende Mittel mit einem herbizid wirksamen Gehalt an Verbindung der Formel I können durch Zugabe von Sprühtank-Adjuvantien in ihrer Wirksamkeit gesteigert werden.
Diese Adjuvantien können beispielsweise sein: nichtionische Tenside, Mischungen von nichtionischen Tensiden, Mischungen von anionischen Tensiden mit nichtionischen Tensiden, kationische Tenside, silizium-organische Tenside, Mineralölderivate mit und ohne Tenside , Pflanzenölderivate mit und ohne Tensidzusatz, alkylierte Derivate von Ölen pflanzlichen oder mineralischen Ursprungs mit und ohne Tenside, Fischöle und andere tierische Öle tierischer Natur sowie deren Alkylderivate mit und ohne Tenside, natürlich vorkommende höhere Fettsäuren, vorzugsweise mit 8 bis 28 Kohlenstoffatomen, und deren Alkylesterderivate, organische Säuren enthaltend ein aromatisches Ringsystem und einen oder mehrere Carbonsäurerester, sowie deren Alkylderivaten, ferner Suspensionen von Polymeren des Vinylacetats oder Copolymeren von Vinylacetat-Acrylsäureestern. Mischungen einzelner Adjuvantien untereinander sowie in Kombination mit organischen Lösungsmitteln können zu einer weiteren Steigerung der Wirkung führen.

Als nichtionische Tenside kommen beispielsweise Polyglykoletherderivate von aliphatischen oder cycloaliphatischen Alkoholen, gesättigten oder ungesättigten Fettsäuren und Alkylphenolen in Frage, vorzugsweise die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, vorzugsweise 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit vorzugsweise 1 bis 10 Kohlenstoffatomen in der Alkylkette. Die genannten Verbindungen enthalten üblicherweise pro Propylenglykol-Einheit 1 bis 5 Ethylenglykoleinheiten.

Als weitere Beispiele nichtionischer Tenside seien auch Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei anionischen Tensiden werden vor allem Alkylsulfate, Alkylsulfonate, Alkylarylsulfonate, alkylierte Phosphorsäuren bevorzugt sowie deren ethoxylierte Derivate. Die Alkylreste enthalten üblicherweise 8 bis 24 Kohlenstoffatome.

Bevorzugte nicht-ionische Tenside sind unter den folgenden Handelsnamen bekannt:

Polyoxyethylen Cocoalkylamin (z.B. AMIET® 105 (Kao Co.)), Polyoxyethylen Oleylamin (z.B. AMIET® 415 (Kao Co.)), Nonylphenolpolyethoxyethanole, Polyoxyethylen Stearylamin (z.B. AMIET® 320 (Kao Co.)), N-polyethoxyethylamines (z.B. GENAMIN® (Hoechst AG)), N,N,N',N'-Tetra(Polyethoxypolypropoxyethyl)ethylen-diamine (z.B. TERRONIL® und TETRONIC® (BASF Wyandotte Corp.)), BRIJ® (Atlas Chemicals), ETHYLAN® CD und ETHYLAN® D (Diamond Shamrock), GENAPOL® C, GENAPOL® O, GENAPOL® S und GENAPOL® X080 (Hoechst AG), EMULGEN® 104P, EMULGEN® 109P und EMULGEN® 408 (Kao Co.); DISTY® 125 (Geronazzo), SOPROPHOR® CY 18 (Rhone Poulenc S.A.); NONISOL® (Ciba-Geigy), MRYJ® (ICI); TWEEN® (ICI); EMULSOGEN® (Hoechst AG); AMIDOX® (Stephan Chemical Co.), ETHOMID® (Armak Co.); PLURONIC® (BASF Wyandotte Corp.), SOPROPHOR® 461 P (Rhône Poulenc S.A.), SOPROPHOR® 496/P (Rhone Poulenc S.A.), ANTAROX FM-63 (Rhone Poulenc S.A.), SLYGARD 309 (Dow Coming), SILWET 408, SILWET L-7607N (Osi-Specialities).

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die verwendeten Öle sind entweder von mineralischer oder natürlicher Herkunft. Die natürlichen Öle können zudemnoch von tierischem oder pflanzlichen Ursprung sein. Bei tierischen Ölen werden vor allem Derivate von Rindertalg bevorzugt, aber auch Fischöle (z.B. Sardinenöl) und deren Derivate werden verwendet. Pflanzliche Öle sind meist Saatöle verschiedener Herkunft. Als Beispiele für besonders verwendete Pflanzenöle können Kokos-, Raps- oder Sonnenblumenöle sowie deren Derivate genannt werden.

In dem erfindungsgemäßen Mittel betragen die Aufwandmengen an Öladditiv in der Regel zwischen 0,01 und 2 % in Bezug auf die Spritzbrühe. Beispielsweise kann das Öladditv nach Herstellung der Spritzbrühe in der gewünschten Konzentration in den Sprühtank gegeben werden.

Im erfindungsgemäßen Mittel bevorzugte Öladditive enthalten ein Öl pflanzlichen Ursprungs wie beispielsweise Rapsöl oder Sonnenblumenöl, Alkylester von Ölen pflanzlichen Ursprungs wie beispielsweise die Methylderivate, oder Mineralöle.

Besonders bevorzugte Öladditive enthalten Alkylester von höheren Fettsäuren (C₈-C₂₂). insbesondere die Methylderivate von C₁₂-C₁₈ Fettsäuren, beispielsweise die Methylester der Laurinsäure, Palmitinsäure und Ölsäure. Diese Ester sind bekannt als Methyllaurat (CAS-111-82-0), Methylpalmitat (CAS-112-39-0) und Methyloleat (CAS-112-62-9).

Das Ausbringen und die Wirkung der Öladditive kann durch deren Kombination mit oberflächenaktiven Substanzen wie nichtionische-, anionische oder kationische Tenside verbessert werden. Beispiele für geeignete anionische, nichtionische und kationische Tenside sind in der WO 97/34485 auf den Seiten 7 und 8 aufgezählt.

Bevorzugte oberflächenaktive Substanzen sind anionische Tenside vom Typ der Dodecylbenzylsulfonate, insbesondere die Calciumsalze davon sowie nichtionische Tenside vom Typ der Fettalkoholethoxylate. Insbeondere bevorzugt sind ethoxylierte C₁₂-C₂₂-Fettalkohole mit einem Ethoxylierungsgrad zwischen 5 und 40. Beispiele für kommerziell erhältliche, bevorzugte Tenside sind die Genapol Typen (Clariant AG, Muttenz, Schweiz). Die Konzentration der oberflächenaktiven Substanzen in Bezug auf das gesamte Additiv beträgt im allgemeinen zwischen 1 und 30 Gew.%.

Beispiele für Öladditive, die aus Mischungen von Ölen bzw. Mineralölen oder deren Derivaten mit Tensiden bestehen, sind Edenor ME SU®, Emery 2231 ® (Henkel Tochtergesellschaft Cognis GMBH, DE), Turbocharge® (Zeneca Agro, Stoney Creek, Ontario , CA) oder, besonders bevorzugt, Actipron® (BP Oil UK Limited, GB).

Ferner kann die Zugabe eines organischen Lösungsmittels zu dem Öladditiv/Tensidgemisch eine weitere Steigerung der Wirkung bewirken. Geeignete Lösungsmittel sind beispielsweise Solvesso® (ESSO) oder Aromatic Solvent® (Exxon Corporation) Typen.

Die Konzentration derartiger Lösungsmittel kann von 10 bis 80 Gew.% des Gesamtgewichtes betragen.

Derartige Öladditive, die beispielsweise auch in US-A-4,834,908 beschrieben sind, sind für das erfindungsgemäße Mittel besonders bevorzugt. Ein ganz besonders bevorzugtes Ötadditiv ist unter dem Namen MERGE® bekannt, kann von der BASF Corporation bezogen werden und ist beispielsweise in US-A-4,834,908 in col. 5, als Example COC-1 im wesentlichen beschrieben. Ein weiteres erfindungsgemäß bevorzugtes Öladditiv ist SCORE® (Novartis Crop Protection Canada.)

In der Formulier- und Adjuvanttechnik gebräuchliche Tenside, Öle, insbesondere Pflanzenöle, Derivate davon wie alkylierte Fettsäuren und Mischungen davon, z.B. mit vorzugsweise anionischen Tensiden wie alkylierten Phosphorsäuren, Alkylsulfate und Alkylarylsulfonaten sowie höheren Fettsäuren, die auch in den erfindungsgemäßen Mitteln und Sprühtanklösungen davon verwendet werden können, sind u.a. in "Mc Cutcheon's Detergents and Emulsifiers Annual" MC Publishing Corp., Ridgewood New Jersey, 1998, Stache, H., "Tensid-Taschenbuch", Carl Hanser Verlag, München/Wien, 1990, M. und J. Ash, "Encyclopedia of Surfactants", Vol I-IV, Chemical Publishing Co., New York, 1981-89, G. Kapusta, "A Compendium of Herbicide Adjuvants", Southem Illinois Univ. , 1998, L. Thomson Harvey, "A Guide to Agricultural Spray Adjuvants Used in the United States", Thomson Pubns., 1992 beschrieben.

Die herbiziden Formulierungen enthalten in der Regel 0,1 bis 99 Gew%, insbesondere 0,1 bis 95 Gew.-% Herbizid, 1 bis 99,9 Gew.-%, insbesondere 5 bis 99,8 Gew.-%, eines festen oder flüssigen Formulierungshilfsstoffes und 0 bis 25 Gew.-%, insbesondere 0,1 bis 25 Gew.-%, eines Tensides. Während als Handelsware eher konzentrierte Mittel bevorzugt werden, verwendet der Endverbraucher in der Regel verdünnte Mittel. Die Mittel können auch weitere Zusätze wie Stabilisatoren z.B. gegebenenfalls epoxydierte Pflanzenöle (epoxydiertes Kokosnußöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe enthalten.

Die Wirkstoffe der Formel I werden in der Regel auf die Pflanze oder deren Lebensraum mit Aufwandmengen von 0,001 bis 4 kg/ha, insbesondere 0,005 bis 2 kg/ha eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Die Verbindungen der Formel I zeichnen sich durch herbizide und wuchshemmende Eigenschaften aus, die sie zum Einsatz in Kulturen von Nutzpflanzen, insbesondere in Getreide, Baumwolle, Soja, Zuckerrüben, Zuckerrohr, Plantagen, Raps, Mais und Reis sowie zur nicht-selektiven Unkrautkontrolle befähigen. Unter Kulturen sind auch solche zu verstehen, die durch konventionelle züchterische oder gentechnologische Methoden gegen Herbizide bzw. Herbizidklassen tolerant gemacht worden sind. Diese sind z.B. IMI Maize, Poast Protected Maize (Sethoxydim-Toleranz), Liberty Link Maize, B.t./Liberty Link Maize, IMI/Liberty Link Maize, IMI/Liberty Link /B.t. Maize, Roundup Ready Maize und Roundup Ready/B.t. Maize.

Bei den zu bekämpfenden Unkräutern kann es sich sowohl um mono- als auch um dikotyle Unkräuter handeln, wie zum Beispiel Stellaria, Nasturtium, Agrostis, Digitaria, Avena, Setaria, Sinapis, Lolium, Solanum, Echinochloa, Scirpus, Monochoria, Sagittaria, Bromus, Alopecurus, Sorghum halepense, Rottboellia, Cyperus, Abutilon, Sida, Xanthium, Amaranthus, Chenopodium, Ipomoea, Chrysanthemum, Galium, Viola und Veronica.

Es hat sich überraschenderweise gezeigt, daß spezielle, aus US-A-5,041,157, US-A-5,541,148, US-A-5,006,656, EP-A-0 094 349, EP-A-0 551 650, EP-A-0 268 554, EP-A-0 375 061, EP-A-0 174 562, EP-A-492 366, WO 91/7874, WO 94/987, DE-A-19612943 , WO 96/29870, WO 98/13361, WO 98/39297, WO 98/27049, EP-A- 0 716 073, EP-A- 0 613 618, US-A-5,597,776 EP-A-0430 004, DE-A-4 331 448, WO 99/16744, WO 00/30447 und WO 00/00020 bekannte Safener zur Mischung mit dem erfindungsgemäßen herbiziden Mittel geeignet sind. Daher betrifft die vorliegende Erfindung auch ein selektiv-herbizides Mittel zur Bekämpfung von Gräsern und Unkräutern in Kulturen von Nutzpflanzen, insbesondere in Kulturen von Mais und Getreide, welches ein Herbizid der Formel I und einen Safener (Gegenmittel, Antidot) enthält und welches die Nutzpflanzen, nicht aber die Unkräuter vor der phytotoxischen Wirkung des Herbizides bewahrt, sowie die Verwendung dieses Mittels zur Unkrautbekämpfung in Nutzpflanzenkulturen.

Erfindungsgemäß wird somit ein selektiv-herbizides Mittel vorgeschlagen, welches dadurch gekennzeichnet ist, daß es neben üblichen inerten Formulierungshilfsmitteln wie Trägerstoffen, Lösungsmitteln und Netzmitteln als Wirkstoff eine Mischung aus
a) einer herbizid-wirksamen Menge einer Verbindung der Formel I 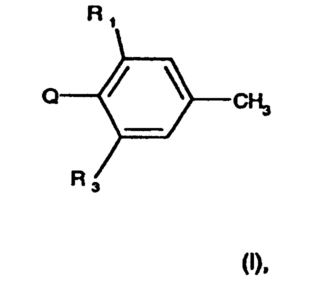 worin R₁, R₃ und Q die oben angegebenen Bedeutungen haben, mit der Maßgabe, daß Q verschieden von Q₁ ist; und
b) einer herbizid-antagonistisch wirksamen Menge entweder eine Verbindung der Formel X 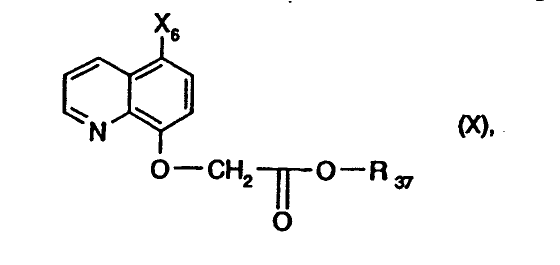
worin
R₃₇ Wasserstoff, C₁-C₈-Alkyl oder durch C₁-C₆-Alkoxy oder C₃-C₆-Alkenyloxy substituiertes C₁-C₈-Alkyl; und X₆ Wasserstoff oder Chlor bedeutet; oder einer Verbindung der Formel XI 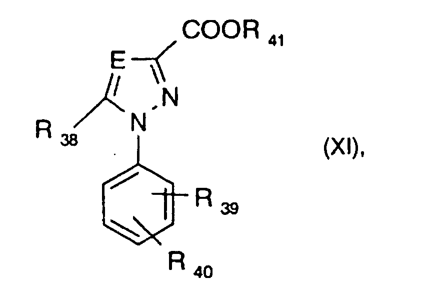 worin E Stickstoff oder Methin;
R₃₈ -CCl₃, Phenyl oder durch Halogen substituiertes Phenyl;
R₃₉ und R₄₀ unabhängig voneinander Wasserstoff oder Halogen; und
R₄₁ C₁-C₄-Alkyl bedeuten; oder einer Verbindung der Formel XII 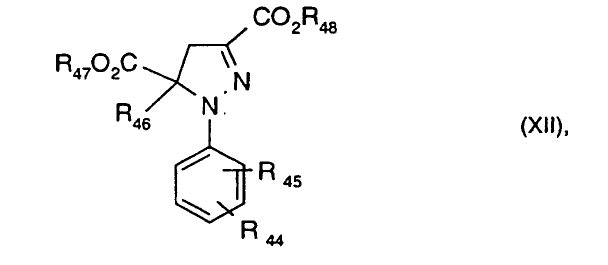 worin R₄₄ und R₄₅ unabhängig voneinander Wasserstoff oder Halogen und
R₄₆, R₄₇ und R₄₈ unabhängig voneinander C₁-C₄-Alkyl bedeuten, oder einer Verbindung der Formel XIII 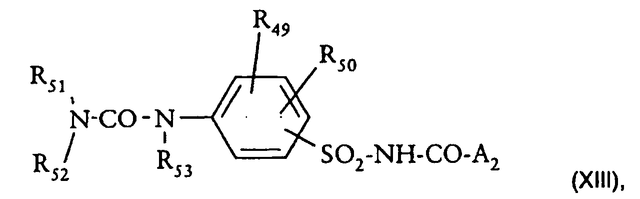 worin A₂ für eine Gruppe 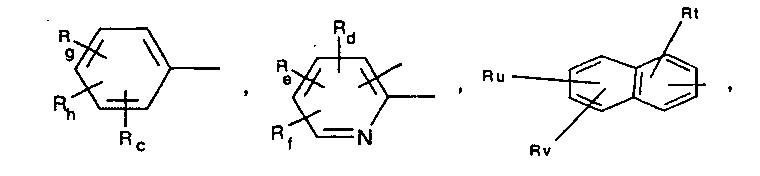 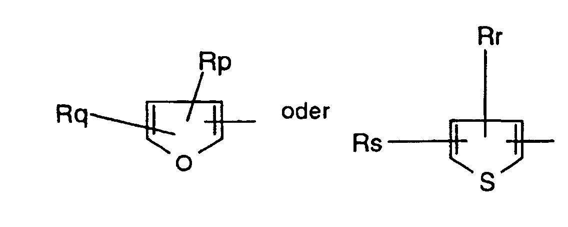 steht,
R₅₁ und R₅₂ unabhängig voneinander Wasserstoff, C₁-C₈-Alkyl, C₃-C₈-Cycloalkyl, C₃-C₆-Alkenyl, C₃-C₆-Alkinyl, 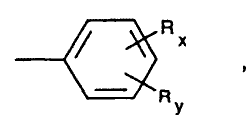 oder durch C₁-C₄-Alkoxy oder 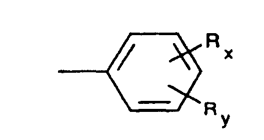 substituiertes C₁-C₄-Alkyl bedeutet; oder R₅₁ und R₅₂ bilden zusammen eine C₄-C₆-Alkylenbrücke, die durch Sauerstoff, Schwefel, SO, SO₂, NH oder -N(C₁-C₄-Alkyl)- unterbrochen sein kann,
R₅₃ für Wasserstoff oder C₁-C₄-Alkyl;
R₄₉ für Wasserstoff, Halogen, Cyano, Trifluoromethyl, Nitro, C₁-C₄-Alkyl, C₁-C₄-Alkoxy, C₁-C₄-Alkylthio, C₁-C₄-Alkylsulfinyl, C₁-C₄-Alkylsulfonyl, -COORⱼ, -CONRₖRₘ, -CORₙ, -SO₂NRₖRₘ oder -OSO₂-C₁-C₄-Alkyl;
R_{g} für Wasserstoff, Halogen, Cyano, Nitro, C₁-C₄-Alkyl, C₁-C₄-Hatogenalkyl, C₁-C₄-Alkylthio, C₁-C₄-Alkylsulfinyl, C₁-C₄-Alkylsulfonyl, -COORⱼ, -CONRₖRₘ, -CORₙ, -SO₂NRₖRₘ, -OSO₂-C₁-C₄-Alkyl, C₁-C₆-Alkoxy, oder C₁-C₆alkoxy substituiert durch C₁-C₄-Alkoxy oder Halogen, C₃-C₆-Alkenyloxy, oder C₃-C₆-Alkenyloxy substituiert durch Halogen, oder C₃-C₆-Alkinyloxy, oder R₄₉ und R₅₀ zusammen bilden eine C₃-C₄-Alkylenbrücke, die durch Halogen oder C₁-C₄-Alkyl substituiert sein kann, oder bilden eine C₃-C₄-Alkenylenbrücke, die durch Halogen oder C₁-C₄-Alkyl substituiert sein kann, oder bilden eine C₄-Alkadienylenbrücke, die durch Halogen oder C₁-C₄-Alkyl substituiert sein kann;
R₅₀ und Rₕ unabhängig voneinander für Wasserstoff, Halogen, C₁-C₄-Alkyl, Trifluormethyl, C₁-C₆-Alkoxy, C₁-C₆-Alkylthio oder -COORⱼ;
R_{c} für Wasserstoff, Halogen, Nitro, C₁-C₄-Alkyl oder Methoxy; R_{d} für Wasserstoff, Halogen, Nitro, C₁-C₄-Alkyl, C₁-C₄-Alkoxy, C₁-C₄Alkylthio, C₁-C₄-Alkylsulfinyl, C₁-C₄-Alkylsulfonyl, -COORⱼ oder CONRₖRₘ;
Rₑ für Wasserstoff, Halogen, C₁-C₄-Alkyl, -COORⱼ, Trifluormethyl or Methoxy, oder R_{d} und Rₑ bilden zusammen eine C₃-C₄-Alkylenbrücke;
Rp für Wasserstoff, Halogen, C₁-C₄-Alkyl, -COORⱼ, Trifluormethyl or Methoxy; Rq für Wasserstoff, Halogen, Nitro, C₁-C₄-Alkyl, C₁-C₄-Alkoxy, C₁-C₄Alkylthio, C₁-C₄-Alkylsulfinyl, C₁-C₄-Alkylsulfonyl, -COORⱼ oder CONRₖRₘ; , oder Rp und Rq bilden zusammen eine C₃-C₄-Alkylenbrücke;
Rr für Wasserstoff, Halogen, C₁-C₄-Alkyl, -COORⱼ, Trifluormethyl or Methoxy; Rs für Wasserstoff, Halogen, Nitro, C₁-C₄-Alkyl, C₁-C₄-Alkoxy, C₁-C₄Alkylthio, C₁-C₄-Alkylsulfinyl, C₁-C₄-Alkylsulfonyl, -COORⱼ oder CONRₖRₘ; , oder Rr und Rs bilden zusammen eine C₃-C₄-Alkylenbrücke;
Rt für Wasserstoff, Halogen, C₁-C₄-Alkyl, -COORⱼ, Trifluormethyl or Methoxy; Ru für Wasserstoff, Halogen, Nitro, C₁-C₄-Alkyl, C₁-C₄-Alkoxy, C₁-C₄Alkylthio, C₁-C₄-Alkylsulfinyl, C₁-C₄-Alkylsulfonyl, -COORⱼ oder CONRₖRₘ; , oder Rv und Ru bilden zusammen eine C₃-C₄-Alkylenbrücke;
R_{f} und Rv für Wasserstoff, Halogen oder C₁-C₄-Alkyl;
Rₓ und R_{y} unabhängig voneinander für Wasserstoff, Halogen, C₁-C₄-Alkyl, C₁-C₄-Alkoxy, C₁-C₄-Alkylthio, -COOR₅₄, Trifluoromethyl, Nitro oder Cyano;
Rⱼ, Rₖ und Rₘ unabhängig voneinander für Wasserstoff oder C₁-C₄-Alkyl; oder
Rₖ und Rₘ bilden zusammen eine C₄-C₆-Alkylenbrücke, die durch Sauerstoff, NH oder -N(C₁-C₄-Alkyl)- unterbrochen sein kann;
Rₙ für C₁-C₄-Alkyl, Phenyl, oder durch Halogen, C₁-C₄-Alkyl, Methoxy, Nitro oder Trifluormethyl substituiertes Phenyl;
R₅₄ für Wasserstoff, C₁-C₁₀-Alkyl, C₁-C₄-Alkoxy-C₁-C₄-alkyl, C₁-C₄-Alkylthio-C₁-C₄-alkyl, Di-C₁-C₄-alkylamino-C₁-C₄-alkyl, Halogen-C₁-C₈-alkyl, C₂-C₈-Alkenyl, Halogen-C₂-C₈-alkenyl, C₃-C₈-Alkinyl, C₃-C₇-Cycloalkyl, Halogen-C₃-C₇-cycloalkyl, C₁-C₈-Alkylcarbonyl, Allylcarbonyl, C₃-C₇-Cycloalkylcarbonyl, Benzoyl, das unsubstituiert oder am Phenylring gleich oder verschieden bis zu dreifach durch Halogen, C₁-C₄-Alkyl, Halogen-C₁-C₄-alkyl, Halogen-C₁-C₄-alkoxy oder C₁-C₄-Alkoxy substituiert ist; oder Furoyl, Thienyl; oder C₁-C₄-Alkyl substituiert durch Phenyl, Halogenphenyl, C₁-C₄-Alkylphenyl, C₁-C₄-Alkoxyphenyl, Halogen-C₁-C₄-alkylphenyl, Halogen-C₁-C₄-alkoxyphenyl, C₁-C₆-Alkoxycarbonyl, C₁-C₄-Alkoxy-C₁-C₈--alkoxycarbonyl, C₃-C₈-Alkenyloxycarbonyl, C₃-C₈-Alkinyloxycarbonyl, C₁-C₈--Alkylthiocarbonyl, C₃-C₈-Alkenylthiocarbonyl, C₃-C₈-Alkinylthiocarbonyl, Carbamoyl, Mono--C₁-C₄-alkylaminocarbonyl, Di-C₁-C₄-alkylaminocarbonyl; oder Phenylaminocarbonyl, das unsubstituiert oder am Phenyl gleich oder verschieden bis zu dreifach durch Halogen, C₁-C₄-Alkyl, Halogen-C₁-C₄-alkyl, Halogen-C₁-C₄-alkoxy oder C₁-C₄-Alkoxy oder einfach durch Cyano oder Nitro substituiert ist, oder Dioxolan-2-yl, das unsubstituiert ist oder durch ein oder zwei C₁-C₄-Alkylreste substituiert ist, oder Dioxan-2-yl, das unsubstituiert ist oder durch ein oder zwei C₁-C₄-Alkylreste substituiert ist, oder C₁-C₄-Alkyl, das durch Cyano, Nitro, Carboxyl oder C₁-C₈-Alkylthio-C₁-C₈-alkoxycarbonyl substituiert ist, bedeutet;
oder einer Verbindung der Formel XIV 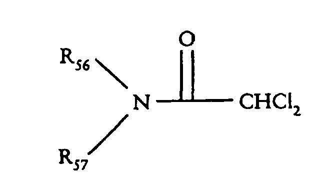 (XIV), worin R₅₆ und R₅₇ unabhängig voneinander für C₁-C₆-Alkyl oder C₂-C₆-Alkenyl; oder R₅₆ und R₅₇ zusammen für 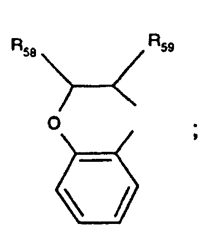 R₅₈ und R₅₉ unabhängig voneinander für Wasserstoff oder C₁-C₆-Alkyl; oder R₅₆ und R₅₇ zusammen für 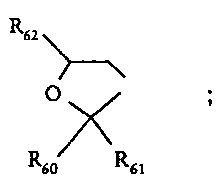 R₆₀ und R₆₁ unabhängig voneinander für C₁-C₄-Alkyl, oder R₆₀ und R₆₁ zusammen -(CH₂)₅- ;
R₆₂ für Wasserstoff, C₁-C₄-Alkyl oder 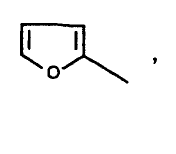 oder R₅₆ und R₅₇ zusammen für 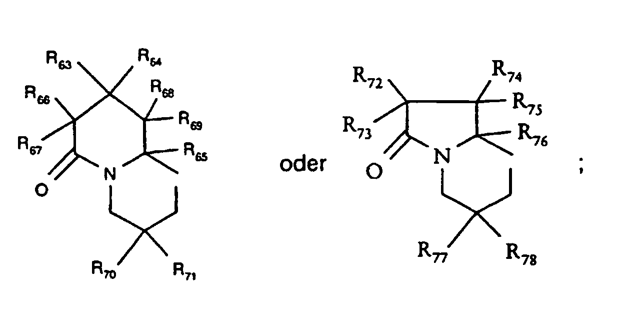 R₆₃, R₆₄, R₆₅, R₆₆, R₆₇, R₆₈, R₆₉, R₇₀, R₇₁, R₇₂, R₇₃, R₇₄, R₇₅, R₇₆, R₇₇ und R₇₈ unabhängig voneinander für Wasserstoff oder C₁-C₄-Alkyl stehen;
oder einer Verbindung der Formel XV 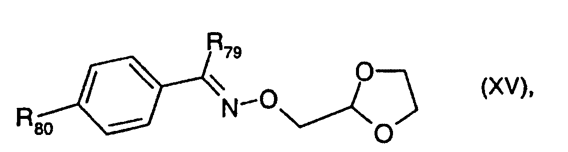 worin R₈₀ Wasserstoff oder Chlor und R₇₉ Cyano oder Trifluormethyl bedeutet,
oder eine Verbindung der Formel XVI 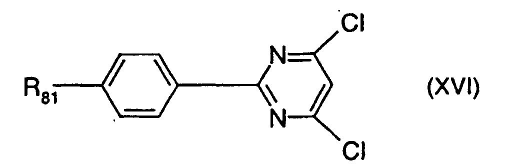 worin R₈₁ Wasserstoff oder Methyl bedeutet,
oder einer Verbindung der Formel XVII 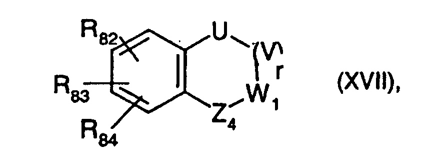 worin
R₈₂ Wasserstoff, C₁-C₄-Alkyl oder C₁-C₄-Alkyl substituiert durch C₁-C₄-Alkyl-X₂- oder C₁-C₄-Halogenalkyl-X₂- bedeutet, oder für C₁-C₄-Halogenalkyl, Nitro, Cyano, -COOR₈₅, -NR₈₆R₈₇, -SO₂NR₈₈R₈₉ oder -CONR₉₀R₉₁ steht;
R₈₃ Wasserstoff, Halogen, C₁-C₄-Alkyl, Trifluormethyl, C₁-C₄-Alkoxy oder C₁-C₄-Halogenalkoxy bedeutet;
R₈₄ Wasserstoff, Halogen oder C₁-C₄-Alkyl bedeutet;
U, V, W₁ und Z₄ unabhängig voneinander Sauerstoff, Schwefel, C(R₉₂)R₉₃, Carbonyl, NR₉₄, eine Gruppe 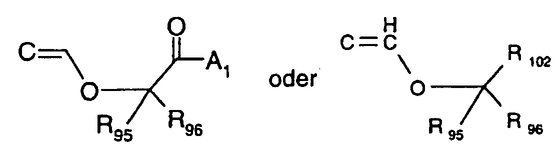 bedeuten, worin R₁₀₂ C₂-C₄-Alkenyl oder C₂-C₄-Alkinyl bedeutet; mit den Maßgaben, daß
a) mindestens eines der Ringglieder U, V, W₁ oder Z₄ Carbonyl ist, und ein zu diesem bzw. diesen Ringgliedern benachbartes Ringglied die Gruppe 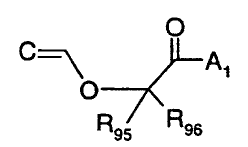 oder 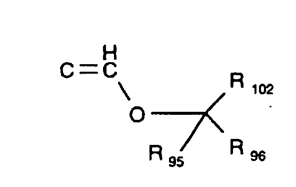 bedeutet, wobei diese Gruppe nur einmal vorkommt; und
b) zwei benachbarte Ringglieder U und V, V und W₁ und W₁ und Z₄ nicht gleichzeitig Sauerstoff bedeuten können;
   R₉₅ und R₉₆ unabhängig voneinander Wasserstoff oder C₁-C₈-Alkyl bedeuten; oder
   R₉₅ und R₉₆ zusammen eine C₂-C₆-Alkylengruppe bilden;
   A₁ R₉₉-Y₁- oder -NR₉₇R₉₈;
   X₂ Sauerstoff oder -S(O)ₛ ;
   Y₁ Sauerstoff oder Schwefel bedeuten;
   R₉₉ Wasserstoff, C₁-C₈-Alkyl, C₁-C₈-Halogenalkyl, C₁-C₄-Alkoxy-C₁-C₈-alkyl, C₃-C₆-Alkenyloxy-C₁-C₈-alkyl oder Phenyl-C₁-C₈-alkyl bedeutet, wobei der Phenylring durch Halogen, C₁-C₄-Alkyl, Trifluormethyl, Methoxy oder Methyl-S(O)ₛ- substituiert sein kann, oder C₃-C₆-Alkenyl, C₃-C₆-Halogenalkenyl, Phenyl-C₃-C₆-alkenyl, C₃-C₆-Alkinyl, Phenyl-C₃-C₆-alkinyl, Oxetanyl, Furyl oder Tetrahydrofuryl bedeutet;
   R₈₅ Wasserstoff oder C₁-C₄-Alkyl;
   R₈₆ Wasserstoff, C₁-C₄-Alkyl oder C₁-C₄-Alkylcarbonyl bedeuten;
   R₈₇ Wasserstoff oder C₁-C₄-Alkyl bedeutet; oder
   R₈₆ und R₈₇ zusammen eine C₄- oder C₅-Alkylengruppe bilden;
   R₈₈, R₈₉, R₉₀ und R₉₁ unabhängig voneinander Wasserstoff oder C₁-C₄-Alkyl bedeuten; oder R₈₈ zusammen mit R₈₉ oder R₉₀ zusammen mit R₉₁ unabhängig voneinander C₄- oder C₅-Alkylen sind, wobei ein Kohlenstoffatom durch Sauerstoff oder Schwefel, oder ein oder zwei Kohlenstoffatome durch -NR₁₀₀- ersetzt sein können;
   R₉₂, R₁₀₀ und R₉₃ unabhängig voneinander Wasserstoff oder C₁-C₈-Alkyl sind; oder
   R₉₂ und R₉₃ zusammen C₂-C₆-Alkylen sind;
   R₉₄ Wasserstoff oder C₁-C₈-Alkyl bedeutet;
   R₉₇ Wasserstoff, C₁-C₈-Alkyl, Phenyl oder Phenyl-C₁-C₈-alkyl bedeutet, wobei die Phenylringe durch Fluor, Chlor, Brom, Nitro, Cyano, -OCH₃, C₁-C₄-Alkyl oder CH₃SO₂-substituiert sein können, oder für C₁-C₄-Alkoxy-C₁-C₈-alkyl, C₃-C₆-Alkenyl oder C₃-C₆-Alkinyl;
   R₉₈ Wasserstoff, C₁-C₈-Alkyl, C₃-C₆-Alkenyl oder C₃-C₆-Alkinyl steht; oder
   R₉₇ und R₉₈ zusammen C₄- oder C₅-Alkylen bedeuten, wobei ein Kohlenstoffatom durch Sauerstoff oder Schwefel, oder ein oder zwei Kohlenstoffatome durch
   -NR₁₀₁- ersetzt sein können;
   R₁₀₁ Wasserstoff oder C₁-C₄-Alkyl bedeutet;
   r 0 oder 1 ist; und
   s 0, 1 oder 2 bedeutet,
   oder eine Verbindung der Formel XVIII 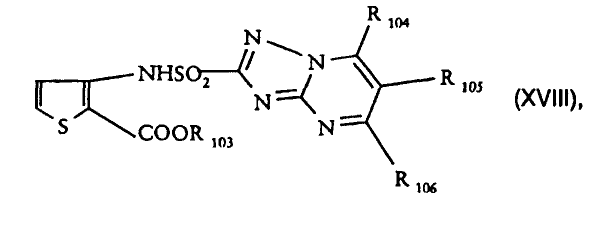 worin R₁₀₃ Wasserstoff, C₁-C₆-Alkyl, C₃-C₆-Cycloalkyl, C₃-C₆-Alkenyl oder C₃-C₆-Alkinyl; und R₁₀₄, R₁₀₅ und R₁₀₆ unabhängig voneinander Wasserstoff, C₁-C₆-Alkyl, C₃-C₆-Cycloalkyl oder C₁-C₆-Alkoxy bedeuten, mit der Maßgabe, daß einer der Substituenten R₁₀₄, R₁₀₅ und R₁₀₆ verschieden von Wasserstoff ist;
   oder eine Verbindung der Formel XIX 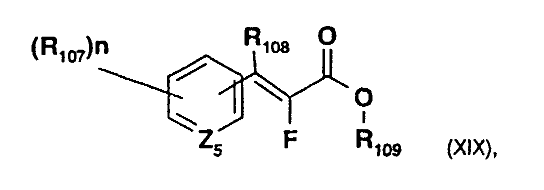 worin Z₅ N oder CH, n für den Fall, daß Z₅ gleich N ist, 0, 1, 2 oder 3 und für den Fall, daß Z₅ CH ist, 0, 1, 2, 3 oder 4 bedeutet, R₁₀₇ Halogen, C₁-C₄-Alkyl, C₁-C₄-Halogenalkyl, C₁-C₄-Alkoxy, C₁-C₄-Halogenalkoxy, Nitro, C₁-C₄-Alkylthio, C₁-C₄-Alkylsulfonyl, C₁-C₄-Alkoxycarbonyl, Phenyl oder Phenoxy, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Phenyl oder Phenoxy bedeutet;
   R₁₀₈ Wasserstoff oder C₁-C₄-Alkyl bedeutet, R₁₀₉ Wasserstoff, C₁-C₄-Alkyl, C₃-C₆-Cycloalkyl, C₂-C₆-Alkenyl, C₂-C₆-Alkinyl, C₁-C₄-Halogenalkyl, C₂-C₆-Halogenalkenyl, C₂-C₆-Halogenalkinyl, C₁-C₄-Alkylthio-C₁-C₄-alkyl, C₁-C₄-Alkylsulfonyl- C₁-C₄-alkyl, C₁-C₄-Alkoxy-C₁-C₄-alkyl, C₁-C₄-Alkenyloxy- C₁-C₄-alkyl oder C₁-C₄-Alkinyloxy-C₁-C₄-alkyl ist;
   oder eine Verbindung der Formel XX 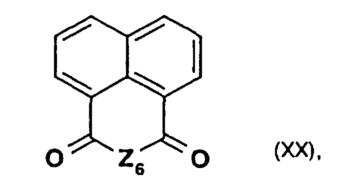 worin Z₆ Sauerstoff oder N-R₁₁₀ und R₁₁₀ eine Gruppe der Formel 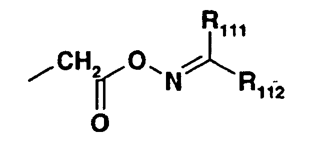 bedeutet, worin R₁₁₁ und R₁₁₂ unabhängig voneinander Cyano, Wasserstoff, C₁-C₄-Alkyl, C₃-C₆-Cycloalkyl, C₂-C₆-Alkenyl, Aryl, Phenyl oder Heteroaryl, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Phenyl, Aryl oder Heteroaryl bedeuten;
   oder eine Verbindung der Formel XXI 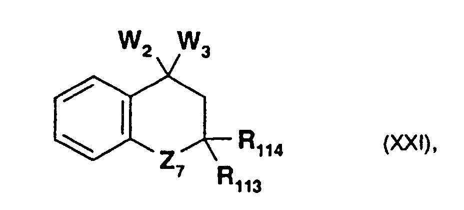 worin Z₇ Sauerstoff, Schwefel, S=O, SO₂ oder CH₂ bedeutet, R₁₁₃ und R₁₁₄ unabhängig voneinander Wasserstoff, Halogen oder C₁-C₄-Alkyl bedeuten, W₂ und W₃ unabhängig voneinander CH₂COOR₁₁₅ oder COOR₀₁₁₅ oder zusammen eine Gruppe der Formel -(CH₂)C(O)-O-C(O)-(CH₂)- bedeuten, und R₁₁₅ und R₀₁₁₅ unabhängig voneinander Wasserstoff, C₁-C₄-Alkyl, C₂-C₄-Alkenyl, C₂-C₆-Alkinyl, C₃-C₆-Cycloalkyl, C₁-C₄-Halogenalkyl, ein Metall- oder ein Ammonium- Kation bedeuten;
   oder eine Verbindung der Formel XXII 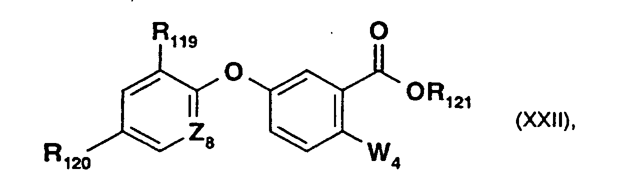 worin R₁₁₉ und R₁₂₀ unabhängig voneinander Wasserstoff, Halogen oder C₁-C₄-Halogenalkyl sind, R₁₂₁ Wasserstoff, C₁-C₄-Alkyl, C₃-C₄-Alkenyl, C₃-C₄-Alkinyl, C₁-C₄-Halogenalkyl, C₃-C₆-Cycloalkyl, ein Metalkation oder ein Ammoniumkation bedeuten, Z₈ N, CH, C-F oder C-Cl bedeuten und W₄ eine Gruppe der Formel 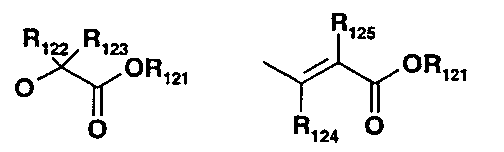 bedeutet, worin R₁₂₂ und R₁₂₃ unabhängig voneinander Wasserstoff oder C₁-C₄-Alkyl und R₁₂₄ und R₁₂₅ unabhängig voneinander Wasserstoff oder C₁-C₄-Alkyl bedeuten;
   oder eine Verbindung der Formel XXIII 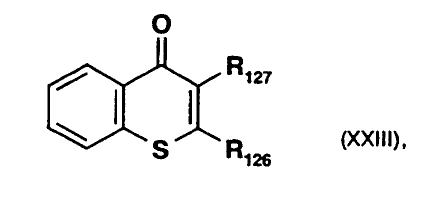 worin R₁₂₆ Wasserstoff, Cyano, Halogen, C₁-C₄-Alkyl, C₃-C₆-Cycloalkyl, C₁-C₄-Alkoxy, C₁-C₄-Alkoxycarbonyl, C₁-C₄-Alkylthiocarbonyl, -NH-R_{128,} -C(O)NH-R₀₁₂₈, Aryl oder Heteroaryl, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Aryl oder Heteroaryl bedeutet;
   R₁₂₇ Wasserstoff, Cyano, Nitro, Halogen, C₁-C₄-Alkyl, C₁-C₄-Halogenalkyl, C₁-C₄-Alkoxy, C₁-C₄-Thioalkyl bedeuten, und
   R₁₂₈ und R₀₁₂₈ unabhängig voneinander C₁-C₄-Alkyl, C₁-C₄-Halogenalkyl, C₃-C₄-Alkenyl, C₃-C₄-Alkinyl, C₃-C₄-Cycloalkyl, Aryl oder Heteroaryl, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Aryl oder Heteroaryl, Formyl, C₁-C₄-Alkylcarbonyl, C₁-C₄-Alkylsufonyl bedeuten;
   oder eine Verbindung der Formel XXIV 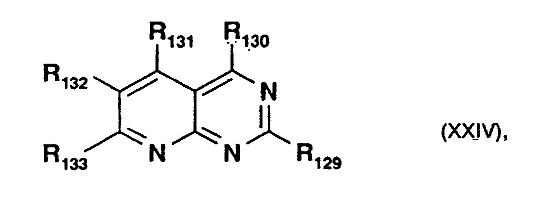 worin R₁₂₉ und R₁₃₀ unabhängig voneinander Wasserstoff, C₁-C₄-Alkyl, C₁-C₄-Halogenalkyl, C₁-C₄-Alkoxy, Mono-C₁-C₈- oder Di-C₁-C₈-Alkylamino, C₃-C₆-Cycloalkyl, C₁-C₄-Thioalkyl, Phenyl oder Heteroaryl sind, R₁₃₁ die Bedeutung von R₁₂₉ hat und zusätzlich OH, NH₂, Halogen, Di- C₁-C₄-Aminoalkyl, C₁-C₄-Alkylthio, C₁-C₄-Alkylsulfonyl oder C₁-C₄-Alkoxycarbonyl ist, R₁₃₂ die Bedeutung von R₁₂₉ hat und zusätzlich Cyano, Nitro, Carboxyl, C₁-C₄-Alkoxycarbonyl, Di- C₁-C₄-Aminoalkyl, C₁-C₄-Alkylthio, C₁-C₄- Alkylsulfonyl, SO₂-OH, i- C₁-C₄-Aminoalkylsulfonyl oder C₁-C₄-Alkoxysulfonyl ist, R₁₃₃ die Bedeutung von R₁₂₉ hat und zusätzlich OH, NH₂, Halogen, Di- C₁-C₄-Aminoalkyl, Pyrrolidin1-yl, Piperidin-1-yl, Morpholin-1-yl, C₁-C₄-Alkylthio, C₁-C₄-Alkylsulfonyl, C₁-C₄-Alkoxycarbonyl, Phenoxy, Naphtoxy, Phenylamino, Benzoyloxy oder Phenylsulfonyloxy ist;
   oder eine Verbindung der Formel XXV 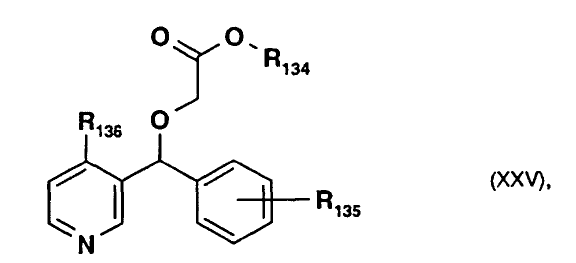 worin R₁₃₄ Wasserstoff, C₄-Alkyl, C₁-C₄-Halogenalkyl, C₂-C₄-Alkenyl, C₂-C₄-Alkinyl oder C₁-C₄-Alkoxy-C₁-C₄-Alkyl bedeutet, R₁₃₅ Wasserstoff, Halogen, C₁-C₄-Alkyl, C₁-C₄-Halogenalkyl oder C₁-C₄-Alkoxy und R₁₃₆ Wasserstoff, Halogen, C₁-C₄-Alkyl, C₁-C₄-Halogenalkyl oder C₁-C₄-Alkoxy bedeuten, mit der Maßgabe, daß R₁₃₅ und R₁₃₆ nicht gleichzeitig Wasserstoff bedeuten,
   oder der Formel XXVI 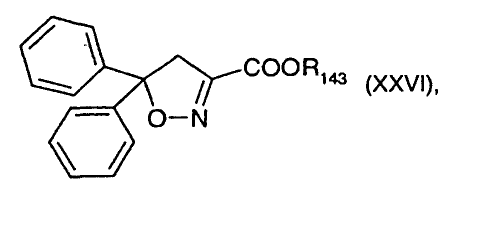 worin
   R₁₄₃ Wasserstoff, ein Alkali-, Erdalkali-, Sulfonium- oder Ammonium-Kation oder Ethyl bedeutet;
   oder der Formel XXVII 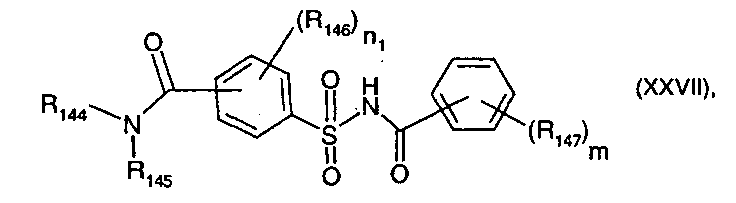 worin R₁₄₄ und R₁₄₅ unabhängig voneinander Wasserstoff, C₁-C₆-Alkyl, C₂-C₆-Alkenyl, C₂-C₆-Alkinyl oder C₃-C₆-Cycloalkyl bedeuten;
   R₁₄₆ Wasserstoff, Halogen, C₁-C₄-Alkyl, C₁-C₆-Halogenalkyl oder C₁-C₆-Halogenalkoxy bedeutet;
   R₁₄₇ Wasserstoff, Halogen, C₁-C₄-Alkyl, C₁-C₄-Halogenalkyl, C₁-C₄-Alkoxy, C₁-C₄-Halogenalkoxy, C₁-C₄-Alkylthio, C₁₋C₄-Alkoxycarbonyl oder Nitro bedeutet;
   n, 0, 1, 2 oder 3; und
   m 1 oder 2 bedeutet;
   oder der Formel XXVIII 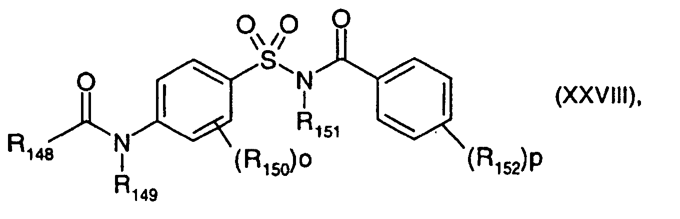 worin
   R₁₄₈ Wasserstoff, C₁-C₆-Alkyl, C₁-C₆-Alkoxy, C₁-C₆-Alkylthio, C₃-C₈-Cycloalkyl, Phenyl, Phenyl-C₁-C₆-alkyl oder Heteroaryl bedeutet; wobei die genannten Gruppen durch Halogen, Cyano, Nitro, Amino, Hydroxy, Carbonyl, Carboxyl, Formyl, Carbonamid oder Sulfonamid substituiert sein können;
   R₁₄₉ Wasserstoff, C₁-C₆-Alkyl oder C₁-C₄-Halogenalkyl bedeutet;
   jedes R₁₅₀ unabhängig Wasserstoff, Halogen, C₁-C₄-Alkyl, C₁-C₄-Halogenalkyl, C₁-C₄-Alkoxy, C₁-C₄-Alkylthio, C₁-C₄-Alkylsulfonyl, Cyano, Nitro, Formyl oder Carboxyl bedeutet;
   R₁₅₁ Wasserstoff, C₁-C₆-Alkyl oder C₁-C₄-Halogenalkyl bedeutet;
   jedes R₁₅₂ unabhängig Wasserstoff, Halogen, C₁-C₄-Alkyl, C₁-C₄-Halogenalkyl, C₁-C₄-Alkoxy, C₁-C₄-Alkylthio, C₁-C₄-Alkylsulfonyl, Cyano, Nitro, Formyl oder Carboxyl bedeutet;
   O für 0, 1, oder 2 steht, und
   p 0, 1 oder 2 bedeutet;
   oder der Formel XXIX 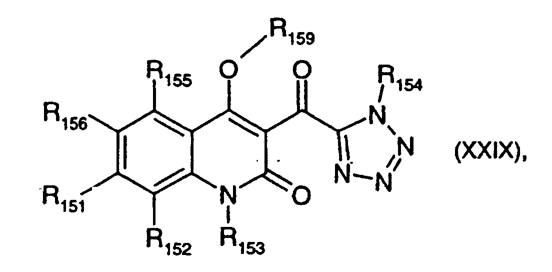 worin
   R₁₅₉ Wasserstoff, Formyl, C₁₋₆-Alkylcarbonyl, C₁₋₆-Alkenylcarbonyl, C₁₋₆-Alkinylcarbonyl, C₁₋₆-Alkoxycarbonyl, C₁₋₆-Alkylthiocarbonyl, C₃₋₈-Cycloalkylcarbonyl, Phenyl-C₁₋₆-alkylcarbonyl, Phenylcarbonyl. C₁₋₆-Alkylsulfonyl, C₁₋₆-Alkenylsulfonyl oder Phenylsulfonyl bedeutet, wobei die vorstehend genannten Kohlenwasserstoffgruppen durch ein oder mehrere Halogenatome, Cyano, Nitro, Amino, Methoxy, Ethoxy oder Phenyl substituiert sein können; R₁₅₃ Wasserstoff, C₁₋₆-Alkyl, C₁₋₆-Alkenyl, C₁₋₆-Alkinyl, C₃₋₈-Cycloalkyl, Formyl, C₁₋₆-Alkylcarbonyl, C₁₋₆-Alkenylcarbonyl, C₁₋₆-Alkinylcarbonyl, C₁₋₆-Alkoxycarbonyl, C₁₋₆-Alkylthiocarbonyl, C₃₋₈-Cycloalkylcarbonyl, C₁₋₆-Alkylsulfonyl, C₁₋₆-Alkenylsulfonyl oder Phenylsulfonyl bedeutet, wobei die vorstehend genannten Kohlenwasserstoffgruppen durch ein oder mehrere Halogenatome, Cyano, Nitro, Amino, Methoxy, Ethoxy oder Phenyl substituiert sein können;
   R₁₅₄ Wasserstoff, C₁₋₆-Alkyl, C₁₋₆-Alkenyl, C₁₋₆-Alkinyl, C₃₋₈-Cycloalkyl, Formyl, C₁₋₆-Alkylcarbonyl, C₁₋₆-Alkenylcarbonyl, C₁₋₆-Alkinylcarbonyl, C₁₋₆-Alkoxycarbonyl, C₁₋₆-Alkylthiocarbonyl, C₃₋₈-Cycloalkylcarbonyl, C₁₋₆-Alkylsulfonyl, C₁₋₆-Alkenylsulfonyl oder Phenylsulfonyl bedeutet, wobei die vorstehend genannten Kohlenwasserstoffgruppen durch ein oder mehrere Halogenatome, Cyano, Nitro, Amino, Methoxy, Ethoxy oder Phenyl substituiert sein können;
   R₁₅₅, R₁₅₆, R₁₅₇, und R₁₅₈ unabhängig voneinander Wasserstoff, Halogen, Amino, C₁₋₃-Alkylamino, C₁₋₆-Dialkylamino, Hydroxy, Cyano, Nitro, Formyl, Carboxyl, C₁₋₆-Alkoxy, C₁₋₆-Halogenalkoxy, C₁₋₆-Alkylcarbonyl, C₁₋₆-Alkoxycarboxyl, C₁₋₆-Alkyl, C₁₋₆-Halogenalkyl, C₁₋₆-Alkenyl oder C₁₋₆-Alkinyl bedeuten;
   oder R₁₅₃ und R₁₅₈ bilden gemeinsam mit den Ringatomen, an die sie gebunden sind, einen fünf- oder sechsgliedrigen teiigesättigten oder ungesättigten Ring, der bis zu 2 gleiche oder verschiedene Heteroatome aus der Gruppe Sauerstoff, Schwefel und Stickstoff enthalten kann, wobei dieser Ring durch einen Rest Oxo substituiert sein kann;
   enthält.

Bevorzugt enthalten die erfindungsgemäßen Mittel eine herbizid-antagonistisch wirksame Menge eines Safeners der Formel X, XI, XII, XIII, XIV, XV, XVI, XVII, XVIII, XIX, XX, XXI, XXII, XXIII, XXIV oder XXV.
Vorzugsweise enthält das erfindungsgemäße selektiv-herbizide Mittel als herbizid-antagonistisch wirksame Menge entweder eine Verbindung der Formel X 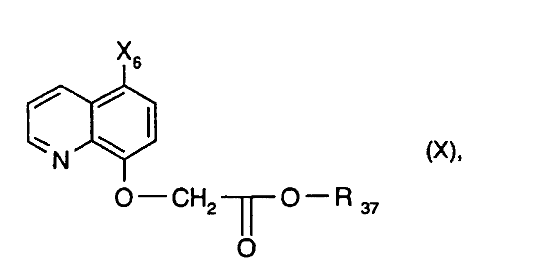 worin R₃₇ Wasserstoff, C₁-C₈-Alkyl oder durch C₁-C₆-Alkoxy oder C₃-C₆-Alkenyloxy substituiertes C₁-C₈-Alkyl; und X₆ Wasserstoff oder Chlor bedeutet; oder eine Verbindung der Formel XI 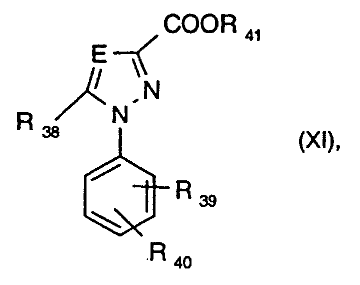 worin
E Stickstoff oder Methin; R₃₈ -CCl₃, Phenyl oder durch Halogen substituiertes Phenyl;
R₃₉ und R₄₀ unabhängig voneinander Wasserstoff oder Halogen; und
R₄₁ C₁-C₄-Alkyl bedeuten; oder eine Verbindung der Formel XII 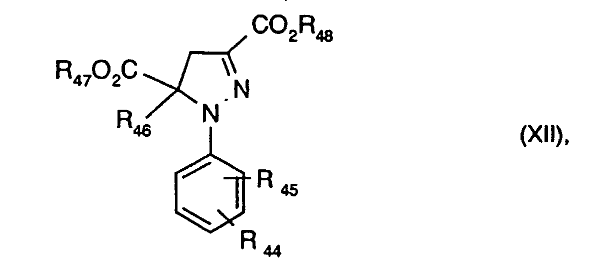 worin R₄₄ und R₄₅ unabhängig voneinander Wasserstoff oder Halogen und
R₄₆, R₄₇ und R₄₈ unabhängig voneinander C₁-C₄-Alkyl bedeuten.

Die oben genannten Bevorzugungen der Verbindungen der Formel I gelten auch bei Mischungen der Verbindungen der Formel I mit den Safenern der Formeln X bis XVIII.

Bevorzugte erfindungsgemäße Mittel enthalten einen Safener ausgewählt aus der Gruppe
der Formel Xa 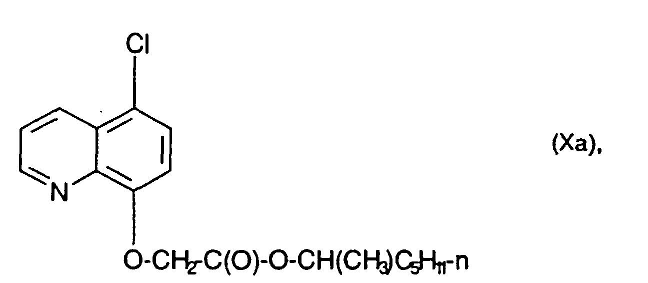 der Formel Xb 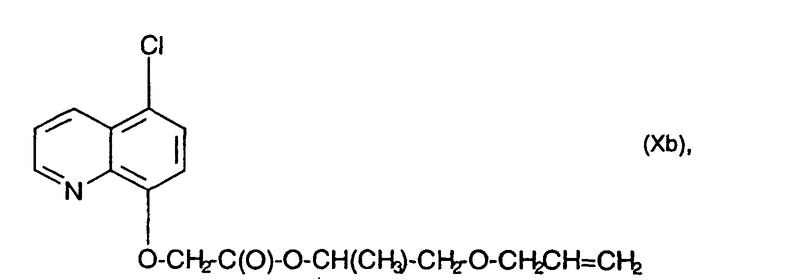 und der Formel XIa

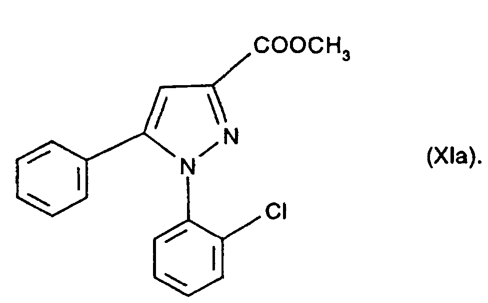

Weitere bevorzugte Verbindungen der Formeln X, XI und XII sind auch in den Tabellen 9, 10 und 11 aufgelistet.

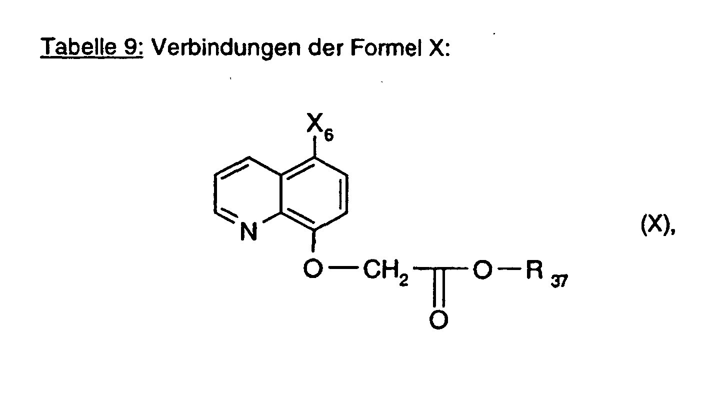

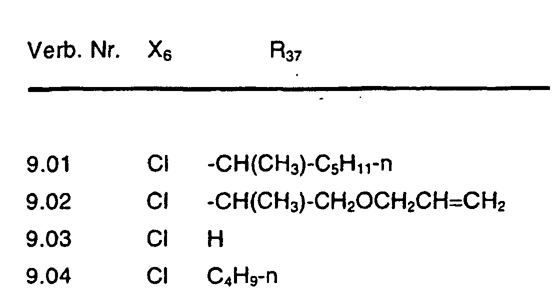

Bevorzugte Verbindungen der Formel XI sind in der folgenden Tabelle 10 aufgelistet.

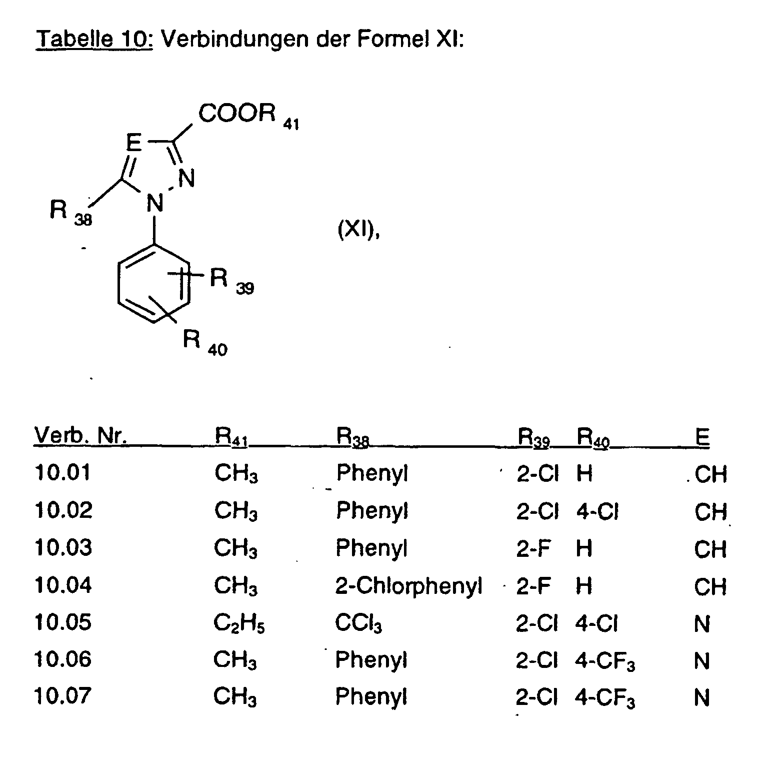

Bevorzugte Verbindungen der Formel XII sind in der folgenden Tabelle 11 aufgetistet.

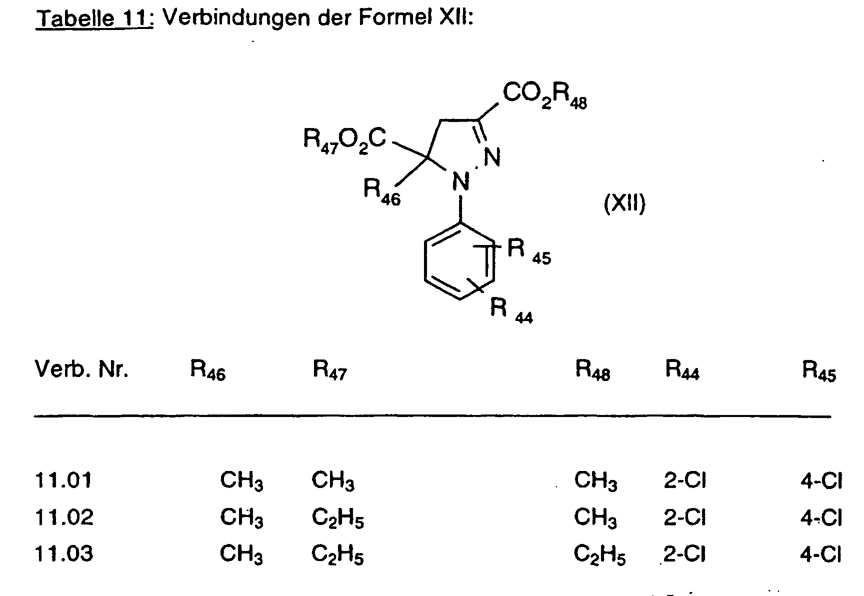

Bevorzugte Verbindungen der Formel XIII sind in der folgenden Tabelle 12 als Verbindungen der Formel XIIIa aufgelistet:

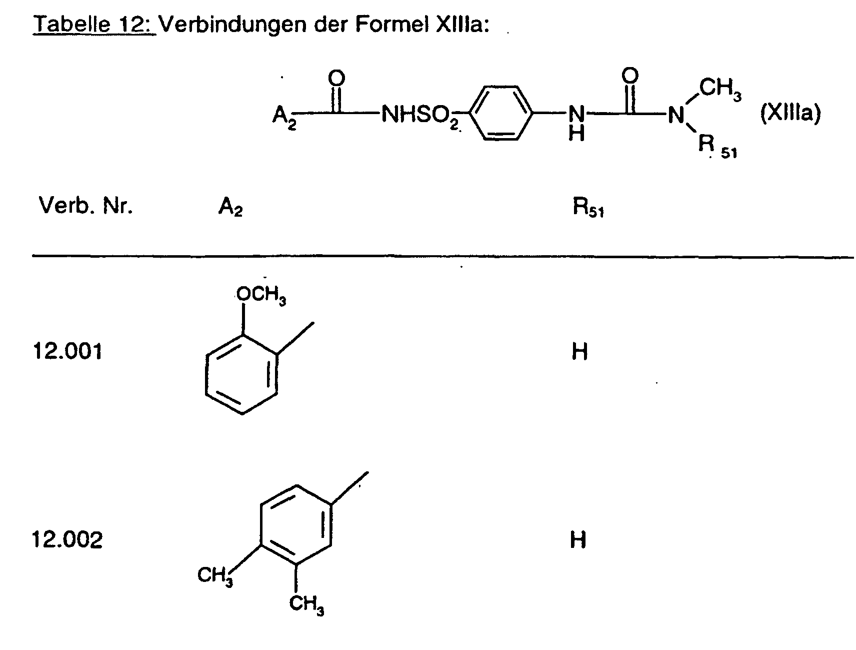

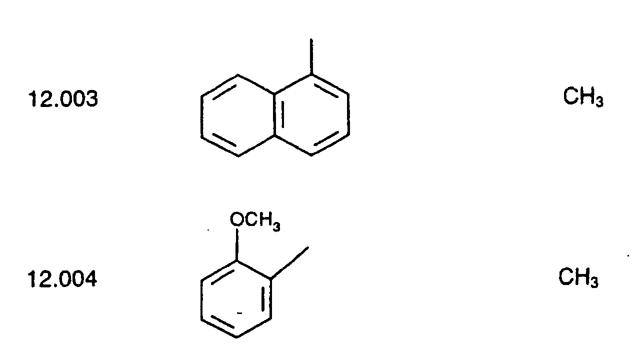

Bevorzugte Verbindungen der Formel XIV sind in der folgenden Tabelle 13 aufgelistet:

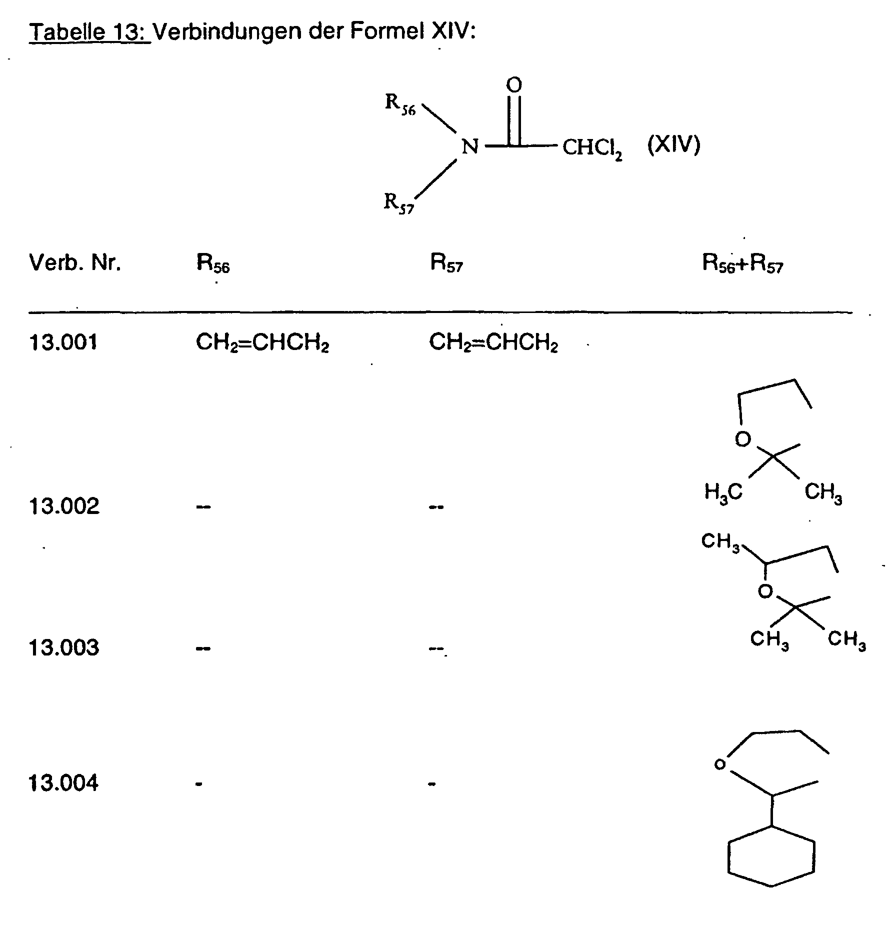

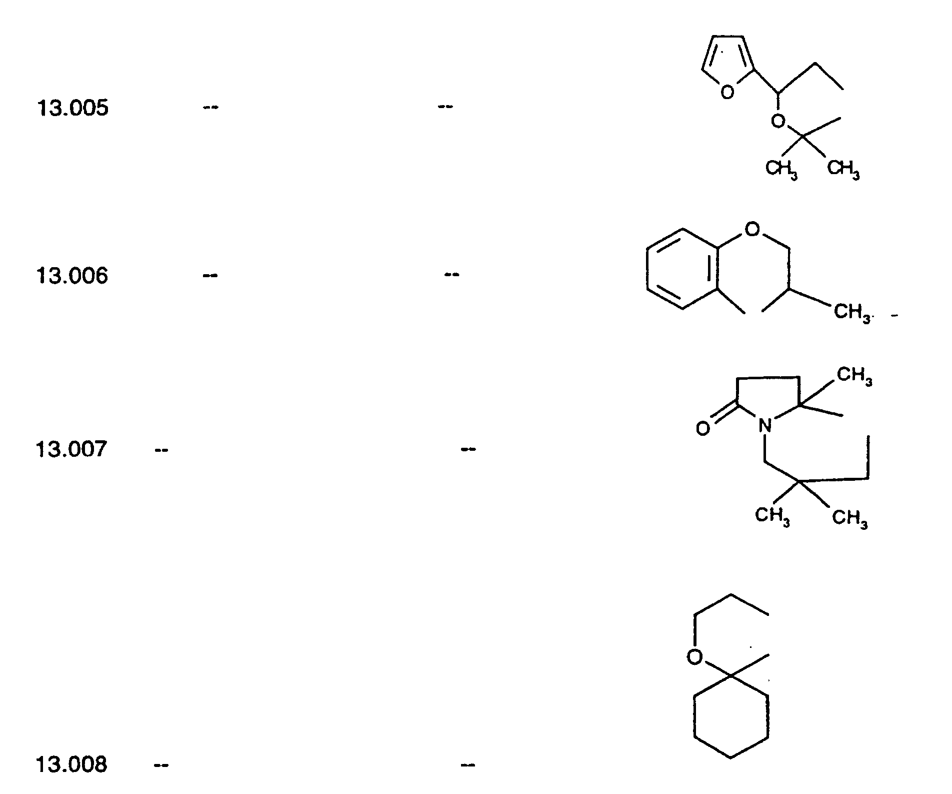

Bevorzugte Verbindungen der Formel XV sind in der folgenden Tabelle 14 aufgelistet:

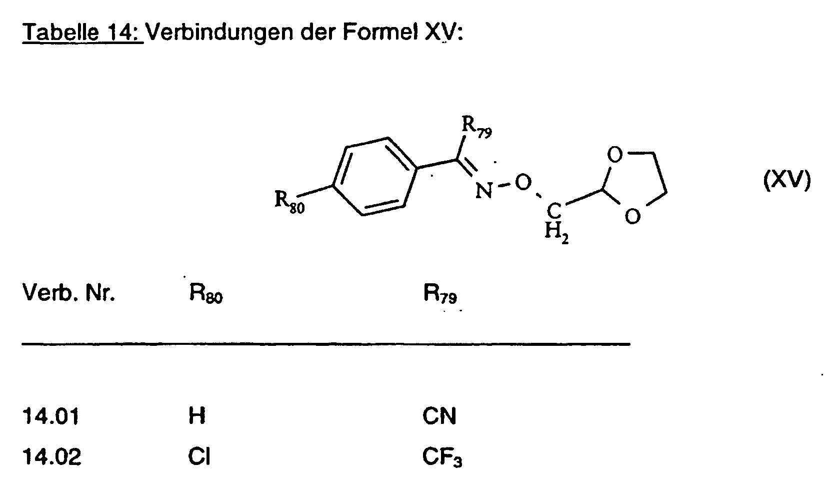

Bevorzugte Verbindungen der Formel XVI sind in der folgenden Tabelle 15 aufgelistet:

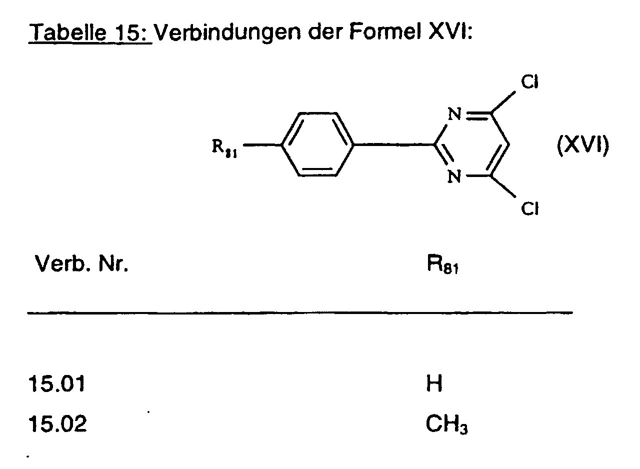

Bevorzugte Verbindungen der Formel XVII sind in der folgenden Tabelle 16 als Verbindungen der Formel XVIIa aufgelistet:

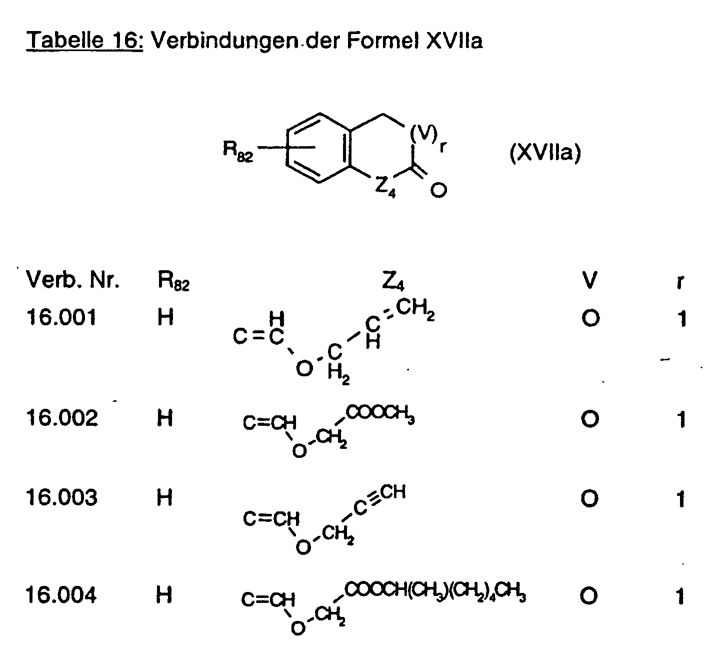

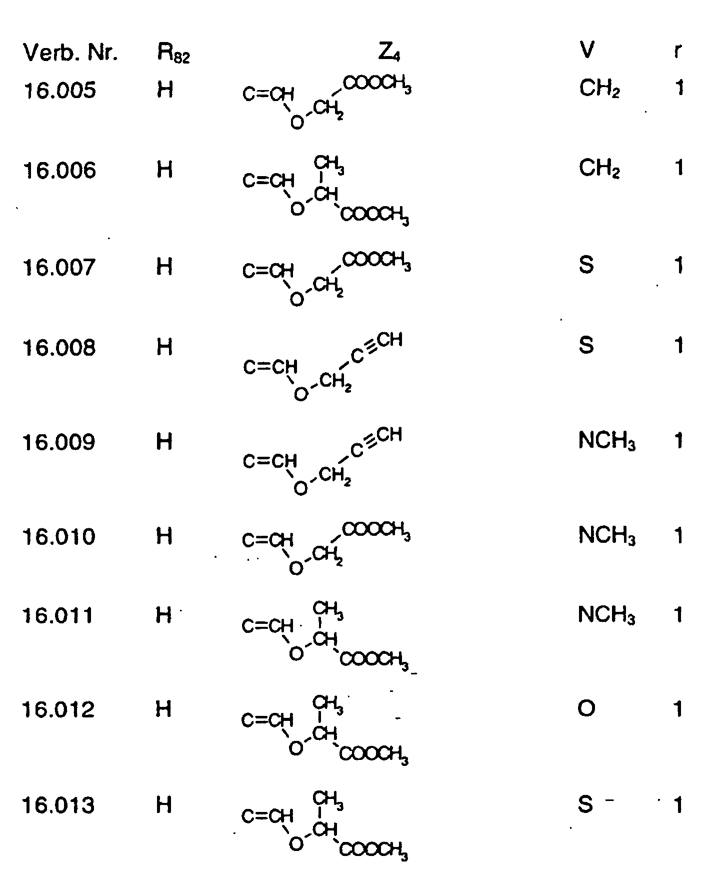

Bevorzugte Verbindungen der Formel XVII sind in der folgenden Tabelle 17 als Verbindungen der Formel XVIIb aufgelistet:

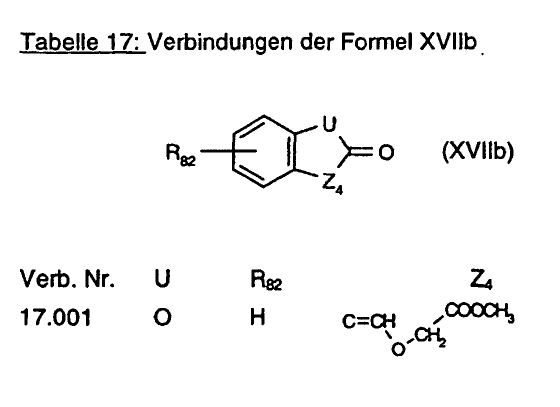

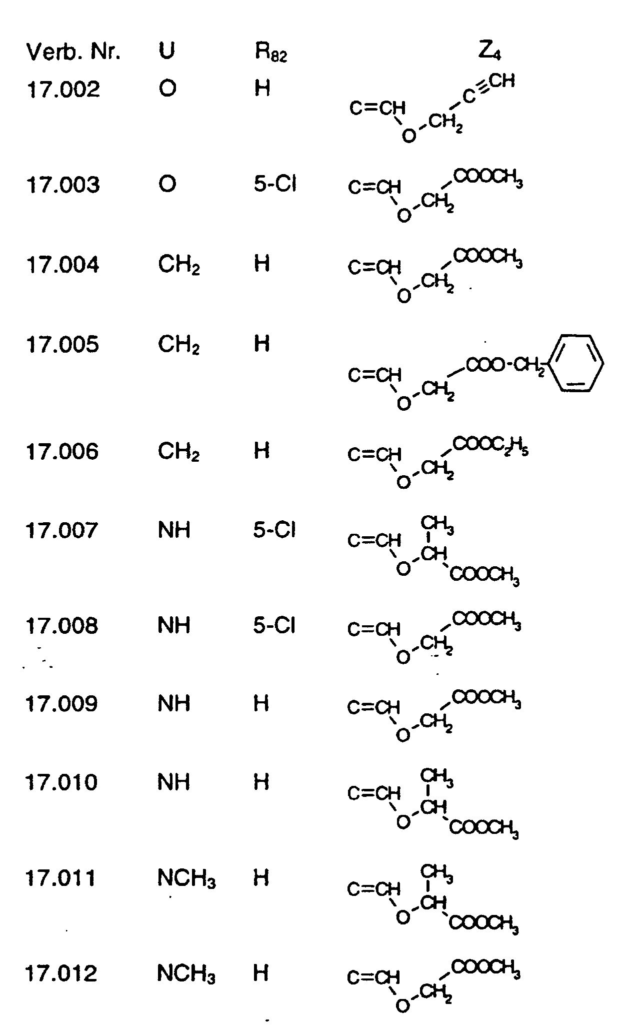

Bevorzugte Verbindungen der Formel XVII sind in der folgenden Tabelle 18 als Verbindungen der Formel XVIIc aufgelistet:

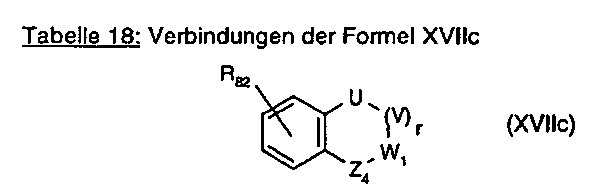

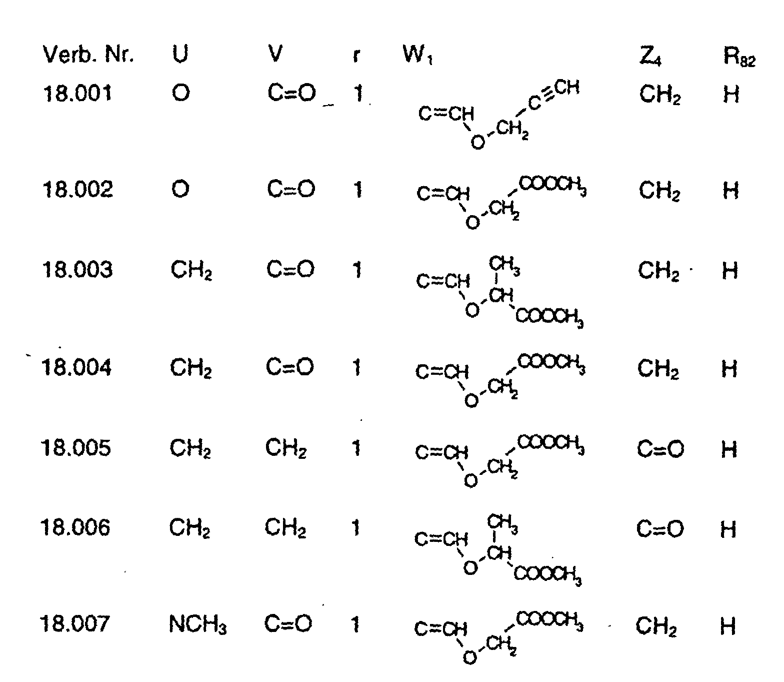

Bevorzugte Verbindungen der Formel XVII sind in der folgenden Tabelle 19 als Verbindungen der Formel XVIId aufgelistet:

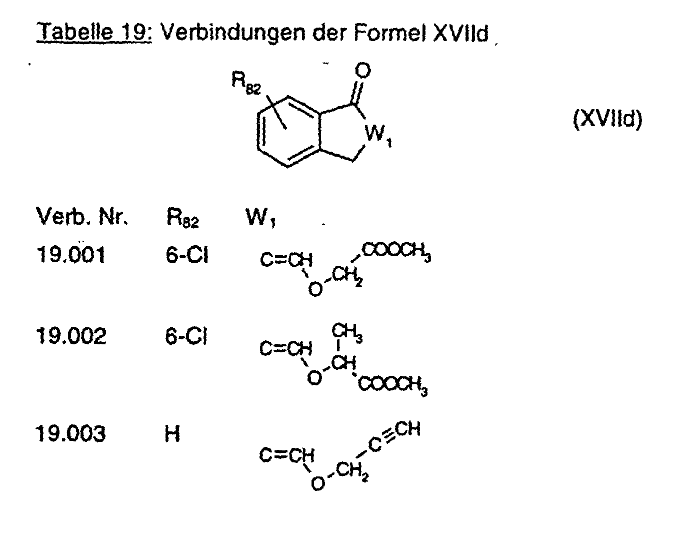

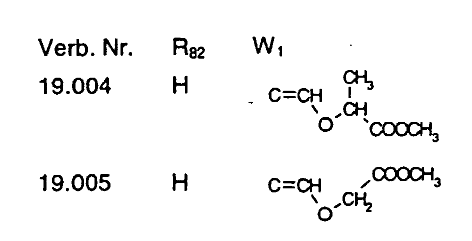

Bevorzugte Verbindungen der Formel XVIII sind in der folgenden Tabelle 20 aufgelistet:

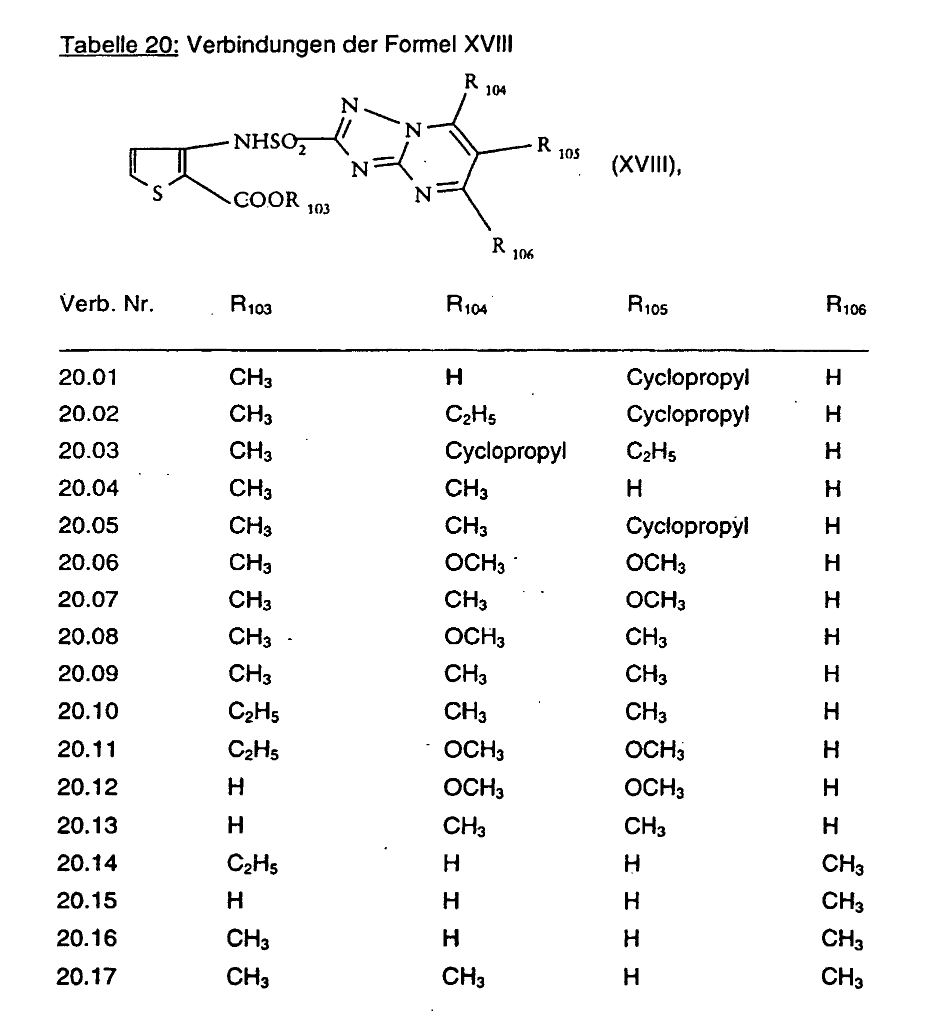

Von den Verbindungen der Formel XXVIII sind diejenigen bevorzugt, worin
R₁₄₈ Wasserstoff, C₁-C₆-Alkyl, C₃-C₈-Cycloalkyl oder Phenyl bedeutet, wobei die genannten Gruppen durch Halogen, Cyano, Nitro, Amino, Hydroxy, Carbonyl, Carboxyl Formyl, Carbonamid oder Sulfonamid substituiert sein können;
R₁₄₉ Wasserstoff bedeutet;
jedes R₁₅₀ unabhängig Wasserstoff, Halogen, C₁-C₄-Alkyl, C₁-C₄-Halogenalkyl, C₁-C₄-Alkoxy, C₁-C₄-Alkylthio, Cyano, Nitro oder Formyl bedeutet;
R₁₅₁ Wasserstoff bedeutet; und
jedes R₁₅₂ unabhängig Wasserstoff, Halogen, C₁-C₄-Alkyl, C₁-C₄-Halogenalkyl, C₁-C₄-Alkoxy, C₁-C₄-Alkylthio, Cyano, Nitro oder Formyl bedeutet.

Besonders bevorzugte Verbindungen der Formel XXVIII sind ausgewählt aus der Gruppe
2-Methoxy-N-[4-(2-methoxybenzoylsulfamoyl)phenyl]-acetamid,
N-[4-(2-Methoxybenzoylsulfamoyl)phenyl]-cyclopropancarboxamid,
N-[4-(2-Methoxy-benzoylsulfamoyl)-phenyl]- cyclobutancarboxamid,
N-[4-(2-Chlorobenzoylsulfamoyl)phenyl]-cyclopropancarboxamid,
N-[4-(2-Chloro-benzoylsulfamoyl)-phenyl]-acetamid,
N-[4-(2-Trifluoromethoxy-benzoylsulfamoyl)-phenyl]-acetamid,
N-[4-(2-Trifluormethylbenzoylsulfamoyl)phenyl]-cyclopropancarboxamid,
N-[4-(2-Trifluormethoxybenzoylsulfamoyl)phenyl]-cyclopropancarboxamid,
N-[4-(2-Trifluormethoxybenzoylsulfamoyl)phenyl]-cyclobutancarboxamid und
N-[4-(2-Trifluoromethyl-benzoylsulfamoyl)-phenyl]-acetamid.

Von den Verbindungen der Formel XXIX sind diejenigen bevorzugt, worin
R₁₅₉ Wasserstoff, Formyl. C₁₋₆-Alkylcarbonyl, C₁₋₆-Alkenylcarbonyl, C₁₋₆-Alkinylcarbonyl, C₁₋₆-Alkoxycarbonyl, C₁₋₆-Alkylthiocarbonyl, C₃₋₈-Cyccloalkylcarbonyl, Phenyl- C₁₋₆-alkylcarbonyl oder Phenylcarbonyl bedeutet, wobei die vorstehend genannten Kohlenwasserstoffreste durch ein oder mehrere Halogenatome, Cyano, Nitro, Amino, Methoxy, Ethoxy oder Phenyl substituiert sein können;
R₁₅₃ Wasserstoff, C₁₋₆-Alkyl, C₁₋₆-Alkenyl, C₁₋₆-Alkinyl, Formyl, C₁₋₆-Alkylcarbonyl oder C₁₋₆-Alkoxycarbonyl bedeutet, wobei die vorstehend genannten Kohlenwasserstoffreste durch ein oder mehrere Halogenatome, Cyano, Nitro, Amino, Methoxy, Ethoxy oder Phenyl substituiert sein können;
R₁₅₄ Wasserstoff, C₁₋₆-Alkyl, C₁₋₆-Alkenyl, C₁₋₆-Alkinyl, Formyl, C₁₋₆-Alkylcarbonyl oder C₁₋₆-Alkoxycarbonyl bedeutet, wobei die vorstehend genannten Kohlenwasserstoffreste durch ein oder mehrere Halogenatome, Cyano, Nitro, Amino, Methoxy, Ethoxy oder Phenyl substituiert sein können;
R₁₅₅, R₁₅₆, R₁₅₇, und R₁₅₈ unabhängig voneinander Wasserstoff, Halogen, Cyano, Nitro, Formyl, Carboxyl, C₁₋₆-Alkoxy, C₁₋₆-Halogenalkoxy, C₁₋₆-Alkylcarbonyl, C₁₋₆-Alkoxycarboxyl, C₁₋₆-Alkyl oder C₁₋₆-Halogenalkyl bedeuten;
oder R₁₅₃ und R₁₅₈ bilden gemeinsam mit den Ringatomen, an die sie gebunden sind, einen fünf oder sechsgliedrigen teilgesättigten oder ungesättigten Ring, der bis zu 2 gleiche oder verschiedene Heteroatome aus der Gruppe Sauerstoff, Schwefel und Stickstoff enthalten kann, wobei dieser Ring durch einen Rest Oxo substituiert sein kann.

Besonders bevorzugte Verbindungen der Formel XXIX sind dadurch gekennzeichnet, daß
R₁₅₉ Wasserstoff, Formyl, C₁₋₆-Alkylcarbonyl, C₁₋₆-Alkenylcarbonyl, C₁₋₆-Alkinylcarbonyl, C₁₋₆-Alkoxycarbonyl, C₁₋₆-Alkylthiocarbonyl, C₃₋₈-Cyccloalkylcarbonyl oder Phenylcarbonyl bedeutet;
R₁₅₃ Wasserstoff, C₁₋₆-Alkyl, C₁₋₆-Alkenyl, C₁₋₆-Alkinyl, Formyl, C₁₋₆-Alkylcarbonyl oder C₁₋₆-Alkoxycarbonyl bedeutet;
R₁₅₄: Wasserstoff, C₁₋₆-Alkyl, C₁₋₆-Alkenyl, C₁₋₆-Alkinyl, Formyl, C₁₋₆-Alkylcarbonyl oder C₁₋₆-Alkoxycarbonyl bedeutet;
R₁₅₅, R₁₅₆, R₁₅₇, und R₁₅₈ unabhängig voneinander Wasserstoff, Halogen, Cyano, Nitro, Formyl, C₁₋₆-Alkyl, C₁₋₆-Halogenalkyl, C₁₋₆-Alkoxy oder C₁₋₆-Halogenalkoxy bedeuten;
oder R₁₅₃ und R₁₅₈ bilden gemeinsam mit den Ringatomen, an die sie gebunden sind, einen fünf oder sechsgliedrigen teilgesättigten oder ungesättigten Ring, der bis zu 2 gleiche oder verschiedene Heteroatome aus der Gruppe Sauerstoff, Schwefel und Stickstoff enthalten kann, wobei dieser Ring durch einen Rest Oxo substituiert sein kann.

Ganz besonders bevorzugte Verbindungen der Formel XXIX sind ausgewählt aus der Gruppe
4-Hydroxy-1-methyl-3-(1.H.-tetrazol-5-carbonyl)-1.H.-chinolin-2-on,
1-Ethyl-4-hydroxy-3-(1.H.-tetrazol-5-carbonyl)-1.H.-chinolin-2-on,
6-Hydroxy-5-(1.H.-tetrazol-5-carbonyl)-1,2-dihydro-pyrrolo[3,2,1-.ij.]chinolin-4-on,
3-(1-Acetyl-1.H.-tetrazol-5-carbonyl)-4-hydroxy-1-methyl-1.H.-chinolin-2-on,
6-Chloro-4-hydroxy-1-methyl-3-(1.H.-tetrazol-5-carbonyl)-1.H.-chinolin-2-on,
6-Fluoro-4-hydroxy-1-methyl-3-(1.H.-tetrazol-5-carbonyl)-1.H.-chinolin-2-on,
4-Hydroxy-1,6-dimethyl-3-(1.H.-tetrazol-5-carbonyl)-1.H.-chinolin-2-on,
4-Hydroxy-6-methoxy-1-methyl-3-(1.H.-tetrazol-5-carbonyl)-1.H.-chinolin-2-on,
4-Hydroxy-6-methoxy-1-methyl-3-(1.H.-tetrazole-5-carbonyl)-1.H.-chinolin-2-on,
Acetic acid 1-methyl-2-oxo-3-(1.H.-tetrazol-5-carbonyl)-1,2-dihydro-chinolin-4-yl ester und
2,2-Dimethyl-propionic acid 1-methyl-2-oxo-3-(1.H.-tetrazol-5-carbonyl)-1,2-dihydro-chinolin-4-yl ester.

Die Erfindung betrifft auch ein Verfahren zum selektiven Bekämpfen von Unkräutern in Nutzpflanzenkulturen, welches darin besteht, daß man die Nutzpflanzen, deren Samen oder Stecklinge oder deren Anbaufläche gleichzeitig oder getrennt mit einer herbizid wirksamen Menge des Herbizids der Formel I und einer herbizid-antagonistisch wirksamen Menge des Safeners der Formel X, XI, XII, XIII, XIV, XV, XVI, XVII, XVIII, XIX, XX, XXI, XXII, XXIII, XXIV, XXV, XXVI, XXVII, XXVIII oder XXIX, vorzugsweise der Formel X, XI, XII, XIII, XIV, XV, XVI, XVII oder XVIII behandelt.
Als Kulturpflanzen, welche durch die Safener der Formel X, XI, XII, XIII, XIV, XV, XVI, XVII oder XVIII gegen die schädigende Wirkung der oben erwähnten Herbizide geschützt werden können, kommen insbesondere Getreide, Baumwolle, Soja, Zuckerrüben, Zuckerrohr, Plantagen, Raps, Mais und Reis, ganz besonders in Mais und Getreide in Betracht. Unter Kulturen sind auch solche zu verstehen, die durch konventionelle züchterische oder gentechnologische Methoden gegen Herbizide bzw. Herbizidklassen tolerant gemacht worden sind.

Bei den zu bekämpfenden Unkräutern kann es sich sowohl um monokotyle wie um dikotyle Unkräuter handeln, wie zum Beispiel die monokotylen Unkräuter Avena, Agrostis, Phalaris, Lolium, Bromus, Alopecurus, Setaria, Digitaria Brachiaria, Echinochloa, Panicum, Sorghum hal./bic., Rottboellia, Cyperus, Brachiaria, Echinochloa, Scirpus, Monochoria, Sagittaria, und Stellaria und die dikotylen Unkräuter Sinapis, Chenopodium, Galium, Viola, Veronica, Matricaria, Papaver, Solanum Abutilon, Sida, Xanthium, Amaranthus, Ipomoea und Chrysanthemum.

Als Anbauflächen gelten die bereits mit den Kulturpflanzen bewachsenen oder mit dem Saatgut dieser Kulturpflanzen beschickten Bodenareale wie auch die zur Bebauung mit diesen Kulturpflanzen bestimmten Böden.

Ein Safener der Formel X, XI, XII, XIII, XIV, XV, XVI, XVII, XVIII, XIX, XX, XXI, XXII, XXIII, XXIV, XXV, XXVI, XXVII, XXVIII oder XXIX kann je nach Anwendungszweck zur Vorbehandlung des Saatgutes der Kulturpflanze (Beizung des Samens oder der Stecklinge) eingesetzt oder vor oder nach der Saat in den Boden gegeben werden. Er kann aber auch für sich allein oder zusammen mit dem Herbizid nach dem Auflaufen der Pflanzen appliziert werden. Die Behandlung der Pflanzen oder des Saatgutes mit dem Safener kann daher grundsätzlich unabhängig vom Zeitpunkt der Applikation des Herbizids erfolgen. Die Behandlung der Pflanze kann man jedoch auch durch gleichzeitige Applikation von Herbizid und Safener (z.B. als Tankmischung) vornehmen. Die zu applizierende Aufwandmenge Safener zu Herbizid richtet sich weitgehend nach der Anwendungsart. Bei einer Feldbehandlung, welche entweder unter Verwendung einer Tankmischung mit einer Kombination von Safener und Herbizid oder durch getrennte Applikation von Safener und Herbizid erfolgt, liegt in der Regel ein Verhältnis von Herbizid zu Safener von 100:1 bis 1:10, bevorzugt 20:1 bis 1:1, vor. In der Regel werden bei der Feldbehandlung 0,001 bis 1,0 kg Safener/ha, vorzugsweise 0,001 bis 0,25 kg Safener/ha, appliziert.

Die Aufwandmengen an Herbizid liegt in der Regel zwischen 0,001 bis 2 kg/ha, vorzugsweise jedoch zwischen 0,005 bis 0,5 kg/ha.

Die erfindungsgemäßen Mittel sind für alle in der Landwirtschaft üblichen Applikationsmethoden wie z.B. preemergente Applikation, postemergente Applikation und Saatbeizung geeignet.

Bei der Samenbeizung werden im allgemeinen 0,001 bis 10 g Safener/kg Samen, vorzugsweise 0,05 bis 2 g Safener/kg Samen, appliziert. Wird der Safener in flüssiger Form kurz vor der Aussaat unter Samenquellung appliziert, so werden zweckmäßigerweise Safenerlösungen verwendet, welche den Wirkstoff in einer Konzentration von 1 bis 10000, vorzugsweise von 100 bis 1000 ppm, enthalten.

Zur Applikation werden die Safener der Formel X, XI, XII, XIII, XIV, XV, XVI, XVII, XVIII, XIX, XX, XXI, XXII, XXIII, XXIV, XXV, XXVI, XXVII, XXVIII oder XXIX oder Kombinationen von diesen Safenem mit den Herbiziden der Formel I zweckmäßigerweise zusammen mit den in der Formulierungstechnik üblichen Hilfsmitteln zu Formulierungen verarbeitet, z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten oder Mikrokapseln.

Solche Formulierungen sind beispielsweise in der WO 97/34485 auf den Seiten 9 bis 13 beschrieben. Die Formulierungen werden in bekannter Weise hergestellt, z.B. durch inniges Vermischen und/oder Vermahlen der Wirkstoffe mit flüssigen oder festen Formulierungshilfsmitteln wie z.B. Lösungsmitteln oder festen Trägerstoffen. Ferner können zusätzlich oberflächenaktive Verbindungen (Tenside) bei der Herstellung der Formulierungen verwendet werden. Für diesen Zweck geeignete Lösungsmittel und feste Trägerstoffe sind z.B. in der WO 97/34485 auf der Seite 6 angegeben.

Als oberflächenaktive Verbindungen kommen je nach der Art des zu formulierenden Wirkstoffes der Formel I nichtionogene, kation- und/oder anionaktive Tenside und Tensidgemische mit guten Emulgier-, Dispergier- und Netzeigenschaften in Betracht. Beispiele für geeignete anionische, nichtionische und kationische Tenside sind beispielsweise in der WO 97/34485 auf den Seiten 7 und 8 aufgezählt. Ferner sind auch die in der Formulierungstechnik gebräuchlichen Tenside, die u.a. in "Mc Cutcheon's Detergents and Emulsifiers Annual" MC Publishing Corp., Ridgewood New Jersey, 1981, Stache, H., "Tensid-Taschenbuch", Carl Hanser Verlag, München/Wien, 1981 und M. und J. Ash, "Encyclopedia of Surfactants", Vol I-III, Chemical Publishing Co., New York, 1980-81 beschrieben sind, zur Herstellung der erfindungsgemäßen herbiziden Mittel geeignet.

Die herbiziden Formulierungen enthalten in der Regel 0,1 bis 99 Gew%, insbesondere 0,1 bis 95 Gew.-% Wirkstoffgemisch aus der Verbindung der Formel I mit den Verbindungen der Formeln X, XI, XII, XIII, XIV, XV, XVI, XVII, XVIII, XIX, XX, XXI, XXII, XXIII, XXIV, XXV, XXVI, XXVII, XXVIII oder XXIX, 1 bis 99,9 Gew.% eines festen oder flüssigen Formulierungshilfstoffes und 0 bis 25 Gew.%, insbesondere 0,1 bis 25 Gew.% eines Tensides. Während als Handelsware üblicherweise konzentrierte Mittel bevorzugt werden, verwendet der Endverbraucher in der Regel verdünnte Mittel.

Die Mittel können auch weitere Zusätze wie Stabilisatoren z.B. gegebenenfalls epoxydierte Pflanzenöle (epoxydiertes Kokosnußöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe enthalten. Für die Verwendung von Safenem der Formeln X, XI, XII, XIII, XIV, XV, XVI, XVII, XVIII, XIX, XX, XXI, XXII, XXIII, XXIV, XXV, XXVI, XXVII, XXVIII oder XXIX oder sie enthaltender Mittel zum Schützen von Kulturpflanzen gegen schädigende Wirkungen von Herbiziden der Formell kommen verschiedene Methoden und Techniken in Betracht, wie beispielsweise die folgenden:

### i) Samenbeizung

a) Beizung der Samen mit einem als Spritzpulver formulierten Wirkstoff der Formeln X, XI, XII, XIII, XIV, XV, XVI, XVII, XVIII, XIX, XX, XXI, XXII, XXIII, XXIV, XXV, XXVI, XXVII, XXVIII oder XXIX, durch Schütteln in einem Gefäß bis zur gleichmäßigen Verteilung auf der Samenoberfläche (Trockenbeizung). Man verwendet dabei etwa 1 bis 500 g Wirkstoff der Formel X, XI, XII, XIII, XIV, XV, XVI, XVII, XVIII, XIX, XX, XXI, XXII, XXIII, XXIV, XXV, XXVI, XXVII, XXVIII oder XXIX (4 g bis 2 kg Spritzpulver) pro 100 kg Saatgut.
b) Beizung der Samen mit einem Emulsionskonzentrat des Wirkstoffs der Formel X, XI, XII, XIII, XIV, XV, XVI, XVII, XVIII, XIX, XX, XXI, XXII, XXIII, XXIV, XXV, XXVI, XXVII, XXVIII oder XXIX nach der Methode a) (Naßbeizung).
c) Beizung durch Tauchen des Saatguts in eine Brühe mit 100-1000 ppm Wirkstoff der Formel X, XI, XII, XIII, XIV, XV, XVI, XVII, XVIII, XIX, XX, XXI, XXII, XXIII, XXIV, XXV, XXVI, XXVII, XXVIII oder XXIX während 1 bis 72 Stunden und gegebenenfalls nachfolgendes Trocknen der Samen (Tauchbeizung).

Die Beizung des Saatguts oder die Behandlung des angekeimten Sämlings sind naturgemäß die bevorzugten Methoden der Applikation, weil die Wirkstoffbehandlung vollständig auf die Zielkultur gerichtet ist. Man verwendet in der Regel 1 bis 1000 g Antidot, vorzugsweise 5 bis 250 g Antidot, pro 100 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

### ii) Applikation als Tankmischung

Eine flüssige Aufarbeitung eines Gemisches von Antidot und Herbizid (gegenseitiges Mengenverhältnis zwischen 10:1 und 1:100) wird verwendet, wobei die Aufwandmenge an Herbizid 0,005 bis 5,0 kg pro Hektar beträgt. Solche Tankmischungen werden vor oder nach der Aussaat appliziert.

### iii) Applikation in der Saatfurche

Der Wirkstoff der Formel X, XI, XII, XIII, XIV, XV, XVI, XVII, XVIII, XIX, XX, XXI, XXII, XXIII, XXIV, XXV, XXVI, XXVII, XXVIII oder XXIX wird als Emulsionskonzentrat, Spritzpulver oder als Granulat in die offene besäte Saatfurche eingebracht. Nach dem Decken der Saatfurche wird in üblicher Weise das Herbizid im Vorauflaufverfahren appliziert.

### iv) Kontrollierte Wirkstoffabgabe

Der Wirkstoff der Formel X, XI, XII, XIII, XIV, XV, XVI, XVII, XVIII, XIX, XX, XXI, XXII, XXIII, XXIV, XXV, XXVI, XXVII, XXVIII oder XXIX wird in Lösung auf mineralische Granulatträger oder polymerisierte Granulate (Harnstoff/Formaldehyd) aufgezogen und getrocknet. Gegebenenfalls kann ein Überzug aufgebracht werden (Umhüllungsgranulate), der es erlaubt, den Wirkstoff über einen bestimmten Zeitraum dosiert abzugeben.

Erfindungsgemäße herbizide und den Pflanzenwuchs hemmende Mittel mit einem herbizid wirksamen Gehalt an Verbindung der Formel I sowie einem herbizid-antagonistisch wirksamen Menge an Verbindung der Formeln X, XI, XII, XIII, XIV, XV, XVI, XVII, XVIII, XIX, XX, XXI, XXII, XXIII, XXIV, XXV, XXVI, XXVII, XXVIII oder XXIX können durch Zugabe von Sprühtank-Adjuvantien in ihrer Wirksamkeit gesteigert werden.
Diese Adjuvantien können beispielsweise sein: nichtionische Tenside, Mischungen von nichtionischen Tensiden, Mischungen von anionischen Tensiden mit nichtionischen Tensiden, kationische Tenside, silizium-organische Tenside, Mineralölderivate mit und ohne Tenside , Pflanzenölderivate mit und ohne Tensidzusatz, alkylierte Derivate von Ölen pflanzlichen oder mineralischen Ursprungs mit und ohne Tenside, Fischöle und andere tierische Öle tierischer Natur sowie deren Alkylderivate mit und ohne Tenside, natürlich vorkommende höhere Fettsäuren, vorzugsweise mit 8 bis 28 Kohlenstoffatomen, und deren Alkylesterderivate, organische Säuren enthaltend ein aromatisches Ringsystem und einen oder mehrere Carbonsäurerester, sowie deren Alkylderivaten, ferner Suspensionen von Polymeren des Vinylacetats oder Copolymeren von Vinylacetat-Acrylsäureestem. Mischungen einzelner Adjuvantien untereinander sowie in Kombination mit organischen Lösungsmitteln können zu einer weiteren Steigerung der Wirkung führen.

Als nichtionische Tenside kommen beispielsweise Polyglykoletherderivate von aliphatischen oder cycloaliphatischen Alkoholen, gesättigten oder ungesättigten Fettsäuren und Alkylphenolen in Frage, vorzugsweise die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, vorzugsweise 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit vorzugsweise 1 bis 10 Kohlenstoffatomen in der Alkylkette. Die genannten Verbindungen enthalten üblicherweise pro Propylenglykol-Einheit 1 bis 5 Ethylenglykoleinheiten.

Als weitere Beispiele nichtionischer Tenside seien auch Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei anionischen Tensiden werden vor allem Alkylsulfate, Alkylsulfonate, Alkylarylsulfonate, alkylierte Phosphorsäuren bevorzugt sowie deren ethoxylierte Derivate. Die Alkylreste enthalten üblicherweise 8 bis 24 Kohlenstoffatome.

Bevorzugte nicht-ionische Tenside sind unter den folgenden Handelsnamen bekannt:

Polyoxyethylen Cocoalkylamin (z.B. AMIET® 105 (Kao Co.)), Polyoxyethylen Oleylamin (z.B. AMIET® 415 (Kao Co.)), Nonylphenolpolyethoxyethanole, Polyoxyethylen Stearylamin (z.B. AMIET® 320 (Kao Co.)), N-polyethoxyethylamines (z.B. GENAMIN® (Hoechst AG)), N,N,N',N'-Tetra(Polyethoxypolypropoxyethyl)ethylen-diamine (z.B. TERRONIL® und TETRONIC® (BASF Wyandotte Corp.)), BRIJ® (Atlas Chemicals), ETHYLAN® CD und ETHYLAN® D (Diamond Shamrock), GENAPOL® C, GENAPOL® O, GENAPOL® S und GENAPOL® X080 (Hoechst AG), EMULGEN® 104P, EMULGEN® 109P und EMULGEN® 408 (Kao Co.); DISTY® 125 (Geronazzo), SOPROPHOR® CY 18 (Rhone Poulenc S.A.); NONISOL® (Ciba-Geigy), MRYJ® (ICI); TWEEN® (ICI); EMULSOGEN® (Hoechst AG); AMIDOX® (Stephan Chemical Co.), ETHOMID® (Armak Co.); PLURONIC® (BASF Wyandotte Corp.), SOPROPHOR® 461 P (Rhöne Poulenc S.A.), SOPROPHOR® 496/P (Rhone Poulenc S.A.), ANTAROX FM-63 (Rhone Poulenc S.A.), SLYGARD 309 (Dow Coming), SILWET 408, SILWET L-7607N (Osi-Specialities).

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die verwendeten Öle sind entweder von mineralischer oder natürlicher Herkunft. Die natürlichen Öle können zudemnoch von tierischem oder pflanzlichen Ursprung sein. Bei tierischen Ölen werden vor allem Derivate von Rindertalg bevorzugt, aber auch Fischöle (z.B. Sardinenöl) und deren Derivate werden verwendet. Pflanzliche Öle sind meist Saatöle verschiedener Herkunft. Als Beispiele für besonders verwendete Pflanzenöle können Kokos-, Raps- oder Sonneblumenöle sowie deren Derivate genannt werden.

In dem erfindungsgemäßen Mittel betragen die Aufwandmengen an Öladditiv in der Regel zwischen 0,01 und 2 % in Bezug auf die Spritzbrühe. Beispielsweise kann das Öladditv nach Herstellung der Spritzbrühe in der gewünschten Konzentration in den Sprühtank gegeben werden.

Im erfindungsgemäßen Mittel bevorzugte Öladditive enthalten ein Öl pflanzlichen Ursprungs wie beispielsweise Rapsöl oder Sonnenblumenöl, Alkylester von Ölen pflanzlichen Ursprungs wie beispielsweise die Methylderivate, oder Mineralöle.

Besonders bevorzugte Öladditive enthalten Alkylester von höheren Fettsäuren (C₈-C₂₂). insbesondere die Methylderivate von C₁₂-C₁₈ Fettsäuren, beispielsweise die Methylester der Laurinsäure, Palmitinsäure und Ölsäure. Diese Ester sind bekannt als Methyllaurat (CAS-111-82-0), Methylpalmitat (CAS-112-39-0) und Methyloleat (CAS-112-62-9).

Das Ausbringen und die Wirkung der Öladditive kann durch deren Kombination mit oberflächenaktiven Substanzen wie nichtionische-, anionische oder kationische Tenside verbessert werden. Beispiele für geeignete anionische, nichtionische und kationische Tenside sind in der WO 97/34485 auf den Seiten 7 und 8 aufgezählt.

Bevorzugte oberflächenaktive Substanzen sind anionische Tenside vom Typ der Dodecylbenzylsulfonate, insbesondere die Calciumsalze davon sowie nichtionische Tenside vom Typ der Fettalkoholethoxylate. Insbeondere bevorzugt sind ethoxylierte C₁₂-C₂₂-Fettalkohole mit einem Ethoxylierungsgrad zwischen 5 und 40. Beispiele für kommerziell erhältliche, bevorzugte Tenside sind die Genapol Typen (Clariant AG, Muttenz, Schweiz). Die Konzentration der oberflächenaktiven Substanzen in Bezug auf das gesamte Additiv beträgt im allgemeinen zwischen 1 und 30 Gew.%.

Beispiele für Öladditive, die aus Mischungen von Ölen bzw. Mineralölen oder deren Derivaten mit Tensiden bestehen, sind Edenor ME SU®, Emery 2231® (Henkel Tochtergesellschaft Cognis GMBH, DE), Turbocharge® (Zeneca Agro, Stoney Creek, Ontario , CA) oder, besonders bevorzugt, Actipron® (BP Oil UK Limited, GB).

Ferner kann die Zugabe eines organischen Lösungsmittels zu dem Öladditiv/Tensidgemisch eine weitere Steigerung der Wirkung bewirken. Geeignete Lösungsmittel sind beispielsweise Solvesso® (ESSO) oder Aromatic Solvent® (Exxon Corporation) Typen. Die Konzentration derartiger Lösungsmittel kann von 10 bis 80 Gew.% des Gesamtgewichtes betragen.

Derartige Öladditive, die beispielsweise auch in US-A-4,834,908 beschrieben sind, sind für das erfindungsgemäße Mittel besonders bevorzugt. Ein ganz besonders bevorzugtes Öladditiv ist unter dem Namen MERGE® bekannt, kann von der BASF Corporation bezogen werden und ist beispielsweise in US-A-4,834,908 in col. 5, als Example COC-1 im wesentlichen beschrieben. Ein weiteres erfindungsgemäß bevorzugtes Öladditiv ist SCORE® (Novartis Crop Protection Canada.)

In der Formulier- und Adjuvanttechnik gebräuchliche Tenside, Öle, insbesondere Pflanzenöle, Derivate davon wie alkylierte Fettsäuren und Mischungen davon, z.B. mit vorzugsweise anionischen Tensiden wie alkylierten Phosphorsäuren, Alkylsulfate und Alkylarylsulfonaten sowie höheren Fettsäuren, die auch in den erfindungsgemäßen Mitteln und Sprühtanklösungen davon verwendet werden können, sind u.a. in "Mc Cutcheon's Detergents and Emulsifiers Annual" MC Publishing Corp., Ridgewood New Jersey, 1998, Stache, H., "Tensid-Taschenbuch", Carl Hanser Verlag, München/Wien, 1990, M. und J. Ash, "Encyclopedia of Surfactants", Vol I-IV, Chemical Publishing Co., New York, 1981-89, G. Kapusta, "A Compendium of Herbicide Adjuvants", Southern Illinois Univ. , 1998, L. Thomson Harvey, "A Guide to Agricultural Spray Adjuvants Used in the United States", Thomson Pubns., 1992 beschrieben.

Insbesondere setzen sich bevorzugte Formulierungen folgendermaßen zusammen: (% = Gewichtsprozent)

### Emulgierbare Konzentrate:

| | |
|---|---|
| Aktives Wirkstoffgemisch | 1 bis 90 %, vorzugsweise 5 bis 20 % |
| oberflächenaktives Mittel | 1 bis 30 %, vorzugsweise 10 bis 20 % |
| flüssiges Trägermittel | 5 bis 94 %, vorzugsweise 70 bis 85 % |

### Stäube:

| | |
|---|---|
| Aktives Wirkstoffgemisch | 0,1 bis 10 %, vorzugsweise 0,1 bis 5 % |
| festes Trägermittel | 99,9 bis 90 %, vorzugsweise 99,9 bis 99 % |

### Suspensions-Konzentrate:

| | |
|---|---|
| Aktives Wirkstoffgemisch | 5 bis 75 %, vorzugsweise 10 bis 50 % |
| Wasser | 94 bis 24 %, vorzugsweise 88 bis 30 % |
| oberflächenaktives Mittel | 1 bis 40 %, vorzugsweise 2 bis 30 % |

### Benetzbare Pulver:

| | |
|---|---|
| Aktives Wirkstoffgemisch | 0,5 bis 90 %, vorzugsweise 1 bis 80 % |
| oberflächenaktives Mittel | 0,5 bis 20 %, vorzugsweise-1 bis 15 % |
| festes Trägermaterial | 5 bis 95 %, vorzugsweise 15 bis 90 % |

### Granulate:

| | |
|---|---|
| Aktives Wirkstoffgemisch | 0,1 bis 30 %, vorzugsweise 0,1 bis 15 % |
| festes Trägermittel | 99,5 bis 70 %, vorzugsweise 97 bis 85 % |

Die folgenden Beispiele erläutern die Erfindung weiter, ohne sie zu beschränken.
Formulierungsbeispiele für Mischungen aus Herbiziden der Formel I und Safener der Formel X, XI, XII, XIII, XIV, XV, XVI, XVII, XVIII, XIX, XX, XXI, XXII, XXIII, XXIV, XXV, XXVI, XXVII, XXVIII oder XXIX (% = Gewichtsprozent)

| F1. Emulsionskonzentrate | a) | b) | c) | d) |
|---|---|---|---|---|
| Wirkstoffgemisch | 5 % | 10 % | 25 % | 50 % |
| Ca-Dodecylbenzolsulfonat | 6 % | 8 % | 6 % | 8 % |
| Ricinusöl-polyglykolether | 4 % | - | 4 % | 4 % |
| (36 Mol EO) | | | | |
| Octylphenol-polyglykolether | - | 4 % | - | 2 % |
| (7-8 Mol EO) | | | | |
| Cyclohexanon | - | - | 10 % | 20 % |
| Arom. Kohlenwasserstoffgemisch C₉-C₁₂ | 85 % | 78 % | 55 % | 16 % |

Aus solchen Konzentraten können durch Verdünnung mit Wasser Emulsionen jeder gewünschten Konzentration hergestellt werden.

| F2. Lösungen | a) | b) | c) | d) |
|---|---|---|---|---|
| Wirkstoffgemisch | 5 % | 10 % | 50 % | 90 % |
| 1-Methoxy-3-(3-methoxypropoxy)-propan | - | 20 % | 20 % | - |
| Polyethylenglykol MG 400 | 20 % | 10 % | - | - |
| N-Methyl-2-pyrrolidon | - | - | 30 % | 10 % |
| Arom. Kohlenwasserstoffgemisch C₉-C₁₂ | 75 % | 60 % | - | - |

Die Lösungen sind zur Anwendung in Form kleinster Tropfen geeignet.

| F3. Spritzpulver | a) | b) | c) | d) |
|---|---|---|---|---|
| Wirkstoffgemisch | 5 % | 25 % | 50 % | 80 % |
| Na-Ligninsulfonat | 4 % | - | 3 % | - |
| Na-Laurylsulfat | 2 % | 3 % | - | 4 % |
| Na-Diisobutyl-naphthalinsulfonat | - | 6 % | 5 % | 6 % |
| Octylphenol-polyglykolether (7-8 Mol EO) | - | 1 % | 2 % | - |
| Hochdisperse Kieselsäure | 1 % | 3 % | 5 % | 10 % |
| Kaolin | 88 % | 62 % | 35 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F4. Umhüllungs-Granulate | a) | b) | c) |
|---|---|---|---|
| Wirkstoffgemisch | 0.1 % | 5 % | 15 % |
| Hochdisperse Kieselsäure | 0.9 % | 2 % | 2 % |
| Anorg. Trägermaterial (Æ 0.1 - 1 mm) | 99.0 % | 93 % | 83 % |

wie z.B. CaCO₃ oder SiO₂

Der Wirkstoff wird in Methylenchlorid gelöst, auf den Träger aufgesprüht und das Lösungsmittel anschließend im Vakuum abgedampft.

| F5. Umhüllungs-Granulate | a) | b) | c) |
|---|---|---|---|
| Wirkstoffgemisch | 0.1 % | 5 % | 15 % |
| Polyethylenglykol MG 200 | 1.0 % | 2 % | 3 % |
| Hochdisperse Kieselsäure | 0.9 % | 1 % | 2 % |
| Anorg. Trägermaterial (Æ 0.1 - 1 mm) | 98.0 % | 92 % | 80 % |

wie z.B. CaCO₃ oder SiO₂

Der fein gemahlene Wirkstoff wird in einem Mischer auf das mit Polyethylenglykol angefeuchtete Trägermaterial gleichmäßig aufgetragen. Auf diese Weise erhält man staubfreie Umhüllungs-Granutate.

| F6. Extruder-Granulate | a) | b) | c) | d) |
|---|---|---|---|---|
| Wirkstoffgemisch | 0.1 % | 3 % | 5 % | 15 % |
| Na-Ligninsulfonat | 1.5 % | 2 % | 3 % | 4 % |
| Carboxymethylcellulose | 1.4 % | 2 % | 2 % | 2 % |
| Kaolin | 97.0 % | 93 % | 90 % | 79 % |

Der Wirkstoff wird mit den Zusatzstoffen vermischt, vermahlen und mit Wasser angefeuchtet. Dieses Gemisch wird extrudiert und anschließend im Luftstrom getrocknet.

| F7. Stäubemittel | a) | b) | c) |
|---|---|---|---|
| Wirkstoffgemisch | 0.1 % | 1 % | 5 % |
| Talkum | 39.9% | 49 % | 35 % |
| Kaolin | 60.0 % | 50 % | 60 % |

Man erhält anwendungsfertige Stäubemittel, indem der Wirkstoff mit den Trägerstoffen vermischt und auf einer geeigneten Mühle vermahlen wird.

| F8. Suspensions-Konzentrate | a) | b) | c) | d) |
|---|---|---|---|---|
| Wirkstoffgemisch | 3 % | 10 % | 25 % | 50 % |
| Ethylenglykol | 5 % | 5 % | 5 % | 5 % |
| Nonylphenol-polyglykolether (15 Mol EO) | - | 1 % | 2 % | - |
| Na-Ligninsulfonat | 3 % | 3 % | 4 % | 5 % |
| Carboxymethylcellulose | 1 % | 1 % | 1 % | 1 % |
| 37%ige wäßrige Formaldehyd-Lösung | 0.2 % | 0.2 % | 0.2 % | 0.2 % |
| Silikonöl-Emulsion | 0.8 % | 0.8 % | 0.8 % | 0.8 % |
| Wasser | 87 % | 79 % | 62 % | 38 % |

Der feingemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Es ist oft praktischer, den Wirkstoff der Formel I und den Mischungspartner der Formel X, XI, XII, XIII, XIV, XV, XVI, XVII, XVIII, XIX, XX, XXI, XXII, XXIII, XXIV, XXV, XXVI, XXVII, XXVIII oder XXIX einzeln zu formulieren und sie dann kurz vor dem Ausbringen im Applikator im gewünschten Mischungsverhältnis als "Tankmischung" im Wasser zusammenzubringen.

Die Fähigkeit der Safener der Formel X, XI, XII, XIII, XIV, XV, XVI, XVII, XVIII, XIX, XX, XXI, XXII, XXIII, XXIV, XXV, XXVI, XXVII, XXVIII oder XXIX Kulturpflanzen vor der phytotoxischen Wirkung von Herbiziden der Formel I zu schützen, wird in den folgenden Beispielen veranschaulicht.

### Biologisches Beispiel 1: Safeningwirkung

Unter Gewächshausbedingungen werden die Testpflanzen in Kunstofftöpfen bis zum 4-Blattstadium angezogen. In diesem Stadium werden zum einen das Herbizid allein, als auch die Mischungen des Herbizids mit den als Safener zu prüfenden Testsubstanzen auf die Testpflanzen appliziert. Die Applikation erfolgt als wäßrige Suspension der Prüfsubstanzen, hergestellt aus einem 25 %igen Spritzpulver (Beispiel F3, b)), mit 500 I Wasser/ha. 2 bis 3 Wochen nach Applikation wird die phytotoxische Wirkung des Herbizids auf die Kulturpflanzen wie z.B. Mais und Getreide mit einer Prozentskala ausgewertet. 100 % bedeutet Testpflanze ist abgestorben, 0 % bedeutet keine phytotoxische Wirkung.

Die in diesem Versuch erhaltenen Resultate zeigen, daß mit den Verbindungen der Formel X, XI, XII, XIII, XIV, XV, XVI, XVII, XVIII, XIX, XX, XXI, XXII, XXIII, XXIV, XXV, XXVI, XXVII, XXVIII oder XXIX die durch das Herbizid der Formel I verursachten Schädigungen der Kulturpflanze deutlich reduziert werden können. Beispiele für diese Resultate sind in der folgenden Tabelle B5 angegeben:

**Tabelle B5:**

| Postemergente Wirkung einer erfindungsgemäßen Mischung aus Herbizid und Safener: | | |
|---|---|---|
| Testpflanze | Verb. Nr. 1.01 (60 g/ha) | Verb. Nr. 1.01 (60 g/ha) + Verb. Nr. 11.03 (15 g/ha) |
| Gerste | 20 | 0 |
| Agrostis | 70 | 70 |
| Alopecurus | 70 | 80 |
| Lolium | 70 | 70 |

Tabelle 5 läßt sich entnehmen, daß die Verbindung Nr. 1.01 auf Gerste eine nicht tolerierbare phytotoxische Wirkung von 20 % zeigt. Die Unkräuter Agrostis, Alopecurus und Lolium werden zufriedenstellend kontrolliert.

Im Gegensatz dazu zeigt die erfindungsgemäße Mischung bestehend aus dem Herbizid Nr. 1.01 und dem Safener Nr. 11.03 auf der Kulturpflanze keinerlei phytotoxische Wirkung. Dabei bleibt die herbizide Wirkung auf die Unkräuter nicht nur gleich, sondern wird überraschenderweise bei Alopecurus sogar noch verstärkt (80 % gegenüber 70 % bei der Applikation des Herbizids Nr. 1.01 allein).

Dieselben Resultate werden erhalten, wenn man die Mischungen gemäß den Beispielen F1, F2 und F4 bis F8 formuliert.

Die Verbindung der Formel I läßt sich mit Vorteil mit einer Reihe von weiteren bekannten Herbiziden mischen. Man erhält dadurch beispielsweise eine wesentliche Verbreiterung des Unkrautspektrums und in vielen Fällen auch eine Erhöhung der Selektivität bezüglich der Nutzpflanzen. Insbesondere sind die Mischungen der Verbindung der Formel I mit mindestens einem der folgenden Herbizide von Bedeutung:
Herbizide aus der Klasse der Phenoxy-phenoxypropionsäuren wie beispielsweise Diclofopmethyl, Fluazifop-P-butyl- Quizalafop-P-ethyl, Propaquizafop, Clodinafop-P-propargyl, Cyhalfop-butyl, Fenoxaprop-P-Ethyl, Haloxyfop-methyl oder Haloxyfop-etoethyl;
Herbizide aus der Klasse der Hydroxylamine wie z.B. Sethoxidim, Alloxydim, Clethodim, Cycloxydim, Tepralkoxydim, Tralkoxydim oder Butroxidim;
Herbizide aus der Klasse der Sulfonylharnstoffe, wie z.b. Amidosulfuron, Azimsulfuron, Bensulfuron-methyl, Chlorimuron-ethyl, Cinosulfuron, Chlorsulfuron, Chlorimuron, Cyclosulfamuron, Ethametsulfuron-methyl, Ethoxysulfuron, Fluazasulfuron, Flupyrsulfuron, Imazosulfuron, Iodosulfuron (CAS RN 144550-36-7 und 185119-76-0), Metsulfuron-methyl, Nicosulfuron, Oxasulfuron, Primisulfuron, Pyrazosulfuron-ethyl, Sulfosulfuron, Rimsulfuron, Thifensulfuron-methyl, Triasulfuron, Tribenuron-mehtyl, Triflusulfuron-methyl, Prosulfuron, Flucarbazon oder Tritosulfuron (CAS RN 142469-14-5);
Herbizide aus der Klasse der Imidazolinone, wie Imazethapyr, Imazamethabenz, Imazamethapyr, Imazaquin, Imazamox oder Imazapyr;
Herbizide aus der Klasse der Pyrimidine, wie Pyrithiobac-sodium, Pyriminobac, Bispyribacsodium;
Herbizide aus der Klasse der Triazine, wie z.B. Atrazin, Simazin, Simethryne, Terbutryne, Terbuthylazine;
Herbizide aus der Klasse der Harnstoffe, wie Isoproturon, Chlortoluron, Diuron, Dymron, Fluometuron, Linuron oder Methabenzthiazuron;
Herbizide aus der Klasse der Phosphonsäurederivate, wie z.B. Glyphosate, Glufosinate, Sulfosate oder Phosphinothricin;
Herbizide aus der Klasse der PPO, wie z.B. Nitrofen, Bifenox, Acifluorfen, Lactofen, Oxyfluorfen, Ethoxyfen, Fluoroglycofen, Fomesafen, Halosafen, Azafenidin (CAS RN. - 68049-83-2), Benzfendizone (CAS RN 158755-95-4), Butafenacil (bekannt aus US-A-5,183,492,
   CAS RN 158755-95-4), Carfentrazone-ethyl, Cinidon-ethyl (CAS RN 142891-20-1), Flumichlorac-pentyl, Flumioxazin, Fluthiacet-methyl, Oxadiargyl, Oxadiazon, Pentoxazon, Sulfentrazone, Fluazolate (CAS RN 174514-07-9) oder Pyraflufen-ethyl;
Herbizide aus der Klasse der Chloracetanilide wie z.B. Alachlor, Acetochlor, Butachlor, Dimethachlor, Dimethenamid, S-Dimethenamid, Metazachlor, Metolachlor, S-Metolachlor, Pretilachlor, Propachlor, Propisochlor, Thenylchlor oder Pethoamid (CAS RN 106700-29-2)
Herbizide aus der Klasse der Phenoxyessigsäuren wie z.B. 2,4-D, Fluroxypyr, MCPA, MCPP, MCPB, Trichlorpyr oder Mecropop-P;
Herbizide aus der Klasse der Triazinone wie z.B. Hexazinon, Metamitron oder Metribuzin;
Herbizide aus der Klasse der Dinitroaniline wie z.B. Oryzalin, Pendimethalin oder Trifluralin;
Herbizide aus der Klasse der Azinone wie z.B. Chloridazon oder Norflurazon;
Herbizide aus der Klasse der Carbamate, wie z.B. Chlorpropham, Desmedipham, Phenmedipham oder Propham;
Herbizide aus der Klasse der Oxyacetamide wie z.B. Mefenacet oder Fluthiacet;
Herbizide aus der Klasse der Thiolcarbamate wie z.B. Butylate, Cycloate, Diallate, EPTC, Esprocarb, Molinate, Prosulfocarb, Thiobencarb oder Triallate;
Herbizide aus der Klasse der Azoloharnstoffe wie z.B. Fentrazamide (CAS RN158237-07-1) oder Cafenstrole;
Herbizide aus der Klasse der Benzoesäuren wie z.B. Dicamba oder Picloram;
Herbizide aus der Klasse der Anilide wie z.B. Diflufenican, oder Propanil;
Herbizide aus der Klasse der Nitrile wie z.B. Bromoxynil, Dichlobenil oder loxynil;
Herbizide aus der Klasse der Trione wie z.B. Sulcotrione, Mesotrione (bekannt aus US-A-5,006,158), Isoxaflutole oder Isoxachlortole;
Herbizide aus der Klasse der Sulfonamide wie z.B. Flucarbazone (CAS RN 181274-17-9), Procarbazone (CAS RN 145026-81-9), Chlorasulam, Diclosulam (CAS RN 145701-21-9), Florasulam, Flumetsulam oder Metosulam;
sowie Amitrol, Benfuresate, Bentazone, Cinmethylin, Clomazone, Chlopyralid, Difenzoquat, Dithiopyr, Ethofumesate, Flurochloridone, Indanofane, Isoxaben, Oxaziclomefone, Pyridate, Pyridafol (CAS RN. 40020-01-7), Quinchlorac, Quinmerac, Tridiphane oder Flamprop.

Wenn nicht anders angegeben, sind die oben angegebenen Mischungspartner der Verbindung der Formel I aus The Pesticide Manual, Eleventh Edition, 1997, BCPC bekannt. Die Mischungspartner der Verbindung der Formel I können gegebenenfalls auch in Form von Estern oder Salzen vorliegen, wie sie z. B. in The Pesticide Manual, Eleventh Edition, 1997, BCPC, genannt sind.

Die folgenden Beispiele erläutern die Erfindung weiter.

### Herstellungsbeispiele:

### Beispiel H1: Herstellung von

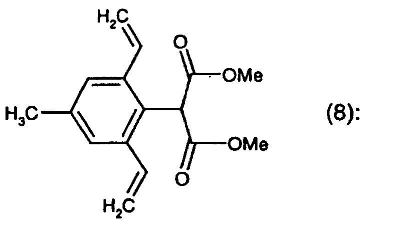

Zu einer Lösung aus 20 g 2-(2,6-Dibrom-4-methyl-phenyl)-malonsäuredimethylester (52,6 mmol) in 400 ml Toluol (3 x entgast, Vakuum/Argon) gibt man zuerst 36,7 g (0,116 mol) Tributylvinylstannan und anschließend 2 g Tetrakis-Triphenylphosphin-Palladium hinzu. Dann wird die Reaktionsmischung für 9 Stunden bei einer Temperatur von 90 bis 95 °C gerührt. Nach Filtration über Hyflo und Einengen am Rotationsverdampfer erhält man nach chromatographischer Reinigung 15,3 g **(8)** in Form eines gelben Öles, welches ohne weitere Reinigung in die nächste Reaktion eingesetzt wird.

### Beispiel H2:

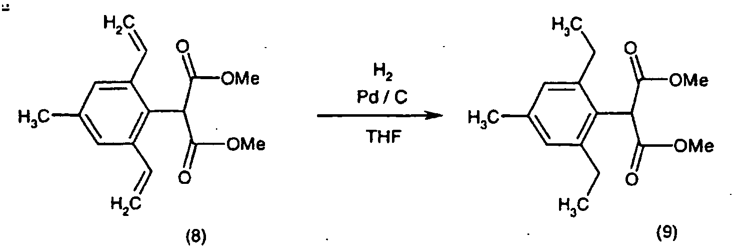

15,2 g der gemäß Beispiel H1 erhaltenen Verbindung **(8)** wird mit Wasserstoff über einen Palladiumkatalysator (Kohlenstoff als Träger, 7 g 5% Pd/C) in 160 ml Tetrahydrofuran bei einer Temperatur von 20 bis 25°C hydriert. Nach Beendigung der Hydrierung wird das Produkt über Hyflo filtriert und das erhaltene Filtrat wird am Rotationsverdampfer eingeengt. Man erhält 13,7 g **(9)** in Form gelber Kristalle mit einem Schmelzpunkt von 47 bis 49 °C.

### Beispiel H3:

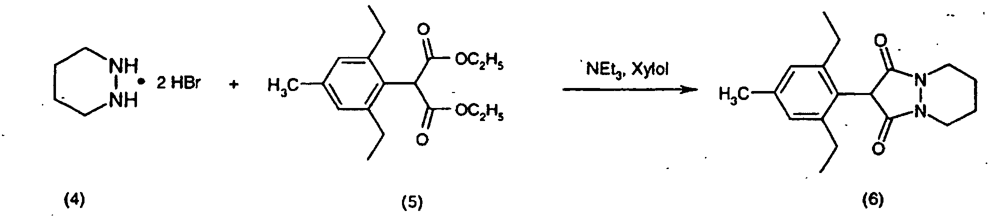

Zu einer Suspension von 40 g (0,15 mol) **(4)** in 1000 ml Xylol gibt man 71,8 g (0,71 mol) Triethylamin hinzu und entgast (4 x Vakuum/Argon). Anschließend wird die gelbe Suspension auf eine Temperatur von 60 °C erwärmt und 3 Stunden lang gerührt. Dann gibt man 42,5 g (0,15 mol) **(5)** hinzu und heizt auf eine Badtemperatur von 150 °C auf, um laufend überschüssiges Triethylamin und das entstandene Ethanol abzudestillieren. Nach 3 Stunden kühlt man die Reaktionsmischung auf eine Temperatur von 40 °C ab und gibt sie in 500 ml eines Eis/Wasser-Gemisches. Mit 100 ml wäßriger 1N-Natriumhydroxidlösung wird das Reaktionsgemisch alkalisch eingestellt und die wäßrige Phase (enthält das Produkt) zweimal mit Essigsäureethylester gewaschen. Nach zweimaligem Zurückwaschen der organischen Phase mit wäßriger 1N-Natriumhydroxidlösung werden die wäßrigen Phasen vereinigt, das verbliebene Xylol abdestilliert und die vereinigten wäßrigen Phasen mit 4 N HCl unter Kühlung auf pH 2-3 eingestellt. Das dabei ausfallende Produkt gibt man auf einen Saugfilter, wäscht den Filterrückstand mit Wasser und kurz mit Hexan und trocknet anschließend den Filterrückstand im Vakuum bei einer Temperatur von 60 °C über P₂O₅. Man erhält 34,6 g **(6)** als einen schwach beigen Feststoff mit einem Schmelzpunkt von 242-244 °C (Zers.).

### Beispiel H4:

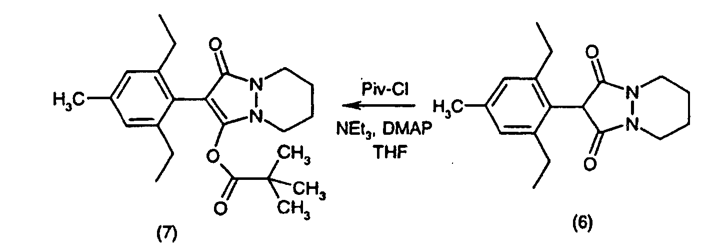

Zu einer auf eine Temperatur von 0 °C gekühlte Lösung aus 3 g (10,4 mmol) **(6)** und 1,6 g (15,8 mmol) Triethylamin in 100 ml Tetrahydrofuran gibt man eine katalytische Menge 4-Dimethylaminopyridin hinzu. Anschließend gibt man 1,57 g (13,0 mmol) Pivaloylchlorid tropfenweise hinzu. Nach 30-minütigem Rühren bei einer Temperatur von 0 °C entfernt man die Kühlung und rührt für weitere 60 Minuten. Anschließend gibt man das Reaktionsgemisch in gesättigte wäßrige Natriumchloridlösung und trennt die organische Phase ab. Die organische Phase wird über Magnesiumsulfat getrocknet, abfiltriert und eingedampft. Nach chromatographischer Reinigung und Umkristallisation aus Diethylether erhält man 2,94 g **(7)** mit einem Schmelzpunkt von 135 bis 136 °C.

### Beispiel H5: Herstellung von 2-(2,6-Diethyl-4-methyl-phenyl)-tetrahydro-pyrazolo[1,2-.a.]pyridazine-1,3-dion:

1,39 g Tetrahydro-pyrazolo[1,2-.a.]pyridazine-1,3-dion und 2,68 g Natriumtertiärbutylat werden in 20 ml Dimethylformamid bei 20° gelöst und mit 3,21 g 2,6-Diethyl-4-methyliodbenzol sowie 0.82 g Pd (TPP)₂ Cl₂ versetzt. Nun wird während 2,5 Stunden bei 125° gerührt. Nach dem Abkühlen auf Raumtemperatur wird mit 200 ml Essigester und 200 ml Ether versetzt und die Reaktionsmischung auf einen Saugfilter gegeben. Der Filterrückstand wird mit je 100 ml Wasser und Methylenchlorid versetzt und mit Salzsäure angesäuert. Die organische Phase wird abgetrennt, getrocknet und eingedampft. Der Rückstand (1.9 g) wird an Kieselgel chromatographiert (Essigester/Hexan 3:1). Man erhält 2-(2,6-Diethyl-4-methyl-phenyl)-tetrahydro-pyrazolo[1,2-.a.]pyridazine-1,3-dion in Form beiger Kristalle mit einem Schmelzpunkt von 174-175°.

### Beispiel H6: Herstellung von 2-(2,6-Diethyl-4-methyl-phenyl)-tetrahydro-pyrazolo[1,2-.a.]pyridazine-1,3-dion:

1,39 g Tetrahydro-pyrazolo[1,2-.a.]pyridazine-1,3-dion und 2,68 g Natriumtertiärbutylat werden in 20 ml Dimethylformamid bei 20° gelöst und mit 2,66 g 2,6-Diethyl-4-methylbrombenzol sowie 0.82 g Pd (TPP)₂ Cl₂ versetzt. Nun wird während 2,5 Stunden bei 125° gerührt. Nach dem Abkühlen auf Raumtemperatur wird mit 200 ml Essigester und 200 ml Ether versetzt und die Reaktionsmischung auf einen Saugfilter gegeben. Der Filterrückstand wird mit je 100 ml Wasser und Methylenchlorid versetzt und mit Salzsäure angesäuert. Die organische Phase wird abgetrennt, getrocknet und eingedampft. Der Rückstand (1.4 g) wird an Kieselgel chromatographiert (Essigester/Hexan 3:1). Man erhält 2-(2,6-Diethyl-4-methyl-phenyl)-tetrahydro-pyrazolo[1,2-.a.]pyridazine-1,3-dion in Form beiger Kristalle mit einem Schmelzpunkt von 174-175°.

In den folgenden Tabellen sind die Schmelzpunkte in °C angegeben. Me bedeutet die Methylgruppe. Ist für die Substituenten G₁ bis G₁₀ sowie R₄ und R₅ (unabhängig voneinander) eine Formel dargestellt, so ist die linke Seite dieser Formel der Verknüpfungspunkt mit dem Sauerstoffatom des Heterocyclus Q₁ bis Q₁₀. Bei der Substituentenbedeutung R₄ und R₅ gemeinsam stellt die rechte Seite des Moleküls die Verknüpfungsstelle mit dem Heterocyclus Q₁ dar. Die übrigen endständigen Valenzen stellen Methylgruppen dar.

In den folgenden Tabellen bedeutet "LC/MS: M+" das positiv geladene molekulare Ion in daltons exprimiert, das aus dem Massenspektrum eruiert wurde bei der Analyse des Produktes mit gekoppelten HPLC (High Performance Liquid Chromatography) und MS (Mass Spectrometry) Geräten.

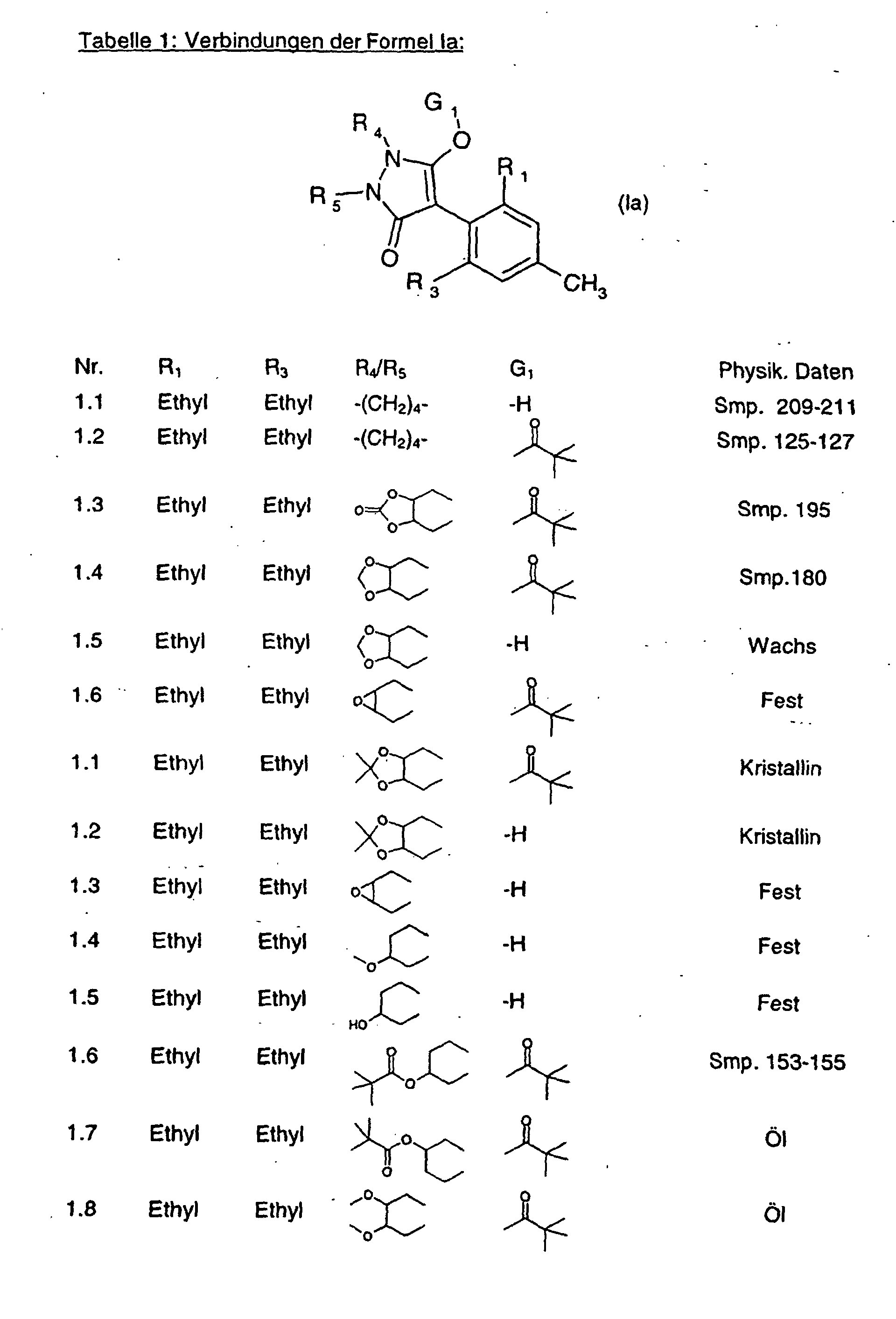

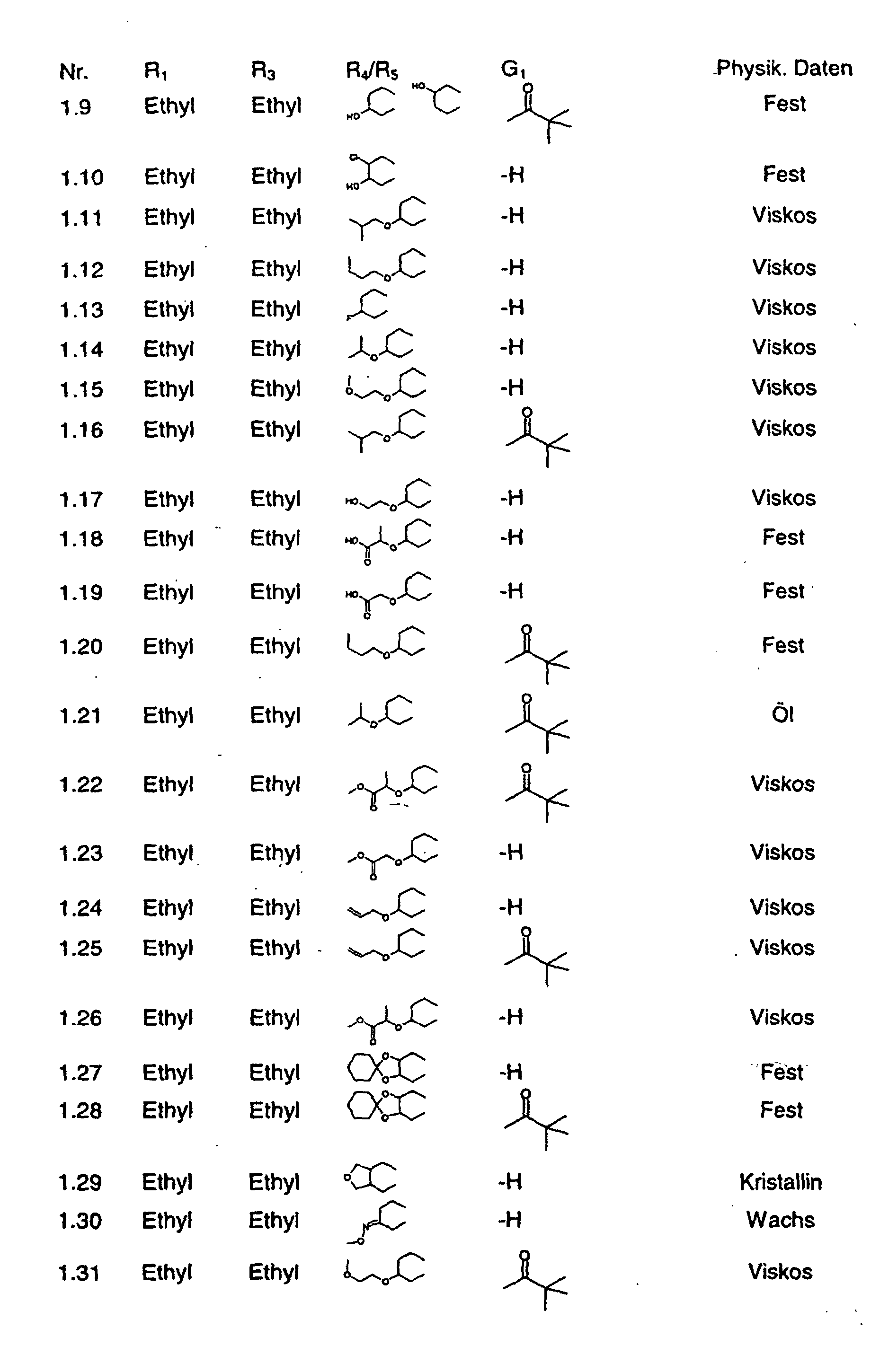

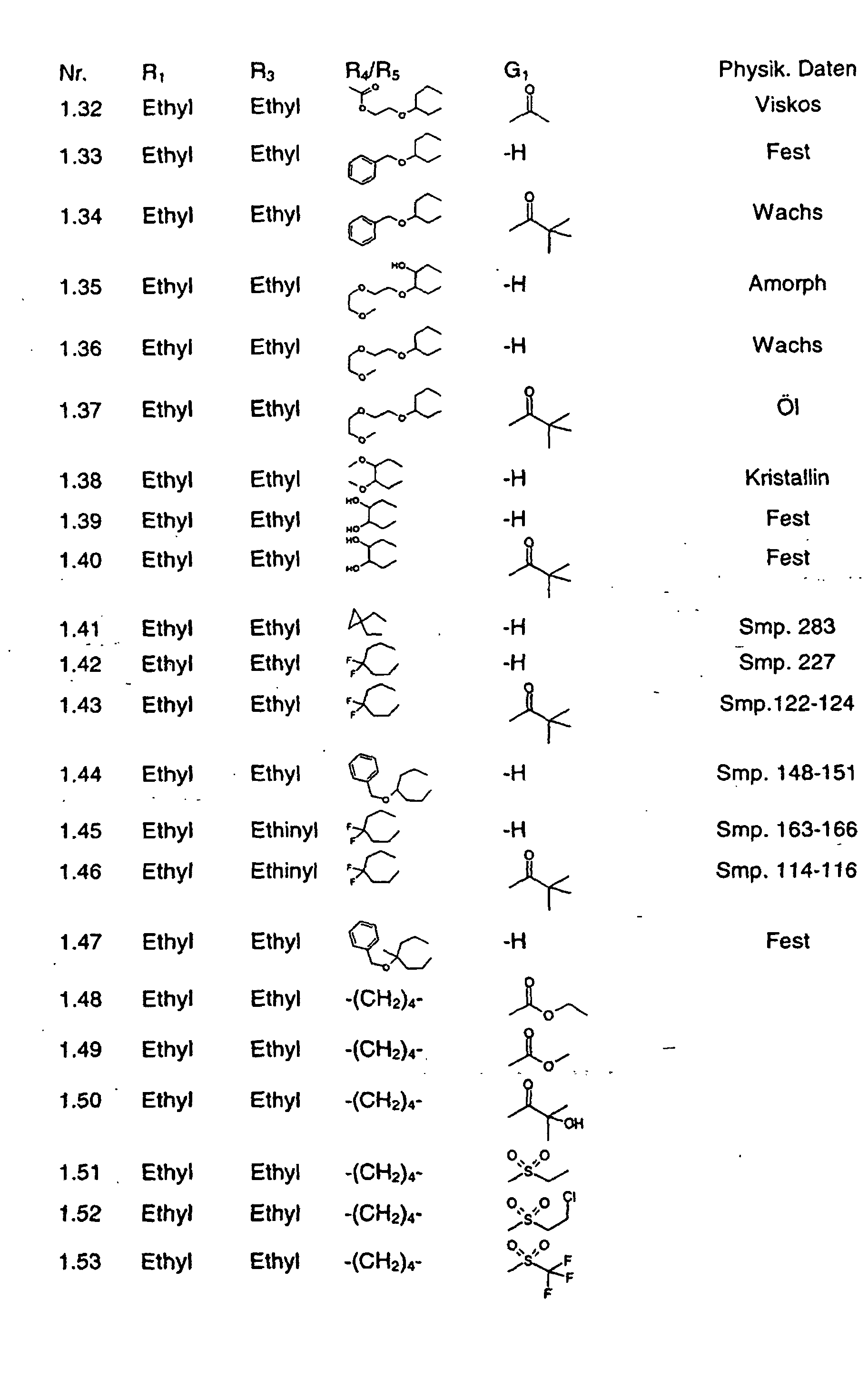

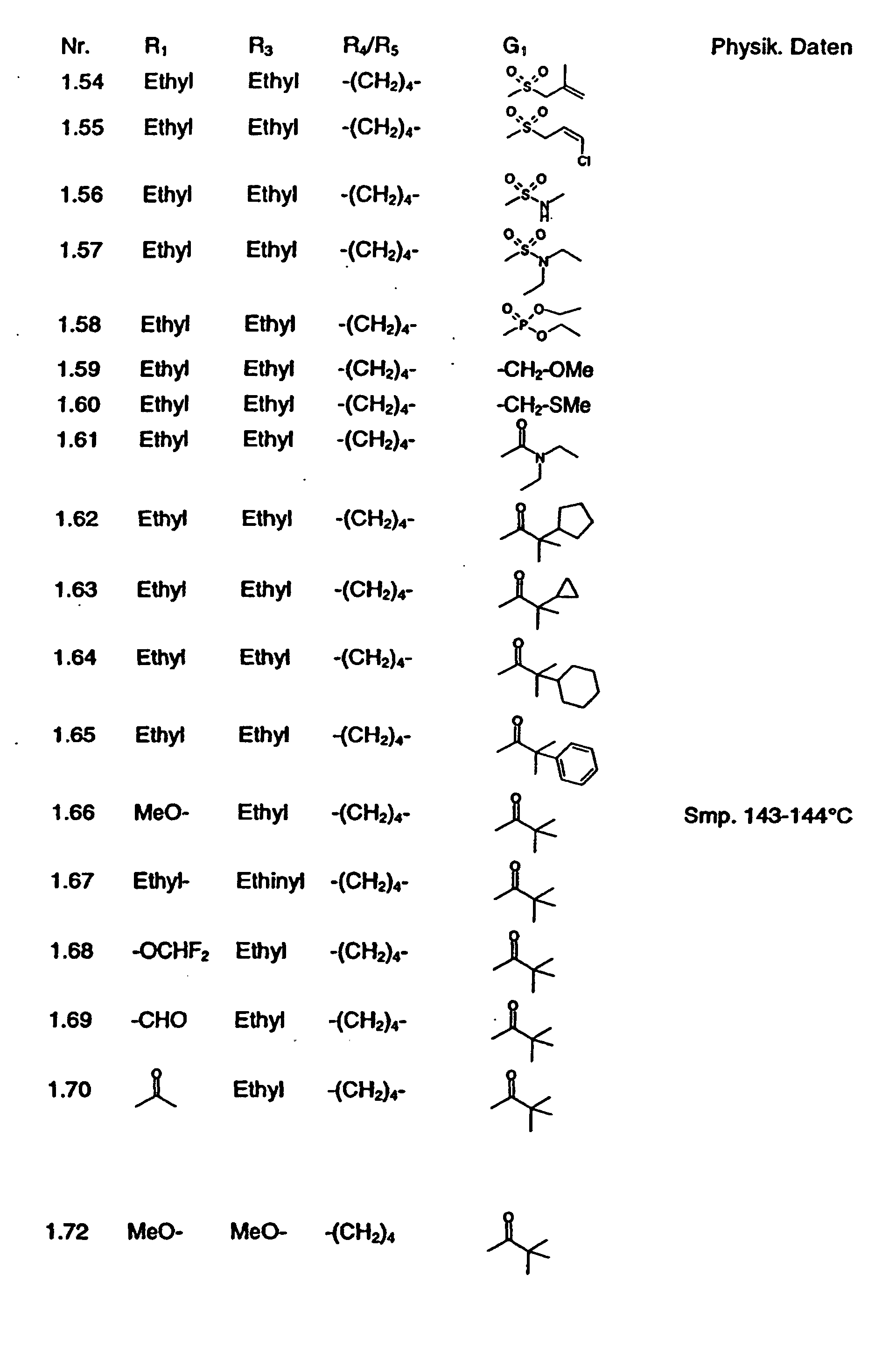

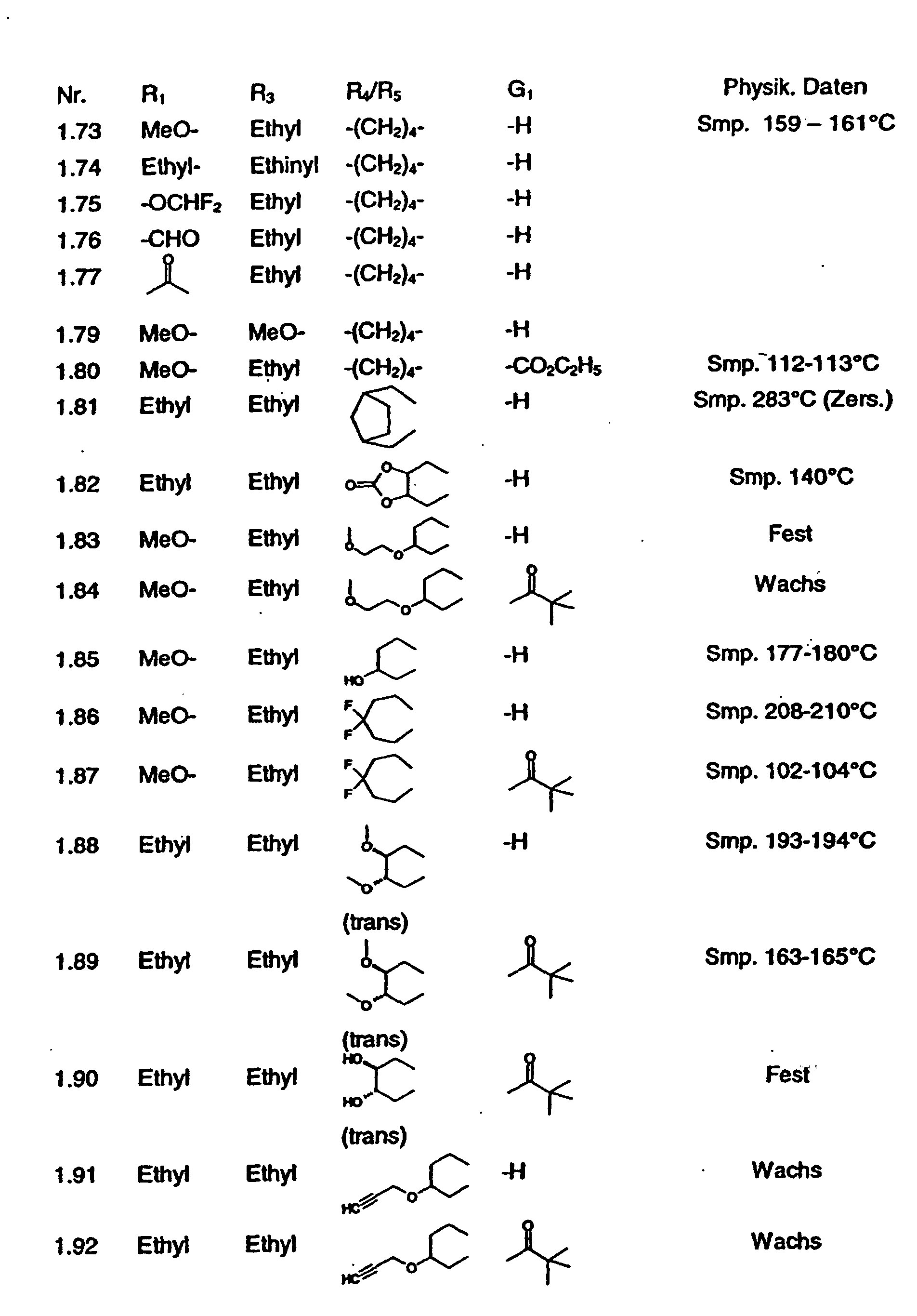

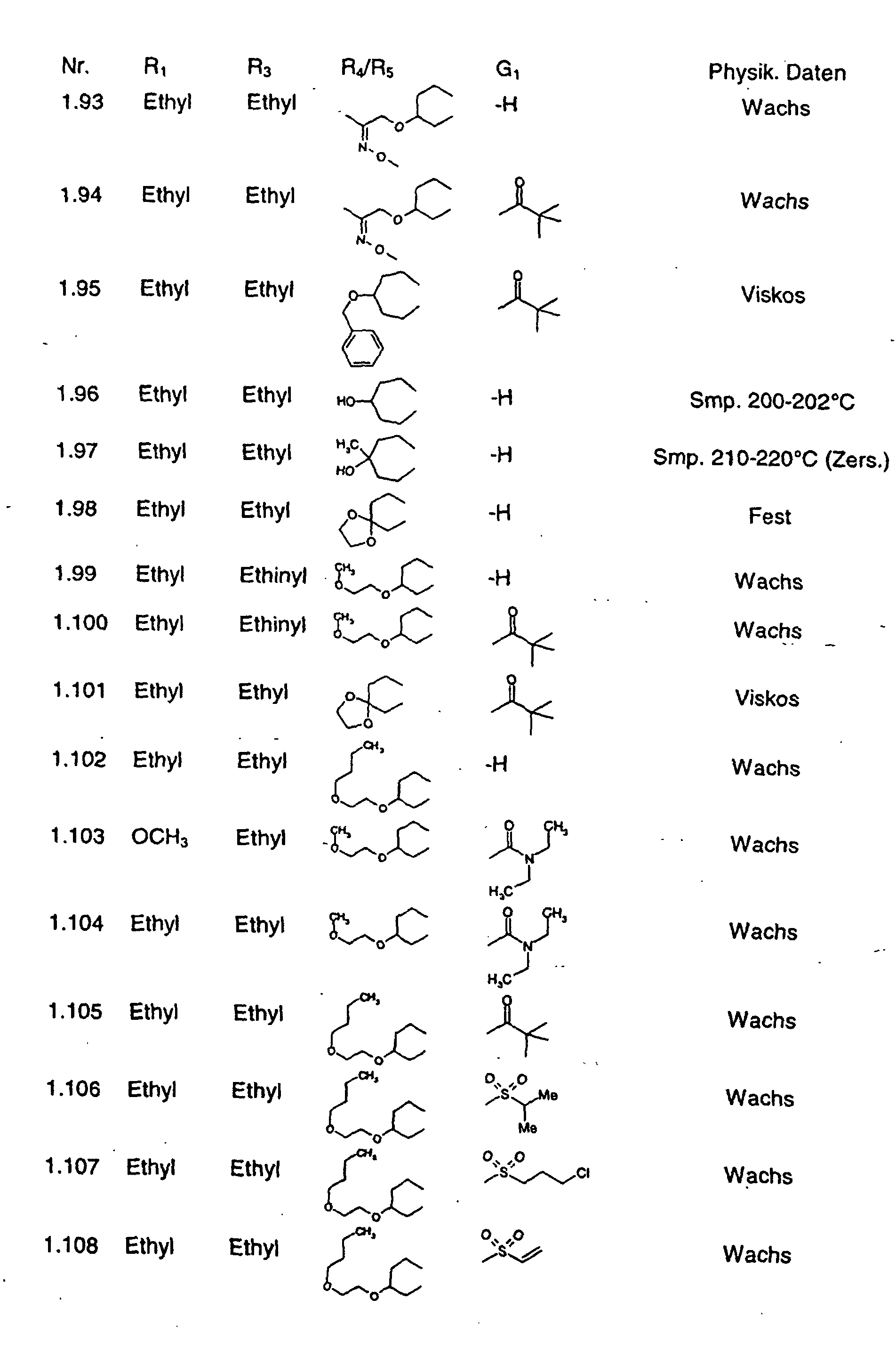

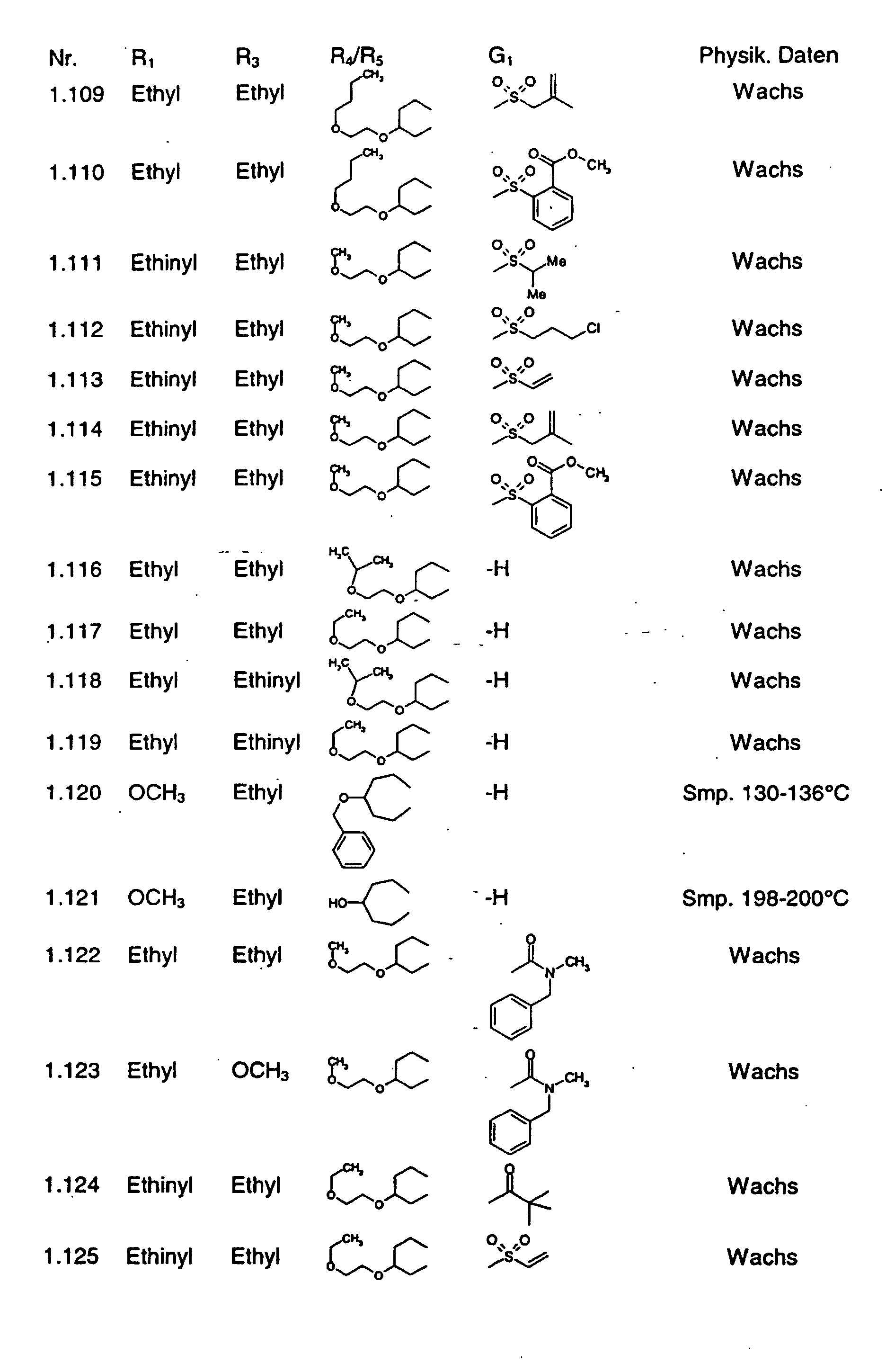

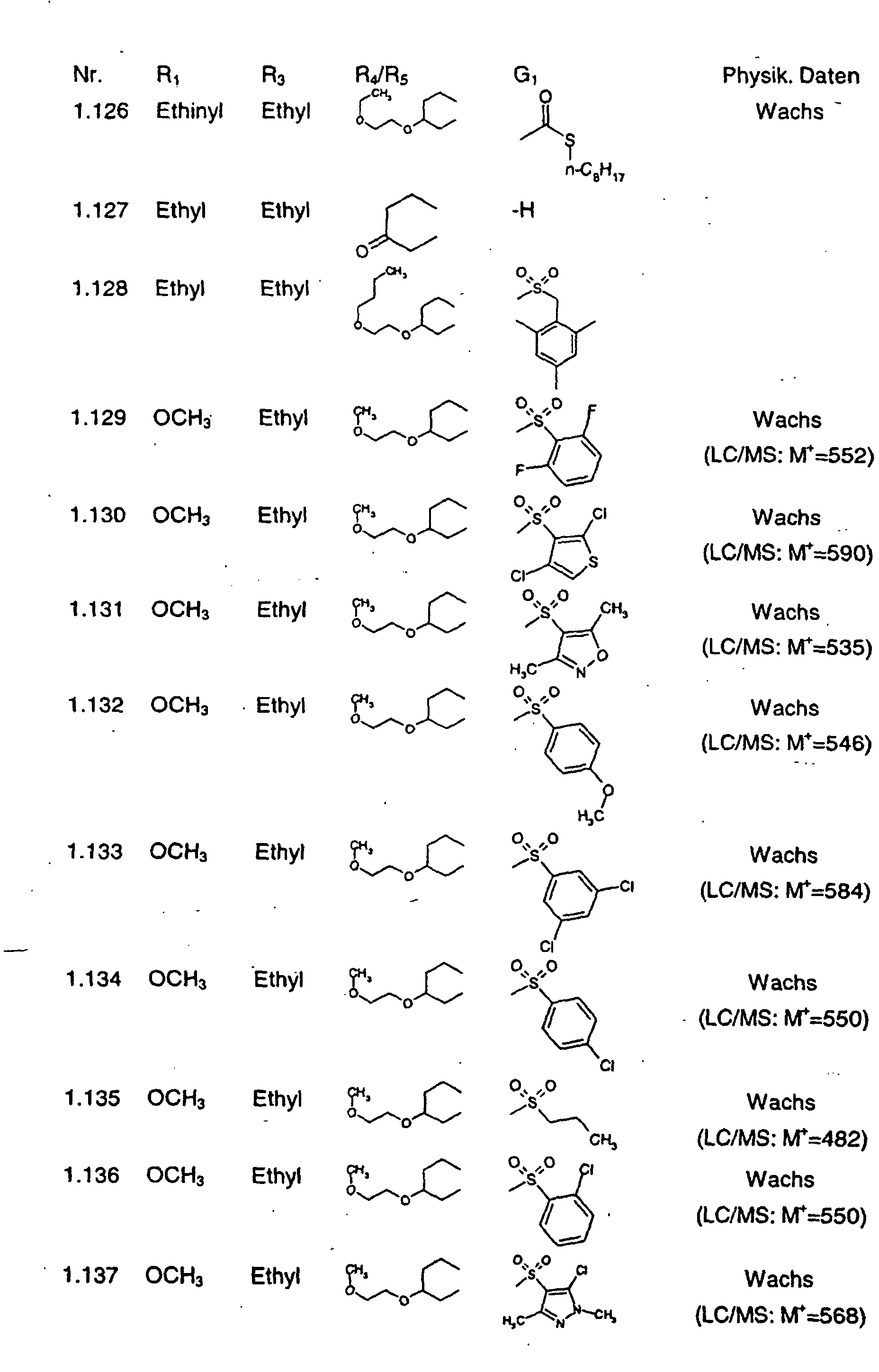

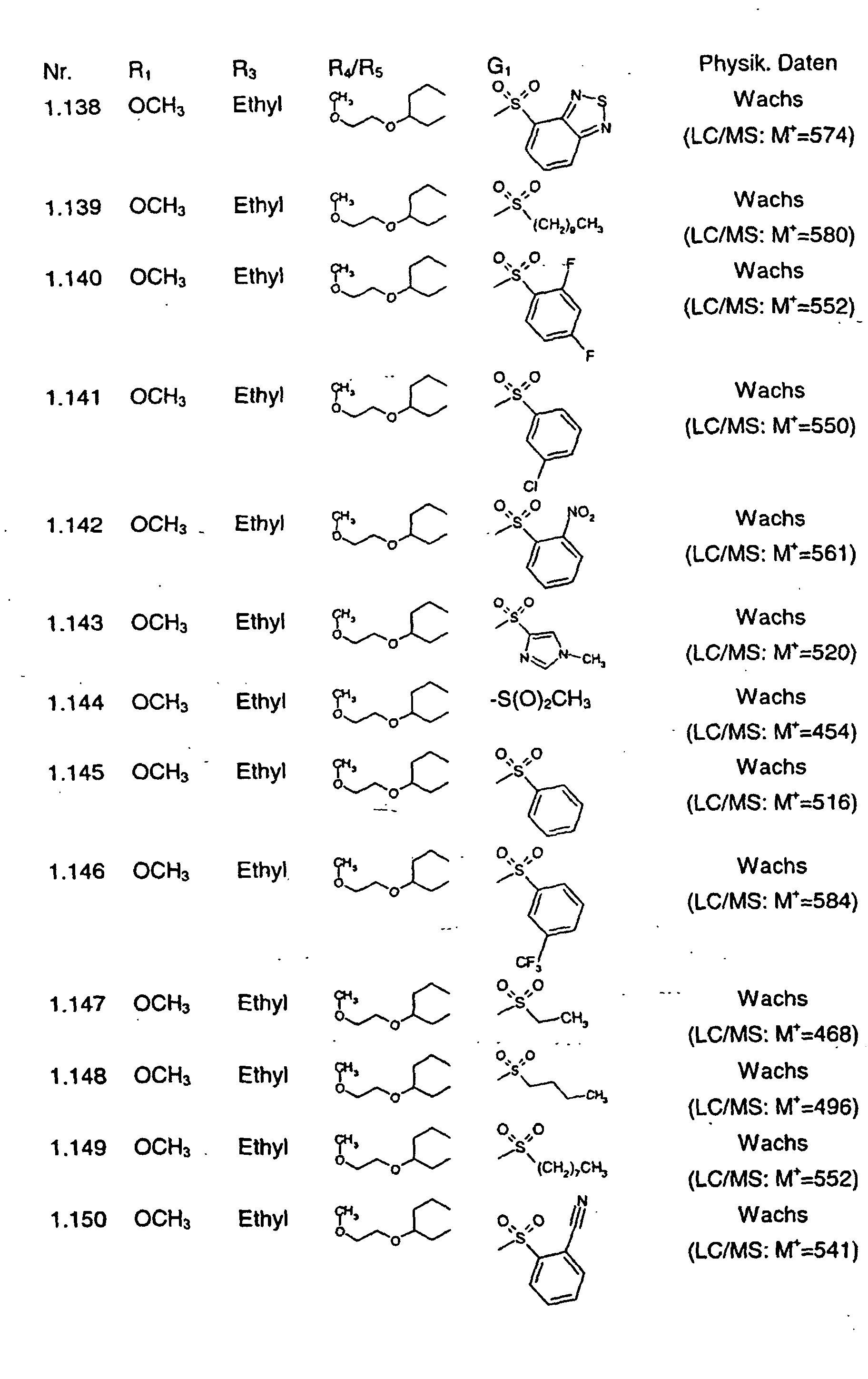

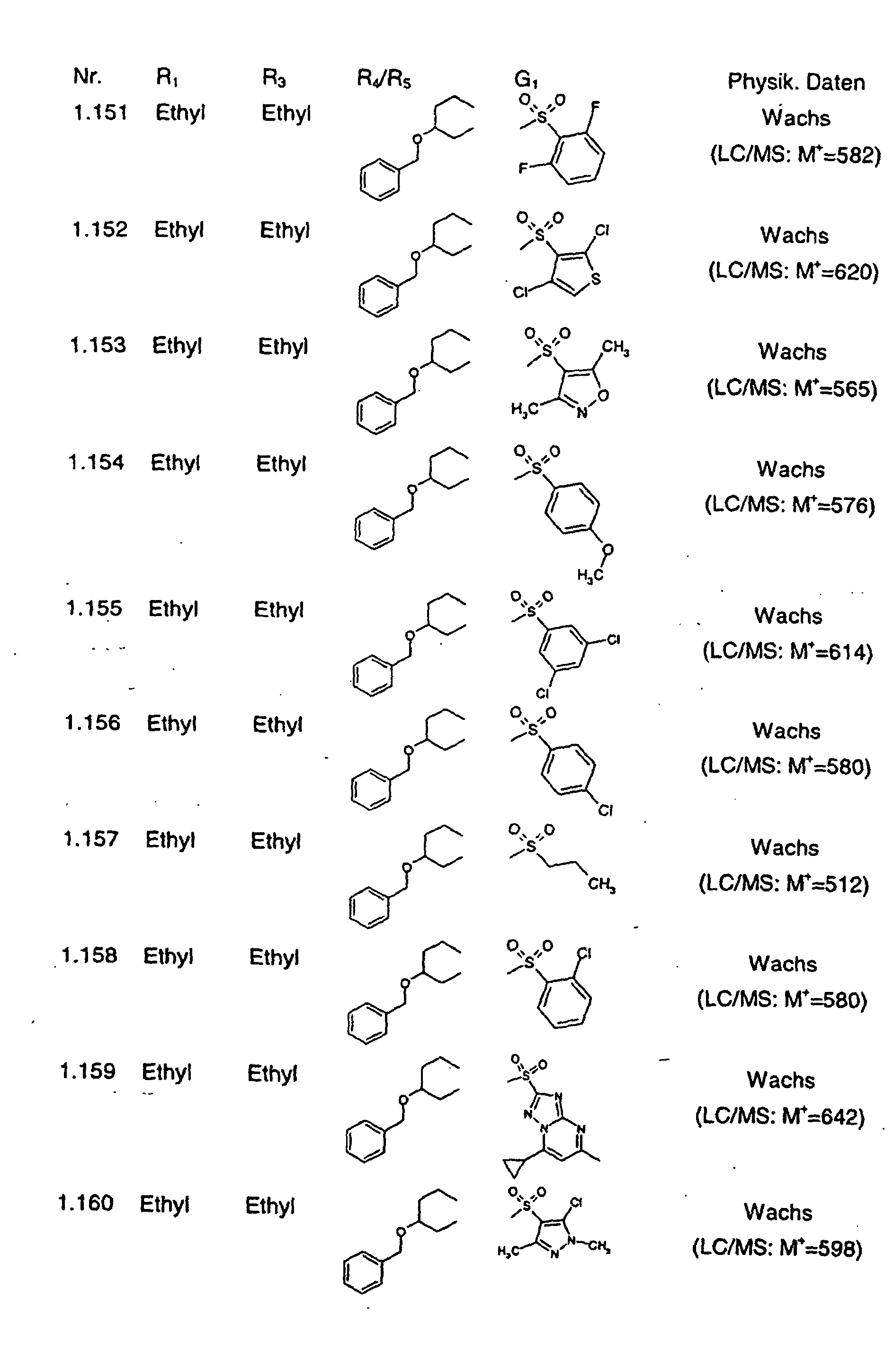

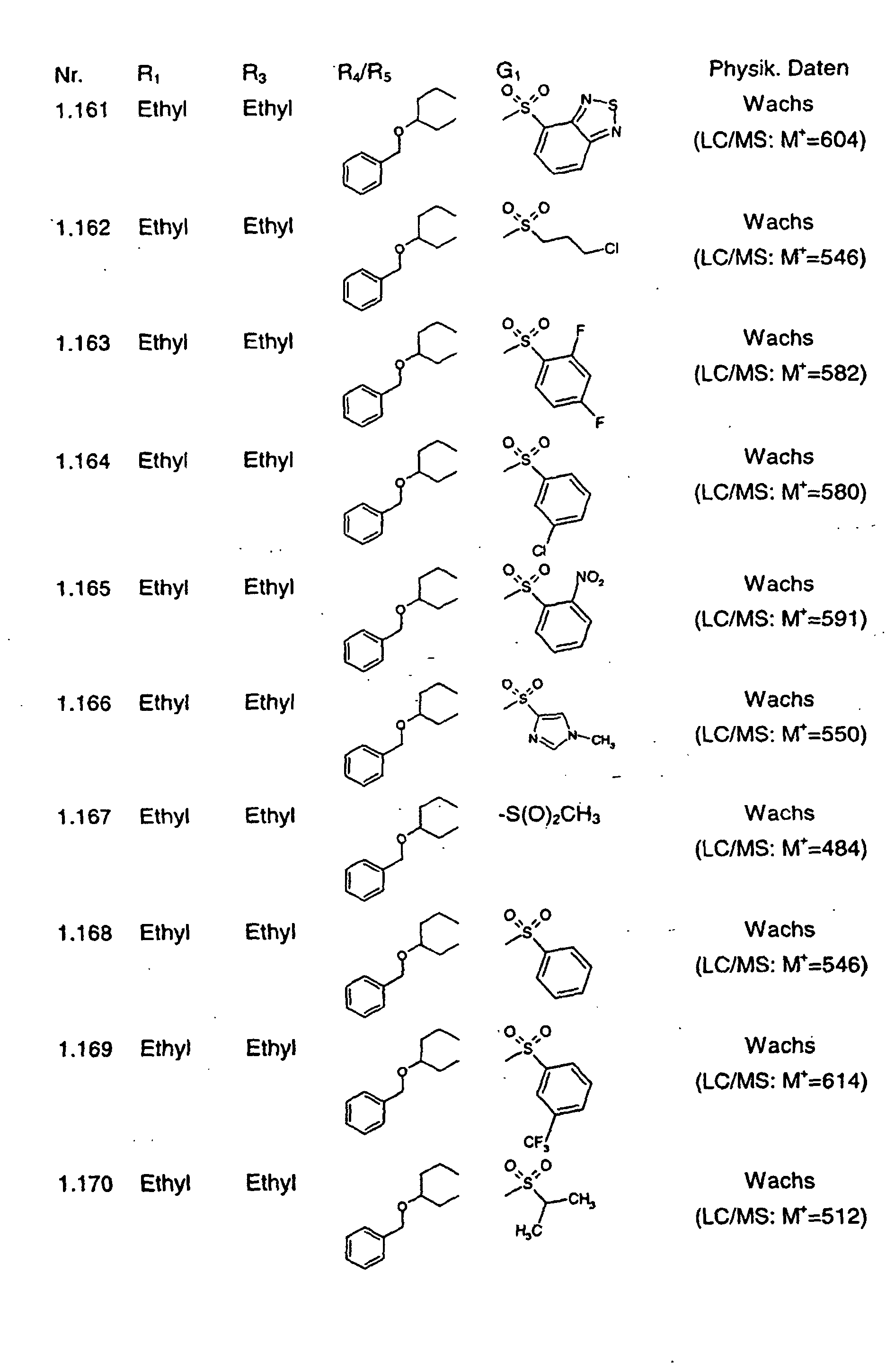

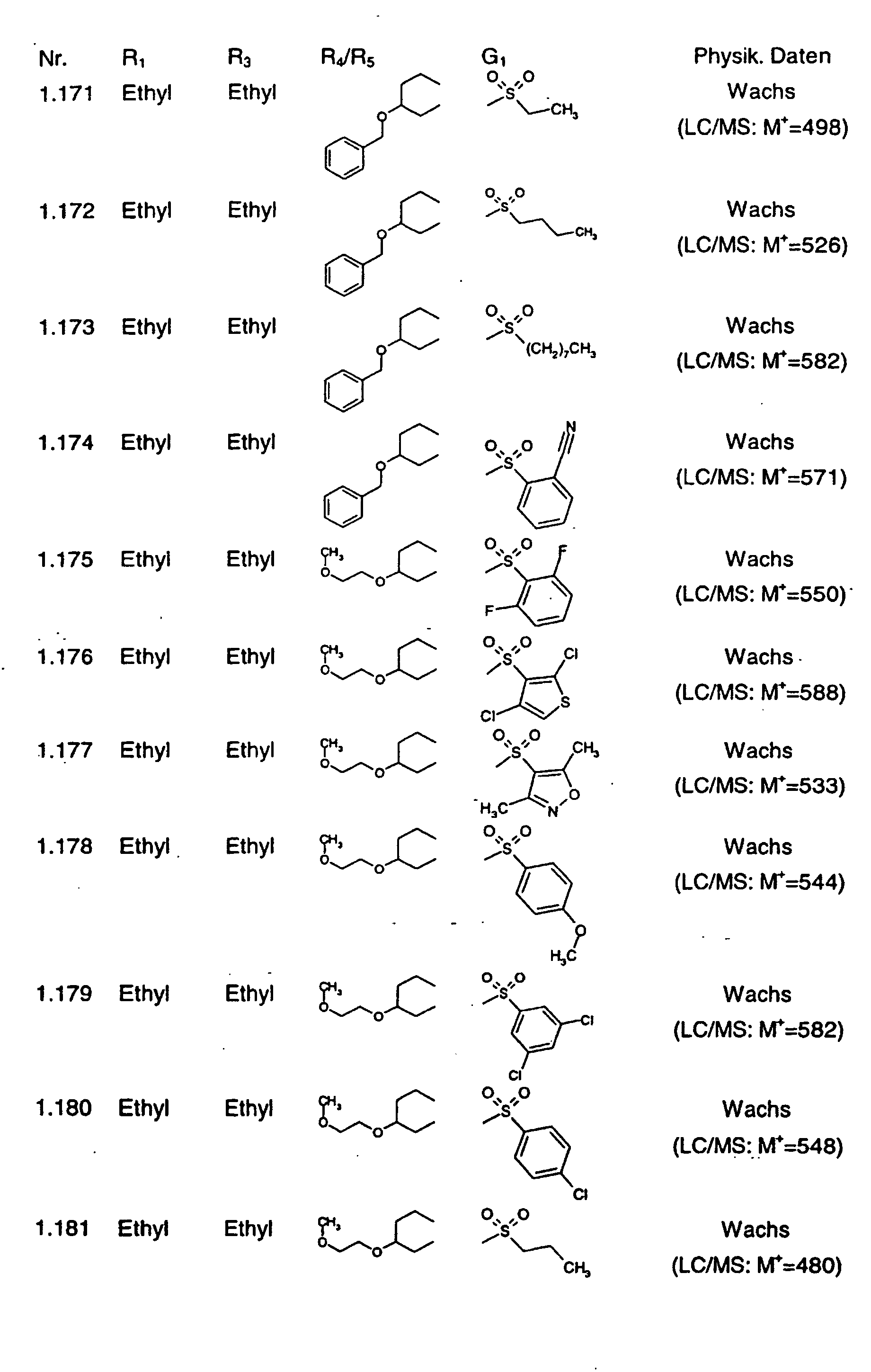

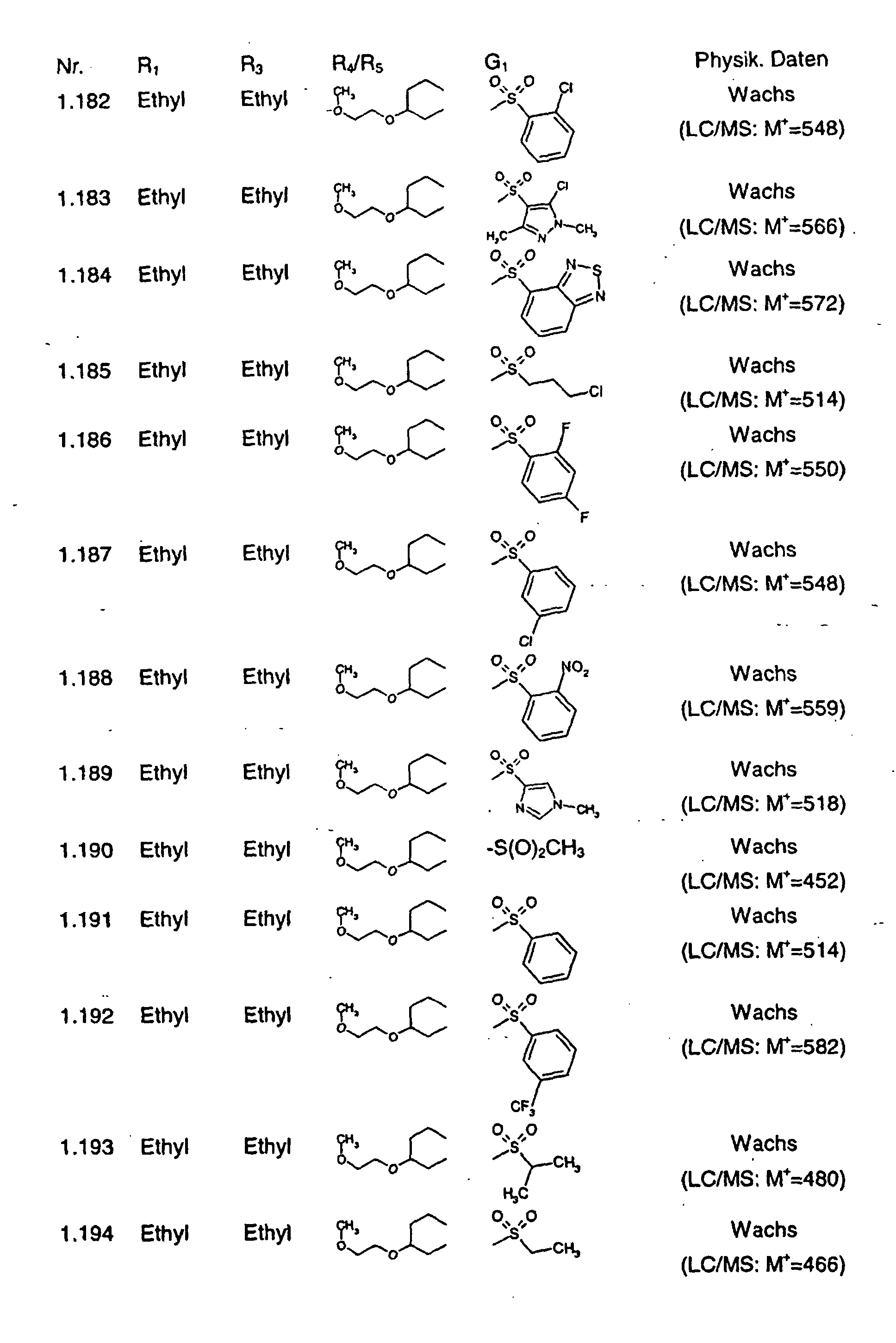

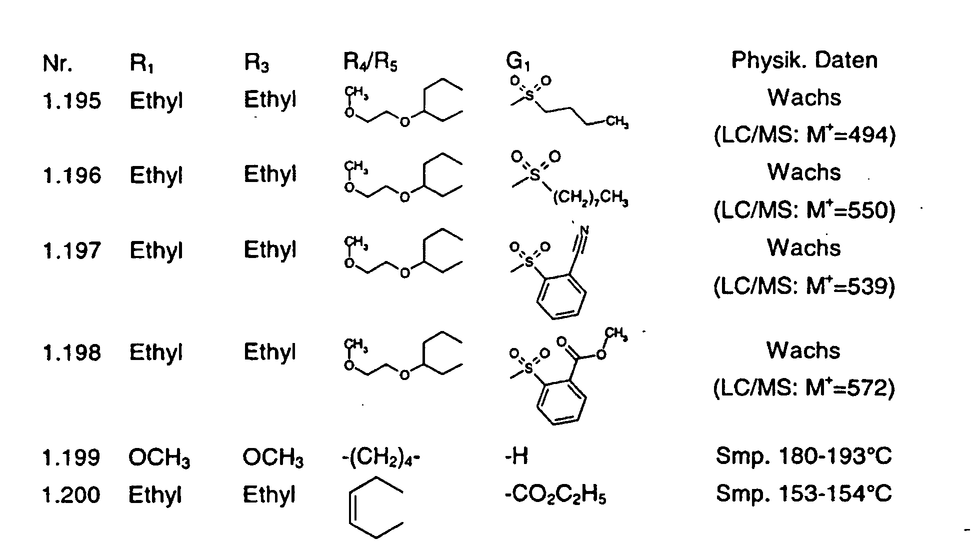

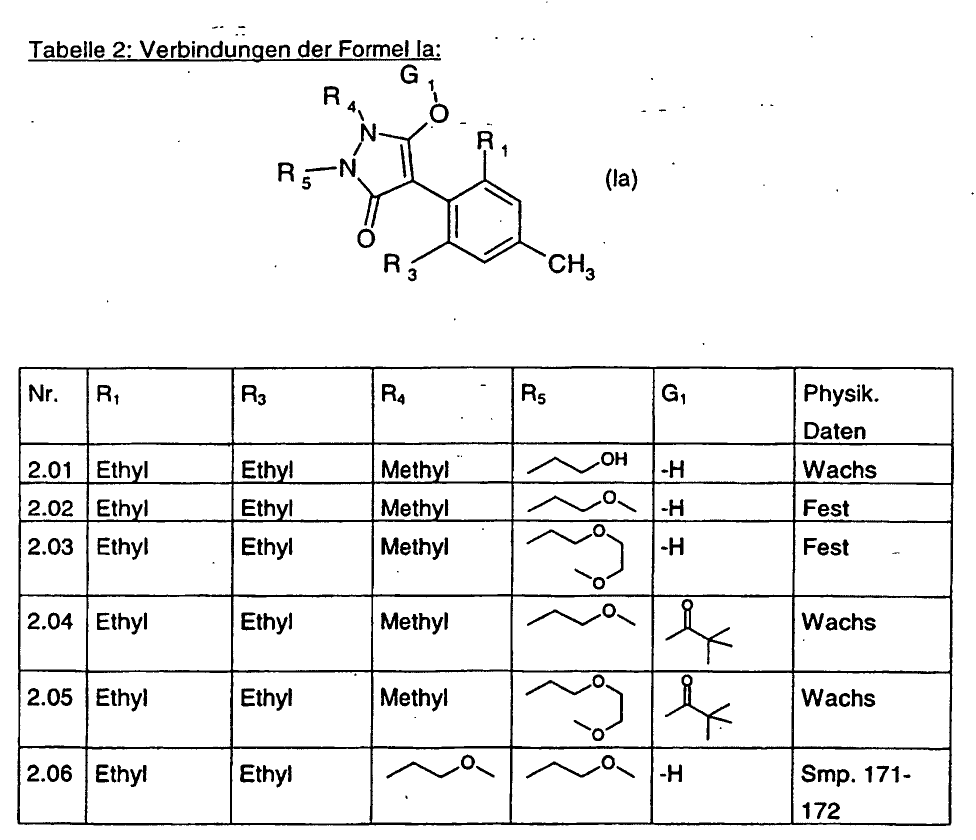

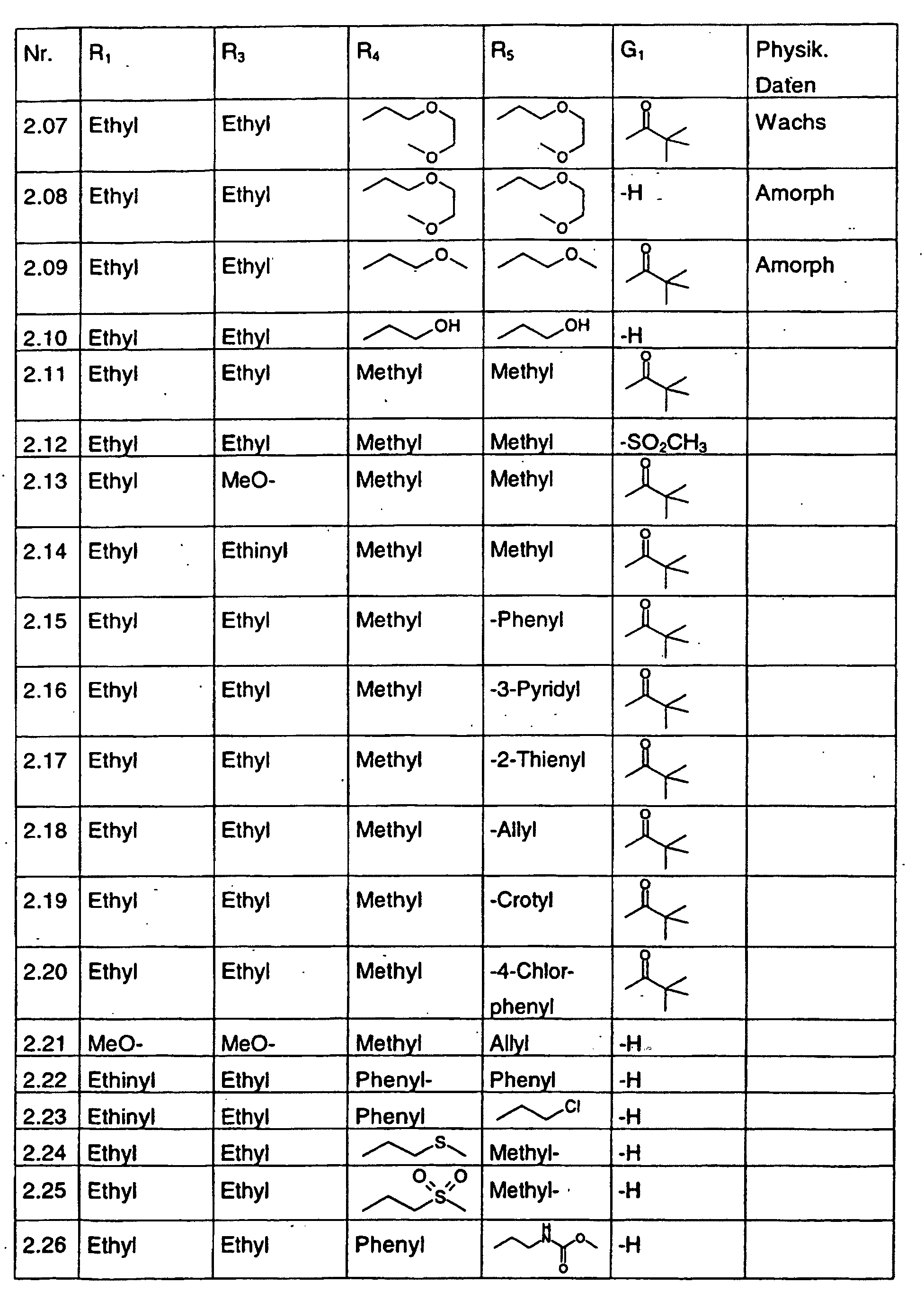

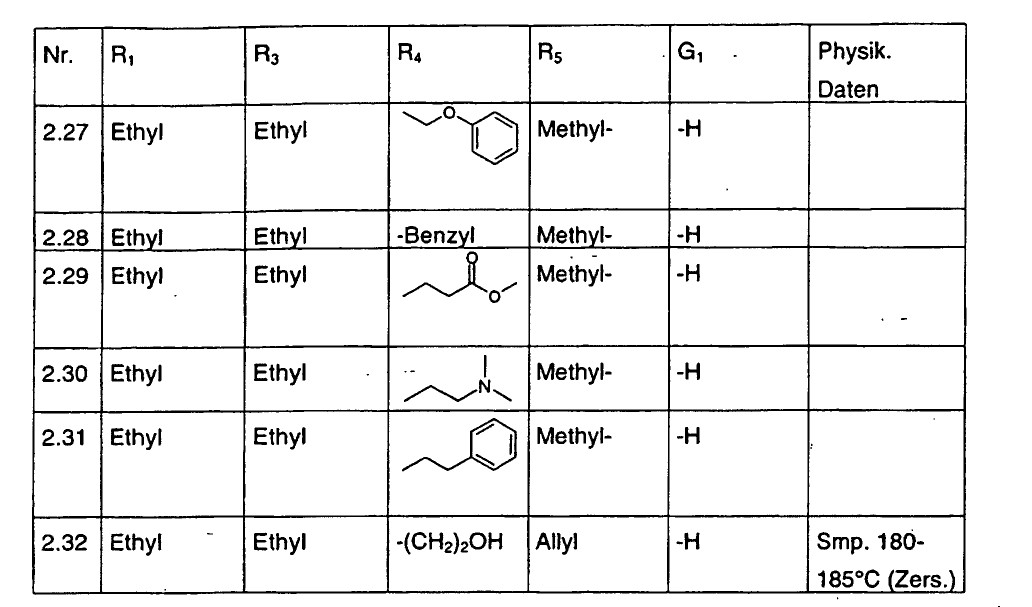

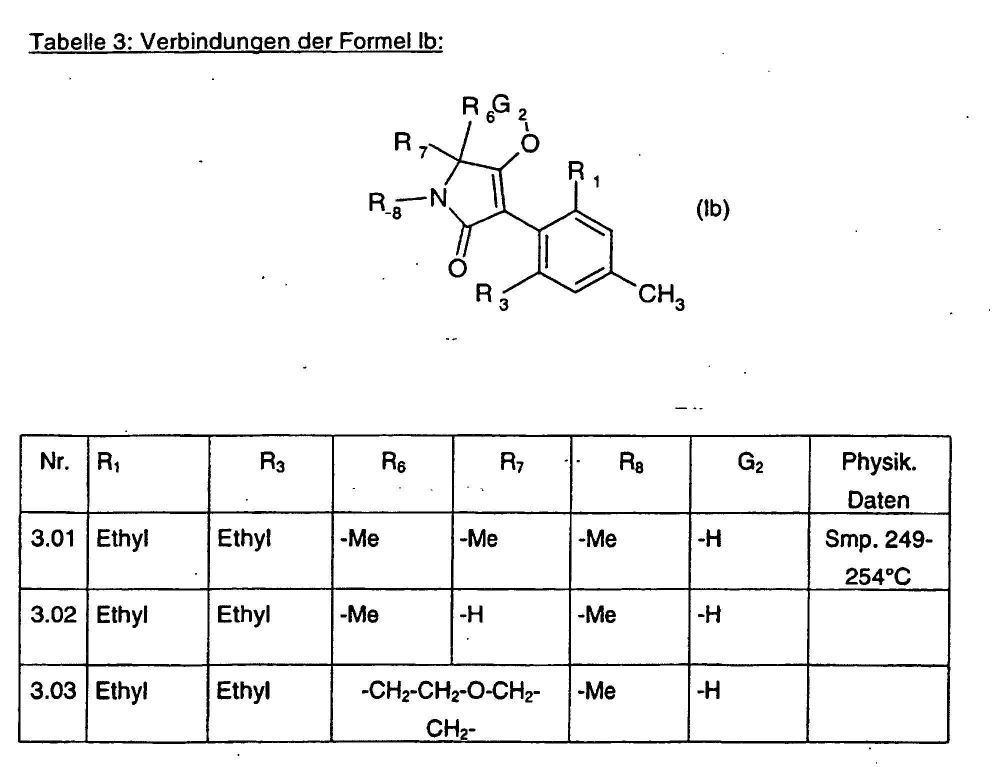

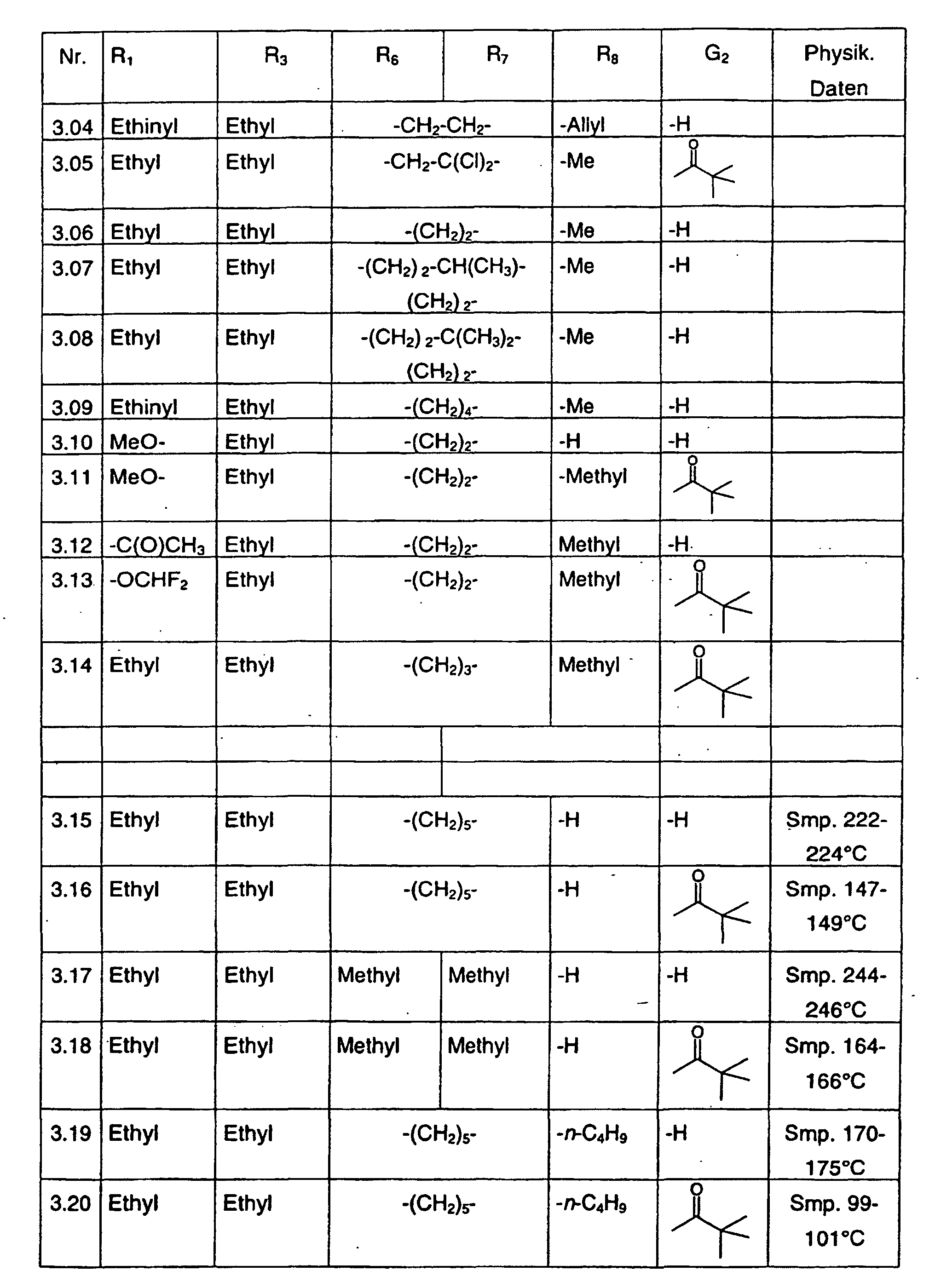

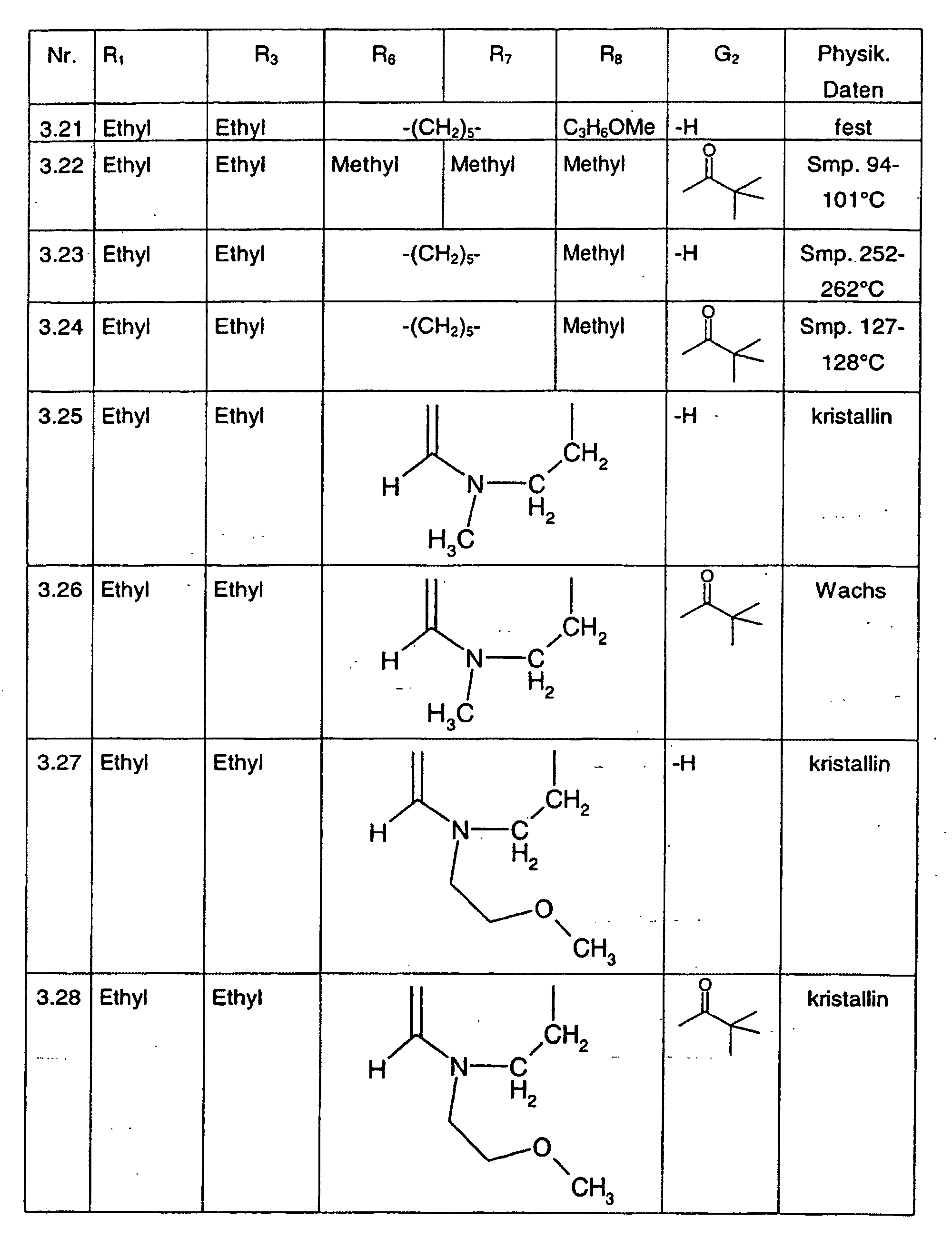

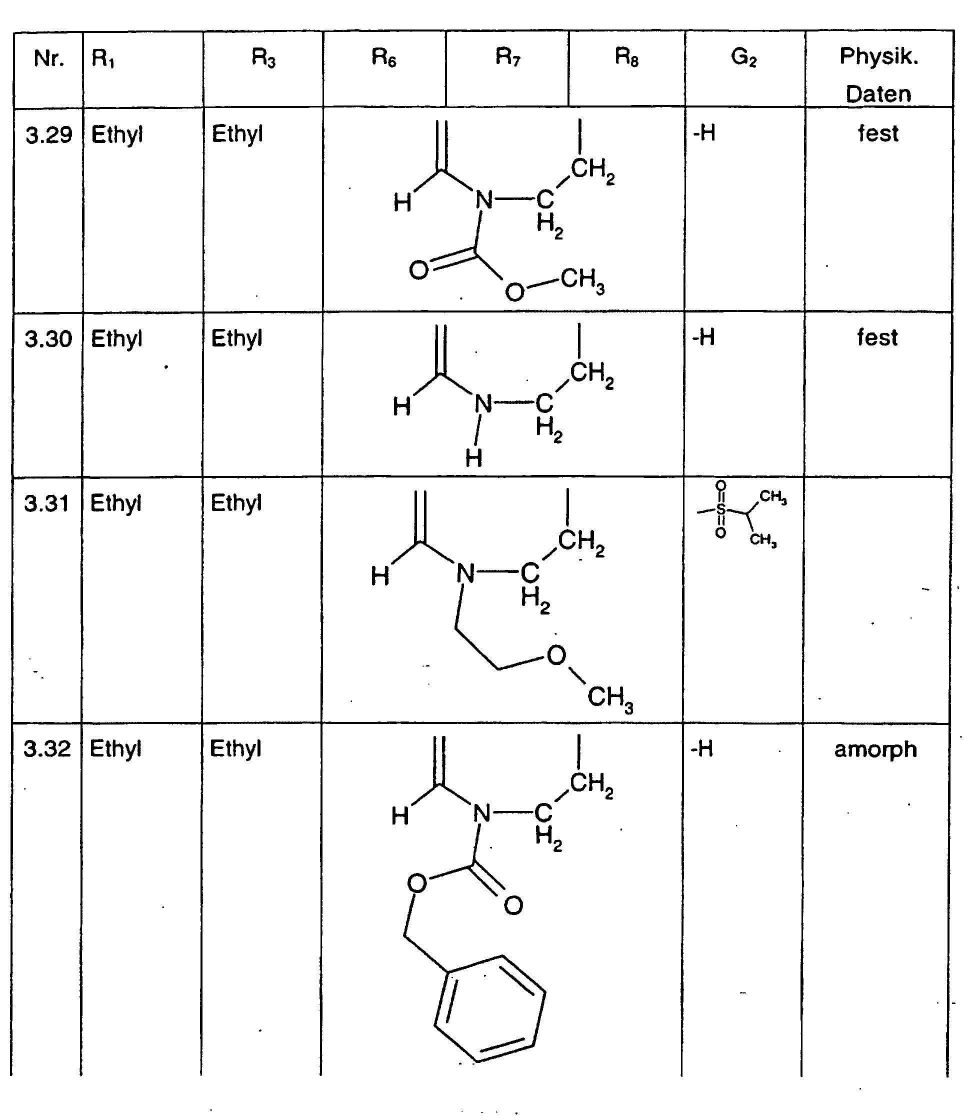

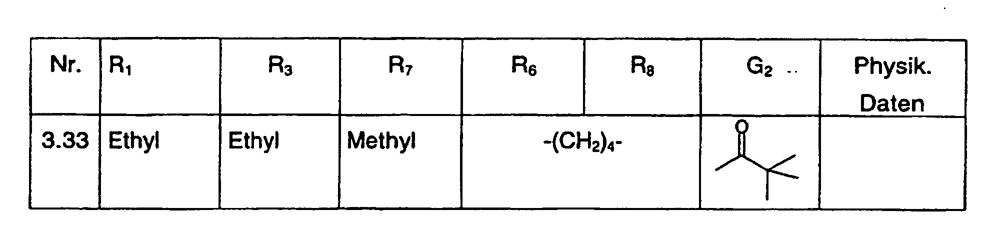

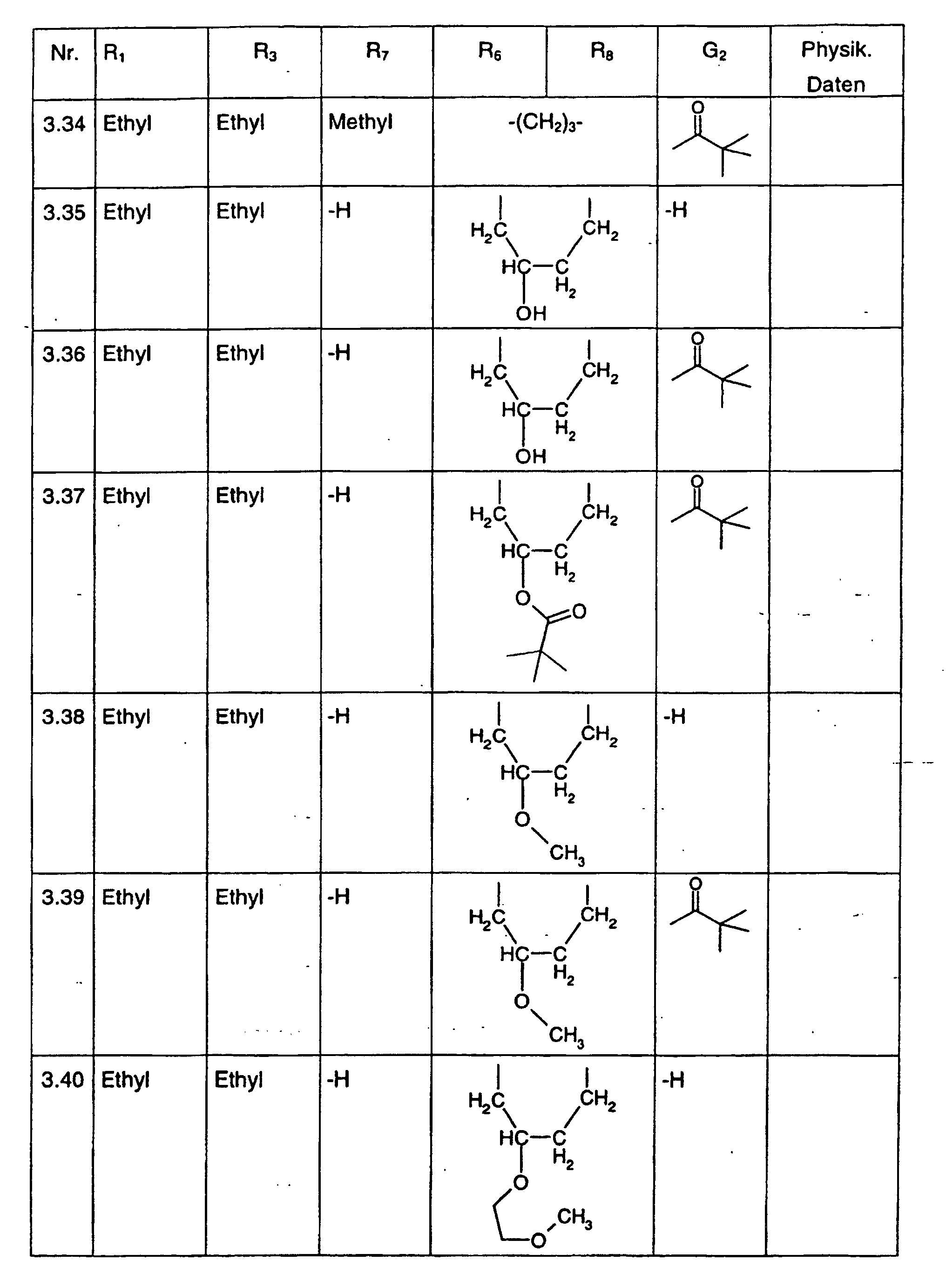

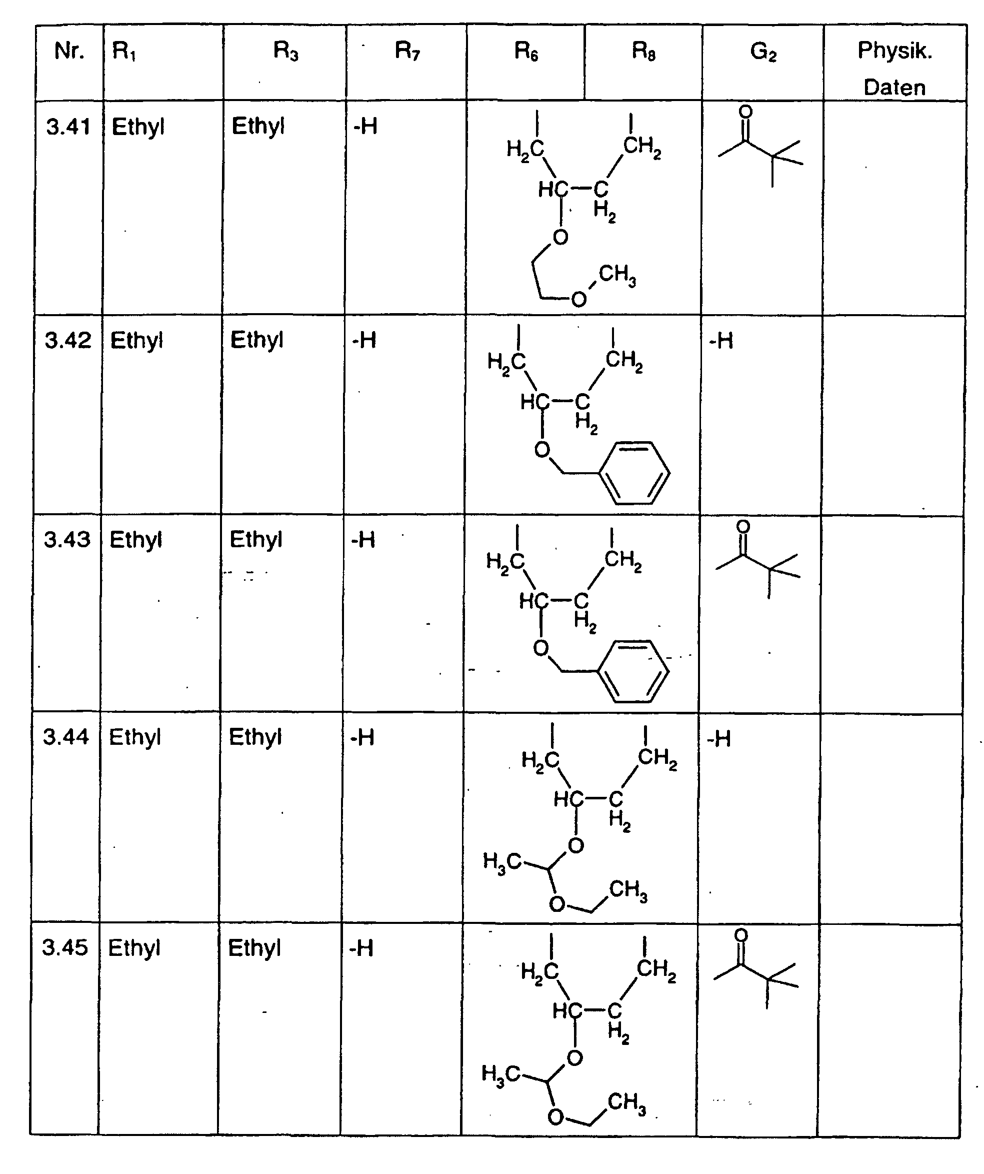

In der folgenden Tabelle 21 steht Me für Methyl, Et für Ethyl, Pr für Propyl und Bu für Butyl:

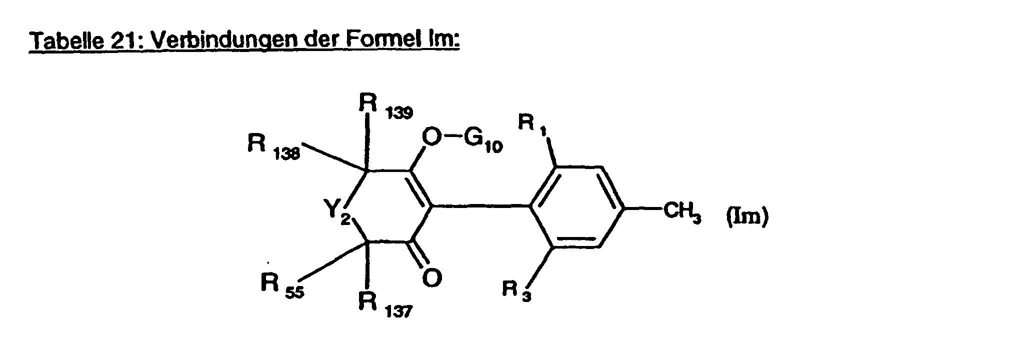

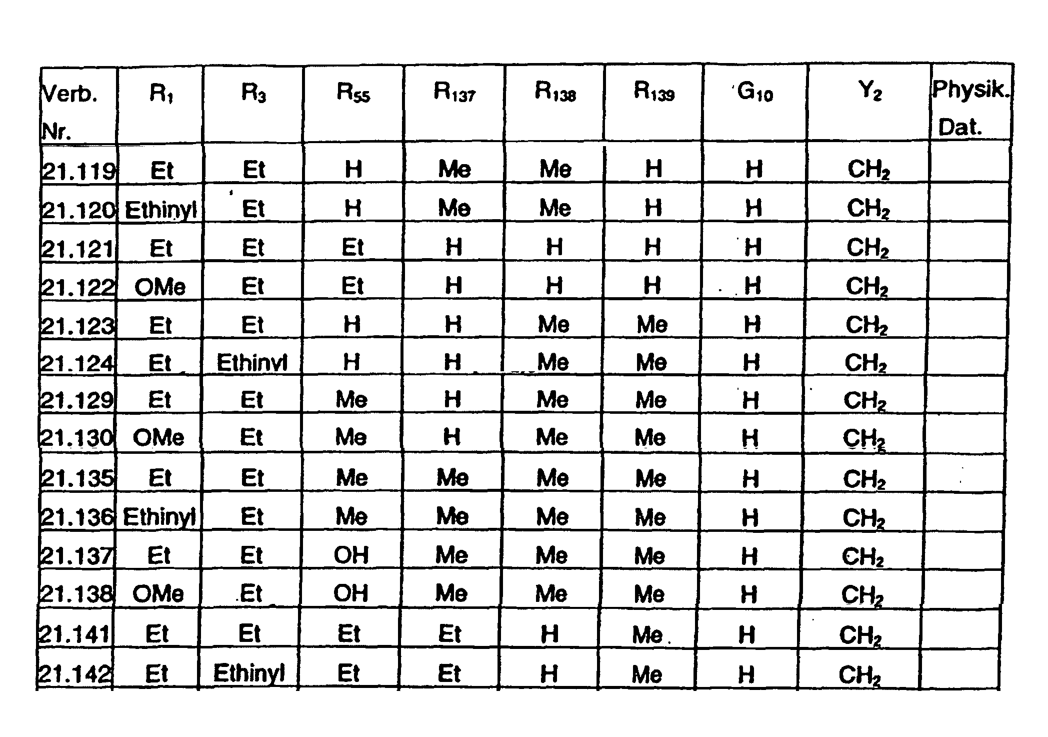

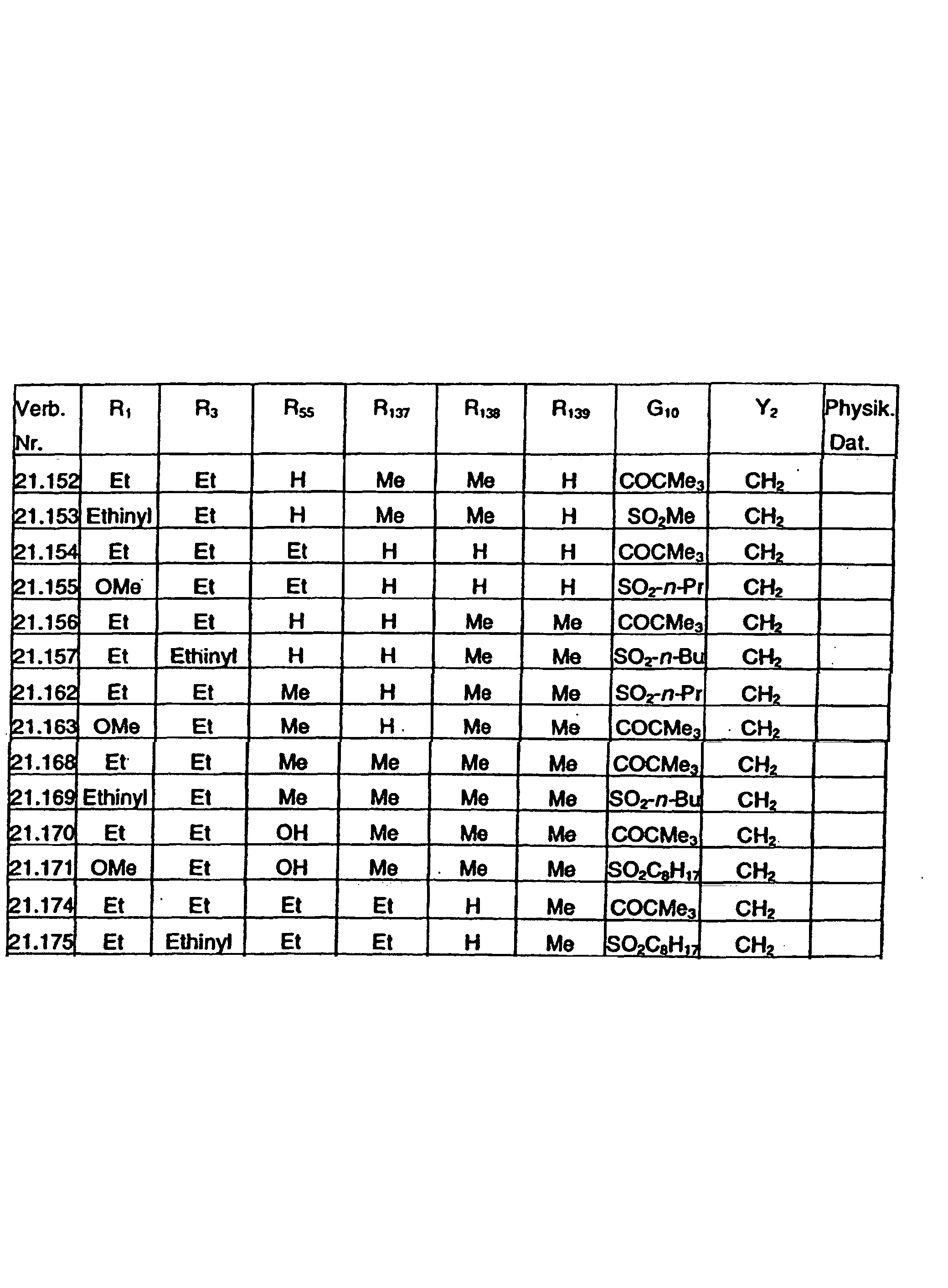

### Biologische Beispiele

### Vergleichsversuch:

Die folgenden Verbindungen wurden auf ihre herbizide Wirkung untersucht:
Verbindung Nr. 1.02 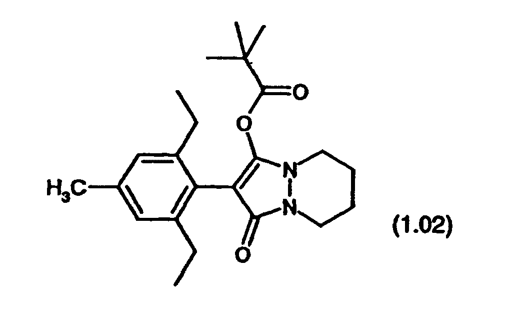 gemäß vorliegender Erfindung, und Verbindung A

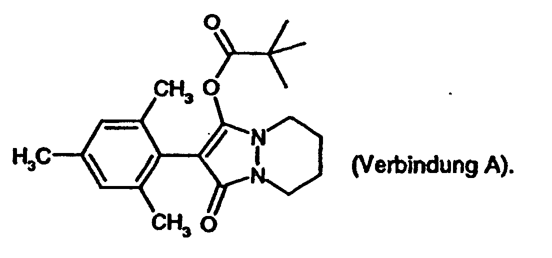

### Beispiel B1: Herbizidwirkung vor dem Auflaufen der Pflanzen (pre-emergente Wirkung)

Monokotyle und dikotyle Unkräuter werden in Kunststofftöpfen in Standarderde ausgesät. Unmittelbar nach der Saat werden die Prüfsubstanzen als wäßrige Suspension (hergestellt aus einem 25 %igen Spritzpulver (Beispiel F3, b)) oder als Emulsion (hergestellt aus einem 25 %igen Emulsionskonzentrat (Beispiel F1, c)) appliziert (500 I Wasser/ha). Die Aufwandmenge beträgt 500 g/ha Aktivsubstanz. Anschließend werden die Testpflanzen im Gewächshaus unter Optimalbedingungen angezogen. Die Auswertung erfolgt 3 Wochen nach Applikation mit einer neunstufigen Boniturskala (1 = vollständige Schädigung, 9 = keine Wirkung). Bonitumoten von 1 bis 4 (insbesondere 1 bis 3) bedeuten eine gute bis sehr gute Herbizidwirkung.
Testpflanzen: Alopecurus (Alo), Avena (Ave), Lolium (Lol), Setaria (Set), Panicum (Pan), Sorghum (Sor), Digitaria (Dig), Echinocloa (Ech) und Brachiaria (Bra).

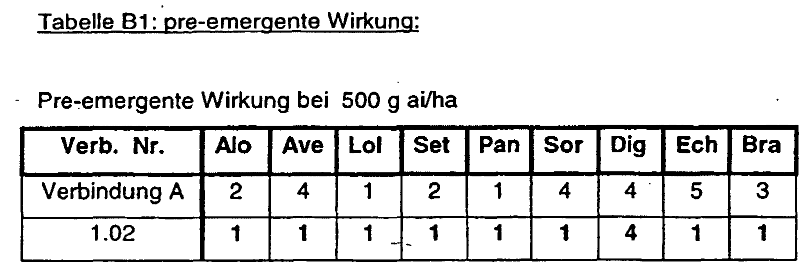

### Beispiel B2: Herbizide Wirkung nach dem Auflaufen der Pflanzen (post-emergente Wirkung):

Monokotyle und dikotyle Unkräuter werden unter Gewächshausbedingungen in Kunststofftöpfen in Standarderde angezogen. Die Applikation der Prüfsubstanzen verfolgt im 3- bis 6-Blattstadium der Testpflanzen. Die Prüfsubstanzen werden als wäßrige Suspension (hergestellt aus einem 25 %igen Spritzpulver (Beispiel F3, b)) oder als Emulsion (hergestellt aus einem 25 %igen Emulsionskonzentrat (Beispiel F1, c)) (500 I Wasser/ha) mit einer Aufwandmenge von 500 g/ha Aktivsubstanz appliziert. Die Auswertung erfolgt 3 Wochen nach Applikation mit einer neunstufigen Boniturskala (1=vollständige Schädigung, 9 = keine Wirkung). Bonitumoten von 1 bis 4 (insbesondere 1 bis 3) bedeuten eine gute bis sehr gute Herbizidwirkung.

Testpflanzen: Alopecurus (Alo), Avena (Ave), Lolium (Lol), Setaria (Set), Panicum (Pan), Sorghum (Sor), Digitaria (Dig), Echinocloa (Ech) und Brachiaria (Bra).

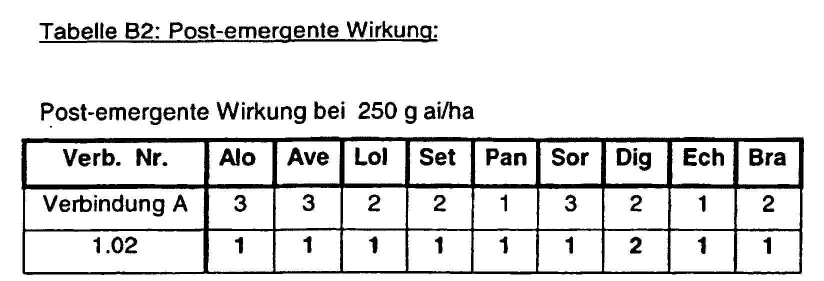

Aus dem Vergleich der herbiziden Wirksamkeit der Verbindung A gegen die Verbindung Nr. 1.02 der vorliegenden Erfindung läßt sich entnehmen, daß die Verbindung Nr. 1.02 bei allen getesteten Unkräutern überraschenderweise eine wesentlich bessere herbizide Wirkung aufweist, obwohl diese Verbindung sich gegenüber der Verbindung A lediglich dadurch unterscheidet, daß zwei Ethylgruppen durch Methylgruppen ersetzt sind.

### Beispiel B3: Herbizidwirkung vor dem Auflaufen der Pflanzen (pre-emergente Wirkung) von Verbindungen der vorliegenden Erfindung:

Monokotyle und dikotyle Unkräuter werden in Kunststofftöpfen in Standarderde ausgesät. Unmittelbar nach der Saat werden die Prüfsubstanzen als wäßrige Suspension (hergestellt aus einem 25 %igen Spritzpulver (Beispiel F3, b)) oder als Emulsion (hergestellt aus einem 25 %igen Emulsionskonzentrat (Beispiel F1, c)) appliziert (500 I Wasser/ha). Die Aufwandmenge beträgt 500 g/ha Aktivsubstanz. Anschließend werden die Testpflanzen im Gewächshaus unter Optimalbedingungen angezogen. Die Auswertung erfolgt 3 Wochen nach Applikation mit einer neunstufigen Boniturskala (1 = vollständige Schädigung, 9 = keine Wirkung). Boniturnoten von 1 bis 4 (insbesondere 1 bis 3) bedeuten eine gute bis sehr gute Herbizidwirkung.
Testpflanzen: Avena (Ave), Lolium (Lol), Setaria (Set).

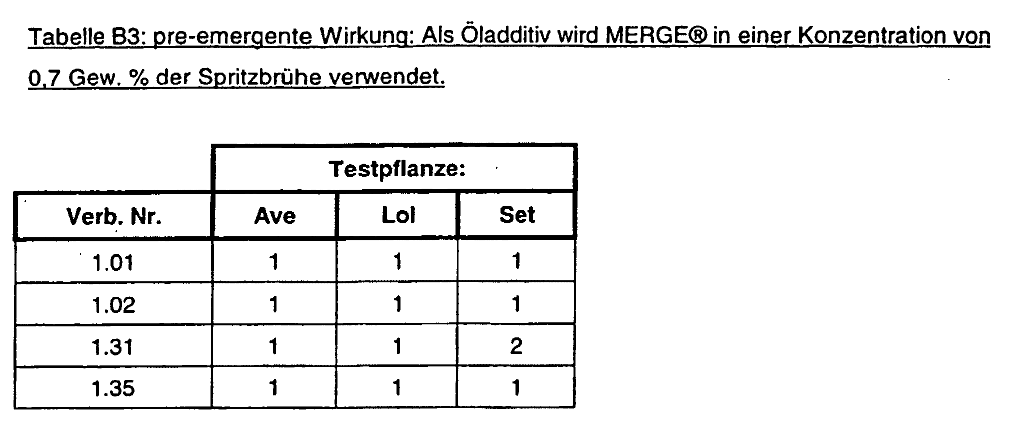

Dieselben Resultate werden erhalten, wenn man die Verbindungen der Formel I gemäß den Beispielen F2 und F4 bis F8 formuliert.

### Beispiel B4: Herbizide Wirkung nach dem Auflaufen der Pflanzen (post-emergente Wirkung) von Verbindungen der vorliegenden Erfindung (Beschreibung siehe Beispiel B2):

Testpflanzen: Avena (Ave), Lolium (Lol), Setaria (Set). Die Resultate sind in der folgenden Tabelle B4 angegeben:

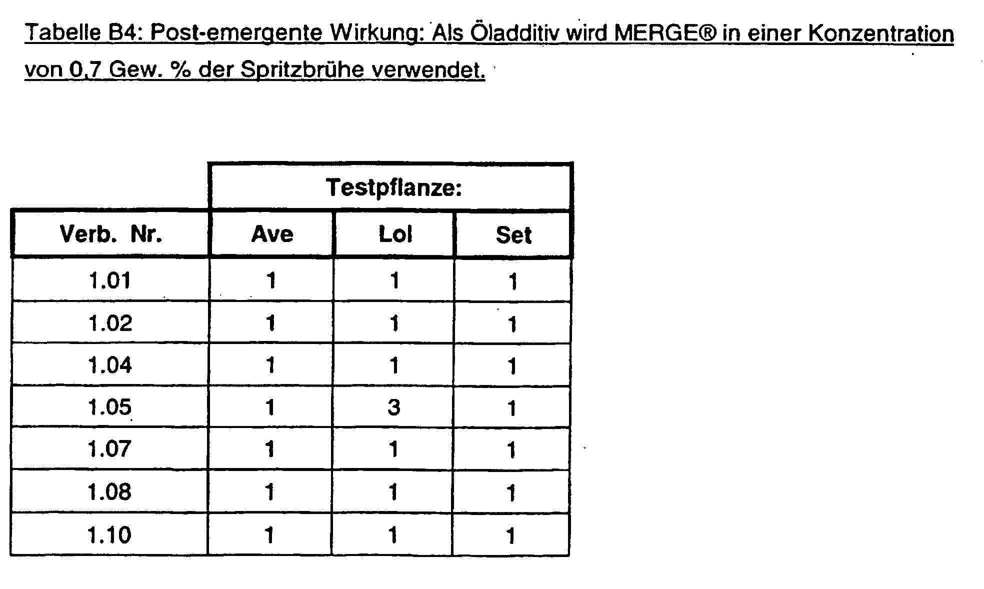

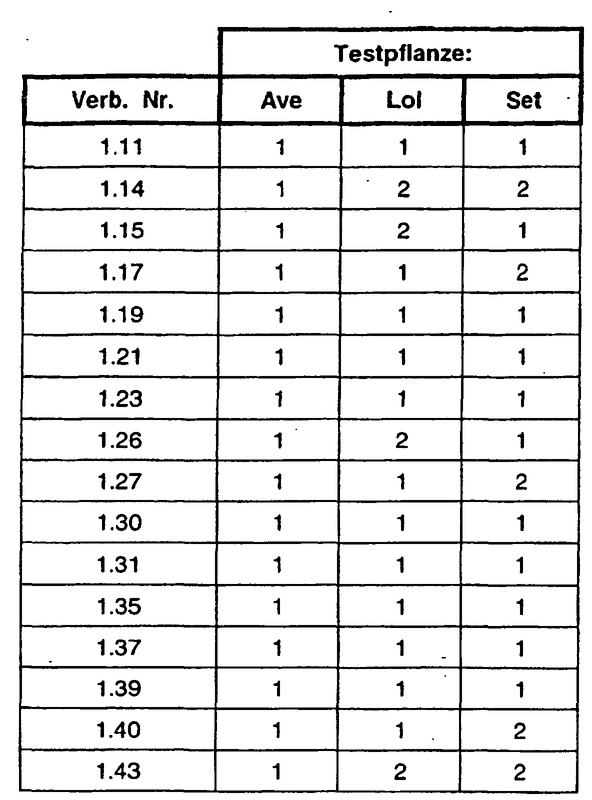

Dieselben Resultate werden erhalten, wenn man die Verbindungen der Formel I gemäß den Beispielen F2 und F4 bis F8 formuliert.

## Patentansprüche

1. Verbindungen der Formel I 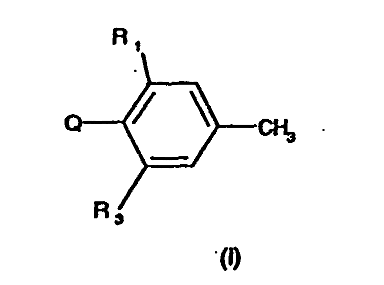 worin
R₁ und R₃ unabhängig voneinander Ethyl, Halogenethyl, Ethinyl, C₁-C₂-Alkoxy, C₁-C₂-Halogenalkoxy oder C₁-C₂-Alkylcarbonyl bedeuten;
Q eine Gruppe 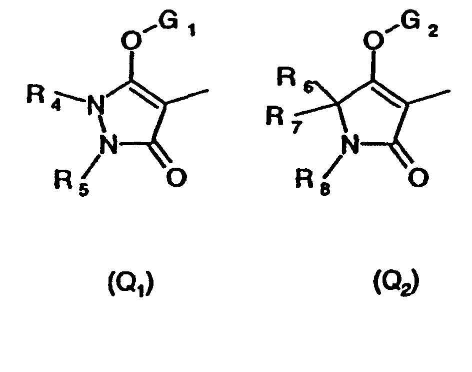 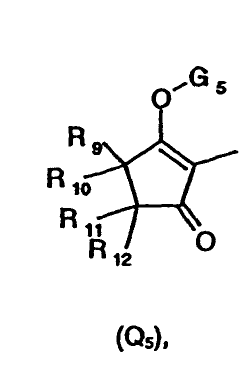 oder 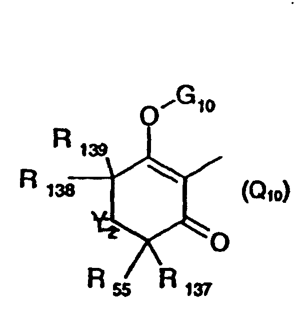 bedeutet;
R₄ und R₅ unabhängig voneinander C₁-C₁₀-Alkyl, C₂-C₁₀-Alkenyl, C₂-C₁₀-Alkinyl, C₁-C₁₀-Halogenalkyl, C₂-C₁₀-Alkoxyalkyl, C₃-C₁₀-Alkenyloxyalkyl, C₃-C₁₀-Alkinyloxyalkyl, C₂-C₁₀-Alkylthioalkyl, C₂-C₁₀-Alkylsulfinylalkyl, C₂-C₁₀-Alkylsulfonylalkyl, C₂-C₁₀-Alkylcarbonyl-alkyl, C₂-C₁₀-N-Alkoxy-iminoalkyl, C₂-C₁₀-Alkoxycarbonylalkyl, C₁-C₁₀-Aminoalkyl, C₃-C₁₀-Dialkylaminoalkyl, C₂-C₁₀-Alkylaminoalkyl, C₁-C₁₀-Cyanoalkyl, C₄-C₁₀-Cycloalkylalkyl, C₁-C₁₀-Phenylalkyl, C₁-C₁₀-Heteroarylalkyl, C₁-C₁₀-Phenoxylalkyl, C₁-C₁₀-Heteroaryloxyalkyl, C₁-C₁₀-Alkylidenaminooxyalkyl, C₁-C₁₀-Nitroalkyl, C₁-C₁₀-Trialkylsilylalkyl, C₂-C₁₀-Alkylaminocarbonylalkyl, C₂-C₁₀-Dialkylaminocarbonylalkyl, C₂-C₁₀-Alkylaminocarbonyloxyalkyl, C₃-C₁₀-Dialkylaminocarbonyloxalkyl, C₂-C₁₀-Alkoxycarbonylaminoalkyl, C₁-C₁₀-N-Alkoxycarbonyl-N-alkylamino-alkyl, C₁-C₁₀-Cycloalkyl, Aryl oder Heteroaryl bedeuten; oder
R₄ und R₅ bilden gemeinsam mit den Atomen, an die sie gebunden sind, einen 5- bis 7-gliedrigen Cyclus, der ein oder zwei Heteroatome ausgewählt aus Stickstoff, Sauerstoff und Schwefel enthalten kann und der zusätzlich eine anellierte oder spirogebundene, aus 2 bis 6 Kohlenstoffatomen bestehende Alkylen- oder Alkenylenkette enthalten kann, die ihrerseits ein oder zwei Heteroatome ausgewählt aus Sauerstoff und Schwefel enthalten kann, wobei dieser Cyclus mit Phenyl oder Benzyl substituiert sein kann, welche ihrerseits durch Halogen, C₁-C₆-Alkyl, C₁-C₆-Halogenalkyl, C₃-C₆-Cycloalkyl, Hydroxy, C₁-C₆-Alkoxy, C₁-C₆-Alkoxy-C₁-C₆-alkoxy, C₁-C₆-Halogenalkoxy oder Nitro substituiert sein können;
R₆ C₁-C₁₀-Alkyl, C₂-C₁₀-Alkenyl, C₂-C₁₀-Alkinyl, C₁-C₁₀-Halogenalkyl, C₂-C₁₀-Alkoxyalkyl, C₃-C₁₀-Alkenyloxyalkyl, C₃-C₁₀-Alkinyloxyalkyl, C₂-C₁₀-Alkylthioalkyl, C₂-C₁₀-Alkylsulfinylalkyl, C₂-C₁₀-Alkylsulfonylalkyl, C₂-C₁₀-Alkylcarbonyl-alkyl, C₃-C₁₀-Cycloalkyl, Aryl oder Heteroaryl bedeutet;
R₇ Wasserstoff, C₁-C₁₀-Alkyl C₂-C₁₀-Alkenyl, C₂-C₁₀-Alkinyl oder C₂-C₁₀-Alkoxyalkyl;
R₈ Wasserstoff, C₁-C₁₀-Alkyl, C₁-C₁₀-Halogenalkyl, C₂-C₁₀-Alkoxyalkyl, C₃-C₁₀-Alkenyloxyalkyl, C₃-C₁₀-Alkinyloxyalkyl, C₂-C₁₀-Alkylthioalkyl, C₂-C₁₀-Alkylsulfinylalkyl, C₂-C₁₀-Alkylsulfonylalkyl, C₃-C₁₀-Cycloalkyl, Aryl oder Heteroaryl bedeuten; oder
R₆ und R₇ bilden gemeinsam mit dem Atom, an das sie gebunden sind, einen gesättigten 3- bis 7- gliedrigen Cyclus, der ein oder zwei Heteroatome ausgewählt aus Stickstoff, Sauerstoff und Schwefel enthalten kann; oder R₆ und R₈ bilden gemeinsam mit den Atomen, an die sie gebunden sind, einen 5-bis 7-gliedrigen Cyclus, der ein oder zwei Heteroatome ausgewählt aus Stickstoff, Sauerstoff und Schwefel enthalten kann;
R₉, R₁₀, R₁₁ und R₁₂ unabängig voneinander C₁-C₁₀-Alkyl, C₂-C₁₀-Alkenyl, C₂-C₁₀-Alkinyl, C₁-C₁₀-Halogenalkyl, C₂-C₁₀-Alkoxyalkyl, C₃-C₁₀-Alkenyloxyalkyl, C₃-C₁₀-Alkinyloxyalkyl, C₂-C₁₀-Alkylthialkyl, C₂-C₁₀-Alkylsulfinylalkyl, C₂-C₁₀-Alkylsulfonylalkyl, C₂-C₁₀-Alkylcarbonyl-alkyl, C₃-C₁₀-Cyclolalkyl, Aryl oder Heteroaryl bedeuten; oder
R₁₀ und R₁₁ oder R₉ und R₁₀ bilden gemeinsam mit den Atomen, an die sie gebunden sind, einen 5- bis 7- gliedrigen Cyclus, der ein oder zwei Heteroatome ausgewählt aus Stickstoff, Sauerstoff und Schwefel enthalten kann;
G₁, G₂, G₅ und G₁₀ unabhängig voneinander Wasserstoff, -C(X₁)-R₂₀, -C(X₂)-X₃-R₂₁, -C(X₄)-N(R₂₂)-R₂₃, -SO₂-R₂₄, ein Alkali-, Erdalkali-, Sulfonium- oder Ammoniumkation, -P(X₅)(R₂₅)-R₂₆ oder -CH₂-X₆-R₂₇ bedeuten;
X₁, X₂, X₃, X₄, X₅ und X₆ unabhängig voneinander Sauerstoff oder Schwefel bedeuten;
R₂₀, R₂₁, R₂₂ und R₂₃ unabhängig voneinander Wasserstoff, C₁-C₁₀-Alkyl, C₂-C₁₀-Alkenyl, C₂-C₁₀-Alkinyl, C₁-C₁₀-Halogenalkyl, C₁-C₁₀-Cyanoalkyl, C₁-C₁₀-Nitroalkyl, C₁-C₁₀-Aminoalkyl, C₁-C₅-Alkylamino-C₁-C₅-alkyl, C₂-C₈-Dialkylamino-C₁-C₅-alkyl, C₃-C₇-Cycloalkyl-C₁-C₅-alkyl, C₂-C₁₀-Alkoxy-alkyl, C₄-C₁₀-Alkenyloxy-alkyl, C₄-C₁₀-Alkinyloxy-alkyl, C₂-C₁₀-Alkylthio-alkyl, C₁-C₅-Alkylsulfoxyl-C₁-C₅-alkyl, C₁-C₅-Alkylsulfonyl-C₁-C₅-alkyl, C₂-C₈-Alkylideneamino-oxy-C₁-C₅-alkyl, C₁-C₅-Alkylcarbonyl-C₁-C₅-alkyl, C₁-C₅-Alkoxycarbonyl-C₁-C₅-alkyl, C₁-C₅-Aminocarbonyl-C₁-C₅-alkyl, C₂-C₈-Dialkylamino-carbonyl-C₁-C₅-alkyl, C₁-C₅-Alkylcarbonylamino-C₁-C₅-alkyl, C₁-C₅-Alkylcarbonyl-(C₂-C₅-alkyl)-aminoalkyl, C₃-C₆-Trialkylsilyl-C₁-C₅-alkyl, Phenyl-C₁-C₅-alkyl, Heteroaryl-C₁-C₅-alkyl, Phenoxy- C₁-C₅-alkyl, Heteroaryloxy-C₁-C₅-alkyl, C₂-C₅-Alkenyl, C₂-C₅-Halogenalkenyl, C₃-C₈-Cycloalkyl, Phenyl, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Phenyl, oder Heteroaryl oder Heteroarylamino, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Heteroaryl oder Heteroarylamino, Diheteroarylamino oder durch C₁-C₃-Alkyl, C₁-C₃-Halogenalkyl, C₁-C₃-Alkoxy, C₁-C₃-Halogenalkoxy, Halogen, Cyano oder Nitro substituiertes Diheteroarylamino, Phenylamino oder durch C₁-C₃-Alkyl, C₁-C₃-Halogenalkyl, C₁-C₃-Alkoxy, C₁-C₃-Halogenalkoxy, Halogen, Cyano oder Nitro substituiertes Phenylamino, Diphenylamino oder durch C₁-C₃-Alkyl, C₁-C₃-Halogenalkyl, C₁-C₃-Alkoxy, C₁-C₃-Halogenalkoxy, Halogen, Cyano oder Nitro substituiertes Diphenylamino, oder C₃-C₇-Cycloalkylamino, Di-C₃-C₇-cycloalkylamino oder C₃-C₇-Cycloalkoxy bedeuten;
R₂₄, R₂₅ und R₂₆ Wasserstoff, C₁-C₁₀-Alkyl, C₂-C₁₀-Alkenyl, C₂-C₁₀-Alkinyl, C₁-C₁₀-Halogenalkyl, C₁-C₁₀-Cyanoalkyl, C₁-C₁₀-Nitroalkyl, C₁- C₁₀-Aminoalkyl, C₁-C₅-Alkylamino-C₁-C₅-alkyl, C₂-C₈-Dialkylamino- C₁-C₅-alkyl, C₃-C₇-Cycloalkyl-C₁-C₅-alkyl, C₂-C₁₀-Alkoxy-alkyl, C₄-C₁₀-Alkenyloxy-alkyl, C₄-C₁₀-Alkinyloxy-alkyl, C₂- C₁₀-Alkylthio-alkyl, C₁-C₅-Alkysulfoxyl-C₁-C₅-alkyl, C₁-C₅-Alkylsulfonyl-C₁-C₅-alkyl, C₂-C₈-Alkylideneamino-oxy-C₁-C₅-alkyl, C₁-C₅-Alkylcarbonyl-C₁-C₅-alkyl, C₁-C₅-Alkoxycarbonyl-C₁-C₅-alkyl, C₁-C₅-Amino-carbonyl-C₁-C₅-alkyl, C₂-C₈-Dialkylamino-carbonyl-C₁-C₅-alkyl, C₁-C₅-Alkylcarbonylamino-C₁-C₅-alkyl, C₁-C₅-Alkylcarbonyl-(C₂-C₅-alkyl)-aminoalkyl, C₃-C₆-Trialkylsilyl-C₁-C₅-alkyl, Phenyl-C₁-C₅-alkyl, Heteroaryl-C₁-C₅-alkyl, Phenoxy- C₁-C₅-alkyl, Heteroaryloxy- C₁-C₅-alkyl, C₂-C₅-Alkenyl, C₂-C₅-Halogenalkenyl, C₃-C₈-Cycloalkyl, Phenyl, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Phenyl, oder Heteroaryl oder Heteroarylamino, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Heteroaryl oder Heteroarylamino, Diheteroarylamino, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Diheteroarylamino, Phenylamino, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Phenylamino, Diphenylamino oder durch C₁-C₃-Alkyl, C₁-C₃-Halogenalkyl, C₁-C₃-Alkoxy, C₁-C₃-Halogenalkoxy, Halogen, Cyano oder Nitro substituiertes Diphenylamino, oder C₃-C₇-Cycloalkylamino, Di-C₃-C₇-cycloalkylamino, C₃-C₇-Cycloalkoxy, C₁-C₁₀-Alkoxy, C₁-C₁₀-Halogenalkoxy, C₁-C₅-Alkylamino, C₂-C₈-Dialkylamino, Benzyloxy oder Phenoxy, wobei die Benzyl- und phenylgruppen ihrerseits durch C₁-C₃-Alkyl, C₁-C₃-Halogenalkyl, C₁-C₃-Alkoxy, C₁-C₃-Halogenalkoxy, Halogen, Cyano oder Nitro substituiert sein können, bedeuten;
R₂₇ C₁-C₁₀-Alkyl, C₂-C₁₀-Alkenyl, C₂-C₁₀-Alkinyl, C₁-C₁₀-Halogenalkyl, C₁- C₁₀-Cyanoalkyl, C₁-C₁₀-Nitroalkyl, C₁-C₁₀-Aminoalkyl, C₁-C₅-Alkylamino-C₁-C₅-alkyl, C₂-C₈-Dialkylamino-C₁-C₅-alkyl, C₃-C₇-Cycloalkyl-C₁-C₅-alkyl, C₂-C₁₀-Alkoxy-alkyl, C₄-C₁₀-Alkenyloxy-alkyl, C₄-C₁₀-Alkinyloxy-alkyl, C₂-C₁₀-Alkylthio-alkyl, C₁-C₅-Alkylsulfoxyl- C₁-C₅-alkyl, C₁-C₅-Alkylsulfonyl-C₁-C₅-alkyl, C₂-C₈-Alkylideneamino-oxy-C₁-C₅-alkyl, C₁-C₅-Alkylcarbonyl-C₁-C₅-alkyl, C₁-C₅-Alkoxycarbonyl-C₁-C₅-alkyl, C₁-C₅-Amino-carbonyl-C₁-C₅-alkyl, C₂-C₈-Dialkylamino-carbonyl-C₁-C₅-alkyl, C₁-C₅-Alkylcarbonylamino-C₁-C₅-alkyl, C₁-C₅-Alkylcarbonyl-(C₂-C₅-alkyl)-aminoalkyl, C₃-C₆-Trialkylsilyl-C₁-C₅-alkyl, Phenyl-C₁-C₅-alkyl, Heteroaryl-C₁-C₅-alkyl, Phenoxy-C₁-C₅-alkyl, Heteroaryloxy-C₁-C₅-alkyl, C₂-C₅-Alkenyl, C₂-C₅-Halogenalkenyl, C₃-C₈-Cycloalkyl, Phenyl, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Phenyl, oder Heteroaryl, oder Heteroarylamino, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Heteroaryl oder Heteroarylamino, Diheteroarylamino, durch C₁-C₃-Alkyl, C₁-C₃-Halogenalkyl, C₁-C₃-Alkoxy, C₁-C₃-Halogenalkoxy, Halogen, Cyano oder Nitro substituiertes Diheteroarylamino, oder Phenylamino, durch C₁-C₃-Alkyl, C₁-C₃-Halogenalkyl, C₁-C₃-Alkoxy, C₁-C₃-Halogenalkoxy, Halogen, Cyano oder Nitro substituiertes Phenylamino, Diphenylamino, durch C₁-C₃-Alkyl, C₁-C₃-Halogenalkyl, C₁-C₃-Alkoxy, C₁-C₃-Halogenalkoxy, Halogen, Cyano oder Nitro substituiertes Diphenylamino, C₃-C₇-Cycloalkylamino, Di-C₃-C₇-cycloalkylamino, C₃-C₇-Cycloalkoxy oder C₁-C₁₀-Alkylcarbonyl bedeutet;
R₅₅ C₁-C₁₀-Alkyl
bedeutet;
R₁₃₇ Wasserstoff oder C₁-C₁₀-Alkyl
bedeutet; und
R₁₃₈ und R₁₃₉ unabhängig voneinander Wasserstoff oder C₁-C₁₀-Alkyl, sind;
sowie agronomisch verträgliche Salze, Isomere und Enantiomere dieser Verbindungen.

2. Verbindungen nach Anspruch 1, **dadurch gekennzeichnet, daß** Q für Q₁, Q₂ oder Q₅, steht.

3. Verfahren zur Herstellung von Verbindungen der Formel I gemäß Anspruch 1, **dadurch gekennzeichnet, daß** man eine Verbindung der Formel XXX
Q-H (XXX)
worin Q für Q₁, Q₂, Q₅, oder Q₁₀ steht, wobei deren Substituenten mit Ausnahme von G₁, G₂,G₅, und G₁₀ die oben angegebene Bedeutung haben und G₁, G₂, G₅, und G₁₀ Wasserstoff bedeutet, mit einer Verbindung der Formel XXXI 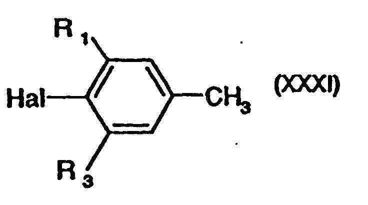 worin R₁ und R₃ die unter Formel I angegebene Bedeutung haben und Hal für Chlor, Brom oder Jod steht, in Gegenwart eines inerten Lösungsmittels, einer Base und eines Palladiumkatalysators bei Temperaturen von 30 bis 250 °C umsetzt.

4. Herbizides und den Pflanzenwuchs hemmendes Mittel, **dadurch gekennzeichnet, daß** es auf einem inerten Träger einen herbizid wirksamen Gehalt an Verbindung der Formel I aufweist.

5. Verfahren zur Bekämpfung unerwünschten Pflanzenwachstums, **dadurch gekennzeichnet, daß** man einen Wirkstoff der Formel I, oder ein diesen Wirkstoff enthaltendes Mittel in einer herbizid wirksamen Menge auf die Pflanzen oder deren Lebensraum appliziert.

6. Verfahren zur Hemmung des Pflanzenwachstums, **dadurch gekennzeichnet, daß** man einen Wirkstoff der Formel I, oder ein diesen Wirkstoff enthaltendes Mittel in einer herbizid wirksamen Menge auf die Pflanzen ode deren Lebensraum appliziert.

7. Selektiv-herbizides Mittel, **dadurch gekennzeichnet daß** es neben üblichen inerten Formulierungshilfsmitteln als Wirkstoff eine Mischung aus
a) einer herbizid-wirksamen Menge einer Verbindung der Formel I gemäß Anspruch 1 mit der Maßgabe, daß Q verschieden von Q₁ ist;
und
b) einer herbizid-antagonistisch wirksamen Menge entweder einer Verbindung der Formel X 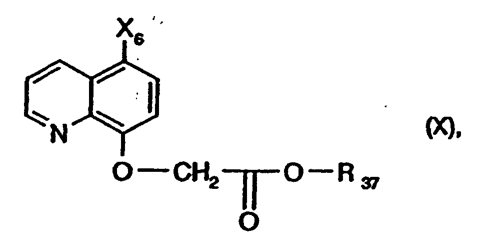
worin
R₃₇ Wasserstoff, C₁-C₈-Alkyl oder durch C₁-C₆-Alkoxy oder C₃-C₆-Alkenyloxy substituiertes C₁-C₈-Alkyl; und X₆ Wasserstoff oder Chlor bedeutet; oder einer Verbindung der Formel XI 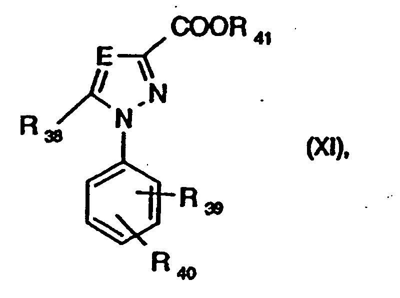 worin
E Stickstoff oder Methin; R₃₈ -CCl₃, Phenyl oder durch Halogen substituiertes Phenyl;
R₃₉ und R₄₀ unabhängig voneinander Wasserstoff oder Halogen; und
R₄₁ C₁-C₄-Alkyl bedeuten; oder einer Verbindung der Fonnel XII 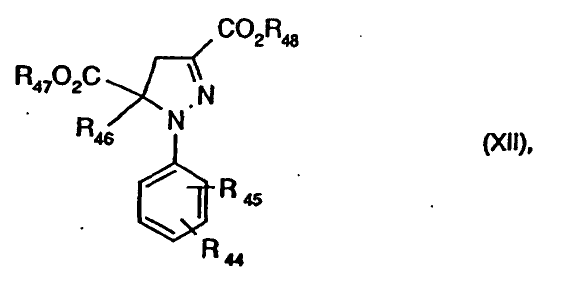 worin R₄₄ und R₄₅ unabhängig voneinander Wasserstoff oder Halogen und
R₄₆, R₄₇ und R₄₈ unabhängig voneinander C₁-C₄-Alkyl bedeuten, oder einer Verbindung der Formel XIII 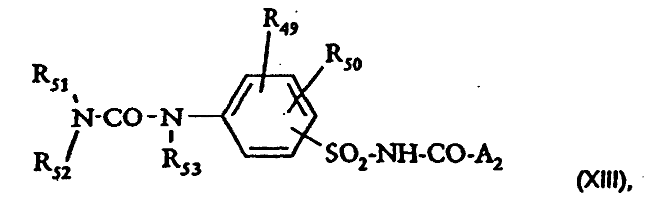 worin A₂ für eine Gruppe 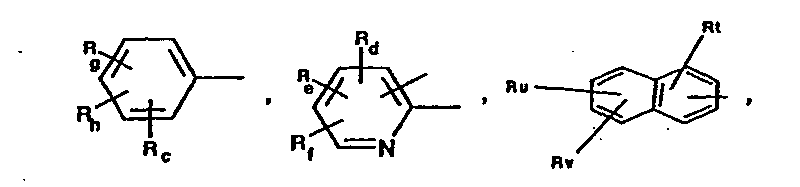 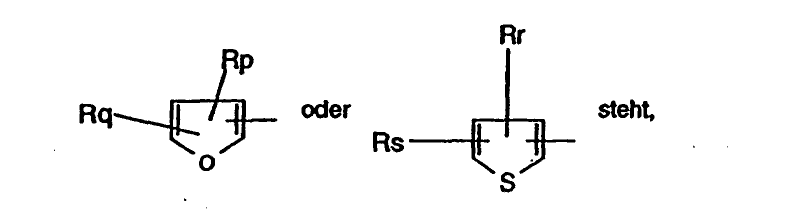 R₅₁ und R₅₂ unabhängig voneinander Wasserstoff, C₁-C₈-Alkyl, C₃-C₈-Cycloalkyl, C₃-C₆-Alkenyl, C₃-C₆-Alkinyl, 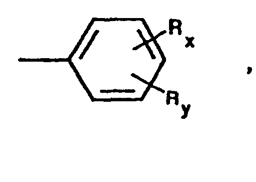 oder durch C₁-C₄-Alkoxy oder 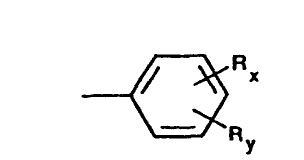 substituiertes C₁-C₄-Alkyl bedeutet; oder R₅₁ und R₅₂ bilden zusammen eine C₄-C₆-Alkylenbrücke, die durch Sauerstoff, Schwefel SO, SO₂, NH oder -N(C₁-C₄-Alkyl)- unterbrochen sein kann,
R₅₃ für Wasserstoff oder C₁-C₄-Alkyl;
R₄₉ für Wasserstoff, Halogen, Cyano, Trifluoromethyl, Nitro, C₁-C₄-Alkyl, C₁-C₄-Alkoxy, C₁-C₄-Alkylthio, C₁-C₄-Alkylsulfinyl, C₁-C₄-Alkylsulfonyl, -COORⱼ, -CONRₖRₘ, -CORₙ, -SO₂NRₖRₘ oder -OSO₂-C₁-C₄-Alkyl;
R₉ für Wasserstoff, Halogen, Cyano, Nitro, C₁-C₄-Alkyl, C₁-C₄-Halogenalkyl, C₁-C₄-Alkylthio, C₁-C₄-Alkylsulfinyl, C₁-C₄-Alkylsulfonyl, -COORⱼ, -CONRₖRₘ, -CORₙ, -SO₂NRₖRₘ, -OSO₂-C₁-C₄-Alkyl, C₁-C₆-Alkoxy, oder C₁-C₆alkoxy substituiert durch C₁-C₄-Alkoxy oder Halogen, C₃-C₆-Alkenyloxy, oder C₃-C₆-Alkenyloxy substituiert durch Halogen, oder C₃-C₆-Alkinyloxy, oder R₄₉ und R₅₀ zusammen bilden eine C₃-C₄-Alkylenbrücke, die durch Halogen oder C₁-C₄-Alkyl substituiert sein kann, oder bilden eine C₃-C₄-Alkenylenbrücke, die durch Halogen oder C₁-C₄-Alkyl substituiert sein kann, oder bilden eine C₄-Alkadienylenbrücke, die durch Halogen oder C₁-C₄-Alkyl substituiert sein kann;
R₅₀ und Rₕ unabhängig voneinander für Wasserstoff, Halogen, C₁-C₄-Alkyl, Trifluormethyl, C₁-C₆-Alkoxy, C₁-C₆-Alkylthio oder -COORⱼ;
R_{c} für Wasserstoff, Halogen, Nitro, C₁-C₄-Alkyl oder Methoxy; R_{d} für Wasserstoff, Halogen, Nitro, C₁-C₄-Alkyl, C₁-C₄-Alkoxy, C₁-C₄Alkylthio, C₁-C₄-Alkylsulfinyl, C₁-C₄-Alkylsulfonyl, -COORⱼ oder CONRₖRₘ;
Rₑ für Wasserstoff, Halogen, C₁-C₄-Alkyl, -COORⱼ, Trifluormethyl or Methoxy, oder R_{d} und Rₑ bilden zusammen eine C₃-C₄-Alkylenbrücke;
Rp für Wasserstoff, Halogen, C₁-C₄-Alkyl, -COORⱼ, Trifluormethyl or Methoxy; Rq für Wasserstoff, Halogen, Nitro, C₁-C₄-Alkyl, C₁-C₄-Alkoxy, C₁-C₄Alkylthio, C₁-C₄-Alkylsulfinyl, C₁-C₄-Alkylsulfonyl, -COORⱼ oder CONRₖRₘ; , oder Rp und Rq bilden zusammen eine C₃-C₄-Alkylenbrücke;
Rr für Wasserstoff, Halogen, C₁-C₄-Alkyl, -COORⱼ, Trifluormethyl or Methoxy; Rs für Wasserstoff, Halogen, Nitro, C₁-C₄-Alkyl, C₁-C₄-Alkoxy, C₁-C₄Alkylthio, C₁-C₄-Alkylsulfinyl, C₁-C₄-Alkylsulfonyl, -COORⱼ oder CONRₖRₘ; , oder Rr und Rs bilden zusammen eine C₃-C₄-Alkylenbrücke;
Rt für Wasserstoff, Halogen, C₁-C₄-Alkyl, -COORⱼ, Trifluormethyl or Methoxy; Ru für Wasserstoff, Halogen, Nitro, C₁-C₄-Alkyl, C₁-C₄-Alkoxy, C₁-C₄Alkylthio, C₁-C₄-Alkylsulfinyl, C₁-C₄-Alkylsulfonyl, -COORⱼ oder CONRₖRₘ;, oder Rv und Ru bilden zusammen eine C₃-C₄-Alkylenbrücke;
R_{f} und Rv für Wasserstoff, Halogen oder C₁-C₄-Alkyl;
Rₓ und R_{y} unabhängig voneinander für Wasserstoff, Halogen, C₁-C₄-Alkyl, C₁-C₄-Alkoxy, C₁-C₄-Alkylthio, -COOR₅₄, Trifluoromethyl, Nitro oder Cyano;
Rⱼ, Rₖ und Rₘ unabhängig voneinander für Wasserstoff oder C₁-C₄-Alkyl; oder
Rₖ und Rₘ bilden zusammen eine C₄-C₆-Alkylenbrücke, die durch Sauerstoff, NH oder -N(C₁-C₄-Alkyl)- unterbrochen sein kann;
Rₙ für C₁-C₄-Alkyl, Phenyl, oder durch Halogen, C₁-C₄-Alkyl, Methoxy, Nitro oder Trifluormethyl substituiertes Phenyl;
R₅₄ für Wasserstoff, C₁-C₁₀-Alkyl, C₁-C₄-Alkoxy-C₁-C₄-alkyl, C₁-C₄-Alkylthio-C₁-C₄-alkyl, Di-C₁-C₄-alkylamino-C₁-C₄-alkyl, Halogen-C₁-C₈-alkyl, C₂-C₈-Alkenyl, Halogen-C₂-C₈-alkenyl, C₃-C₈-Alkinyl, C₃-C₇-Cycloalkyl, Halogen-C₃-C₇-cycloalkyl, C₁-C₈-Alkylcarbonyl, Allylcarbonyl, C₃-C₇-Cycloalkylcarbonyl, Benzoyl, das unsubstituiert oder am Phenylring gleich oder verschieden bis zu dreifach durch Halogen, C₁-C₄-Alkyl, Halogen-C₁-C₄-alkyl, Halogen-C₁-C₄-alkoxy oder C₁-C₄-Alkoxy substituiert ist; oder Furoyl, Thienyl; oder C₁-C₄-Alkyl substituiert durch Phenyl, Halogenphenyl, C₁-C₄-Alkylphenyl, C₁-C₄-Alkoxyphenyl, Halogen-C₁-C₄-alkylphenyl, Halogen-C₁-C₄-alkoxyphenyl, C₁-C₆-Alkoxycarbonyl, C₁-C₄-Alkoxy-C₁-C₈-alkoxycarbonyl, C₃-C₈-Alkenyloxycarbonyl, C₃-C₈-Alkinyloxycarbonyl, C₁-C₈-Alkylthiocarbonyl, C₃-C₈-Alkenylthiocarbonyl, C₃-C₈-Alkinylthiocarbonyl, Carbamoyl, Mono-C₁-C₄-alkylaminocarbonyl, Di-C₁-C₄-alkylaminocarbonyl; oder Phenylaminocarbonyl, das unsubstituiert oder am Phenyl gleich oder verschieden bis zu dreifach durch Halogen, C₁-C₄-Alkyl, Halogen-C₁-C₄-alkyl, Halogen-C₁-C₄-alkoxy oder C₁-C₄-Alkoxy oder einfach durch Cyano oder Nitro substituiert ist, oder Dioxolan-2-yl, das unsubstituiert ist oder durch ein oder zwei C₁-C₄-Alkylreste substituiert ist, oder Dioxan-2-yl, das unsubstituiert ist oder durch ein oder zwei C₁-C₄-Alkylreste substituiert ist, oder C₁-C₄-Alkyl, das durch Cyano, Nitro, Carboxyl oder C₁-C₈-Alkylthio-C₁-C₈-alkoxycarbonyl substituiert ist, bedeutet;
oder einer Verbindung der Formel XIV 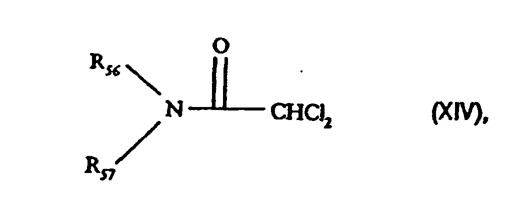 worin R₅₆ und R₅₇ unabhängig voneinander für C₁-C₆-Alkyl oder C₂-C₆-Alkenyl; oder R₅₆ und R₅₇ zusammen für 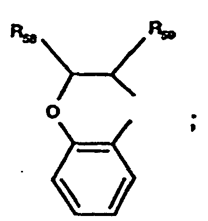 R₅₈ und R₅₉ unabhängig voneinander für Wasserstoff oder C₁-C₆-Alkyl; oder R₅₆ und R₅₇ zusammen für 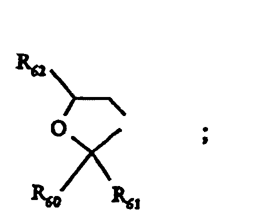 R₆₀ und R₆₁ unabhängig voneinander für C₁-C₄-Alkyl, oder R₆₀ und R₆₁ zusammen -(CH₂)₅- ;
R₆₂ für Wasserstoff, C₁-C₄-Alkyl oder 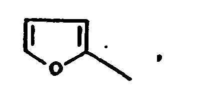 oder R₅₆ und R₅₇ zusammen für 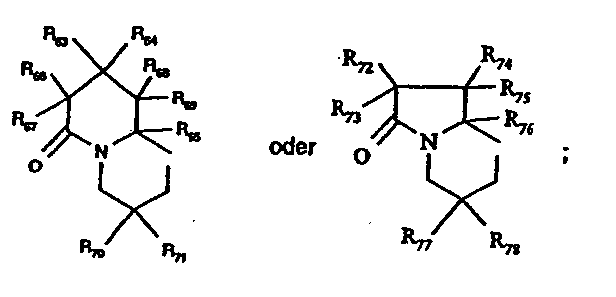 R₆₃, R₆₄, R₆₅, R₆₆, R₆₇, R₆₈, R₆₉, R₇₀, R₇₁, R₇₂, R₇₃, R₇₄, R₇₅, R₇₆, R₇₇ und R₇₈ unabhängig voneinander für Wasserstoff oder C₁-C₄-Alkyl stehen;
oder einer Verbindung der Formel XV 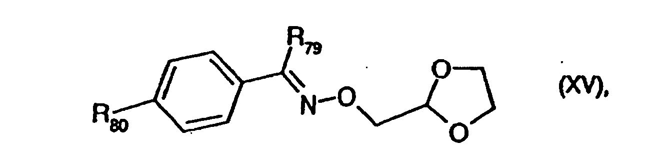 worin R₈₀ Wasserstoff oder Chlor und R₇₉ Cyano oder Trifluormethyl bedeutet,
oder eine Verbindung der Formel XVI 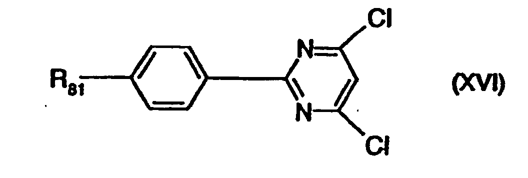 worin R₈₁ Wasserstoff oder Methyl bedeutet,
oder einer Verbindung der Formel XVII 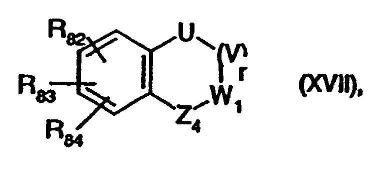 worin
R₈₂ Wasserstoff, C₁-C₄-Alkyl oder C₁-C₄-Alkyl substituiert durch C₁-C₄-Alkyl-X₂- oder C₁-C₄-Halogenalkyl-X₂- bedeutet, oder für C₁-C₄-Halogenalkyl, Nitro, Cyano, -COOR₈₅, -NR₈₆R₈₇, -SO₂NR₈₈R₈₉ oder -CONR₉₀R₉₁ steht;
R₈₃ Wasserstoff, Halogen, C₁-C₄-Alkyl, Trifluormethyl, C₁-C₄-Alkoxy oder C₁-C₄-Halogenalkoxy bedeutet;
R₈₄ Wasserstoff, Halogen oder C₁-C₄-Alkyl bedeutet;
U, V, W₁ und Z₄ unabhängig voneinander Sauerstoff, Schwefel, C(R₉₂)R₉₃, Carbonyl, NR₉₄, eine Gruppe 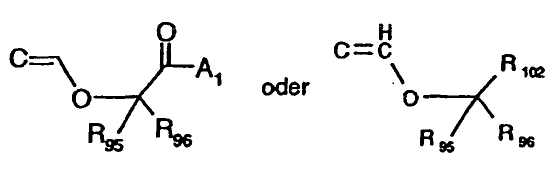 bedeuten, worin R₁₀₂ C₂-C₄-Alkenyl oder C₂-C₄-Alkinyl bedeutet; mit den Maßgaben, daß
a) mindestens eines der Ringglieder U, V, W₁ oder Z₄ Carbonyl ist, und ein zu diesem bzw. diesen Ringgliedem benachbartes Ringglied die Gruppe 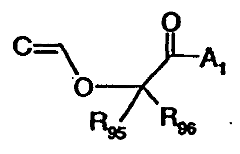 oder 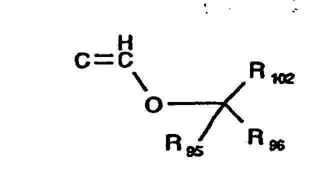 bedeutet, wobei diese Gruppe nur einmal vorkommt; und
b) zwei benachbarte Ringglieder U und V, V und W₁ und W₁ und Z₄ nicht gleichzeitig Sauerstoff bedeuten können;
R₉₅ und R₉₆ unabhängig voneinander Wasserstoff oder C₁-C₈-Alkyl bedeuten; oder
R₉₅ und R₉₆ zusammen eine C₂-C₆-Alkylengruppe bilden;
A₁ R₉₉-Y₁- oder -NR₉₇R₉₈;
X₂ Sauerstoff oder -S(O)ₛ;
Y₁ Sauerstoff oder Schwefel bedeuten;
R₉₉ Wasserstoff, C₁-C₈-Alkyl, C₁-C₈-Halogenalkyl, C₁-C₄-Alkoxy-C₁-C₈-alkyl, C₃-C₆-Alkenyloxy-C₁-C₈-alkyl oder Phenyl-C₁-C₈-alkyl bedeutet, wobei der Phenylring durch Halogen, C₁-C₄-Alkyl, Trifluormethyl, Methoxy oder Methyl-S(O)ₛ- substituiert sein kann, oder C₃-C₆-Alkenyl, C₃-C₆-Halogenalkenyl, Phenyl-C₃-C₆-alkenyl, C₃-C₆-Alkinyl, Phenyl-C₃-C₆-alkinyl, Oxetanyl, Furyl oder Tetrahydrofuryl bedeutet;
R₈₅ Wasserstoff oder C₁-C₄-Alkyl;
R₈₆ Wasserstoff, C₁-C₄-Alkyl oder C₁-C₄-Alkylcarbonyl bedeuten;
R₈₇ Wasserstoff oder C₁-C₄-Alkyl bedeutet; oder
R₈₆ und R₈₇ zusammen eine C₄- oder C₅-Alkylengruppe bilden;
R₈₈, R₈₉, R₉₀ und R₉₁ unabhängig voneinander Wasserstoff oder C₁-C₄-Alkyl bedeuten; oder
R₈₈ zusammen mit R₈₉ oder R₉₀ zusammen mit R₉₁ unabhängig voneinander C₄- oder C₅-Alkylen sind, wobei ein Kohlenstoffatom durch Sauerstoff oder Schwefel, oder ein oder zwei Kohlenstoffatome durch -NR₁₀₀- ersetzt sein können;
R₉₂, R₁₀₀ und R₉₃ unabhängig voneinander Wasserstoff oder C₁-C₈-Alkyl sind; oder
R₉₂ und R₉₃ zusammen C₂-C₆-Alkylen sind;
R₉₄ Wasserstoff oder C₁-C₈-Alkyl bedeutet;
R₉₇ Wasserstoff, C₁-C₈-Alkyl, Phenyl oder Phenyl-C₁-C₈-alkyl bedeutet, wobei die Phenylringe durch Fluor, Chlor, Brom, Nitro, Cyano, -OCH₃, C₁-C₄-Alkyl oder CH₃SO₂-substituiert sein können, oder für C₁-C₄-Alkoxy-C₁-C₈-alkyl, C₃-C₆-Alkenyl oder C₃-C₆-Alkinyl;
R₉₈ Wasserstoff, C₁-C₈-Alkyl, C₃-C₆-Alkenyl oder C₃-C₆-Alkinyl steht; oder
R₉₇ und R₉₈ zusammen C₄- oder C₅-Alkylen bedeuten, wobei ein Kohlenstoffatom durch Sauerstoff oder Schwefel, oder ein oder zwei Kohlenstoffatome durch -NR₁₀₁- ersetzt sein können;
R₁₀₁ Wasserstoff oder C₁-C₄-Alkyl bedeutet;
r 0 oder 1 ist; und
s 0, 1 oder 2 bedeutet,
oder eine Verbindung der Formel XVIII 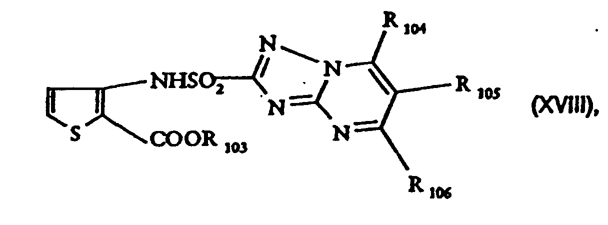 worin R₁₀₃ Wasserstoff, C₁-C₆-Alkyl, C₃-C₆-Cycloalkyl, C₃-C₆-Alkenyl oder C₃-C₆-Alkinyl; und R₁₀₄, R₁₀₅ und R₁₀₆ unabhängig voneinander Wasserstoff, C₁-C₆-Alkyl, C₃-C₆-Cycloalkyl oder C₁-C₆-Alkoxy bedeuten, mit der Maßgabe, daß einer der Substituenten R₁₀₄, R₁₀₅ und R₁₀₆ verschieden von Wasserstoff ist;
oder eine Verbindung der Formel XIX 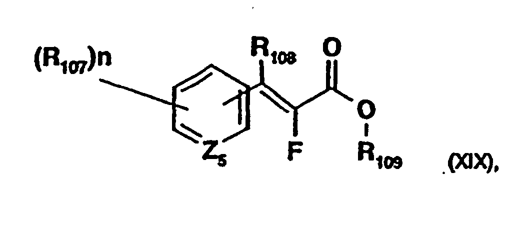 worin Z₅ N oder CH, n für den Fall, daß Z₅ gleich N ist, 0, 1, 2 oder 3 und für den Fall, daß Z₅ CH ist, 0, 1, 2, 3 oder 4 bedeutet, R₁₀₇ Halogen, C₁-C₄-Alkyl, C₁-C₄-Halogenalkyl, C₁-C₄-Alkoxy, C₁-C₄-Halogenalkoxy, Nitro, C₁-C₄-Alkylthio, C₁-C₄-Alkylsulfonyl, C₁-C₄-Alkoxycarbonyl, Phenyl oder Phenoxy, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Phenyl oder Phenoxy bedeutet;
R₁₀₈ Wasserstoff oder C₁-C₄-Alkyl bedeutet, R₁₀₉ Wasserstoff, C₁-C₄-Alkyl, C₃-C₆-Cycloalkyl, C₂-C₆-Alkenyl, C₂-C₆-Alkinyl, C₁-C₄-Halogenalkyl, C₂-C₆-Halogenalkenyl, C₂-C₆-Halogenalkinyl, C₁-C₄-Alkylthio-C₁-C₄-alkyl, C₁-C₄-Alkylsulfonyl-C₁-C₄-alkyl, C₁-C₄-Alkoxy-C₁-C₄-alkyl, C₁-C₄-Alkenyloxy- C₁-C₄-alkyl oder C₁-C₄-Alkinyloxy-C₁-C₄-alkyl ist;
oder eine Verbindung der Formel XX 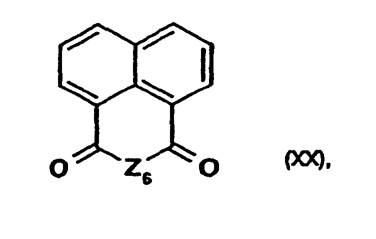 worin Z₆ Sauerstoff oder N-R₁₁₀ und R₁₁₀ eine Gruppe der Formel 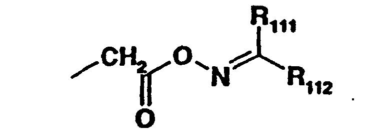 bedeutet, worin R₁₁₁ und R₁₁₂ unabhängig voneinander Cyano, Wasserstoff, C₁-C₄-Alkyl, C₃-C₆-Cycloalkyl, C₂-C₆-Alkenyl, Aryl, Phenyl oder Heteroaryl, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Phenyl, Aryl oder Heteroaryl bedeuten;
oder eine Verbindung der Formel XXI 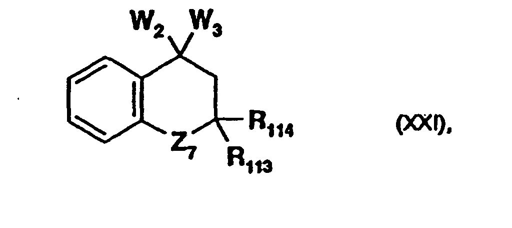 worin Z₇ Sauerstoff, Schwefel, S=O, SO₂ oder CH₂ bedeutet, R₁₁₃ und R₁₁₄ unabhängig voneinander Wasserstoff, Halogen oder C₁-C₄-Alkyl bedeuten, W₂ und W₃ unabhängig voneinander CH₂COOR₁₁₅ oder COOR₀₁₁₅ oder zusammen eine Gruppe der Formel -(CH₂)C(O)-O-C(O)-(CH₂)- bedeuten, und R₁₁₅ und R₀₁₁₅ unabhängig voneinander Wasserstoff, C₁-C₄-Alkyl, C₂-C₄-Alkenyl, C₂-C₆-Alkinyl, C₃-C₆-Cycloalkyl, C₁-C₄-Halogenalkyl, ein Metall- oder ein Ammonium- Kation bedeuten;
oder eine Verbindung der Formel XXII 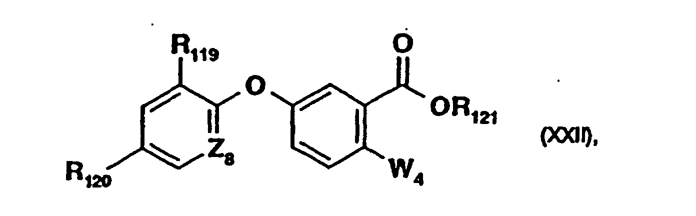 worin R₁₁₉ und R₁₂₀ unabhängig voneinander Wasserstoff, Halogen oder C₁-C₄-Halogenalkyl sind, R₁₂₁ Wasserstoff, C₁-C₄-Alkyl, C₃-C₄-Alkenyl, C₃-C₄-Alkinyl, C₁-C₄-Halogenalkyl, C₃-C₆-Cycloalkyl, ein Metalkation oder ein Ammoniumkation bedeuten, Z₈ N, CH, C-F oder C-Cl bedeuten und W₄ eine Gruppe der Formel 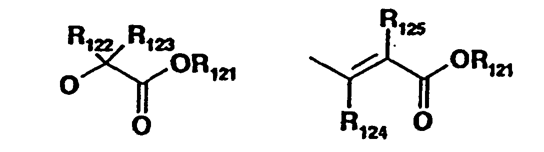 bedeutet, worin R₁₂₂ und R₁₂₃ unabhängig voneinander Wasserstoff oder C₁-C₄-Alkyl und R₁₂₄ und R₁₂₅ unabhängig voneinander Wasserstoff oder C₁-C₄-Alkyl bedeuten;
oder eine Verbindung der Formel XXIII 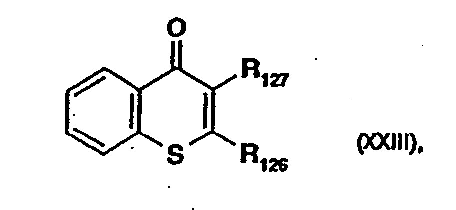 worin R₁₂₆ Wasserstoff, Cyano, Halogen, C₁-C₄-Alkyl, C₃-C₆-Cycloalkyl, C₁-C₄-Alkoxy, C₁-C₄-Alkoxycarbonyl, C₁-C₄-Alkylthiocarbonyl, -NH-R_{128,} -C(O)NH-R₀₁₂₈, Aryl oder Heteroaryl, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Aryl oder Heteroaryl bedeutet;
R₁₂₇ Wasserstoff, Cyano, Nitro, Halogen, C₁-C₄-Alkyl, C₁-C₄-Halogenalkyl, C₁-C₄-Alkoxy, C₁-C₄-Thioalkyl bedeuten, und
R₁₂₈ und R₀₁₂₈ unabhängig voneinander C₁-C₄-Alkyl, C₁-C₄-Halogenalkyl, C₃-C₄-Alkenyl, C₃-C₄-Alkinyl, C₃-C₄-Cycloalkyl, Aryl oder Heteroaryl, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Aryl oder Heteroaryl, Formyl, C₁-C₄-Alkylcarbonyl, C₁-C₄-Alkylsufonyl bedeuten;
oder eine Verbindung der Formel XXIV 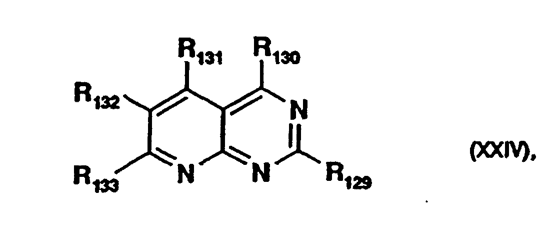 worin R₁₂₉ und R₁₃₀ unabhängig voneinander Wasserstoff, C₁-C₄-Alkyl, C₁-C₄-Halogenalkyl, C₁-C₄-Alkoxy, Mono-C₁-C₈- oder Di-C₁-C₈-Alkylamino, C₃-C₆-Cycloalkyl, C₁-C₄-Thioalkyl, Phenyl oder Heteroaryl sind, R₁₃₁ die Bedeutung von R₁₂₉ hat und zusätzlich OH, NH₂, Halogen, Di- C₁-C₄-Aminoalkyl, C₁-C₄-Alkylthio, C₁-C₄-Alkylsulfonyl oder C₁-C₄-Alkoxycarbonyl ist, R₁₃₂ die Bedeutung von R₁₂₉ hat und zusätzlich Cyano, Nitro, Carboxyl, C₁-C₄-Alkoxycarbonyl, Di- C₁-C₄-Aminoalkyl, C₁-C₄-Alkylthio, C₁-C₄- Alkylsulfonyl, SO₂-OH, i- C₁-C₄-Aminoalkylsulfonyl oder C₁-C₄-Alkoxysulfonyl ist, R₁₃₃ die Bedeutung von R₁₂₉ hat und zusätzlich OH, NH₂, Halogen, Di- C₁-C₄-Aminoalkyl, Pyrrolidin1-yl, Piperidin-1-yl, Morpholin-1-yl, C₁-C₄-Alkylthio, C₁-C₄-Alkylsulfonyl, C₁-C₄-Alkoxycarbonyl, Phenoxy, Naphtoxy, Phenylamino, Benzoyloxy oder Phenylsulfonyloxy ist;
oder eine Verbindung der Formel XXV 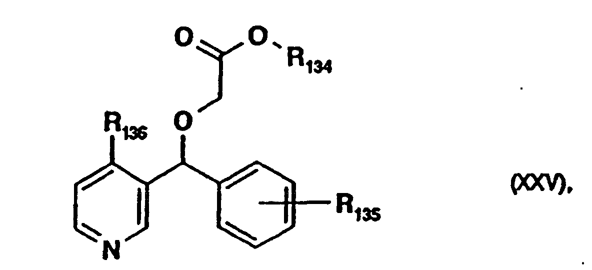 worin R₁₃₄ Wasserstoff, C₄-Alkyl, C₁-C₄-Halogenalkyl, C₂-C₄-Alkenyl, C₂-C₄-Alkinyl oder C₁-C₄-Alkoxy-C₁-C₄-Alkyl bedeutet, R₁₃₅ Wasserstoff, Halogen, C₁-C₄-Alkyl, C₁-C₄-Halogenalkyl oder C₁-C₄-Alkoxy und R₁₃₆ Wasserstoff, Halogen, C₁-C₄-Alkyl, C₁-C₄-Halogenalkyl oder C₁-C₄-Alkoxy bedeuten, mit der Maßgabe, daß R₁₃₅ und R₁₃₆ nicht gleichzeitig Wasserstoff
bedeuten,
oder der Formel XXVI 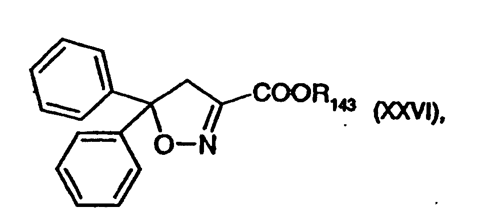 worin
R₁₄₃ Wasserstoff, ein Alkali-, Erdalkali-, Sulfonium- oder Ammonium-Kation oder Ethyl bedeutet;
oder der Formel XXVII 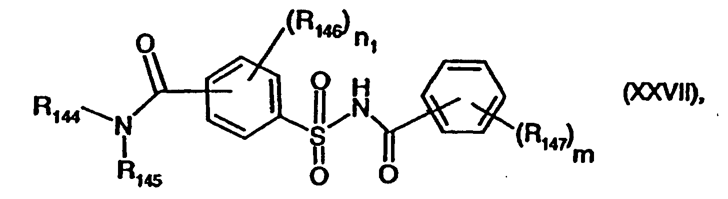 worin R₁₄₄ und R₁₄₅ unabhängig voneinander Wasserstoff, C₁-C₆-Alkyl, C₂-C₆-Alkenyl, C₂-C₆-Alkinyl oder C₃-C₆-Cycloalkyl bedeuten;
R₁₄₆ Wasserstoff, Halogen, C₁-C₄-Alkyl, C₁-C₆-Halogenalkyl oder C₁-C₆-Halogenalkoxy bedeutet;
R₁₄₇ Wasserstoff, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkoxycarbonyl oder Nitro bedeutet;
n₁ 0, 1, 2 oder 3; und
m 1 oder 2 bedeutet;
oder der Formel XXVIII 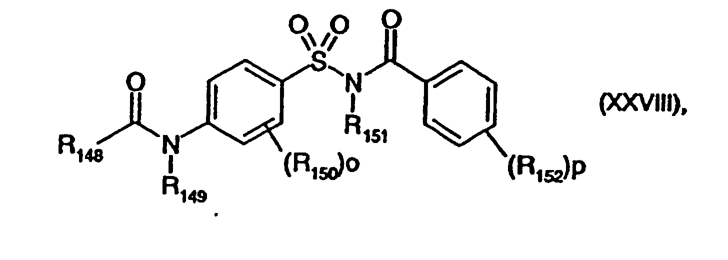 worin
R₁₄₈ Wasserstoff, C₁-C₆-Alkyl, C₁-C₆-Alkoxy, C₁-C₆-Alkylthio, C₃-C₈-Cycloalkyl, Phenyl, Phenyl-C₁-C₆-alkyl oder Heteroaryl bedeutet; wobei die genannten Gruppen durch Halogen, Cyano, Nitro, Amino, Hydroxy, Carbonyl, Carboxyl, Formyl, Carbonamid oder Sulfonamid substituiert sein können;
R₁₄₉ Wasserstoff, C₁-C₆-Alkyl oder C₁-C₄-Halogenalkyl bedeutet;
jedes R₁₅₀ unabhängig Wasserstoff, Halogen, C₁-C₄-Alkyl, C₁-C₄-Halogenalkyl, C₁-C₄-Alkoxy, C₁-C₄-Alkylthio, C₁-C₄-Alkylsulfonyl, Cyano, Nitro, Formyl oder Carboxyl bedeutet; R₁₅₁ Wasserstoff, C₁-C₆-Alkyl oder C₁-C₄-Halogenalkyl bedeutet;
jedes R₁₅₂ unabhängig Wasserstoff, Halogen, C₁-C₄-Alkyl, C₁-C₄-Halogenalkyl, C₁-C₄-Alkoxy, C₁-C₄-Alkylthio, C₁-C₄-Alkylsulfonyl, Cyano, Nitro, Formyl oder Carboxyl bedeutet;
O für 0, 1, oder 2 steht, und
p 0, 1 oder 2 bedeutet;
oder der Formel XXIX 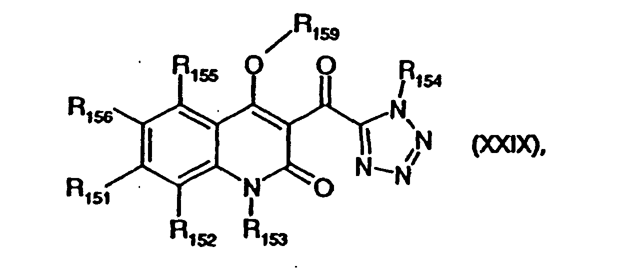 worin
R₁₅₉ Wasserstoff, Formyl, C₁₋₆-Alkylcarbonyl, C₁₋₆-Alkenylcarbonyl, C₁₋₆-Alkinylcarbonyl, C₁₋₆-Alkoxycarbonyl, C₁₋₆-Alkylthiocarbonyl, C₃₋₈-Cycloalkylcarbonyl, Phenyl-C₁₋₆-alkylcarbonyl, Phenylcarbonyl. C₁₋₆-Alkylsulfonyl, C₁₋₆-Alkenylsulfonyl oder Phenylsulfonyl bedeutet, wobei die vorstehend genannten Kohlenwasserstoffgruppen durch ein oder mehrere Halogenatome, Cyano, Nitro, Amino, Methoxy, Ethoxy oder Phenyl substituiert sein können;
R₁₅₃ Wasserstoff, C₁₋₆-Alkyl, C₁₋₆-Alkenyl, C₁₋₆-Alkinyl, C₃₋₈-Cycloalkyl, Formyl, C₁₋₆-Alkylcarbonyl, C₁₋₆-Alkenylcarbonyl, C₁₋₆-Alkinylcarbonyl, C₁₋₆-Alkoxycarbonyl, C₁₋₆-Alkylthiocarbonyl, C₃₋₈-Cycloalkylcarbonyl, C₁₋₆-Alkylsulfonyl, C₁₋₆-Alkenylsulfonyl oder Phenylsulfonyl bedeutet, wobei die vorstehend genannten Kohlenwasserstoffgruppen durch ein oder mehrere Halogenatome, Cyano, Nitro, Amino, Methoxy, Ethoxy oder Phenyl substituiert sein können;
R₁₅₄ Wasserstoff, C₁₋₆-Alkyl, C₁₋₆-Alkenyl, C₁₋₆-Alkinyl, C₃₋₈-Cycloalkyl, Formyl, C₁₋₆-Alkylcarbonyl, C₁₋₆-Alkenylcarbonyl, C₁₋₆-Alkinylcarbonyl, C₁₋₆-Alkoxycarbonyl, C₁₋₆-Alkylthiocarbonyl, C₃₋₆-Cycloalkylcarbonyl, C₁₋₆-Alkylsulfonyl, C₁₋₆-Alkenylsulfonyl oder Phenylsulfonyl bedeutet, wobei die vorstehend genannten Kohlenwasserstoffgruppen durch ein oder mehrere Halogenatome, Cyano, Nitro, Amino, Methoxy, Ethoxy oder Phenyl substituiert sein können;
R₁₅₅, R₁₅₆, R₁₅₇, und R₁₅₈ unabhängig voneinander Wasserstoff, Halogen, Amino, C₁₋₃-Alkylamino, C₁₋₆-Dialkylamino, Hydroxy, Cyano, Nitro, Formyl, Carboxyl, C₁₋₆-Alkoxy, C₁₋₆-Halogenalkoxy, C₁₋₆-Alkylcarbonyl, C₁₋₆-Alkoxycarboxyl, C₁₋₆-Alkyl, C₁₋₆-Halogenalkyl, C₁₋₆-Alkenyl oder C₁₋₆-Alkinyl bedeuten;
oder R₁₅₃ und R₁₅₈ bilden gemeinsam mit den Ringatomen, an die sie gebunden sind, einen fünf- oder sechsgliedrigen teilgesättigten oder ungesättigten Ring, der bis zu 2 gleiche oder verschiedene Heteroatome aus der Gruppe Sauerstoff, Schwefel und Stickstoff enthalten kann, wobei dieser Ring durch einen Rest Oxo substituiert sein kann;
enthält.

8. Mittel nach Anspruch 7, **dadurch gekennzeichnet, daß** es eine herbizid-antagonistisch wirksamen Menge eines Safeners der Formel X, XI, XII, XIII, XIV, XV, XVI, XVII, XVIII, XIX, XX, XXI, XXII, XXIII, XXIV oder XXV enthält.

9. Verfahren zum selektiven Bekämpfen von Unkräutern und Gräsern in Nutzpflanzenkulturen, **dadurch gekennzeichnet, daß** man die Nutzpflanzen, deren Samen oder Stecklinge oder deren Anbaufläche mit einer herbizid wirksamen Menge eines Herbizids der Formel I und einer herbizid-antagonistisch wirksamen Menge eines Safeners der Formel X, XI, XII, XIII, XIV, XV, XVI, XVII, XVIII, XIX, XX, XXI, XXII, XXIII, XXIV, XXV, XXVI, XXVII, XXVIII oder XXIX behandelt.

10. Verfahren nach Anspruch 9, **dadurch gekennzeichnet, daß** man die Nutzpflanzen, deren Samen oder Stecklinge oder deren Anbaufläche mit einer herbizid-antagonistisch wirksamen Menge eines Safeners der Formel X, XI, XII, XIII, XIV, XV, XVI, XVII, XVIII, XIX, XX, XXI, XXII, XXIII, XXIV oder XXV behandelt.

11. Mittel nach Anspruch 4, **dadurch gekennzeichnet, daß** es Sprühtank-Adjuvantien enthält.

12. Mittel nach Anspruch 7, **dadurch gekennzeichnet, daß** es Sprühtank-Adjuvantien enthält.

## Claims

1. A compound of formula I 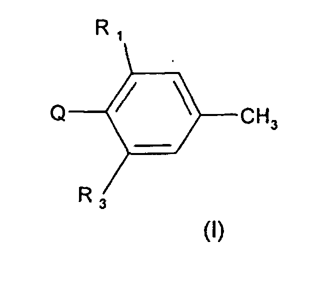 wherein
R₁ and R₃ are each independently of the other ethyl, haloethyl, ethynyl, C₁-C₂alkoxy, C₁-C₂haloalkoxy or C₁-C₂alkylcarbonyl;
Q is a group 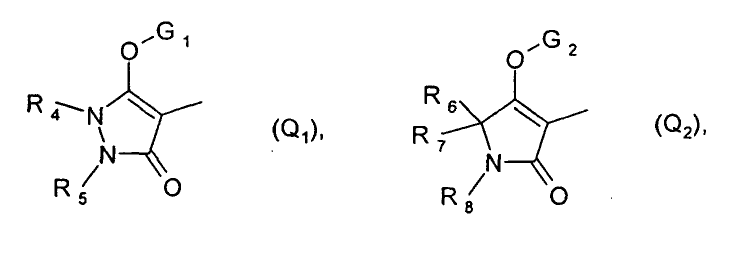 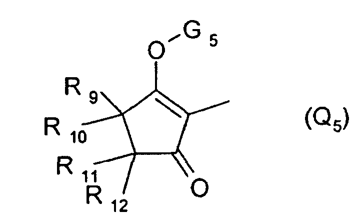 or 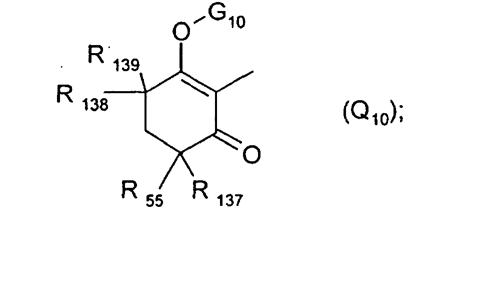 R₄ and R₅ are each independently of the other C₁-C₁₀alkyl, C₂-C₁₀alkenyl, C₂-C₁₀alkynyl, C₁-C₁₀haloalkyl, C₂-C₁₀alkoxyalkyl, C₃-C₁₀alkenyloxyalkyl, C₃-C₁₀alkynyloxyalkyl, C₂-C₁₀alkylthioalkyl, C₂-C₁₀alkylsulfinylalkyl, C₂-C₁₀alkylsulfonylalkyl, C₂-C₁₀alkylcarbonyl-alkyl, C₂-C₁₀-N-alkoxy-iminoalkyl, C₂-C₁₀alkoxycarbonylalkyl, C₁-C₁₀aminoalkyl, C₃-C₁₀dialkylaminoalkyl, C₂-C₁₀alkylaminoalkyl, C₁-C₁₀cyanoalkyl, C₄-C₁₀cycloalkylalkyl, C₁-C₁₀phenylalkyl, C₁-C₁₀-heteroarylalkyl, C₁-C₁₀phenoxyalkyl, C₁-C₁₀heteroaryloxyalkyl, C₁-C₁₀alkylideneaminooxyalkyl, C₁-C₁₀nitroalkyl, C₁-C₁₀trialkylsilylalkyl, C₂-C₁₀alkylaminocarbonylalkyl, C₂-C₁₀dialkylaminocarbonylalkyl, C₂-C₁₀alkylaminocarbonyloxyalkyl, C₃-C₁₀dialkylaminocarbonyloxalkyl, C₂-C₁₀alkoxycarbonylaminoalkyl, C₁-C₁₀-N-alkoxycarbonyl-N-alkylamino-alkyl, C₁-C₁₀cycloalkyl, aryl or heteroaryl; or
R₄ and R₅, together with the atoms to which they are bonded, form a 5- to 7-membered cyclic group that may contain one or two hetero atoms selected from nitrogen, oxygen and sulfur and that, in addition, may contain a fused or spire-bound alkylene or alkenylene chain consisting of from 2 to 6 carbon atoms, which chain may in turn contain one or two hetero atoms selected from oxygen and sulfur, wherein the cyclic group may be substituted by phenyl or benzyl, which in turn may be substituted by halogen, C₁-C₆alkyl, C₁-C₆haloalkyl, C₃-C₆cycloalkyl, hydroxy, C₁-C₆alkoxy, C₁-C₆alkoxy-C₁-C₆alkoxy, C₁-C₆haloalkoxy or by nitro;
R₆ is C₁-C₁₀alkyl, C₂-C₁₀alkenyl, C₂-C₁₀alkynyl, C₁-C₁₀haloalkyl, C₂-C₁₀alkoxyalkyl, C₃-C₁₀-alkenyloxyalkyl, C₃-C₁₀alkynyloxyalkyl, C₂-C₁₀alkylthioalkyl, C₂-C₁₀alkylsulfinylalkyl, C₂-C₁₀-alkylsulfonylalkyl, C₂-C₁₀alkylcarbonyl-alkyl, C₃-C₁₀cycloalkyl, aryl or heteroaryl;
R₇ is hydrogen, C₁-C₁₀alkyl, C₂-C₁₀alkenyl, C₂-C₁₀alkynyl or C₂-C₁₀alkoxyalkyl;
R₈ is hydrogen, C₁-C₁₀alkyl, C₁-C₁₀haloalkyl, C₂-C₁₀alkoxyalkyl, C₃-C₁₀alkenyloxyalkyl, C₃-C₁₀-alkynyloxyalkyl, C₂-C₁₀alkylthioalkyl, C₂-C₁₀alkylsulfinylalkyl, C₂-C₁₀alkylsulfonylalkyl, C₃-C₁₀-cycloalkyl, aryl or heteroaryl; or
R₆ and R₇, together with the atom to which they are bonded, form a saturated 3- to 7-membered cyclic group that may contain one or two hetero atoms selected from nitrogen, oxygen and sulfur; or R₆ and R₈, together with the atoms to which they are bonded, form a 5-to 7-membered cyclic group that may contain one or two hetero atoms selected from nitrogen, oxygen and sulfur;
R₉, R₁₀, R₁₁ and R₁₂ are each independently of the others C₁-C₁₀alkyl, C₂-C₁₀alkenyl, C₂-C₁₀-alkynyl, C₁-C₁₀haloalkyl, C₂-C₁₀alkoxyalkyl, C₃-C₁₀alkenyloxyalkyl, C₃-C₁₀alkynyloxyalkyl, C₂-C₁₀alkylthialkyl, C₂-C₁₀alkylsulfinylalkyl, C₂-C₁₀alkylsulfonylalkyl, C₂-C₁₀alkylcarbonyl-alkyl, C₃-C₁₀cycloalkyl, aryl or heteroaryl; or
R₁₀ and R₁₁ or R₉ and R₁₀, together with the atoms to which they are bonded, form a 5- to 7-membered cyclic group that may contain one or two hetero atoms selected from nitrogen, oxygen and sulfur;
G₁, G₂, G₅ and G₁₀ are each independently of the others hydrogen, -C(X₁)-R₂₀, -C(X₂)-X₃-R₂₁, -C(X₄)-N(R₂₂)-R₂₃, -SO₂-R₂₄, an alkali metal cation, alkaline earth metal cation, sulfonium cation or ammonium cation, -P(X₅)(R₂₅)-R₂₆ or -CH₂-X₆-R₂₇;
X₁, X₂, X₃, X₄, X₅ and X₆ are each independently of the others oxygen or sulfur;
R₂₀, R₂₁, R₂₂ and R₂₃ are each independently of the others hydrogen, C₁-C₁₀alkyl, C₂-C₁₀-alkenyl, C₂-C₁₀alkynyl, C₁-C₁₀haloalkyl, C₁-C₁₀cyanoalkyl, C₁-C₁₀nitroalkyl, C₁-C₁₀aminoalkyl, C₁-C₅alkylamino-C₁-C₅alkyl, C₂-C₈dialkylamino-C₁-C₅alkyl, C₃-C₇cycloalkyl-C₁-C₅alkyl, C₂-C₁₀alkoxy-alkyl, C₄-C₁₀alkenyloxy-alkyl, C₄-C₁₀alkynyloxy-alkyl, C₂-C₁₀alkylthio-alkyl, C₁-C₅alkylsulfoxyl-C₁-C₅alkyl, C₁-C₅alkylsulfonyl-C₁-C₅alkyl, C₂-C₈alkylideneaminooxy-C₁-C₅alkyl, C₁-C₅alkylcarbonyl-C₁-C₅alkyl, C₁-C₅alkoxycarbonyl-C₁-C₅alkyl, C₁-C₅aminocarbonyl-C₁-C₅alkyl, C₂-C₈dialkylamino-carbonyl-C₁-C₅alkyl, C₁-C₅alkylcarbonylamino-C₁-C₅alkyl, C₁-C₅alkylcarbonyl-(C₂-C₅alkyl)-aminoalkyl, C₃-C₆trialkylsilyl-C₁-C₅alkyl, phenyl-C₁-C₅alkyl, heteroaryl-C₁-C₅alkyl, phenoxy-C₁-C₅alkyl, heteroaryloxy-C₁-C₅alkyl, C₂-C₅-alkenyl, C₂-C₅haloalkenyl, C₃-C₈cycloalkyl, phenyl, or phenyl substituted by C₁-C₃alkyl, C₁-C₃haloalkyl, C₁-C₃alkoxy, C₁-C₃haloalkoxy, halogen, cyano or by nitro, or heteroaryl or heteroarylamino, or heteroaryl or heteroarylamino substituted by C₁-C₃alkyl, C₁-C₃haloalkyl, C₁-C₃alkoxy, C₁-C₃haloalkoxy, halogen, cyano or by nitro, diheteroarylamino, or diheteroarylamino substituted by C₁-C₃alkyl, C₁-C₃haloalkyl, C₁-C₃alkoxy, C₁-C₃haloalkoxy, halogen, cyano or by nitro, phenylamino, or phenylamino substituted by C₁-C₃alkyl, C₁-C₃haloalkyl, C₁-C₃alkoxy, C₁-C₃haloalkoxy, halogen, cyano or by nitro, diphenylamino, or diphenylamino substituted by C₁-C₃alkyl, C₁-C₃haloalkyl, C₁-C₃alkoxy, C₁-C₃haloalkoxy, halogen, cyano or by nitro, or C₃-C₇cycloalkylamino, di-C₃-C₇cycloalkylamino or C₃-C₇cycloalkoxy;
R₂₄, R₂₅ and R₂₆ are hydrogen, C₁-C₁₀alkyl, C₂-C₁₀alkenyl, C₂-C₁₀alkynyl, C₁-C₁₀haloalkyl, C₁-C₁₀cyanoalkyl, C₁-C₁₀nitroalkyl, C₁-C₁₀aminoalkyl, C₁-C₅alkylamino-C₁-C₅alkyl, C₂-C₈-dialkylamino-C₁-C₅alkyl, C₃-C₇cycloalkyl-C₁-C₅alkyl, C₂-C₁₀alkoxy-alkyl, C₄-C₁₀alkenyloxyalkyl, C₄-C₁₀alkynyloxy-alkyl, C₂-C₁₀alkylthio-alkyl, C₁-C₅alkylsulfoxyl-C₁-C₅alkyl, C₁-C₅alkylsulfonyl-C₁-C₅alkyl, C₂-C₈alkylideneaminooxy-C₁-C₅alkyl, C₁-C₅alkylcarbonyl-C₁-C₅alkyl, C₁-C₅alkoxycarbonyl-C₁-C₅alkyl, C₁-C₅amino-carbonyl-C₁-C₅alkyl, C₂-C₈dialkylaminocarbonyl-C₁-C₅alkyl, C₁-C₅alkylcarbonylamino-C₁-C₅alkyl, C₁-C₅alkylcarbonyl-(C₂-C₅alkyl)-aminoalkyl, C₃-C₆trialkylsilyl-C₁-C₅alkyl, phenyl-C₁-C₅alkyl, heteroaryl-C₁-C₅alkyl, phenoxy-C₁-C₅alkyl, heteroaryloxy-C₁-C₅alkyl, C₂-C₅alkenyl, C₂-C₅haloalkenyl, C₃-C₈cycloalkyl, phenyl, or phenyl substituted by C₁-C₃alkyl, C₁-C₃haloalkyl, C₁-C₃alkoxy, C₁-C₃haloalkoxy, halogen, cyano or by nitro, or heteroaryl or heteroarylamino, or heteroaryl or heteroarylamino substituted by C₁-C₃alkyl, C₁-C₃haloalkyl, C₁-C₃alkoxy, C₁-C₃haloalkoxy, halogen, cyano or by nitro, diheteroarylamino, or diheteroarylamino substituted by C₁-C₃alkyl, C₁-C₃haloalkyl, C₁-C₃alkoxy, C₁-C₃haloalkoxy, halogen, cyano or by nitro, phenylamino, or phenylamino substituted by C₁-C₃alkyl, C₁-C₃haloalkyl, C₁-C₃alkoxy, C₁-C₃haloalkoxy, halogen, cyano or by nitro, diphenylamino, or diphenylamino substituted by C₁-C₃alkyl, C₁-C₃haloalkyl, C₁-C₃-alkoxy, C₁-C₃haloalkoxy, halogen, cyano or by nitro, or C₃-C₇cycloalkylamino, di-C₃-C₇cycloalkylamino, C₃-C₇cycloalkoxy, C₁-C₁₀alkoxy, C₁-C₁₀haloalkoxy, C₁-C₅alkylamino, C₂-C₈dialkylamino, benzyloxy or phenoxy, wherein the benzyl and phenyl groups may in turn be substituted by C₁-C₃alkyl, C₁-C₃haloalkyl, C₁-C₃alkoxy, C₁-C₃haloalkoxy, halogen, cyano or by nitro;
R₂₇ is C₁-C₁₀alkyl, C₂-C₁₀alkenyl, C₂-C₁₀alkynyl, C₁-C₁₀haloalkyl, C₁-C₁₀cyanoalkyl, C₁-C₁₀-nitroalkyl, C₁-C₁₀aminoalkyl, C₁-C₅alkylamino-C₁-C₅alkyl, C₂-C₈dialkylamino-C₁-C₅alkyl, C₃-C₇cycloalkyl-C₁-C₅alkyl, C₂-C₁₀alkoxy-alkyl, C₄-C₁₀alkenyloxy-alkyl, C₄-C₁₀alkynyloxy-alkyl, C₂-C₁₀alkylthio-alkyl, C₁-C₅alkylsulfoxyl-C₁-C₅alkyl, C₁-C₅alkylsulfonyl-C₁-C₅alkyl, C₂-C₈alkylideneaminooxy-C₁-C₅alkyl, C₁-C₅alkylcarbonyl-C₁-C₅alkyl, C₁-C₅alkoxycarbonyl-C₁-C₅alkyl, C₁-C₅amino-carbonyl-C₁-C₅alkyl, C₂-C₈dialkylamino-carbonyl-C₁-C₅alkyl, C₁-C₅alkylcarbonylamino-C₁-C₅alkyl, C₁-C₅alkylcarbonyl-(C₂-C₅alkyl)-aminoalkyl, C₃-C₆trialkylsilyl-C₁-C₅alkyl, phenyl-C₁-C₅alkyl, heteroaryl-C₁-C₅alkyl, phenoxy-C₁-C₅alkyl, heteroaryloxy-C₁-C₅alkyl, C₂-C₅alkenyl, C₂-C₅haloalkenyl, C₃-C₈cycloalkyl, phenyl, or phenyl substituted by C₁-C₃alkyl, C₁-C₃haloalkyl, C₁-C₃alkoxy, C₁-C₃haloalkoxy, halogen, cyano or by nitro, or heteroaryl or heteroarylamino, or heteroaryl or heteroarylamino substituted by C₁-C₃alkyl, C₁-C₃haloalkyl, C₁-C₃alkoxy, C₁-C₃haloalkoxy, halogen, cyano or by nitro, diheteroarylamino, diheteroarylamino substituted by C₁-C₃alkyl, C₁-C₃haloalkyl, C₁-C₃alkoxy, C₁-C₃haloalkoxy, halogen, cyano or by nitro, or phenylamino, phenylamino substituted by C₁-C₃alkyl, C₁-C₃haloalkyl, C₁-C₃alkoxy, C₁-C₃haloalkoxy, halogen, cyano or by nitro, diphenylamino, diphenylamino substituted by C₁-C₃alkyl, C₁-C₃haloalkyl, C₁-C₃alkoxy, C₁-C₃haloalkoxy, halogen, cyano or by nitro, C₃-C₇cycloalkylamino, di-C₃-C₇cycloalkylamino, C₃-C₇cycloalkoxy or C₁-C₁₀alkylcarbonyl;
R₅₅ is C₁-C₁₀alkyl;
R₁₃₇ is hydrogen or C₁-C₁₀alkyl and
R₁₃₈ and R₁₃₉ are each independently of the other hydrogen or C₁-C₁₀alkyl;
or an agronomically tolerable salt, isomer or enantiomer of such a compound.

2. A compound according to claim 1, wherein Q is Q₁, Q₂ or Q₅.

3. A process for the preparation of a compound of formula I according to claim 1, wherein a compound of formula XXX
Q-H (XXX)
wherein Q is Q₁, Q₂, Q₅ or Q₁₀, the substituents of which, with the exception of G₁, G₂, G₅ and G₁₀, have the meanings given above, and G₁, G₂, G₅ and G₁₀ are hydrogen, is reacted with a compound of formula XXXI 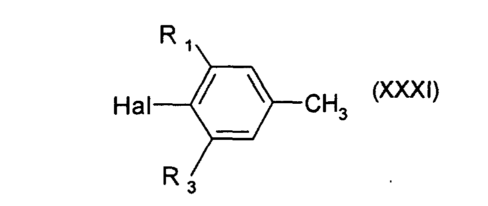 wherein R₁ and R₃ are as defined for formula I and Hal is chlorine, bromine or iodine, in the presence of an inert solvent, a base and a palladium catalyst at temperatures of from 30 to 250°C.

4. A herbicidal and plant growth-inhibiting composition that comprises a herbicidally effective amount of a compound of formula I on an inert carrier.

5. A method of controlling undesired plant growth that comprises applying a herbicidally effective amount of an active ingredient of formula I, or of a composition comprising such an active ingredient, to the plants or to the locus thereof.

6. A method of inhibiting plant growth that comprises applying a herbicidally effective amount of an active ingredient of formula I, or of a composition comprising such an active ingredient, to the plants or to the locus thereof.

7. A selective-herbicidal composition that comprises as active ingredient, in addition to customary inert formulation adjuvants, a mixture of
a) a herbicidally effective amount of a compound of formula I according to claim 1, with the proviso that Q is other than Q₁;
and
b) a herbicide-antagonistically effective amount either of a compound of formula X 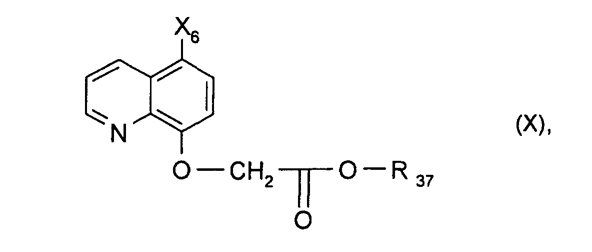
wherein
R₃₇ is hydrogen, C₁-C₈alkyl, or C₁-C₈alkyl substituted by C₁-C₆alkoxy or by C₃-C₆alkenyloxy; and X₆ is hydrogen or chlorine; or of a compound of formula XI 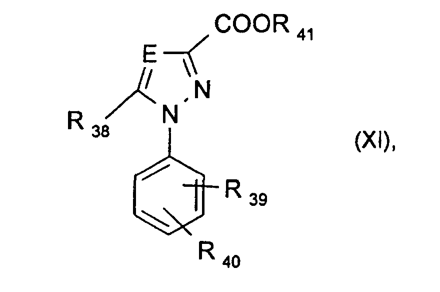 wherein
E is nitrogen or methine;
R₃₈ is -CCl₃, phenyl or phenyl substituted by halogen;
R₃₉ and R₄₀ are each independently of the other hydrogen or halogen; and
R₄₁ is C₁-C₄alkyl; or of a compound of formula XII 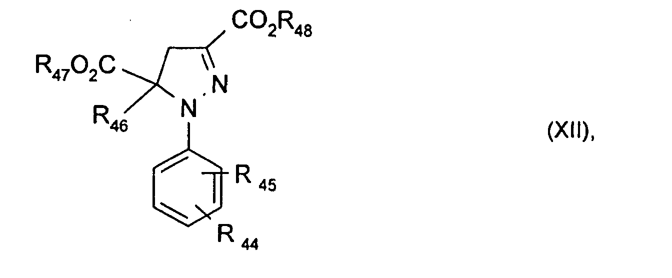 wherein R₄₄ and R₄₅ are each independently of the other hydrogen or halogen, and R₄₆, R₄₇ and R₄₈ are each independently of the others C₁-C₄alkyl, or of a compound of formula XIII 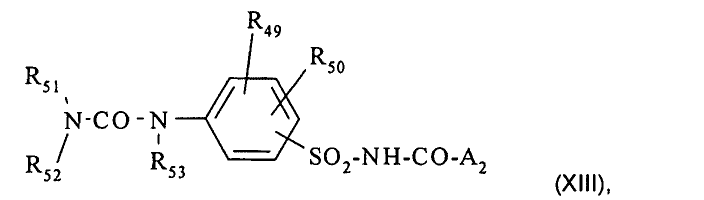 wherein A₂ is a group 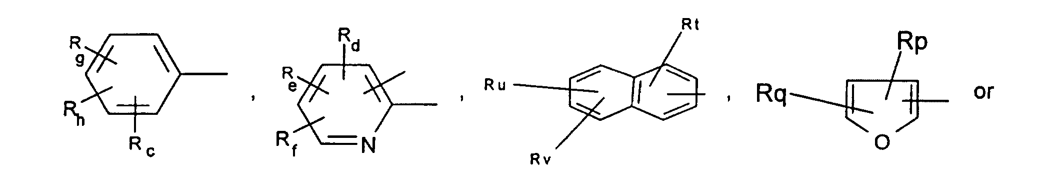 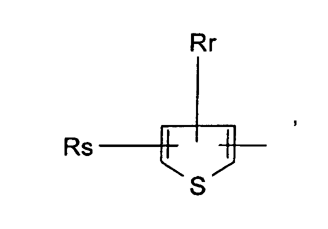 R₅₁ and R₅₂ are each independently of the other hydrogen, C₁-C₈alkyl, C₃-C₈cycloalkyl, C₃-C₆alkenyl, C₃-C₆alkynyl, 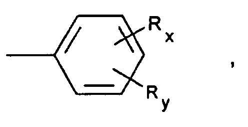 or C₁-C₄alkyl substituted by C₁-C₄alkoxy or by 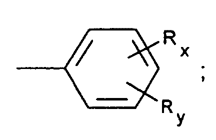 or R₅₁ and R₅₂ together form a C₄-C₆alkylene bridge that may be interrupted by oxygen, sulfur, SO, SO₂, NH or by -N(C₁-C₄alkyl)-;
R₅₃ is hydrogen or C₁-C₄alkyl;
R₄₉ is hydrogen, halogen, cyano, trifluoromethyl, nitro, C₁-C₄alkyl, C₁-C₄alkoxy, C₁-C₄alkylthio, C₁-C₄alkylsulfinyl, C₁-C₄alkylsulfonyl, -COORⱼ, -CONRₖRₘ, -CORₙ, -SO₂NRₖRₘ or -OSO₂-C₁-C₄alkyl;
R_{g} is hydrogen, halogen, cyano, nitro, C₁-C₄alkyl, C₁-C₄haloalkyl, C₁-C₄alkylthio, C₁-C₄alkylsulfinyl, C₁-C₄alkylsulfonyl, -COORⱼ, -CONRₖRₘ, -CORₙ, -SO₂NRₖRₘ, -OSO₂-C₁-C₄alkyl, C₁-C₆alkoxy, or C₁-C₆alkoxy substituted by C₁-C₄alkoxy or by halogen, C₃-C₆alkenyloxy, or C₃-C₆alkenyloxy substituted by halogen, or C₃-C₆alkynyloxy,
or R₄₉ and R₅₀ together form a C₃-C₄alkylene bridge that may be substituted by halogen or by C₁-C₄alkyl, or together form a C₃-C₄alkenylene bridge that may be substituted by halogen or by C₁-C₄alkyl, or together form a C₄alkadienylene bridge that may be substituted by halogen or by C₁-C₄alkyl;
R₅₀ and Rₕ are each independently of the other hydrogen, halogen, C₁-C₄alkyl, trifluoromethyl, C₁-C₆alkoxy, C₁-C₆alkylthio or -COORⱼ;
R_{c} is hydrogen, halogen, nitro, C₁-C₄alkyl or methoxy; R_{d} is hydrogen, halogen, nitro, C₁-C₄alkyl, C₁-C₄alkoxy, C₁-C₄alkylthio, C₁-C₄alkylsulfinyl, C₁-C₄alkylsulfonyl, -COORⱼ or CONRₖRₘ;
Rₑ is hydrogen, halogen, C₁-C₄alkyl, -COORⱼ, trifluoromethyl or methoxy, or R_{d} and Rₑ together form a C₃-C₄alkylene bridge;
Rp is hydrogen, halogen, C₁-C₄alkyl, -COORⱼ, trifluoromethyl or methoxy; Rq is hydrogen, halogen, nitro, C₁-C₄alkyl, C₁-C₄alkoxy, C₁-C₄alkylthio, C₁-C₄alkylsulfinyl, C₁-C₄alkylsulfonyl, -COORⱼ or CONRₖRₘ; or Rp and Rq together form a C₃-C₄alkylene bridge;
Rr is hydrogen, halogen, C₁-C₄alkyl, -COORⱼ, trifluoromethyl or methoxy; Rs is hydrogen, halogen, nitro, C₁-C₄alkyl, C₁-C₄alkoxy, C₁-C₄alkylthio, C₁-C₄alkylsulfinyl, C₁-C₄alkylsulfonyl, -COORⱼ or CONRₖRₘ; or Rr and Rs together form a C₃-C₄alkylene bridge;
Rt is hydrogen, halogen, C₁-C₄alkyl, -COORⱼ, trifluoromethyl or methoxy; Ru is hydrogen, halogen, nitro, C₁-C₄alkyl, C₁-C₄alkoxy, C₁-C₄alkylthio, C₁-C₄alkylsulfinyl, C₁-C₄alkylsulfonyl, -COORⱼ or CONRₖRₘ; or Rv and Ru together form a C₃-C₄alkylene bridge;
R_{f} and Rv are hydrogen, halogen or C₁-C₄alkyl;
Rₓ and R_{y} are each independently of the other hydrogen, halogen, C₁-C₄alkyl, C₁-C₄alkoxy, C₁-C₄alkylthio, -COOR₅₄, trifluoromethyl, nitro or cyano;
Rⱼ, Rₖ and Rₘ are each independently of the others hydrogen or C₁-C₄alkyl; or
Rₖ and Rₘ together form a C₄-C₆alkylene bridge that may be interrupted by oxygen, NH or by -N(C₁-C₄alkyl)-;
Rₙ is C₁-C₄alkyl, phenyl, or phenyl substituted by halogen, C₁-C₄alkyl, methoxy, nitro or by trifluoromethyl;
R₅₄ is hydrogen, C₁-C₁₀alkyl, C₁-C₄alkoxy-C₁-C₄alkyl, C₁-C₄alkylthio-C₁-C₄alkyl, di-C₁-C₄-alkylamino-C₁-C₄alkyl, halo-C₁-C₈alkyl, C₂-C₈alkenyl, halo-C₂-C₈alkenyl, C₃-C₈alkynyl, C₃-C₇cycloalkyl, halo-C₃-C₇cycloalkyl, C₁-C₈alkylcarbonyl, allylcarbonyl, C₃-C₇cycloalkylcarbonyl, benzoyl, which is unsubstituted or substituted on the phenyl ring identically or differently up to three times by halogen, C₁-C₄alkyl, halo-C₁-C₄alkyl, halo-C₁-C₄alkoxy or C₁-C₄alkoxy; or furoyl, thienyl; or C₁-C₄alkyl substituted by phenyl, halophenyl, C₁-C₄alkylphenyl, C₁-C₄alkoxyphenyl, halo-C₁-C₄alkylphenyl, halo-C₁-C₄alkoxyphenyl, C₁-C₆alkoxycarbonyl, C₁-C₄alkoxy-C₁-C₈alkoxycarbonyl, C₃-C₈alkenyloxycarbonyl, C₃-C₈-alkynyloxycarbonyl, C₁-C₈alkylthiocarbonyl, C₃-C₈alkenylthiocarbonyl, C₃-C₈alkynylthiocarbonyl, carbamoyl, mono-C₁-C₄alkylaminocarbonyl, di-C₁-C₄alkylaminocarbonyl; or phenylaminocarbonyl, which is unsubstituted or substituted on the phenyl identically or differently up to three times by halogen, C₁-C₄alkyl, halo-C₁-C₄alkyl, halo-C₁-C₄alkoxy or C₁-C₄alkoxy or once by cyano or nitro; or dioxolan-2-yl, which is unsubstituted or substituted by one or two C₁-C₄alkyl radicals, or dioxan-2-yl, which is unsubstituted or substituted by one or two C₁-C₄alkyl radicals, or C₁-C₄alkyl, which is substituted by cyano, nitro, carboxyl or by C₁-C₈-alkylthio-C₁-C₈alkoxycarbonyl;
or of a compound of formula XIV 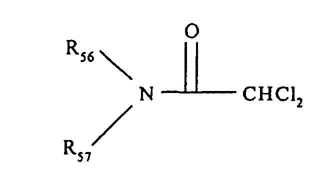 (XIV), wherein R₅₆ and R₅₇ are each independently of the other C₁-C₆alkyl or C₂-C₆alkenyl; or R₅₆ and R₅₇ together are 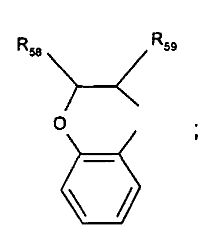 R₅₈ and R₅₉ are each independently of the other hydrogen or C₁-C₆alkyl; or R₅₆ and R₅₇ together are 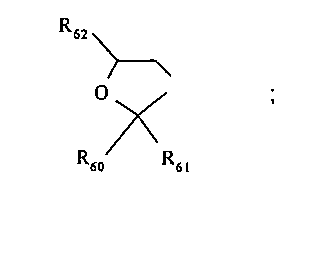 R₆₀ and R₆₁ are each independently of the other C₁-C₄alkyl, or R₆₀ and R₆₁ together are -(CH₂)₅- ;
R₆₂ is hydrogen, C₁-C₄alkyl or 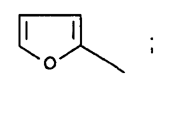 or R₅₆ and R₅₇ together are 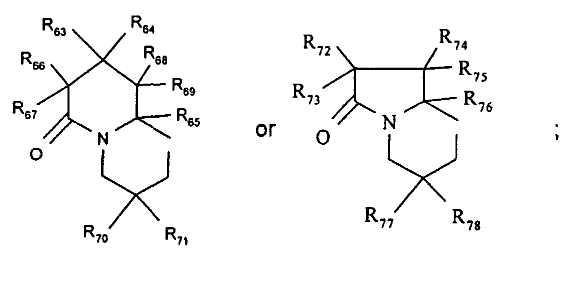 R₆₃, R₆₄, R₆₅, R₆₆, R₆₇, R₆₈, R₆₉, R₇₀, R₇₁, R₇₂, R₇₃, R₇₄, R₇₅, R₇₆, R₇₇ and R₇₈ are each independently of the others hydrogen or C₁-C₄alkyl;
or of a compound of formula XV 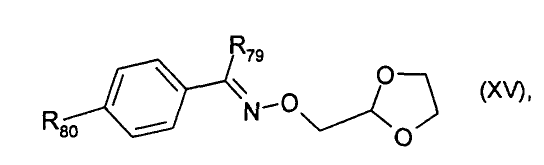 wherein R₈₀ is hydrogen or chlorine and R₇₉ is cyano or trifluoromethyl;
or of a compound of formula XVI 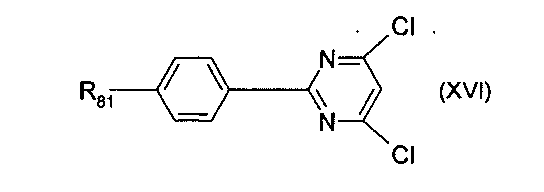 wherein R₈₁ is hydrogen or methyl;
or of a compound of formula XVII 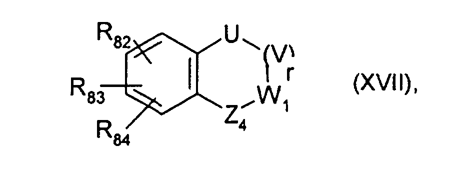 wherein
R₈₂ is hydrogen, C₁-C₄alkyl, or C₁-C₄alkyl substituted by C₁-C₄alkyl-X₂- or by C₁-C₄haloalkyl-X₂-, or is C₁-C₄haloalkyl, nitro, cyano, -COOR₈₅, -NR₈₆R₈₇, -SO₂NR₈₈R₈₉ or -CONR₉₀R₉₁;
R₈₃ is hydrogen, halogen, C₁-C₄alkyl, trifluoromethyl, C₁-C₄alkoxy or C₁-C₄haloalkoxy;
R₈₄ is hydrogen, halogen or C₁-C₄alkyl;
U, V, W₁ and Z₄ are each independently of the others oxygen, sulfur, C(R₉₂)R₉₃, carbonyl, NR₉₄, or a group 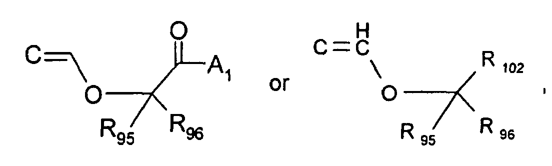 wherein R₁₀₂ is C₂-C₄alkenyl or C₂-C₄alkynyl; with the provisos that
a) at least one of the ring members U, V, W₁ or Z₄ is carbonyl, and a ring member adjacent to that ring member or to those ring members is the group 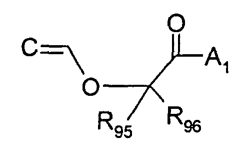 or 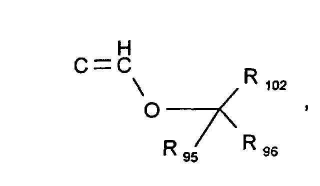 that group appearing only once; and
b) two adjacent ring members U and V, V and W₁ and W₁ and Z₄ cannot simultaneously be oxygen;
R₉₅ and R₉₆ are each independently of the other hydrogen or C₁-C₈alkyl; or
R₉₅ and R₉₆ together form a C₂-C₆alkylene group;
A₁ is R₉₉-Y₁- or -NR₉₇R₉₈;
X₂ is oxygen or -S(O)ₛ ;
Y₁ is oxygen or sulfur;
R₉₉ is hydrogen, C₁-C₈alkyl, C₁-C₈haloalkyl, C₁-C₄alkoxy-C₁-C₈alkyl, C₃-C₆alkenyloxy-C₁-C₈-alkyl, or phenyl-C₁-C₈alkyl in which the phenyl ring may be substituted by halogen, C₁-C₄-alkyl, trifluoromethyl, methoxy or by methyl-S(O)ₛ-, or is C₃-C₆alkenyl, C₃-C₆haloalkenyl, phenyl-C₃-C₆alkenyl, C₃-C₆alkynyl, phenyl-C₃-C₆alkynyl, oxetanyl, furyl or tetrahydrofuryl;
R₈₅ is hydrogen or C₁-C₄alkyl;
R₈₆ is hydrogen, C₁-C₄alkyl or C₁-C₄alkylcarbonyl;
R₈₇ is hydrogen or C₁-C₄alkyl; or
R₈₆ and R₈₇ together form a C₄- or C₅-alkylene group;
R₈₈, R₈₉, R₉₀ and R₉₁ are each independently of the others hydrogen or C₁-C₄alkyl; or R₈₈ together with R₈₉, or R₉₀ together with R₉₁, are each independently of the other C₄- or C₅-alkylene in which one carbon atom may have been replaced by oxygen or by sulfur, or one or two carbon atoms may have been replaced by -NR₁₀₀-;
R₉₂, R₁₀₀ and R₉₃ are each independently of the others hydrogen or C₁-C₈alkyl; or
R₉₂ and R₉₃ together are C₂-C₆alkylene;
R₉₄ is hydrogen or C₁-C₈alkyl;
R₉₇ is hydrogen, C₁-C₈alkyl, phenyl or phenyl-C₁-C₈alkyl, wherein the phenyl rings may be substituted by fluorine, chlorine, bromine, nitro, cyano, -OCH₃, C₁-C₄alkyl or by CH₃SO₂-, or is C₁-C₄alkoxy-C₁-C₈alkyl, C₃-C₆alkenyl or C₃-C₆alkynyl;
R₉₈ is hydrogen, C₁-C₈alkyl, C₃-C₆alkenyl or C₃-C₆alkynyl; or
R₉₇ and R₉₈ together are C₄- or C₅-alkylene in which one carbon atom may have been replaced by oxygen or by sulfur, or one or two carbon atoms may have been replaced by -NR₁₀₁-;
R₁₀₁ is hydrogen or C₁-C₄alkyl;
r is 0 or 1; and
s is 0, 1 or 2,
or of a compound of formula XVIII 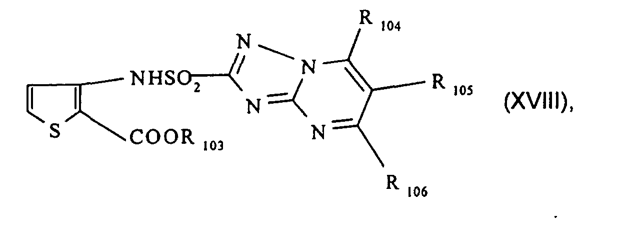 wherein R₁₀₃ is hydrogen, C₁-C₆alkyl, C₃-C₆cycloalkyl, C₃-C₆alkenyl or C₃-C₆alkynyl; and R₁₀₄, R₁₀₅ and R₁₀₆ are each independently of the others hydrogen, C₁-C₆alkyl, C₃-C₆cycloalkyl or C₁-C₆alkoxy, with the proviso that one of the substituents R₁₀₄, R₁₀₅ and R₁₀₆ is other than hydrogen;
or of a compound of formula XIX 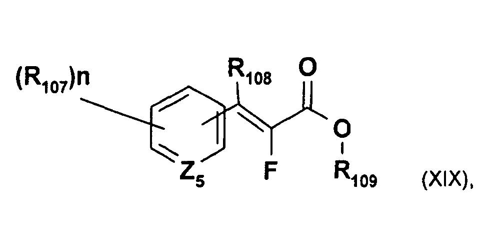 wherein Z₅ is N or CH, n is 0, 1, 2 or 3 when Z₅ is N, and n is 0, 1, 2, 3 or 4 when Z₅ is CH,
R₁₀₇ is halogen, C₁-C₄alkyl, C₁-C₄haloalkyl, C₁-C₄alkoxy, C₁-C₄haloalkoxy, nitro, C₁-C₄alkylthio, C₁-C₄alkylsulfonyl, C₁-C₄alkoxycarbonyl, phenyl or phenoxy, or phenyl or phenoxy substituted by C₁-C₃alkyl, C₁-C₃haloalkyl, C₁-C₃alkoxy, C₁-C₃haloalkoxy, halogen, cyano or by nitro;
R₁₀₈ is hydrogen or C₁-C₄alkyl, R₁₀₉ is hydrogen, C₁-C₄alkyl, C₃-C₆cycloalkyl, C₂-C₆alkenyl, C₂-C₆alkynyl, C₁-C₄haloalkyl, C₂-C₆haloalkenyl, C₂-C₆haloalkynyl, C₁-C₄alkylthio-C₁-C₄alkyl, C₁-C₄alkylsulfonyl-C₁-C₄alkyl, C₁-C₄alkoxy-C₁-C₄alkyl, C₁-C₄alkenyloxy-C₁-C₄alkyl or C₁-C₄-alkynyloxy-C₁-C₄alkyl;
or of a compound of formula XX 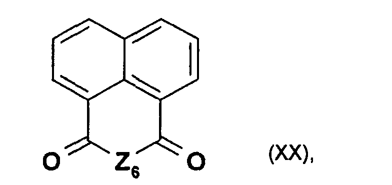 wherein Z₆ is oxygen or N-R₁₁₀ and R₁₁₀ is a group of formula 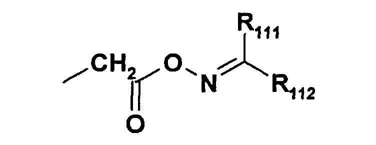 wherein R₁₁₁ and R₁₁₂ are each independently of the other cyano, hydrogen, C₁-C₄alkyl, C₃-C₆cycloalkyl, C₂-C₆alkenyl, aryl, phenyl or heteroaryl, or phenyl, aryl or heteroaryl substituted by C₁-C₃alkyl, C₁-C₃haloalkyl, C₁-C₃alkoxy, C₁-C₃haloalkoxy, halogen, cyano or by nitro;
or of a compound of formula XXI 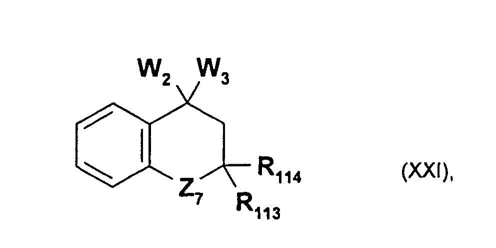 wherein Z₇ is oxygen, sulfur, S=O, SO₂ or CH₂, R₁₁₃ and R₁₁₄ are each independently of the other hydrogen, halogen or C₁-C₄alkyl, W₂ and W₃ are each independently of the other CH₂COOR₁₁₅ or COOR₀₁₁₅ or together are a group of formula -(CH₂)C(O)-O-C(O)-(CH₂)-, and R₁₁₅ and R₀₁₁₅ are each independently of the other hydrogen, C₁-C₄alkyl, C₂-C₄alkenyl, C₂-C₆-alkynyl, C₃-C₆cycloalkyl, C₁-C₄haloalkyl, or a metal cation or an ammonium cation;
or of a compound of formula XXII 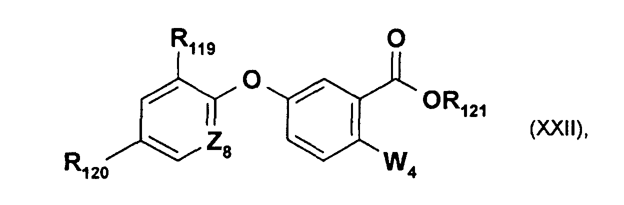 wherein R₁₁₉ and R₁₂₀ are each independently of the other hydrogen, halogen or C₁-C₄haloalkyl, R₁₂₁ is hydrogen, C₁-C₄alkyl, C₃-C₄alkenyl, C₃-C₄alkynyl, C₁-C₄haloalkyl, C₃-C₆cycloalkyl, a metal cation or an ammonium cation, Z₈ is N, CH, C-F or C-Cl and W₄ is a group of formula 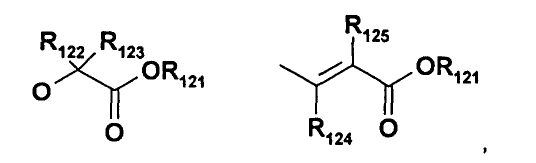 wherein R₁₂₂ and R₁₂₃ are each independently of the other hydrogen or C₁-C₄alkyl and R₁₂₄ and R₁₂₅ are each independently of the other hydrogen or C₁-C₄alkyl;
or of a compound of formula XXIII 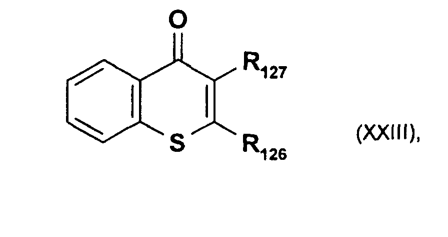 wherein R₁₂₆ is hydrogen, cyano, halogen, C₁-C₄alkyl, C₃-C₆cycloalkyl, C₁-C₄alkoxy, C₁-C₄-alkoxycarbonyl, C₁-C₄alkylthiocarbonyl, -NH-R₁₂₈, -C(O)NH-R₀₁₂₈, aryl or heteroaryl, or aryl or heteroaryl substituted by C₁-C₃alkyl, C₁-C₃haloalkyl, C₁-C₃alkoxy, C₁-C₃haloalkoxy, halogen, cyano or by nitro;
R₁₂₇ is hydrogen, cyano, nitro, halogen, C₁-C₄alkyl, C₁-C₄haloalkyl, C₁-C₄alkoxy or C₁-C₄thioalkyl; and
R₁₂₈ and R₀₁₂₈ are each independently of the other C₁-C₄alkyl, C₁-C₄haloalkyl, C₃-C₄alkenyl, C₃-C₄alkynyl, C₃-C₄cycloalkyl, aryl or heteroaryl, or aryl or heteroaryl substituted by C₁-C₃-alkyl, C₁-C₃haloalkyl, C₁-C₃alkoxy, C₁-C₃haloalkoxy, halogen, cyano or by nitro, formyl, C₁-C₄alkylcarbonyl or C₁-C₄alkylsufonyl;
or of a compound of formula XXIV 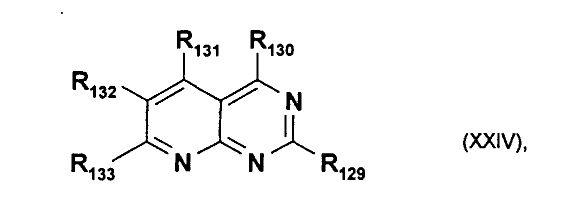 wherein R₁₂₉ and R₁₃₀ are each independently of the other hydrogen, C₁-C₄alkyl, C₁-C₄haloalkyl, C₁-C₄alkoxy, mono-C₁-C₈- or di-C₁-C₈-alkylamino, C₃-C₆cycloalkyl, C₁-C₄thioalkyl, phenyl or heteroaryl, R₁₃₁ has the meanings of R₁₂₉ and in addition is OH, NH₂, halogen, di-C₁-C₄aminoalkyl, C₁-C₄alkylthio, C₁-C₄alkylsulfonyl or C₁-C₄alkoxycarbonyl, R₁₃₂ has the meanings of R₁₂₉ and in addition is cyano, nitro, carboxyl, C₁-C₄alkoxycarbonyl, di-C₁-C₄-aminoalkyl, C₁-C₄alkylthio, C₁-C₄alkylsulfonyl, SO₂-OH, i-C₁-C₄aminoalkylsulfonyl or C₁-C₄-alkoxysulfonyl, R₁₃₃ has the meanings of R₁₂₉ and in addition is OH, NH₂, halogen, di-C₁-C₄-aminoalkyl, pyrrolidin-1-yl, piperidin-1-yl, morpholin-1-yl, C₁-C₄alkylthio, C₁-C₄alkylsulfonyl, C₁-C₄alkoxycarbonyl, phenoxy, naphthyloxy, phenylamino, benzoyloxy or phenylsulfonyloxy; or of a compound of formula XXV 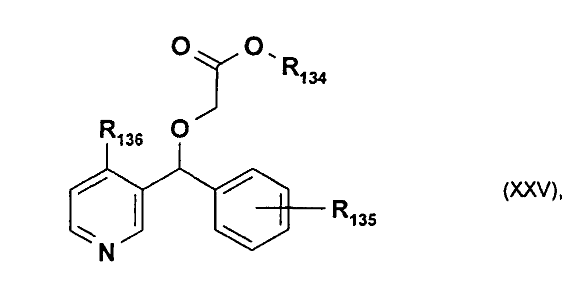 wherein R₁₃₄ is hydrogen, C₄alkyl, C₁-C₄haloalkyl, C₂-C₄alkenyl, C₂-C₄alkynyl or C₁-C₄alkoxy-C₁-C₄alkyl, R₁₃₅ is hydrogen, halogen, C₁-C₄alkyl, C₁-C₄haloalkyl or C₁-C₄alkoxy and R₁₃₆ is hydrogen, halogen, C₁-C₄alkyl, C₁-C₄haloalkyl or C₁-C₄alkoxy, with the proviso that R₁₃₅ and R₁₃₆ are not simultaneously hydrogen,
or of formula XXVI 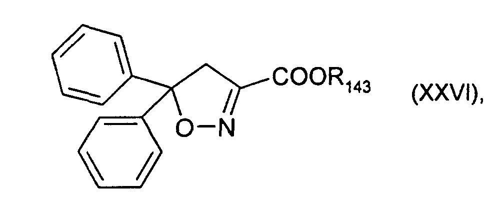 wherein
R₁₄₃ is hydrogen, an alkali metal cation, alkaline earth metal cation, sulfonium cation or ammonium cation or ethyl;
or of formula XXVII 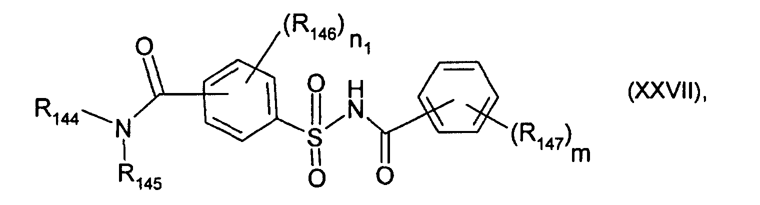 wherein R₁₄₄ and R₁₄₅ are each independently of the other hydrogen, C₁-C₆alkyl, C₂-C₆-alkenyl, C₂-C₆alkynyl or C₃-C₆cycloalkyl;
R₁₄₆ is hydrogen, halogen, C₁-C₄alkyl, C₁-C₆haloalkyl or C₁-C₆haloalkoxy;
R₁₄₇ is hydrogen, halogen, C₁-C₄alkyl, C₁-C₄haloalkyl, C₁-C₄alkoxy, C₁-C₄haloalkoxy, C₁-C₄-alkylthio, C₁-C₄alkoxycarbonyl or nitro;
n, is 0, 1, 2 or 3; and
m is 1 or 2;
or of formula XXVIII 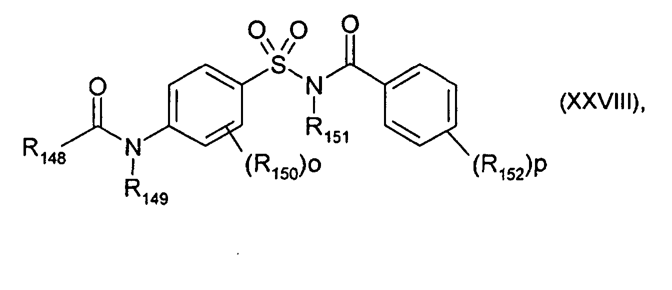 wherein
R₁₄₈ is hydrogen, C₁-C₆alkyl, C₁-C₆alkoxy, C₁-C₆alkylthio, C₃-C₈cycloalkyl, phenyl, phenyl-C₁-C₆alkyl or heteroaryl; wherein the said groups may be substituted by halogen, cyano, nitro, amino, hydroxy, carbonyl, carboxyl, formyl, carbonamide or by sulfonamide;
R₁₄₉ is hydrogen, C₁-C₆alkyl or C₁-C₄haloalkyl;
each R₁₅₀ is independently of any other(s) hydrogen, halogen, C₁-C₄alkyl, C₁-C₄haloalkyl, C₁-C₄alkoxy, C₁-C₄alkylthio, C₁-C₄alkylsulfonyl, cyano, nitro, formyl or carboxyl;
R₁₅₁ is hydrogen, C₁-C₆alkyl or C₁-C₄haloalkyl;
each R₁₅₂ is independently of any other(s) hydrogen, halogen, C₁-C₄alkyl, C₁-C₄haloalkyl, C₁-C₄alkoxy, C₁-C₄alkylthio, C₁-C₄alkylsulfonyl, cyano, nitro, formyl or carboxyl;
o is 0, 1, or 2, and
p is 0, 1 or 2;
or of formula XXIX 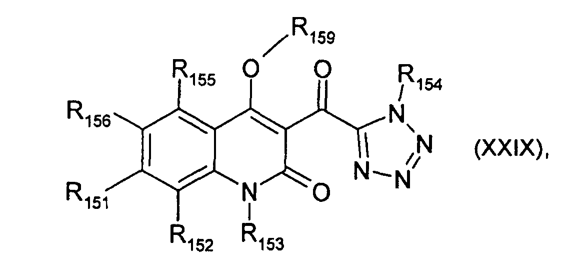 wherein
R₁₅₉ is hydrogen, formyl, C₁₋₆alkylcarbonyl, C₁₋₆alkenylcarbonyl, C₁₋₆alkynylcarbonyl, C₁₋₆alkoxycarbonyl, C₁₋₆alkylthiocarbonyl, C₃₋₈acycloalkylcarbonyl, phenyl-C₁₋₆alkylcarbonyl, phenylcarbonyl, C₁₋₆alkylsulfonyl, C₁₋₆alkenylsulfonyl or phenylsulfonyl, wherein the aforementioned hydrocarbon groups may be substituted by one or more halogen atoms, cyano, nitro, amino, methoxy, ethoxy or phenyl;
R₁₅₃ is hydrogen, C₁₋₆alkyl, C₁₋₆alkenyl, C₁₋₆alkynyl, C₃₋₈cycloalkyl, formyl, C₁₋₆alkylcarbonyl, C₁₋₆alkenylcarbonyl, C₁₋₆alkynylcarbonyl, C₁₋₆alkoxycarbonyl, C₁₋₆alkylthiocarbonyl, C₃₋₈cycloalkylcarbonyl, C₁₋₆alkylsulfonyl, C₁₋₆alkenylsulfonyl or phenylsulfonyl, wherein the aforementioned hydrocarbon groups may be substituted by one or more halogen atoms, cyano, nitro, amino, methoxy, ethoxy or phenyl;
R₁₅₄ is hydrogen, C₁₋₆alkyl, C₁₋₆alkenyl, C₁₋₆alkynyl, C₃₋₈cycloalkyl, formyl, C₁₋₆alkylcarbonyl, C₁₋₆alkenylcarbonyl, C₁₋₆alkynylcarbonyl, C₁₋₆alkoxycarbonyl, C₁₋₆alkylthiocarbonyl, C₃₋₈cycloalkylcarbonyl, C₁₋₆alkylsulfonyl, C₁₋₆alkenylsulfonyl or phenylsulfonyl, wherein the aforementioned hydrocarbon groups may be substituted by one or more halogen atoms, cyano, nitro, amino, methoxy, ethoxy or phenyl;
R₁₅₅, R₁₅₆, R₁₅₇, and R₁₅₈ are each independently of the others hydrogen, halogen, amino, C₁₋₃alkylamino, C₁₋₆dialkylamino, hydroxy, cyano, nitro, formyl, carboxyl, C₁₋₆alkoxy, C₁₋₆haloalkoxy, C₁₋₆alkylcarbonyl, C₁₋₆alkoxycarboxyl, C₁₋₆alkyl, C₁₋₆haloalkyl, C₁₋₆alkenyl or C₁₋₆alkynyl;
or R₁₅₃ and R₁₅₈, together with the ring atoms to which they are bonded, form a five- or six-membered, partially saturated or unsaturated ring that may contain up to 2 identical or different hetero atoms from the group oxygen, sulfur and nitrogen, it being possible for that ring to be substituted by an oxo radical.

8. A composition according to claim 7 that comprises a herbicide-antagonistically effective amount of a safener of formula X, XI, XII, XIII, XIV, XV, XVI, XVII, XVIII, XIX, XX, XXI, XXII, XXIII, XXIV or XXV.

9. A method of selectively controlling weeds and grasses in crops of useful plants that comprises treating the useful plants, the seeds or the cuttings thereof or the area of cultivation thereof with a herbicidally effective amount of a herbicide of formula I and of a herbicide-antagonistically effective amount of a safener of formula X, XI, XII, XIII, XIV, XV, XVI, XVII, XVIII, XIX, XX, XXI, XXII, XXIII, XXIV, XXV, XXVI, XXVII, XXVIII or XXIX.

10. A method according to claim 9 that comprises treating the useful plants, the seeds or cuttings thereof or the area of cultivation thereof with a herbicide-antagonistically effective amount of a safener of formula X, XI, XII, XIII, XIV, XV, XVI, XVII, XVIII, XIX, XX, XXI, XXII, XXIII, XXIV or XXV.

11. A composition according to claim 4 that includes spray tank adjuvants.

12. A composition according to claim 7 that includes spray tank adjuvants.

## Revendications

1. Composés répondant à la formule I 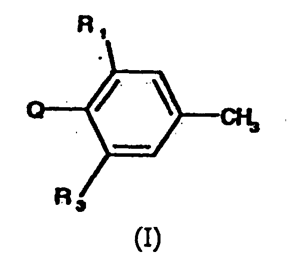 dans laquelle
R₁ et R₃ indépendamment l'un de l'autre représentent un groupe éthyle, un groupe halogénoéthyle, un groupe éthinyle, un groupe alcoxy en C₁-C₂, un groupe halogénoalcoxy en C₁-C₂ ou un groupe alkylcarbonyle en C₁-C₂ ;
Q représente un groupe 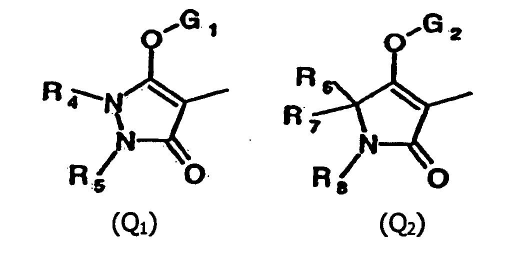 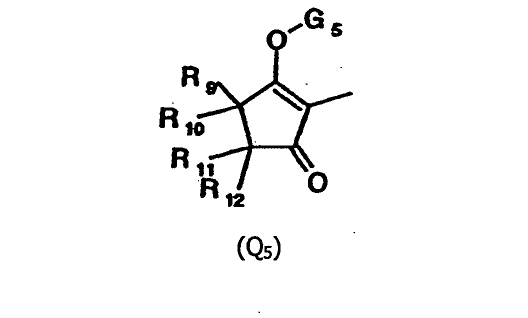 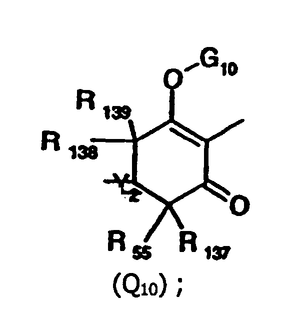 R₄ et R₅ représentent indépendamment l'un de l'autre un groupe alkyle en C₁-C₁₀, un groupe alcényle en C₂-C₁₀, un groupe alcinyle en C₂-C₁₀, un groupe halogénoalkyle en C₁-C₁₀, un groupe alcoxyalkyle en C₂-C₁₀, un groupe alcényloxyalkyle en C₃-C₁₀, un groupe alcinyloxyalkyle en C₃-C₁₀, un groupe alkylthioalkyle en C₂-C₁₀, un groupe alkylsulfinylalkyle en C₂-C₁₀, un groupe alkylsulfonylalkyle en C₂-C₁₀, un groupe alkylcarbonylalkyle en C₂-C₁₀, un groupe -N-alcoxyiminoalkyle en C₂-C₁₀, un groupe alcoxycarbonylalkyle en C₂-C₁₀, un groupe aminoalkyle en C₁-C₁₀, un groupe dialkylaminoalkyle en C₃-C₁₀, un groupe alkylaminoalkyle en C₂-C₁₀, un groupe cyanoalkyle en C₁-C₁₀, un groupe cycloalkylalkyle en C₄-C₁₀, un groupe phényl alkyle en C₁-C₁₀, un groupe hétéroarylalkyle en C₁-C₁₀, un groupe phénoxylalkyle en C₁-C₁₀, un groupe hétéroaryloxy alkyle en C₁-C₁₀, un groupe alkylidène-aminooxyalkyle en C₁-C₁₀, un groupe nitroalkyle en C₁-C₁₀, un groupe trialkylsilylalkyle en C₁-C₁₀, un groupe alkylaminocarbonylalkyle en C₂-C₁₀, un groupe dialkylaminocarbonylalkyle en C₂-C₁₀, un groupe alkylaminocarbonyloxyalkyle en C₂-C₁₀, un groupe dialkylaminocarbonyloxyalkyle en C₃-C₁₀, un groupe alcoxycarbonylaminoalkyle en C₂-C₁₀, un groupe N-alcoxycarbonyl-N-alkylaminoalkyle en C₁-C₁₀, un groupe cycloalkyle en C₁-C₁₀, un groupe aryle ou un groupe hétéroaryle ; ou
R₄ et R₅ forment ensemble avec les atomes auxquels ils sont reliés un cycle de 5 à 7 chaînons qui peut contenir un ou deux hétéroatomes choisis parmi l'azote, l'oxygène et le soufre et qui en plus, peut contenir une chaîne alcénylène ou alkylène se composant de 2 à 6 atomes de carbone spiroliés ou annelés, laquelle chaîne de son côté peut contenir un ou deux hétéroatomes choisis parmi l'oxygène et le soufre, ce cycle pouvant être substitué par un groupe phényle ou un groupe benzyle, lesquels de leur côté peuvent être substitués par un atome d"halogène, un groupe alkyle en C₁-C₆, un groupe halogénoalkyle en C₁-C₆, un groupe cycloalkyle en C₃-C₆, un groupe hydroxy, un groupe alcoxy en C₁-C₆, un groupe alcoxy en C₁-C₆ alcoxy en C₁-C₆, un groupe halogénoalcoxy en C₁-C₆ ou un groupe nitro ;
R₆ représente un groupe alkyle en C₁-C₁₀, un groupe alcényle en C₂-C₁₀, un groupe alcinyle en C₂-C₁₀, un groupe halogénoalkyle en C₁-C₁₀, un groupe alcoxyalkyle en C₂-C₁₀, un groupe alcényloxyalkyle en C₃-C₁₀, un groupe alcinyloxyalkyle en C₃-C₁₀, un groupe alkylthioalkyle en C₂-C₁₀, un groupe alkylsulfinylalkyle en C₂-C₁₀, un groupe alkylsulfonylalkyle en C₂-C₁₀, un groupe alkylcarbonylalkyle en C₂-C₁₀, un groupe cycloalkyle en C₃-C₁₀, un groupe aryle ou un groupe hétéroaryle ;
R₇ représente un atome d'hydrogène, un groupe alkyle en C₁-C₁₀ un groupe alcényle en C₂-C₁₀, un groupe alcinyle en C₂-C₁₀ ou un groupe alcoxyalkyle en C₂-C₁₀ ;
R₈ représente un atome d'hydrogène, un groupe alkyle en C₁-C₁₀, un groupe halogénoalkyle en C₁-C₁₀, un groupe alcoxyalkyle en C₂-C₁₀, un groupe alcényloxyalkyle en C₃-C₁₀, un groupe alcinyloxyalkyle en C₃-C₁₀, un groupe alkylthioalkyle en C₂-C₁₀, un groupe alkylsulfinylalkyle en C₂-C₁₀, un groupe alkylsulfonylalkyle en C₂-C₁₀, un groupe cycloalkyle en C₃-C₁₀, un groupe aryle ou un groupe hétéroaryle ; ou
R₆ et R₇ forment ensemble avec l'atome auquel ils sont liés un cycle de 3 à 7 chaînons saturé qui peut contenir un ou deux hétéroatomes choisis parmi l'azote, l'oxygène et le soufre ; ou R₆ et R₈ forment ensemble avec les atomes auxquels ils sont liés un cycle de 5 à 7 chaînons, qui peut contenir un ou deux hétéroatomes choisis parmi l'azote, l'oxygène et le soufre ;
R₉, R₁₀, R₁₁ et R₁₂ représentent indépendamment les uns des autres un groupe alkyle en C₁-C₁₀, un groupe alcényle en C₂-C₁₀, un groupe alcinyle en C₂-C₁₀, un groupe halogénoalkyle en C₁-C₁₀, un groupe alcoxyalkyle en C₂-C₁₀, un groupe alcényloxyalkyle en C₃-C₁₀, un groupe alcinyloxyalkyle en C₃-C₁₀, un groupe alkylthioalkyle en C₂-C₁₀, un groupe alkylsulfinylalkyle en C₂-C₁₀, un groupe alkylsulfonylalkyle en C₂-C₁₀, un groupe alkylcarbonylalkyle en C₂-C₁₀, un groupe cycloalkyle en C₃-C₁₀, un groupe aryle ou un groupe hétéroaryle ; ou
R₁₀ et R₁₁ ou R₉ et R₁₀ forment ensemble avec les atomes auxquels ils sont liés un cycle de 5 à 7 chaînons qui peut contenir un ou deux hétéroatomes choisis parmi l'azote, l'oxygène et le soufre ;
G₁, G₂, G₅ et G₁₀ représentent indépendamment les uns des autres un atome d'hydrogène, -C(X₁)-R₂₀, -C(X₂)-X₃-R₂₁, -C(X₄)-N(R₂₂)-R₂₃, -SO₂-R₂₄, un cation alcali, un cation alcalino-terreux, un cation sulfonium ou un cation ammonium, -P(X₅)(R₂₅)-R₂₆ ou -CH₂-X₆-R₂₇ ;
X₁, X₂, X₃, X₄, X₅ et X₆ représentent indépendamment les uns des autres un atome d'oxygène ou un atome de soufre ;
R₂₀, R₂₁, R2₂ et R₂₃ représentent indépendamment les uns des autres un atome d'hydrogène, un groupe alkyle en C₁-C₁₀, un groupe alcényle en C₂-C₁₀, un groupe alcinyle en C₂-C₁₀, un groupe halogénoalkyle en C₁-C₁₀, un groupe cyanoalkyle en C₁-C₁₀, un groupe nitroalkyle en C₁-C₁₀, un groupe aminoalkyle en C₁-C₁₀, un groupe alkylamino en C₁-C₅ alkyle en C₁-C₅, un groupe dialkylamino en C₂-C₈ alkyle en C₁-C₅, un groupe cycloalkyle en C₃-C₇ alkyle en C₁-C₅, un groupe alcoxyalkyle en C₂-C₁₀, un groupe alcényloxyalkyle en C₄-C₁₀, un groupe alcinyloxyalkyle en C₄-C₁₀, un groupe alkylthioalkyle en C₂-C₁₀, un groupe alkylsulfoxyle en C₁-C₅ alkyle en C₁-C₅, un groupe alkylsulfonyle en C₁-C₅ alkyle en C₁-C₅, un groupe alkylidène aminooxy en C₂-C₈ alkyle en C₁-C₅, un groupe alkylcarbonyle en C₁-C₅ alkyle en C₁-C₅, un groupe alcoxycarbonyle en C₁-C₅ alkyle en C₁-C₅, un groupe aminocarbonyle en C₁-C₅ alkyle en C₁-C₅, un groupe dialkylaminocarbonyle en C₂-C₈ alkyle en C₁-C₅, un groupe alkylcarbonylamino en C₁-C₅ alkyle en C₁-C₅, un groupe alkylcarbonyle en C₁-C₅ (alkyl en C₂-C₅)aminoalkyle, un groupe trialkylsilyle en C₃-C₆ alkyle en C₁-C₅, un groupe phényl alkyle en C₁-C₅, un groupe hétéroaryl alkyle en C₁-C₅, un groupe phénoxy alkyle en C₁-C₅, un groupe hétéroaryloxy alkyle en C₁-C₅, un groupe alcényle en C₂-C₅, un groupe halogénoalcényle en C₂-C₅, un groupe cycloalkyle en C₃-C₈, un groupe phényle, ou un groupe phényle substitué par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro ou un groupe hétéroaryle ou un groupe hétéroarylamino, ou un groupe hétéroaryle ou un groupe hétéroarylamino substitué par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro, un groupe dihétéroarylamino ou un groupe dihétéroarylamino substitué par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro, un groupe phénylamino ou un groupe phénylamino substitué par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro, un groupe diphénylamino ou un groupe diphénylamino substitué par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro, ou un groupe cycloalkylamino, un groupe dicycloalkylamino en C₃-C₇ ou un groupe cycloalcoxy en C₃-C₇ ;
R₂₄, R₂₅ et R₂₆ représentent un atome d'hydrogène, un groupe alkyle en C₁-C₁₀, un groupe alcényle en C₂-C₁₀, un groupe alcinyle en C₂-C₁₀, un groupe halogénoalkyle en C₁-C₁₀, un groupe cyanoalkyle en C₁-C₁₀, un groupe nitroalkyle en C₁-C₁₀, un groupe aminoalkyle en C₁-C₁₀, un groupe alkylamino en C₁-C₅ alkyle en C₁-C₅, un groupe dialkylamino en C₂-C₈ alkyle en C₁-C₅, un groupe cycloalkyle en C₃-C₇ alkyle en C₁-C₅, un groupe alcoxyalkyle en C₂-C₁₀, un groupe alcényloxyalkyle en C₄-C₁₀, un groupe alcinyloxyalkyle en C₄-C₁₀, un groupe alkylthioalkyle en C₂-C₁₀, un groupe alkysulfoxyle en C₁-C₅ alkyle en C₁-C₅, un groupe alkysulfonyle en C₁-C₅ alkyle en C₁-C₅, un groupe alkylidène aminooxy en C₂-C₈ alkyle en C₁-C₅, un groupe alkylcarbonyle en C₁-C₅ alkyle en C₁-C₅, un groupe alcoxycarbonyle en C₁-C₅ alkyle en C₁-C₅, un groupe aminocarbonyle en C₁-C₅ alkyle en C₁-C₅, un groupe dialkylaminocarbonyle en C₂-C₈ alkyle en C₁-C₅, un groupe alkylcarbonylamino en C₁-C₅ alkyle en C₁-C₅, un groupe alkylcarbonyle en C₁-C₅ (alkyle en C₂-C₅)aminoalkyle, un groupe trialkylsilyle en C₃-C₆ alkyle en C₁-C₅, un groupe phényl alkyle en C₁-C₅, un groupe hétéroaryle alkyle en C₁-C₅, un groupe phénoxy alkyle en C₁-C₅, un groupe hétéroaryloxy alkyle en C₁-C₅, un groupe alcényle en C₂-C₅, un groupe halogénoalcényle en C₂-C₅, un groupe cycloalkyle en C₃-C₈, un groupe phényle ou un groupe phényle substitué par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro ou un groupe hétéroaryle ou un groupe hétéroarylamino ou un groupe hétéroaryle ou un groupe hétéroarylamino substitué par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro, un groupe dihétéroarylamino ou un groupe dihétéroarylamino substitué par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro, un groupe phénylamino ou un groupe phénylamino substitué par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro, un groupe diphénylamino ou un groupe diphénylamino substitué par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro, ou un groupe cycloalkylamino en C₃-C₇, un groupe dicycloalkylamino en C₃-C₇, un groupe cycloalcoxy en C₃-C₇, un groupe alcoxy en C₁-C₁₀, un groupe halogénoalcoxy en C₁-C₁₀, un groupe alkylamino en C₁-C₅, un groupe dialkylamino en C₂-C₈, un groupe benzyloxy ou un groupe phénoxy parmi lesquels les groupes benzyle et phényle peuvent être substitués de leur côté par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro ;
R₂₇ représente un groupe alkyle en C₁-C₁₀, un groupe alcényle en C₂-C₁₀, un groupe alcinyle en C₂-C₁₀, un groupe halogénoalkyle en C₁-C₁₀, un groupe cyanoalkyle en C₁-C₁₀, un groupe nitroalkyle en C₁-C₁₀, un groupe aminoalkyle en C₁-C₁₀, un groupe alkylamino en C₁-C₅ alkyle en C₁-C₅, un groupe dialkylamino en C₂-C₈ alkyle en C₁-C₅, un groupe cycloalkyle en C₃-C₇ alkyle en C₁-C₅, un groupe alcoxyalkyle en C₂-C₁₀, un groupe alcényloxyalkyle en C₄-C₁₀, un groupe alcinyloxyalkyle en C₄-C₁₀, un groupe alkylthioalkyle en C₂-C₁₀, un groupe alkylsulfoxyle en C₁-C₅ alkyle en C₁-C₅, un groupe alkylsulfonyle en C₁-C₅ alkyle en C₁-C₅, un groupe alkylidèneaminooxy en C₂-C₈ alkyle en C₁-C₅, un groupe alkylcarbonyle en C₁-C₅ alkyle en C₁-C₅, un groupe alcoxycarbonyle en C₁-C₅ alkyle en C₁-C₅, un groupe aminocarbonyle en C₁-C₅ alkyle en C₁-C₅, un groupe dialkylaminocarbonyle en C₂-C₈ alkyle en C₁-C₅, un groupe alkylcaronylamino en C₁-C₅ alkyle en C₁-C₅, un groupe alkylcarbonyle en C₁-C₅ (alkyle en C₂-C₅)aminoalkyle, un groupe trialkylsilyle en C₃-C₆ alkyle en C₁-C₅, un groupe phényl alkyle en C₁-C₅, un groupe hétéroarylalkyle en C₁-C₅, un groupe phénoxyalkyle en C₁-C₅, un groupe hétéroaryloxy alkyle en C₁-C₅, un groupe alcényle en C₂-C₅, un groupe halogénoalcényle en C₂-C₅, un groupe cycloalkyle en C₃-C₈, un groupe phényle ou un groupe phényle substitué par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro ou un groupe hétéroaryle ou un groupe hétéroarylamino ou un groupe hétéroaryle ou un groupe hétéroarylamino substitué par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro, un groupe dihétéroarylamino ou un groupe dihétéroarylamino substitué par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro, un groupe phénylamino ou un groupe phénylamino substitué par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro, un groupe diphénylamino ou un groupe diphénylamino substitué par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro, un groupe cycloalkylamino en C₃-C₇, un groupe dicycloalkylamino en C₃-C₇, un groupe cycloalcoxy en C₃-C₇ ou un groupe alkylcarbonyle en C₁-C₁₀ ;
R₅₅ représente un groupe alkyle en C₁-C₁₀ ;
R₁₃₇ représente un atome d'hydrogène ou un groupe alkyle en C₁-C₁₀ ;
R₁₃₈ ou R₁₃₉ représentent indépendamment l'un de l'autre un atome d'hydrogène ou un groupe alkyle en C₁-C₁₀, ainsi que des sels, des isomères et des énantiomères de ces composés agronomiquement acceptables.

2. Composés selon la revendication 1, **caractérisés en ce que** Q représente Q₁, Q₂ ou Q₅.

3. Procédé pour la fabrication des composés répondant à la formule I selon la revendication 1, **caractérisé en ce que** l'on convertit un composé répondant à la formule XXX
Q-H (XXX)
dans laquelle Q représente Q₁, Q₂, Q₅ ou Q₁₀, leurs substituants à l'exception de G₁, G₂, G₅ et G₁₀ ayant la signification précédemment mentionnée et G₁, G₂, G₅ et G₁₀ désignant un atome d'hydrogène, avec un composé répondant à la formule XXXI 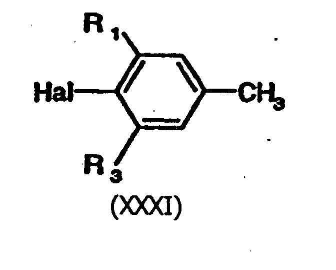 dans laquelle R₁ et R₃ ont la signification précédemment mentionnée sous la formule I et Hal représente un atome de chlore, un atome de brome ou un atome d'iode, en présence d'un solvant inerte, d'une base et d'un catalyseur au palladium à des températures de 30 à 250°C.

4. Agent herbicide et inhibant la croissance des plantes, **caractérisé en ce qu'**il présente sur un support inerte une teneur efficace en tant qu'herbicide d'un composé répondant à la formule I.

5. Procédé pour lutter contre la croissance des plantes indésirables, **caractérisé en ce que** l'on applique un principe actif répondant à la formule I ou un agent contenant ce principe actif en une quantité efficace en tant qu'herbicide sur les plantes ou leur milieu naturel.

6. Procédé pour inhiber la croissance des plantes, **caractérisé en ce que** l'on applique un principe actif répondant à la formule I ou un agent contenant ce principe actif en une quantité efficace en tant qu'herbicide sur des plantes ou leur milieu naturel.

7. Agent herbicide sélectif, **caractérisé en ce qu'**il contient en plus des adjuvants de formulation inertes traditionnelles, comme principe actif, un mélange
a) d'une quantité efficace en tant qu'herbicide d'un composé répondant à la formule I selon la revendication 1, à condition que Q soit différent de Q₁ ;
et
b) d'une quantité efficace en tant qu'antagoniste d'herbicide soit d'un composé répondant à la formule X 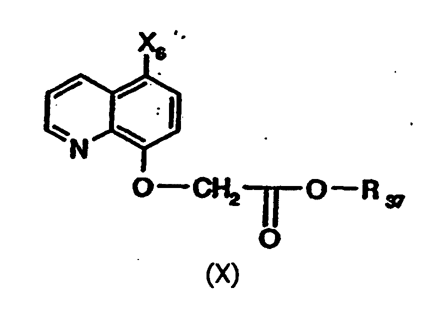
dans laquelle
R₃₇ représente un atome d'hydrogène, un groupe alkyle en C₁-C₈ ou un groupe alkyle en C₁-C₈ substitué par un groupe alcoxy en C₁-C₆ ou par un groupe alcényloxy en C₃-C₆ ; et X₆ représente un atome d'hydrogène ou un atome de chlore ; soit d'un composé répondant à une formule XI 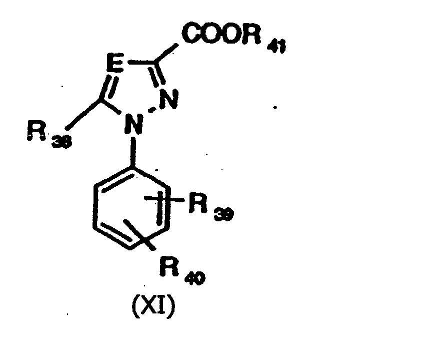 dans laquelle
E représente un atome d'azote ou un groupe méthine ; R₃₈ représente -CCI₃, un groupe phényle ou un groupe phényle substitué par un atome d'halogène ;
R₃₉ et R₄₀ représentent indépendamment l'un de l'autre un atome d'hydrogène ou un atome d'halogène ; et
R₄₁ représente un groupe alkyle en C₁-C₄ ; ou d'un composé répondant à la formule XII 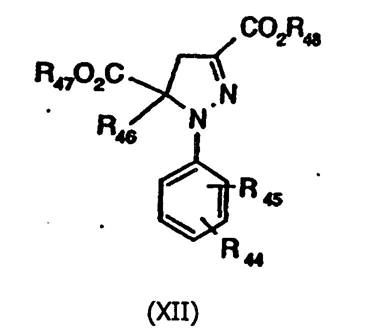 dans laquelle R₄₄ et R₄₅ représentent indépendamment l'un de l'autre un atome d'hydrogène ou un atome d'halogène et R₄₆, R₄₇ et R₄₈ représentent indépendamment les uns des autres un groupe alkyle en C₁-C₄ ; ou d'un composé répondant à la formule XIII 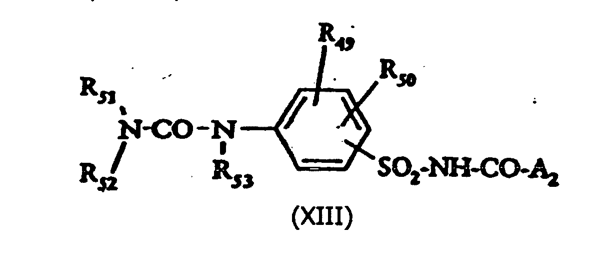 dans laquelle A₂ représente un groupe 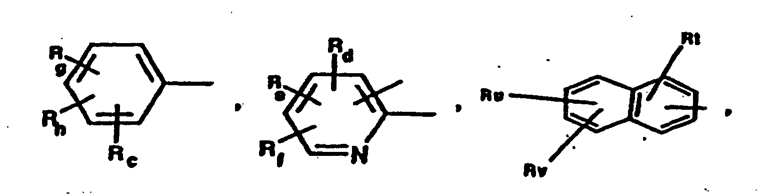 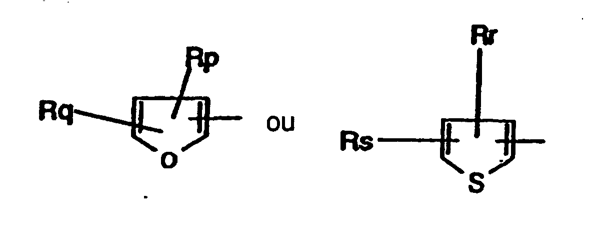 R₅₁ et R₅₂ représentent indépendamment l'un de l'autre un atome d'hydrogène, un groupe alkyle en C₁-C₈, un groupe cycloalkyle en C₃-C₈, un groupe alcényle en C₃-C₆, un groupe alcinyle en C₃-C₆ 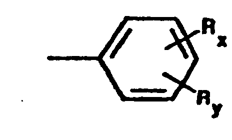 ou un groupe alkyle en C₁-C₄ substitué par 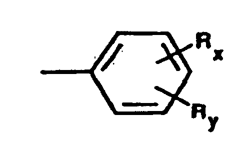 ou par un groupe alcoxy en C₁-C₄ ; ou R₅₁ et R₅₂ forment ensemble un pont alkylène en C₄-C₆ qui peut être interrompu par un atome d'oxygène, par un atome de soufre, SO, SO₂, NH ou N(-alkyle en C₁-C₄),
R₅₃ représente un atome d'hydrogène ou un groupe alkyle en C₁-C₄ ;
R₄₉ représente un atome d'hydrogène, un atome d'halogène, un groupe cyano, un groupe trifluorométhyle, un groupe nitro, un groupe alkyle en C₁-C₄, un groupe alcoxy en C₁-C₄, un groupe alkylthio en C₁-C₄, un groupe alkylsulfinyle en C₁-C₄, un groupe alkylsulfonyle en C₁-C₄, -COORⱼ, -CONRkRₘ, -CORₙ, -SO₂NRₖRₘ ou un groupe -OSO₂-alkyle en C₁-C₄ ;
R_{g} représente un atome d'hydrogène, un atome d'halogène, un groupe cyano, un groupe nitro, un groupe alkyle en C₁-C₄, un groupe halogénoalkyle en C₁-C₄, un groupe alkylthio en C₁-C₄, un groupe alkylsulfinyle en C₁-C₄, un groupe alkylsulfonyle en C₁-C₄, -COORⱼ, -CONRₖRₘ, -CORₙ, -SO2NRₖRₘ, un groupe -OSO₂-alkyle en C₁-C₄, un groupe alcoxy en C₁-C₆, ou un groupe alcoxy en C₁-C₆ substitué par un groupe alcoxy en C₁-C₄ ou par un atome d'halogène ou un groupe alcényloxy en C₃-C₆ ou un groupe alcényloxy en C₃-C₆ substitué par un atome d'halogène ou un groupe alcinyloxy en C₃-C₆, ou R₄₉ et R₅₀ forment ensemble un pont alkylène en C₃-C₄ qui peut être substitué par un atome d'halogène ou par un groupe alkyle en C₁-C₄ ou forment un pont alcénylène en C₃-C₄ qui peut être substitué par un atome d'halogène ou un groupe alkyle en C₁-C₄ ou forment un pont alkadiénylène en C₄ qui peut être substitué par un atome d'halogène ou par un groupe alkyle en C₁-C₄ ;
R₅₀ et Rₕ représentent indépendamment l'un de l'autre un atome d'hydrogène, un atome d'halogène, un groupe alkyle en C₁-C₄, un groupe trifluorométhyle, un groupe alcoxy en C₁-C₆, un groupe alkylthio en C₁-C₆ ou -COORⱼ ;
R_{c} représente un atome d'hydrogène, un atome d'halogène, un groupe nitro, un groupe alkyle en C₁-C₄ ou un groupe méthoxy ; R_{d} représente un atome d'hydrogène, un atome d'halogène, un groupe nitro, un groupe alkyle en C₁-C₄, un groupe alcoxy en C₁-C₄, un groupe alkylthio en C₁-C₄, un groupe alkylsulfinyle en C₁-C₄, un groupe alkylsulfonyle en C₁-C₄, -COORⱼ ou CONRₖRₘ ;
Rₑ représente un atome d'hydrogène, un atome d'halogène, un groupe alkyle en C₁-C₄, -COORⱼ, un groupe trifluorométhyle ou un groupe méthoxy, ou R_{d} et Rₑ forment ensemble un pont alkylène en C₃-C₄ ;
Rₚ représente un atome d'hydrogène, un atome d'halogène, un groupe alkyle en C₁-C₄, -COORⱼ, un groupe trifluorométhyle ou un groupe méthoxy ; R_{q} représente un atome d'hydrogène, un atome d'halogène, un groupe nitro, un groupe alkyle en C₁-C₄, un groupe alcoxy en C₁-C₄, un groupe alkylthio en C₁-C₄, un groupe alkylsulfinyle en C₁-C₄, un groupe alkylsulfonyle en C₁-C₄, -COORⱼ ou CONRₖRₘ ; ou Rₚ et R_{q} forment ensemble un pont alkylène en C₃-C₄ ;
Rᵣ représente un atome d'hydrogène, un atome d'halogène, un groupe alkyle en C₁-C₄, -COORⱼ, un groupe trifluorométhyle ou un groupe méthoxy ; Rₛ représente un atome d'hydrogène, un atome d'halogène, un groupe nitro, un groupe alkyle en C₁-C₄, un groupe alcoxy en C₁-C₄, un groupe alkylthio en C₁-C₄, un groupe alkylsulfinyle en C₁-C₄, un groupe alkylsulfonyle en C₁-C₄, -COORⱼ ou CONRₖRₘ ; ou Rᵣ et Rₛ forment ensemble un pont alkylène en C₃-C₄ ;
Rₜ représente un atome d'hydrogène, un atome d'halogène, un groupe alkyle en C₁-C₄, -COORⱼ, un groupe trifluorométhyle ou un groupe méthoxy ; Rᵤ représente un atome d'hydrogène, un atome d'halogène, un groupe nitro, un groupe alkyle en C₁-C₄, un groupe alcoxy en C₁-C₄, un groupe alkylthio en C₁-C₄, un groupe alkylsulfinyle en C₁-C₄, un groupe alkylsulfonyle en C₁-C₄, -COORⱼ ou CONRₖRₘ ; ou Rᵥ et Rᵤ forment ensemble un pont alkylène en C₃-C₄ ;
Rₜ et Rᵥ représentent un atome d'hydrogène, un atome d'halogène ou un groupe alkyle en C₁-C₄ ;
Rₓ et R_{y} représentent indépendamment les uns des autres un atome d'hydrogène, un atome d'halogène, un groupe alkyle en C₁-C₄, un groupe alcoxy en C₁-C₄, un groupe alkylthio en C₁-C₄, -COOR₅₄, un groupe trifluorométhyle, un groupe nitro ou un groupe cyano ;
Rⱼ, Rₖ et Rₘ représentent indépendamment l'un de l'autre un atome d'hydrogène ou un groupe alkyle en C₁-C₄ ; ou Rₖ et Rₘ forment ensemble un pont alkylène en C₄-C₆ qui peut être interrompu par un atome d'oxygène, NH ou N(alkyle C₁-C₄) ;
Rₙ représente un groupe alkyle en C₁-C₄, un groupe phényle, ou un phényle substitué par un atome d'halogène, par un groupe alkyle en C₁-C₄, par un groupe méthoxy, par un groupe nitro ou par un groupe trifluorométhyle ;
R₅₄ représente un atome d'hydrogène, un groupe alkyle en C₁-C₁₀, un groupe alcoxy en C₁-C₄ alkyle en C₁-C₄, un groupe alkylthio en C₁-C₄ alkyle en C₁-C₄, un groupe dialkylamino en C₁-C₄ alkyle en C₁-C₄, un atome d'halogène alkyle en C₁-C₈, un groupe alcényle en C₂-C₈, un atome d'halogène alcényle en C₂-C₈, un groupe alcinyle en C₃-C₈, un groupe cycloalkyle en C₃-C₇, un atome d'halogène cycloalkyle en C₃-C₇, un groupe alkylcarbonyle en C₁-C₈, un groupe allylcarbonyle, un groupe cycloalkylcarbonyle en C₃-C₇, un groupe benzoyle qui est non substitué ou substitué au niveau du noyau phényle de façon similaire ou différente jusqu'à trois fois par un atome d'halogène, par un groupe alkyle en C₁-C₄, par un atome d'halogène alkyle en C₁-C₄, par un atome d'halogène alcoxy en C₁-C₄ ou par un groupe alcoxy en C₁-C₄; ou un groupe furoyle, un groupe thiényle; ou un groupe alkyle en C₁-C₄ substitué par un groupe phényle, par un groupe halogénophényle, par un groupe alkylphényle en C₁-C₄, par un groupe alcoxyphényle en C₁-C₄, par un atome d'halogène alkylphényle en C₁-C₄, par un atome d'halogène alcoxyphényle en C₁-C₄, par un groupe alcoxycarbonyle en C₁-C₄, par un groupe alcoxy en C₁-C₄ alcoxycarbonyle en C₁-C₈, un groupe alcényloxycarbonyle en C₃-C₈, par un groupe alcinyloxycarbonyle en C₃-C₈, par un groupe alkylthiocarbonyle en C₁-C₈, par un groupe alcénylthiocarbonyle en C₃-C₈, par un groupe alcinylthiocarbonyle en C₃-C₈, par un groupe carbamoyle, par un groupe monoalkylaminocarbonyle en C₁-C₄, par un groupe dialkylaminocarbonyle en C₁-C₄; ou un groupe phénylaminocarbonyle qui est non substitué ou substitué au niveau du groupe phényle de façon identique ou différente jusqu'à trois fois par un atome d'halogène, par un groupe alkyle en C₁-C₄, par un atome d'halogène alkyle en C₁-C₄, par un atome d'halogène alcoxy en C₁-C₄ ou par un groupe alcoxy en C₁-C₄ ou une fois par un groupe cyano ou par un groupe nitro ou un dioxalan-2-yle qui est non substitué ou qui est substitué par un ou deux restes alkyle en C₁-C₄, ou un dioxan-2-yle qui est non substitué ou qui est substitué par un ou deux restes alkyles en C₁-C₄, ou un groupe alkyle en C₁-C₄ qui est substitué par un groupe cyano, par un groupe nitro, par un groupe carboxyle ou par un groupe alkylthio en C₁-C₈ alcoxycarbonyle en C₁-C₈;
ou d'un composé répondant à la formule XIV 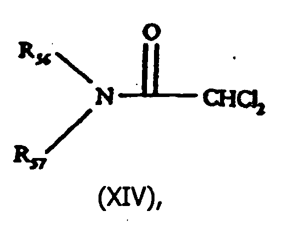 dans laquelle R₅₆ et R₅₇ représentent indépendamment l'un de l'autre un groupe alkyle en C₁-C₆ ou un groupe alcényle en C₂-C₆ ; ou R₅₆ et R₅₇ représentent ensemble 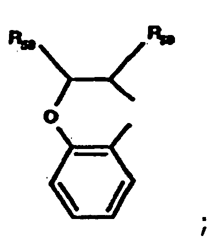 R₅₈ et R₅₉ représentent indépendamment l'un de l'autre un atome d'hydrogène ou un groupe alkyle en C₁-C₆ ; ou R₅₆ et R₅₇ représentent ensemble 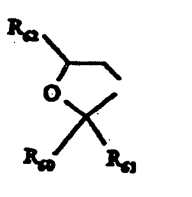 R₆₀ et R₆₁ représentent indépendamment l'un de l'autre un groupe alkyle en C₁-C₄, ou R₆₀ et R₆₁ représentent ensemble -(CH₂)₅- ;
R₆₂ représente un atome d'hydrogène, un groupe alkyle en C₁-C₄ ou 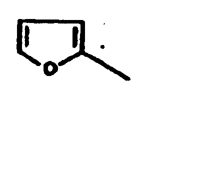 ou R₅₆ et R₅₇ représentent ensemble 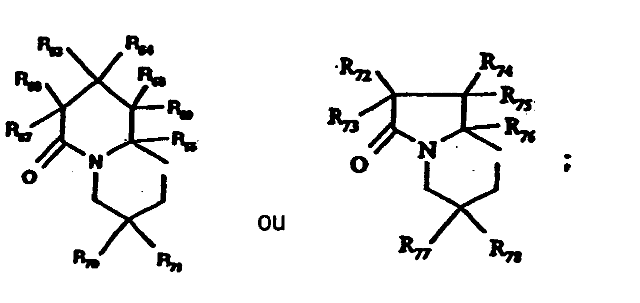 R₆₃, R₆₄, R₆₅, R₆₆, R₆₇, R₆₈, R₆₉, R₇₀, R₇₁, R₇₂, R₇₃, R₇₄, R₇₅, R₇₆, R₇₇ et R₇₈ représentent indépendamment les uns des autres un atome d'hydrogène ou un groupe alkyle en C₁-C₄ ;
ou d'un composé répondant à la formule XV 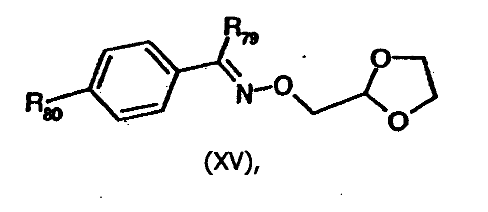 dans laquelle R₈₀ représente un atome d'hydrogène ou un atome de chlore et R₇₉ représente un groupe cyano ou un groupe trifluorométhyle
ou un composé répondant à la formule XVI 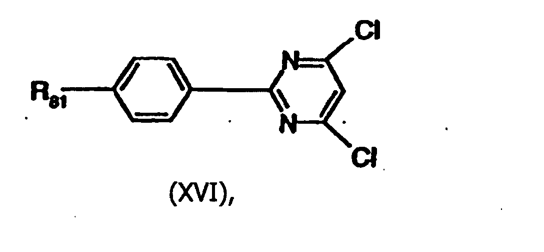 dans laquelle R₈₁ représente un atome d'hydrogène ou un groupe méthyle,
ou d'un composé répondant à la formule XVII 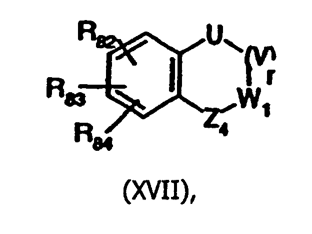 dans laquelle
R₈₂ représente un atome d'hydrogène, un groupe alkyle en C₁-C₄ ou un groupe alkyle en C₁-C₄ substitué par un groupe -X2- alkyle en C₁-C₄ ou un groupe -X2- halogénoalkyle en C₁-C₄, ou représente un groupe halogénoalkyle en C₁-C₄, un groupe nitro, un groupe cyano, un -COOR₈₅, -NR₈₆R₈₇, -SO₂NR₈₈R₈₉ ou -CONR₉₀R₉₁ ;
R₈₃ représente un atome d'hydrogène, un atome d'halogène, un groupe alkyle en C₁-C₄, un groupe trifluorométhyle, un groupe alcoxy en C₁-C₄ ou un groupe halogénoalcoxy en C₁-C₄ ;
R₈₄ représente un atome d'hydrogène, un atome d'halogène ou un groupe alkyle en C₁-C₄ ;
U, V, W1 et Z₄ représentent indépendamment les uns des autres un atome d'oxygène, un atome de soufre, C(R₉₂)R₉₃, un groupe carbonyle, NR₉₄,
ou un groupe, 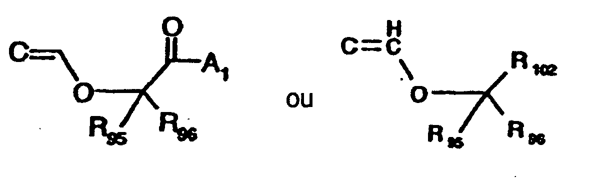 dans lequel R₁₀₂ représente un groupe alcényle en C₂-C₄ ou un groupe alcinyle en C₂-C₄ ; à condition que
a) au moins un des chaînons du noyau U, V ou W₁ ou Z₄ soit un groupe carbonyle, et que l'un chaînon du noyau avoisinant ce ou ces chaînons du noyau représente le groupe 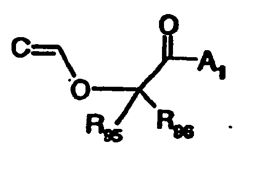 ou 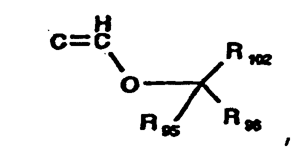 ce groupe n'apparaissant qu'une fois ; et
b) deux des chaînons du noyau voisins U et V, V et W₁ et W₁ et Z₄ ne puissent pas représenter simultanément un atome d'oxygène ;
R₉₅ et R₉₆ représentent indépendamment l'un de l'autre un atome d'hydrogène ou un groupe alkyle en C₁-C₈ ; ou
R₉₅ et R₉₆ forment ensemble un groupe alkylène en C₂-C₆ ;
A₁ représente R₉₉-Y₁- ou -NR₉₇R₉₈ ;
X₂ représente un atome d'oxygène ou -S(O)ₛ ;
Y₁ représente un atome d'oxygène ou un atome de soufre ;
R₉₉ représente un atome d'hydrogène, un groupe alkyle en C₁-C₈, un groupe halogénoalkyle en C₁-C₈, un groupe alcoxy en C₁-C₄ alkyle en C₁-C₈, un groupe alcényloxy en C₃-C₆ alkyle en C₁-C₈ ou un groupe phényl alkyle en C₁-C₈, le noyau phényle pouvant être substitué par un atome d'halogène, par un groupe alkyle en C₁-C₄, par un groupe trifluorométhyle, par un groupe méthoxy ou par un groupe méthyle-S(O)ₛ-, ou un groupe alcényle en C₃-C₆, ou un groupe halogénoalcényle en C₃-C₆, un groupe phényl alcényle en C₃-C₆, un groupe alcinyle en C₃-C₆, un groupe phényl alcinyle en C₃-C₆, un groupe oxétanyle, un groupe furyle ou un groupe tétrahydrofuryle ;
R₈₅ représente un atome d'hydrogène ou un groupe alkyle en C₁-C₄ ;
R₈₆ représente un atome d'hydrogène, un groupe alkyle en C₁-C₄ ou un groupe alkylcarbonyle en C₁-C₄ ;
R₈₇ représente un atome d'hydrogène ou un groupe alkyle en C₁-C₄ ; ou
R₈₆ et R₈₇ forment ensemble un groupe alkylène en C₄ ou C₅ ;
R₈₈, R₈₉, R₉₀ et R₉₁ représentent indépendamment les uns des autres un atome d'hydrogène ou un groupe alkyle en C₁-C₄ ; ou
R₈₈ avec R₈₉ ou R₉₀ avec à R₉₁ représentent indépendamment les uns des autres un groupe alkylène en C₄ ou C₅, un atome de carbone pouvant être remplacé par un atome d'oxygène ou par un atome de soufre, ou un ou deux atomes de carbone pouvant être remplacés par NR₁₀₀,
R₉₂, R₁₀₀ et R₉₃ représentent indépendamment les uns des autres un atome d'hydrogène ou un groupe alkyle en C₁-C₈ ; ou
R₉₂ et R₉₃ représentent ensemble un alkylène en C₂-C₆ ;
R₉₄ représente un atome d'hydrogène ou un groupe alkyle en C₁-C₈ ;
R₉₇ représente un atome d'hydrogène, un groupe alkyle en C₁-C₈, un groupe phényle ou un groupe phényl alkyle en C₁-C₈, les noyaux phényle pouvant être substitués par un atome de fluor, par un atome de chlore, par un atome de brome, par un groupe nitro, par un groupe cyano, par -OCH₃, par un groupe alkyle en C₁-C₄ ou par CH₃SO₂-, ou représente un groupe alcoxy en C₁-C₄ alkyle en C₁-C₈, un groupe alcényle en C₃-C₆ ou un groupe alcinyle en C₃-C₆ ;
R₉₈ représente un atome d'hydrogène, un groupe alkyle en C₁-C₈, un groupe alcényle en C₃-C₆ ou un groupe alcinyle en C₃-C₆ ; ou
R₉₇ et R₉₈ représentent un alkylène en C₄-C₅, un atome de carbone pouvant être remplacé par un atome d'oxygène ou par un atome de soufre ou un ou deux atomes de carbone par -NR₁₀₁- ;
R₁₀₁ représente un atome d'hydrogène ou un groupe alkyle en C₁-C₄ ;
r vaut 0 ou 1 ; et
s vaut 0, 1 ou 2,
ou d'un composé répondant à la formule XVIII 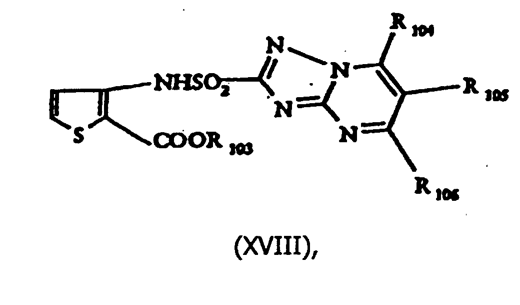 dans laquelle R₁₀₃ représente un atome d'hydrogène, un groupe alkyle en C₁-C₆, un groupe cycloalkyle en C₃-C₆, un groupe alcényle en C₃-C₆ ou un groupe alcinyle en C₃-C₆ ; et R₁₀₄, R₁₀₅ et R₁₀₆ représentent indépendamment les uns des autres un atome d'hydrogène, un groupe alkyle en C₁-C₆, un groupe cycloalkyle en C₃-C₆ ou un groupe alcoxy en C₁-C₆, à condition qu'un des substituants R₁₀₄, R₁₀₅ et R₁₀₆ soit différent de l'atome d'hydrogène ;
ou d'un composé répondant à la formule XIX 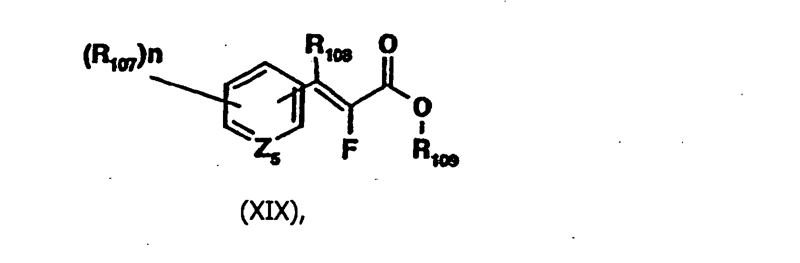 dans laquelle Z₅ représente N ou CH, n dans le cas où Z₅ est identique à N vaut 0, 1, 2 ou 3 et dans le cas où Z₅ représente CH, vaut 0, 1, 2, 3 ou 4, R₁₀₇ représente un atome d'halogène, un groupe alkyle en C₁-C₄, un groupe halogénoalkyle en C₁-C₄, un groupe alcoxy C₁-C₄, un groupe halogénoalcoxy en C₁-C₄, un groupe nitro, un groupe alkylthio en C₁-C₄, un groupe alkylsulfonyle en C₁-C₄, un groupe alcoxycarbonyle en C₁-C₄, ou un groupe phényle ou un groupe phénoxy un groupe phényle ou un groupe phénoxy substitué par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro ;
R₁₀₈ représente un atome d'hydrogène ou un groupe alkyle en C₁-C₄, R₁₀₉ représente un atome d'hydrogène, un groupe alkyle en C₁-C₄, un groupe cycloalkyle en C₃-C₆, un groupe alcényle en C₂-C₆, un groupe alcinyle en C₂-C₆, un groupe halogénoalkyle en C₁-C₄, un groupe halogénoalcényle en C₂-C₆, un groupe halogénoalcinyle en C₂-C₆, un groupe alkylthio en C₁-C₄ alkyle en C₁-C₄, un groupe alkylsulfonyle en C₁-C₄ alkyle en C₁-C₄, un groupe alcoxy en C₁-C₄ alkyle en C₁-C₄, un groupe alcényloxy en C₁-C₄ alkyle en C₁-C₄ ou un groupe alcinyloxy en C₁-C₄ alkyle en C₁-C₄ ;
ou d'un composé répondant à la formule XX 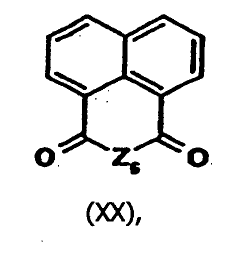 dans laquelle Z₆ représente un atome d'oxygène ou N-R₁₁₀ et R₁₁₀ représente un groupe répondant à la formule 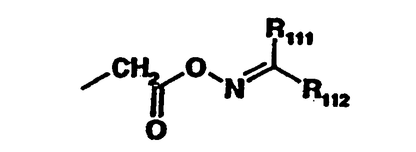 dans laquelle R₁₁₁ et R₁₁₂ représentent indépendamment l'un de l'autre un groupe cyano, un atome d'hydrogène, un groupe alkyle en C₁-C₄, un groupe cycloalkyle en C₃-C₆, un groupe alcényle en C₂-C₆, un groupe aryle, un groupe phényle ou un groupe hétéroaryle, ou un groupe phényle, aryle ou hétéroaryle substitué par un groupe alkyle en C₁-C₃, par un groupe halogénoalkyle en C₁-C₃, par un groupe alcoxy en C₁-C₃, par un groupe halogénoalkyle en C₁-C₃, par un atome d'halogène, par un groupe cyano ou par un groupe nitro ;
ou d'un composé répondant à la formule XXI 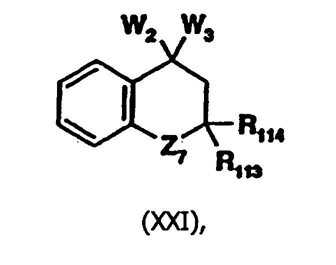 dans laquelle Z₇ représente un atome d'oxygène, un atome de soufre, S=O, SO₂, ou CH₂, R₁₁₃ et R₁₁₄ représentent indépendamment l'un de l'autre un atome d'hydrogène, un atome d'halogène ou un groupe alkyle en C₁-C₄, W₂ et W₃ représentent indépendamment l'un de l'autre CH₂COOR₁₁₅ ou COOR₀₁₁₅ ou ensemble un groupe répondant à la formule -(CH₂)C(O)-O-C(O)-(CH₂)- et R₁₁₅ et R₀₁₁₅ représentent indépendamment l'un de l'autre un atome d'hydrogène, un groupe alkyle en C₁-C₄, un groupe alcényle en C₂-C₄, un groupe alcinyle en C₂-C₆, un groupe cycloalkyle en C₃-C₆, un groupe halogénoalkyle en C₁-C₄, un cation métallique ou un cation ammonium ;
ou d'un composé répondant à la formule XXII 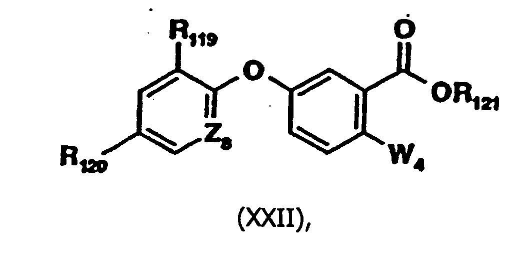 dans laquelle R₁₁₉ et R₁₂₀ représentent indépendamment l'un de l'autre un atome d'hydrogène, un atome d'halogène, un groupe halogénoalkyle en C₁-C₄, R₁₂₁ représente un atome d'hydrogène, un groupe alkyle en C₁-C₄, un groupe alcényle en C₃-C₄, un groupe alcinyle en C₃-C₄, un groupe halogénoalkyle en C₁-C₄, un groupe cycloalkyle en C₃-C₆, un cation métallique ou un cation ammonium, Z₈ représente N, CH, C-F ou C-Cl et W₄ représente un groupe répondant à la formule 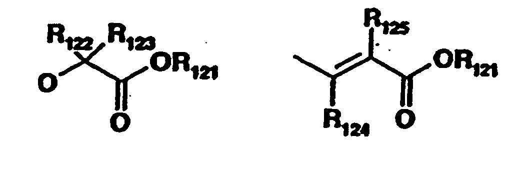 dans laquelle R₁₂₂ et R₁₂₃ représentent indépendamment l'un de l'autre un atome d'hydrogène ou un groupe alkyle en C₁-C₄ et R₁₂₄ et R₁₂₅ représentent indépendamment l'un de l'autre un atome d'hydrogène ou un groupe alkyle en C₁-C₄ ;
ou d'un composé répondant à la formule XXIII 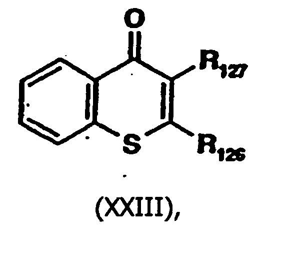 dans laquelle R₁₂₆ représente un atome d'hydrogène, un groupe cyano, un atome d'halogène, un groupe alkyle en C₁-C₄, un groupe cycloalkyle en C₃-C₆, un groupe alcoxy en C₁-C₄, un groupe alcoxycarbonyle en C₁-C₄, un groupe alkylthiocarbonyle en C₁-C₄, -NH-R₁₂₈, -C(O)NH-R₀₁₂₈, ou un groupe aryle ou un groupe hétéroaryle ou un groupe aryle ou un groupe hétéroaryle substitué par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro ;
R₁₂₇ représente un atome d'hydrogène, un groupe cyano, un groupe nitro, un atome d'halogène, un groupe alkyle en C₁-C₄, un groupe halogénoalkyle en C₁-C₄, un groupe alcoxy en C₁-C₄, un groupe thioalkyle en C₁-C₄, et
R₁₂₈ et R₀₁₂₈ représentent indépendamment l'un de l'autre un groupe alkyle en C₁-C₄, un groupe halogénoalkyle en C₁-C₄, un groupe alcényle en C₃-C₄, un groupe alcinyle en C₃-C₄, un groupe cycloalkyle en C₃-C₄, un groupe aryle ou un groupe hétéroaryle un groupe aryle ou un groupe hétéroaryle substitué par un groupe alkyle en C₁-C₃, par un groupe halogénoalkyle en C₁-C₃, par un groupe alcoxy en C₁-C₃, par un groupe halogénoalcoxy en C₁-C₃, par un atome d'halogène, par un groupe cyano ou par un groupe nitro, un groupe formyle, un groupe alkylcarbonyle en C₁-C₄, un groupe arylsulfonyle en C₁-C₄ ;
ou d'un composé répondant à la formule XXIV 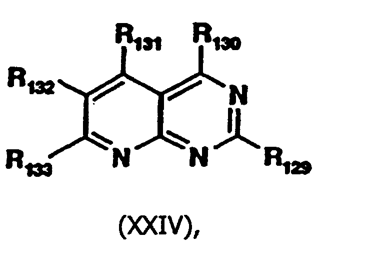 dans laquelle R₁₂₉ et R₁₃₀ représentent indépendamment l'un de l'autre un atome d'hydrogène, un groupe alkyle en C₁-C₄, un groupe halogénoalkyle en C₁-C₄, un groupe alcoxy en C₁-C₄, un groupe monoalkylamino en C₁-C₈ ou un groupe dialkylamino en C₁-C₈, un groupe cycloalkyle en C₃-C₆, un groupe thioalkyle en C₁-C₄, un groupe phényle ou un groupe hétéroaryle, R₁₃₁ a la signification de R₁₂₉ et en plus représente OH, NH₂, un atome d'halogène, un groupe diaminoalkyle en C₁-C₄, un groupe alkylthio en C₁-C₄, un groupe alkylsulfonyle en C₁-C₄ ou un groupe alcoxycarbonyle en C₁-C₄, R₁₃₂ a la signification de R₁₂₉ et en plus représente un groupe cyano, un groupe nitro, un groupe carboxyle, un groupe alcoxycarbonyle en C₁-C₄, un groupe diaminoalkyle en C₁-C₄, un groupe alkylthio en C₁-C₄, un groupe alkylsulfonyle en C₁-C₄, SO₂-OH, un groupe diaminoalkylsulfonyle en C₁-C₄ ou un groupe alcoxysulfonyle en C₁-C₄, R₁₃₃ a la signification de R₁₂₉ et en plus représente OH, NH₂, un atome d'halogène, un groupe diaminoalkyle en C₁-C₄, un groupe pyrrolidin-1-yle, un groupe pipéridin-1-yle, un groupe morpholin-1-yle, un groupe alkylthio en C₁-C₄, un groupe alkylsulfonyle en C₁-C₄, un groupe alcoxycarbonyle en C₁-C₄, un groupe phénoxy, un groupe naphtoxy, un groupe phénylamino, un groupe benzoyloxy ou un groupe phénylsulfonyloxy ;
ou d'un composé répondant à la formule XXV 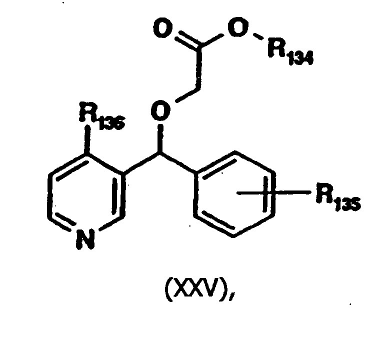 dans laquelle R₁₃₄ représente un atome d'hydrogène, un groupe alkyle, en C₄, un groupe halogénoalkyle en C₁-C₄, un groupe alcényle en C₂-C₄, un groupe alcinyle en C₂-C₄ ou un groupe alcoxy en C₁-C₄ alkyle en C₁-C₄, R₁₃₅ représente un atome d'hydrogène, un atome d'halogène, un groupe alkyle en C₁-C₄, un groupe halogénoalkyle en C₁-C₄, ou un groupe alcoxy en C₁-C₄ et R₁₃₆ représente un atome d'hydrogène, un atome d'halogène, un groupe alkyle en C₁-C₄, un groupe halogénoalkyle en C₁-C₄ ou un groupe alcoxy en C₁-C₄, à condition que R₁₃₅ et R₁₃₆ ne représentent pas simultanément un atome d'hydrogène,
ou d'un composé répondant à la formule XXVI 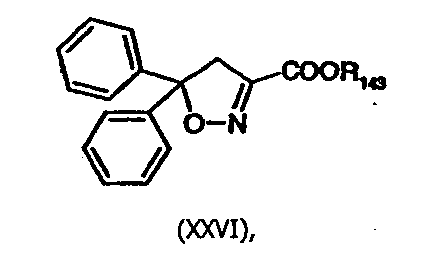 dans laquelle
R₁₄₃ représente un atome d'hydrogène, un cation alcali, un cation alcalino-terreux, un cation sulfonium ou ammonium ou un groupe éthyle ;
ou d'un composé répondant à la formule XXVII 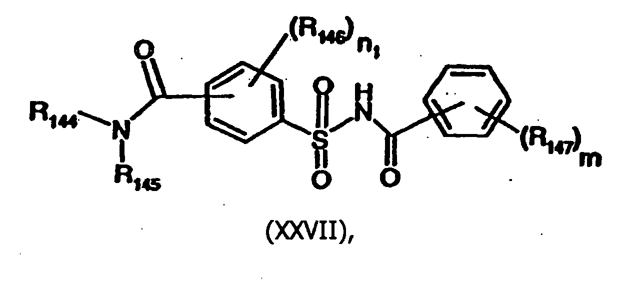 dans laquelle R₁₄₄ et R₁₄₅ représentent indépendamment l'un de l'autre un atome d'hydrogène, un groupe alkyle en C₁-C₆, un groupe alcényle en C₂-C₆, un groupe alcinyle en C₂-C₆ ou un groupe cycloalkyle en C₃-C₆ ;
R₁₄₆ représente un atome d'hydrogène, un atome d'halogène, un groupe alkyle en C₁-C₄, un groupe halogénoalkyle en C₁-C₆ ou un groupe halogénoalcoxy en C₁-C₆ ;
R₁₄₇ représente un atome d'hydrogène, un atome d'halogène, un groupe alkyle en C₁-C₄, un groupe halogénoalkyle en C₁-C₄, un groupe alcoxy en C₁-C₄, un groupe halogénoalcoxy en C₁-C₄, un groupe alkylthio en C₁-C₄, un groupe alcoxycarbonyle en C₁-C₄ ou un groupe nitro ;
n1 vaut 0, 1, 2 ou 3 ; et
m vaut 1 ou 2 ;
ou d'un composé répondant à la formule XXVIII 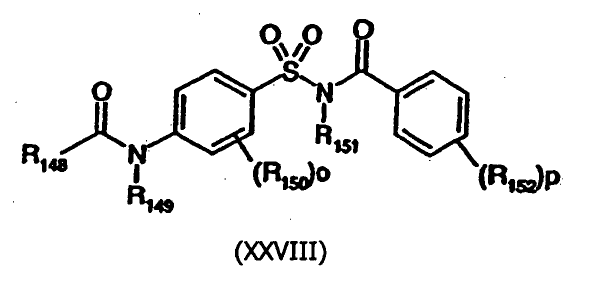 dans laquelle
R₁₄₈ représente un atome d'hydrogène, un groupe alkyle en C₁-C₆, un groupe alcoxy en C₁-C₆, un groupe alkylthio en C₁-C₆, un groupe cycloalkyle en C₃-C₈, un groupe phényle, un groupe phényl alkyle en C₁-C₆ ou un groupe hétéroaryle ; les groupes cités pouvant être substitués par un atome d'halogène, un groupe cyano, par un groupe nitro, par un groupe amino, par un groupe hydroxy, par un groupe carbonyle, par un groupe carboxyle, par un groupe formyle, par un carbonamide ou par un sulfonamide ;
R₁₄₉ représente un atome d'hydrogène, un groupe alkyle en C₁-C₆ ou un groupe halogénoalkyle en C₁-C₄ ;
chaque R₁₅₀ représente indépendamment un atome d'hydrogène, un atome d'halogène, un groupe alkyle en C₁-C₄, un groupe halogénoalkyle en C₁-C₄, un groupe alcoxy en C₁-C₄, un groupe alkylthio en C₁-C₄, un groupe alkylsulfonyle en C₁-C₄, un groupe cyano, un groupe nitro, un groupe formyle ou un groupe carboxyle ;
R₁₅₁ représente un atome d'hydrogène, un groupe alkyle en C₁-C₆ ou un groupe halogénoalkyle en C₁-C₄ ;
chaque R₁₅₂ indépendamment représente un atome d'hydrogène, un atome d'halogène, un groupe alkyle en C₁-C₄, un groupe halogénoalkyle en C₁-C₄, un groupe alcoxy en C₁-C₄, un groupe alkylthio en C₁-C₄, un groupe alkylsulfonyle en C₁-C₄, un groupe cyano, un groupe nitro, un groupe formyle ou un groupe carboxyle ;
O vaut 0, 1 ou 2, et
p vaut 0, 1 ou 2 ;
ou d'un composé répondant à la formule XXIX 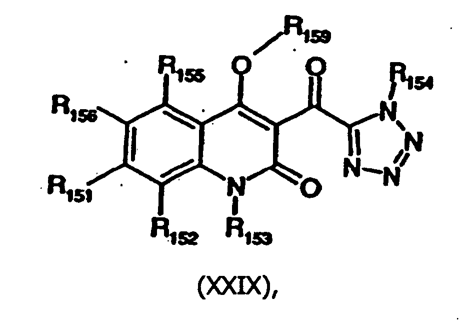 dans laquelle
R₁₅₉ représente un atome d'hydrogène, un groupe formyle, un groupe alkylcarbonyle en C₁₋₆, un groupe alcénylcarbonyle en C₁₋₆, un groupe alcinylcarbonyle en C₁₋₆, un groupe alcoxycarbonyle en C₁₋₆, un groupe alkylthiocarbonyle en C₁₋₆, un groupe cycloalkylcarbonyle en C₃₋₈, un groupe phényl alkylcarbonyle en C₁₋₆, un groupe phénylcarbonyle, un groupe alkylsulfonyle en C₁₋₆, un groupe alcénylsulfonyle en C₁₋₆ ou un groupe phénylsulfonyle, les groupes d'hydrocarbures cités précédemment pouvant être substitués par un ou plusieurs atomes d'halogène, par un groupe cyano, par un groupe nitro, par un groupe amino, par un groupe méthoxy, par un groupe éthoxy ou par un groupe phényle ;
R₁₅₃ représente un atome d'hydrogène, un groupe alkyle en C₁-₆, un groupe alcényle en C₁₋₆, un groupe alcinyle en C₁₋₆, un groupe cycloalkyle en C₃₋₈, un groupe formyle, un groupe alkylcarbonyle en C₁₋₆, un groupe alcénylcarbonyle en C₁₋₆, un groupe alcinylcarbonyle en C₁₋₆, un groupe alcoxycarbonyle en C₁₋₆, un groupe alkylthiocarbonyle en C₁₋₆, un groupe cycloalkylcarbonyle en C₃₋₈, un groupe alkylsulfonyle en C₁₋₆, un groupe alcénylsulfonyle en C₁₋₆ ou un groupe phénylsulfonyle, les groupes d'hydrocarbures précédemment cités pouvant être substitués par un ou plusieurs atomes d'halogène, par un groupe cyano, par un groupe nitro, par un groupe amino, par un groupe méthoxy, par un groupe éthoxy ou par un groupe phényle ;
R₁₅₄ représente un atome d'hydrogène, un groupe alkyle en C₁₋₆, un groupe alcényle en C₁₋₆, un groupe alcinyle en C₁₋₆, un groupe cycloalkyle en C₃₋₈, un groupe formyle, un groupe alkylcarbonyle en C₁₋₆, un groupe alcénylcarbonyle en C₁₋₆, un groupe alcinylcarbonyle en C₁₋₆, un groupe alcoxycarbonyle en C₁₋₆, un groupe alkylthiocarbonyle en C₁₋₆, un groupe cycloalkylcarbonyle en C₃₋₈, un groupe alkylsulfonyle en C₁₋₆, un groupe alcénylsulfonyle en C₁₋₆ ou un groupe phénylsulfonyle, les groupes d'hydrocarbures précédemment cités pouvant être substitués par un ou plusieurs atomes d'halogène, par un groupe cyano, par un groupe nitro, par un groupe amino, par un groupe méthoxy, par un groupe éthoxy ou par un groupe phényle ;
R₁₅₅, R₁₅₆, R₁₅₇ et R₁₅₈ représentent indépendamment les uns des autres un atome d'hydrogène, un atome d'halogène, un groupe amino, un groupe alkylamino en C₁₋₃, un groupe dialkylamino en C₁₋₆, un groupe hydroxy, un groupe cyano, un groupe nitro, un groupe formyle, un groupe carboxyle, un groupe alcoxy en C₁₋₆, un groupe halogénoalcoxy en C₁₋₆, un groupe alkylcarbonyle en C₁₋₆, un groupe alcoxycarboxyle en C₁₋₆, un groupe alkyle en C₁₋₆, un groupe halogénoalkyle en C₁₋₆, un groupe alcényle en C₁₋₆ ou un groupe alcinyle en C₁₋₆ ;
ou R₁₅₃ et R₁₅₈ forment avec les atomes du noyau auquel ils sont liés, un noyau insaturé ou partiellement saturé, de 5 ou 6 chaînons, qui peut contenir jusqu'à 2 hétéroatomes identiques ou différents provenant du groupe constitué par l'atome d'oxygène, l'atome de soufre et l'atome d'azote, ce noyau pouvant être substitué avec un reste oxo.

8. Agent selon la revendication 7, **caractérisé en ce qu'**il contient une quantité efficace en tant qu'antagoniste d'herbicide d'un safener répondant à la formule X, XI, XII, XIII, XIV, XV, XVI, XVII, XVIII, XIX, XX, XXI, XXII, XXIII, XXIV ou XXV.

9. Procédé pour combattre de façon sélective les mauvaises herbes et les graminées dans des cultures de plantes utilitaires, **caractérisé en ce que** l'on traite les plantes utilitaires, leurs semences et leurs boutures ou leur surface cultivée avec une quantité efficace en tant qu'herbicide d'un herbicide répondant à la formule I ou d'une quantité efficace en tant qu'antagoniste d'herbicide d'un safener répondant à la formule X, XI, XII, XIII, XIV, XV, XVI, XVII, XVIII, XIX, XX, XXI, XXII, XXIII, XXIV, XXV, XXVI, XXVII, XXVIII ou XXIX.

10. Procédé selon la revendication 9, **caractérisé en ce que** l'on traite les plantes utilitaires, leurs semences et leurs boutures ou leur surface cultivée avec une quantité efficace en tant qu'antagoniste d'herbicide d'un safener répondant à la formule X, XI, XII, XIII, XIV, XV, XVI, XVII, XVIII, XIX, XX, XXI, XXII, XXIII, XXIV ou XXV.

11. Agent selon la revendication 4, **caractérisé en ce qu'**il contient des adjuvants dans le réservoir de pulvérisation.

12. Agent selon la revendication 7, **caractérisé en ce qu'**il contient des adjuvants dans le réservoir de pulvérisation.
